(19) **Europäisches Patentamt**
**European Patent Office**
**Office européen des brevets**

(11) **EP 2 302 394 A1**

(12) **EUROPEAN PATENT APPLICATION**

(43) Date of publication:
**30.03.2011 Bulletin 2011/13**

(51) Int Cl.:
***G01N 33/68*** (2006.01)

(21) Application number: **10012637.4**

(22) Date of filing: **20.05.2005**

(84) Designated Contracting States:
**AT BE BG CH CY CZ DE DK EE ES FI FR GB GR HU IE IS IT LI LT LU MC NL PL PT RO SE SI SK TR**

(30) Priority: **21.05.2004 US 573593**

(62) Document number(s) of the earlier application(s) in accordance with Art. 76 EPC:
**09153474.3 / 2 093 569**
**05751819.3 / 1 766 412**

(71) Applicant: **The Institute for Systems Biology Seattle, WA 98103-1299 (US)**

(72) Inventors:
• **Aebersold, Rudolph H.**
  **8049 Zürich (CH)**

• **Zhang, Hui**
  **Seattle, WA 98115 (US)**

(74) Representative: **UEXKÜLL & STOLBERG**
**Patentanwälte**
**Beselerstrasse 4**
**22607 Hamburg (DE)**

Remarks:
•The complete document including Reference Tables and the Sequence Listing can be downloaded from the EPO website
•Claims filed after the date of filing of the application/ after the date of    receipt of the divisional application ( Rule 68(4) EPC)
•This application was filed on 30-09-2010 as a divisional application to the application mentioned under INID code 62.

(54) **Compositions and methods for quantification of serum glycoproteins**

(57)     The invention provides compositions and methods for identifying and/or quantifying glycopolypeptides from human serum or plasma. The compositions and methods include a plurality of standard peptides containing glycosylation sites determined for human serum/plasma proteins.

'Quantitative analysis of serum proteins by MALDI-TOF

**Figure 4**

**EP 2 302 394 A1**

**Description**

BACKGROUND OF THE INVENTION

The present invention relates generally to the field of proteomics and more specifically to quantitative analysis of blood, plasma or serum glycoproteins.

Complete genomic sequences and large partial (EST) sequence databases potentially identify every gene in a species. However, the sequences alone do not explain the mechanism of biological and clinical processes because they do not explain how the genes and their products cooperate to carry out a specific process or function. Furthermore, the gene sequence does not predict the amount or the activity of the protein products nor does it answer the questions of whether, how, and at what position(s) a protein may be modified.

Quantitative protein profiling has been recognized as an important approach for profiling the physiological state or pathological state of cells or organisms. Specific expectations of quantitative protein profiles include the possibility to detect diagnostic and prognostic disease markers, to discover proteins as therapeutic targets or to learn about basic biological mechanisms.

Not only do the amounts and type of proteins expressed vary in different pathological states, post-translational modifications of proteins also vary depending on the physiological or pathological state of cells or organisms. Thus, it is important to be able to profile the amount and types of expressed proteins as well as protein modifications.

Glycosylation has long been recognized as the most common post-transtational modification affecting the functions of proteins, such as protein stability, enzymatic activity and protein-protein interactions. Differential Glycosylation is a major source of protein microheterogeneity. Glycoproteins play key roles in cell communication, signaling and cell adhesion. Changes in carbohydrates in cell surface and body fluid are demonstrated in cancer and other disease states and highlights their importance. However, studies on protein glycosylation have been complicated by the diverse structure of protein glycans and the lack of effective tools to identify the glycosylation site(s) on proteins and of glycan structures. Oligosaccharides can be linked to serine or threonine residues (O-glycosylation) or to asparagine residues (N-glycosylation), and glycoproteins can have different oligosaccharides attached to any given possible site(s).

Among the many post-translation modifications of proteins, glycosylation is a modification that is common to proteins that are exposed to an extracellular environment. For example, proteins expressed on the surface of a cell are exposed to the external environment such as blood or surrounding tissue. Similarly, proteins that are secreted from a cell, for example, into the bloodstream, are commonly glycosylated.

Proteins secreted by cells or shed from the cell surface, including hormones, lymphokines, interferons, transferrin, antibodies, proteases, protease inhibitors, and other factors, perform critical functions with respect to the physiological activity of an organism. Examples of physiologically important secreted proteins include the interferons, lymphokines, protein and peptide hormones. Aberrant availability of such proteins can have grave clinical consequences. It is therefore apparent that the ability to precisely quantitatively profile secreted proteins would be of great importance for the discovery of the mechanisms regulating a wide variety of physiological processes in health and disease and for diagnostic or prognostic purposes. Such secreted proteins are present in body fluids such as blood serum and plasma, cerebrospinal fluid, urine, lung lavage, breast milk, pancreatic juice, and saliva. For example, the presence of increased levels of prostate-specific antigen has been used as a diagnostic marker for prostate cancer. Furthermore, the use of agonists or antagonists or the replacement of soluble secreted proteins is an important mode of therapy for a wide range of diseases.

Quantitative proteomics requires the-analysis of complex -protein-samples. In the case of clinical diagnosis, the ability to obtain appropriate specimens for clinical analysis is important for ease and accuracy of diagnosis. As discussed above, a number of biologically important molecules are secreted and are therefore present in body fluids such as blood and serum, cerebrospinal fluid, saliva and the like. In addition to the presence of important, biological molecules, body fluids also provide an attractive specimen source because body fluids are generally readily accessible and available in reasonable quantities for clinical analysis. It is therefore apparent that a general method for the quantitative analysis of the proteins contained in body fluids in health and disease would be of great diagnostic and clinical importance.

A key problem with the proteomic analysis of serum and many other body fluids is the peculiar protein composition of these specimens. The protein composition is dominated by a few proteins that are extraordinarily abundant, with albumin alone representing 50% of the total plasma proteins. Due to the abundance of these major proteins as well as the presence of multiple modified forms of these abundant proteins, the large number of protein species of lower abundance are obscured or inaccessible by traditional proteomics analysis methods such as two-dimensional electrophoresis (2DE).

Proteins secreted and present in body fluids have in common a high propensity for being glycosylated, that is, modified post translationally with a carbohydrate structure of varying complexity at one or several amino acid residues. Thus, the analysis of glycoproteins allows characterization of important biological molecules.

Thus, there exists a need for methods of high throughput and quantitative analysis of blood, serum or plasma

2

glycoproteins and glycoprotein profiling for diagnostic purposes. The present invention satisfies this need and provides related advantages as well.

SUMMARY OF INVENTION

The invention provides compositions and methods for identifying and/or quantifying glycopolypeptides from human serum or plasma. The compositions and methods include a plurality of standard peptides containing glycosylation sites determined for human serum/plasma proteins.

Embodiments of the invention may provide a method for identifying glycopolypeptides in a serum or plasma sample, comprising: (a) derivatizing glycopolypeptides in said sample; (b) immobilizing said derivatized sample glycopolypeptides to a solid support; (c) cleaving said immobilized sample glycopolypeptides, thereby releasing non-glycosylated peptide fragments and retaining immobilized sample glycopeptide fragments: (d) labeling said immobilized sample glycopeptide fragments with an isotope tag; (e) releasing said sample glycopeptide fragments from said solid support; thereby generating released sample glycopeptide fragments; (f) adding to said released sample glycopeptide fragments a plurality of standard peptides selected from peptides containing the glycosylation sites referenced as SEQ ID NOS: 1-3482, wherein said standard peptides correspond to peptides derivatized as in step (a), cleaved as in step (c), and released as in step (e), and wherein said standard peptides are differentially labeled with a corresponding isotope tag as used in step (d): (g) analyzing said released sample glycopeptide fragments using mass spectrometry; and (h) identifying released sample glycopeptide fragments that correspond to standard peptides added in step (f).

In one embodiment, the method further comprises quantifying the amount of said sample glycopeptide fragments identified in step (h).

In another embodiment, the solid support comprises a hydrazide moiety.

In another embodiment the glycopeptides are released from said solid support using a glycosidase.Optionally, the glycosidase is an N-glycosidase or an O-glycosidase, and optionally the glycopeptides are released from said solid using sequential addition of N-glycosidase and O-glycosidase.

In another embodiment the glycopeptides are released from said solid support using chemical cleavage.

In another embodiment the glycopolypcptides are oxidized with periodate.

In another embodiment the said glycopolypeptides are cleaved with a protease. Optionally, the glycopolypeptides are cleaved with trypsin.

Other embodiments of the invention may provide a method for identifying glycopolypeptides in a serum or plasma sample, comprising: (a) immobilizing said sample glycopolypeptides to a solid support; (b) cleaving said immobilized sample glycopolypeptides, thereby releasing non-glycosylated peptide fragments and retaining immobilized sample glycopeptide fragments: (c) labeling said immobilized sample glycopeptide fragments with an isotope tag; (d) releasing said sample, glycopeptide fragments from said solid support, thereby generating released sample glycopeptide fragments; (e) adding to said released sample glycopeptide fragments a plurality of standard peptides selected from peptides containing the glycosylation sites referenced as SEQ ID NOS: 1-3482, wherein said-standard peptides correspond to peptides cleaved as in step (b), and released as in step (d), and wherein said standard peptides are differentially labeled with a corresponding isotope tag as used in step (c); (f) analyzing said released sample glycopeptide fragments using mass spectrometry; and (g) identifying released sample glycopeptide fragments that correspond to standard peptides added in step (e).

In one embodiment the method further comprises quantifying the amount of said sample glycopeptide fragments identified in step (g).

In another embodiment the solid support comprises a hydrazide moiety.

In another embodiment the glycopeptides are released from said solid support using a glycosidase. Optionally, the glycosidase is an N-glycosidase or an O-glycosidase, and optionally the glycopeptides are released from said solid using sequential addition of N-glycosidase and O-glycosidase.

In another embodiment the glycopeptides are released from said solid support using chemical cleavage.

In another embodiment the glycopolypeptides are oxidized with periodate.

In another embodiment the glycopolypeptides are cleaved with a protease. Optionally, the glycopolypeptides are cleaved with trypsin.

Other embodiments of the invention may provide a method for identifying and quantifying glycopolypeptides in a control serum or plasma sample, comprising: (a) derivatizing glycopolypeptides in a said sample; (b) immobilizing said derivatized sample glycopolypeptides to a solid support: (c) cleaving said immobilized sample glycopolypeptides, thereby releasing non-glycosylated peptide fragments and retaining immobilized sample glycopeptide fragments; (d) labeling said immobilized sample glycopeptide fragments with an isotope tag; (e) releasing said sample glycopeptide fragments from said solid support, thereby generating released sample glycopeptide fragments; (f) adding to said released sample glycopeptide fragments a plurality of standard peptides selected from peptides containing the glycosylation sites referenced as SEQ ID NOS: 1-3482, wherein said standard peptides correspond to peptides derivatized as in step (a), cleaved as in step (c), and released as in step (e), and wherein said standard peptides are differentially labeled with a corresponding isotope tag as used in step (d); (g) analyzing said released sample glycopeptide fragments using mass spectrometry; (h) identifying released sample glycopeptide fragments that correspond to standard peptides added in step (f); and (i) quantifying the amount of said sample glycopeptide fragments identified in step (h).

In one embodiment the control serum or plasma sample is normal serum or plasma.

In another embodiment the solid support comprises a hydrazide moiety,

In another embodiment the said glycopeptides are released from said solid support using a glycosidase. Optionally, the glycosidase is an N-glycosidase or an O-glycosidase, and optionally the glycopeptides are released from said solid using sequential addition of N-glycosidase and O-glycosidase.

In another embodiment the glycopeptides are released from said solid support using chemical cleavage.

In another embodiment the glycopolypeptides are oxidized with periodate.

In another embodiment the glycopolypeptides are cleaved with a protease. Optionally, the glycopolypeptides are cleaved with trypsin.

Other embodiments of the invention may provide a method for identifying one or more diagnostic markers for a disease, comprising: (a) derivatizing glycopolypeptides in a serum or plasma sample from an individual having a disease; (b) immobilizing said derivatized sample glycopolypeptides to a solid support; (c) cleaving said immobilized sample glycopolypeptides, thereby releasing non-glycosylated peptide fragments and retaining immobilized sample glycopeptides fragments; (d) labeling said immobilized sample glycopeptide fragments with an isotope tag: (e) releasing said sample glycopeptide fragments from said solid support. thereby generating released sample glycopeptide fragments: (f) adding to said released sample glycopeptide fragments a predetermined amount of a plurality of standard peptides selected from peptides containing the glycosylation sites referenced as SEQ ID NOS: 1-3482, wherein said standard peptides correspond to peptides derivatized as in step (a), cleaved as in step (c), and released as in step (e), and wherein said standard peptides are differentially labeled with a corresponding isotope tag as used in step (d); (g) analyzing said released sample glycopeptide fragments using mass spectrometry; (h) identifying released sample glycopeptide fragments that correspond to standard peptides added in step (f); (i) quantifying the amount of said sample glycopeptide fragments identified in step (h); and (j) comparing the amount of said sample glycopeptide fragments determined in step (i) to the amount of the same glycopeptide fragments determined in a normal serum or plasma sample.

In one embodiment the solid support comprises a hydrazide moiety.

In another embodiment the glycopeptides are released from said solid support using a glycosidase. Optionally, the glycosidase is an N-glycosidase or an O-glycosidase, and optionally, the glycopeptides are released from said solid using sequential addition of N-glycosidase and O-glycosidase.

In another embodiment the glycopeptide are released from said solid support using chemical cleavage.

In another embodiment the glycopolypeptides are oxidized with periodate.

In another embodiment the glycopolypeptides are cleaved with a protease. Optionally, the glycopolypeptides are cleaved with trypsin.

In another embodiment the disease is cancer.

Other embodiments of the invention may provide a composition comprising a plurality of peptides containing the glycosylation sites referenced as SEQ ID NOS: 1-3482, wherein said peptides each correspond to peptide fragments derived by cleavage of polypeptides using the same cleavage reagent.

In one embodiment the cleavage reagent is a protease. Optionally the protease is trypsin.

Other embodiments of the invention may provide a kit comprising a plurality of peptides containing the glycosylation sites sh referenced as SEQ ID NOS: 1-3482, wherein said peptides each correspond to peptide fragments derived by cleavage of polypeptides using the same cleavage reagent.

In one embodiment the kit further comprises a pair of differentially labeled isotope tags.

In another embodiment the kit further comprises the cleavage reagent corresponding to said peptide fragments.

In another embodiment the cleavage reagent is a protease.

In another embodiment the protease is trypsin.

In another embodiment the kit further comprises a hydrazide resin.

In another embodiment the kit further comprises a glycosidase.

BRIEF DESCRIPTION OF THE DRAWINGS

Figure 1 shows oxidation of a carbohydrate to an aldehyde followed by covalent coupling to hydrazide beads.

Figure 2 shows representative chemical reagents that have been tested and proven to be able to label amino groups of glycopeptides. The structures of labeled peptides are shown in the right column.

Figure 3 shows the chemistry and schematic diagram of isotopically labeling the N-termini of immobilized glycopeptides by attaching differentially isotopically labeled forms of the amino acid phenylalanine (Phe) to their N-termini.

Figure 4 shows a schematic of quantitative analysis of serum proteins.

Figure 5 shows an exemplary analysis with the addition of a standard peptide

Figure 6 shows a diagram of a procedure for glycopeptide profiling of serum proteins using liquid chromatograpy-mass spectrometry (LC-MS).

Figure 7 shows the ratio of peptides identified without NXS/T glycosylation motif as a function of peptide identification stringency. The fraction of peptides identified with (center bar) or without (right bar) glycosylation consensus motif are shown for different PeptideProphet (Keller et al., Anal. Chem. 74:5383-5392 (2002)) probabilities. The false positive error rates were estimated by PeptideProphet are indicated (left bar).

Figure 8 shows the reproducibility of the high throughput serum analysis method. Distribution of coefficient of variance (CV) from 9 repeated LC-MS analyses of the same glycopeptide mixture (rectangles), and distribution of CV from 4 repeated sample preparations using glycopeptide capture-and-release method and LC-MS analysis (squares) are shown.

Figure 9 shows identification of peptides exhibiting increased abundance in treated cancer-bearing mice. Figure 9A shows normalized abundances of the peptide at $m/z$ value of 709.7 observed in sera of normal (N1a, N1b, N2) and cancer-bearing mice (C1, C2, C3), determined by LC-MS analysis. Figure 9B shows validation of differential abundance of the same peptide shown in Figure 9A using isotopic labeling of N-termini.

Figure 10 shows a schematic illustration of an offline LC-MALDI TOF/TOF based platform for proteome-screening technology.

Figure 11 shows a search and identification of a specific spike-native peptide pair in a complex background. The native peptide was consistently identified in different runs using the spike-in stable isotope labeled peptide as a search criterion, even though the peptides were deposited on different spot positions in different runs.

Figure 12 shows the complementary approach for peptide identification using specific mass match and peptide sequencing. The search of a specific mass resulted in more than one precursor ion locating at different spot positions. Both of the precursor ions were submitted for MS/MS analysis. The one with the higher intensity, distributing across spot 133 to 138 was identified as the targeted peptide.

Figure 13 shows an exemplary analysis using a method of the invention. Figure 13A shows the base peak chromatogram of a glycopeptide mixture spiked with stable isotope labeled peptides. The sample was fractionated in 192 wells on a MALD plate. Each point on the x-axis indicates a spot position. The elution of the majority of the peptides was between spot 45 and 165. Figure 13B shows the MS spectrum of a representative spot.

Figure 14A shows the number of precursor ions detected in each spot in MS mode. Figure 14B shows the elution profile of the spike-in stable isotope labeled peptides extracted from the complex background. The elution profile of a spiked peptide was used to locate the spot position(s) containing the peptide.

Figure 15 shows the identification of a targeted peptide with low abundance in a complex serum glycopeptide mixture. The pair of the spike-in and native peaks was located and identified using specific mass search against the MS data. The validation of the peptide sequence was accomplished using MS/MS analysis and database searching.

Figure 16 shows a quantitative profile of the selected peptides detected in 4 different serum samples (1S2, 1F2, 3S1, 3F1). The Y axis represents the peptide mass. The y axis indicates the abundance ratio of a native peptide to the corresponding spike-in stable isotope labeled peptide. The peptides and their corresponding proteins are listed in Table 6.

Figure 17 shows a protein network analysis of changes in glycoprotein expression in prostate cancer tissue.

Figure 18A and 18B shows the amino acid preferences around N-1 linked glycosylation sites.

Figure 19 shows a representative output of proteotypic N-linked glycopeptides from a database using UniPep.

Figure 20 shows reproducible CID spectra generated from light and heavy isoforms of the same peptide sequence.

DETAILED DESCRIPTION OF THE Invention

The invention provides methods for quantitative profiting of glycoproteins and glycopeptides on a proteome-wide scale. The methods of the invention can be used to determine changes in the abundance of glycoproteins and changes in the state of glycosylation at individual glycosylation sites on those glycoproteins that occur in response to perturbations of biological systems and organisms in health and disease.

Because the methods of the invention are directed to isolating glypolypeptides, the methods also reduce the complexity of analysis since many proteins and fragments of glycoproteins do not contain carbohydrate, which can simplify the analysis of complex biological samples such as serum, plasma or blood. The methods of the invention are advantageous for the determination of protein glycosylation in glycome studies and can be used to isolate and identify glycoproteins from serum, plasma or blood to determine specific glycoprotein changes related to certain disease states or cancer. The methods of the invention can be used for detecting quantitative changes in protein samples containing glycoproteins and to detect their extent of glycosylation. The methods of the invention are applicable for the identification and/or characterization of diagnostic biomarkers, immunotherapy, or other diagnostic or therapeutic applications. The methods of the invention can also be used to evaluate the effectiveness of drugs during drug development, optimal dosing, toxicology, drug targeting, and related therapeutic applications.

The invention uses methods for identifying and/or quantifying glycopolypeptides in a blood, plasma or serum sample, in particular a human blood, plasma or serum sample. The methods of the invention can also be used to identify and/or quantify glycopolypeptides in other biological fluids. Methods for quantifying glycoproteins have been described previously (see, for example, Zhang et al., Nat. Biotechnol. 21:660-666 (2003); Aebersold and Zhang, U.S. publication 2004/0023306, each of which is incorporated herein by reference.

In one embodiment, the invention provides a method for identifying glycopolypeptides in a serum, plasma or blood sample. The method can include the steps of (a) derivatizing glycopolypeptides in the sample; (b) immobilizing the derivatized sample glycopolypeptides to a solid support; (c) cleaving the immobilized sample glycopolypeptides, thereby releasing non-glyeosylated peptide fragments and retaining immobilized sample glycopeptide fragments; (d) labeling the immobilized sample glycopeptide fragments with an isotope tag; (e) releasing the sample glycopeptide fragments from the solid support thereby generating released sample glycopeptide fragments; (f) adding to the released sample glycopeptide fragments a plurality of standard peptides selected from peptides containing the glycosylation sites shown in Tables 7 (SEQ ID NOS:1-3244), 8 (SEQ ID NOS:3245-3369) or 10 (SEQ ID NOS:3370-3517) and referenced as SEQ ID NOS:1-3517, wherein the standard peptides correspond to peptides derivatized as in step (a), cleaved as in step (c), and released as in step (e), and wherein the standard peptides are differentially labeled with a corresponding isotope tag as used in step (d); (g) analyzing the released sample glycopeptide fragments using mass spectrometry; and (h) identifying released sample glycopeptide fragments that correspond to standard peptides added in step (f). The method can further comprise quantifying the amount of the sample glycopeptide fragments identified in step (h).

As used herein, a plurality of standard peptides refers to a selection of 2 or more peptides containing the glycosylation sites listed in Tables 7, 8 and/or 10. A plurality of standard peptides can include, for example, 3 or more, 5 or more, 10 or more, 20 or more, 30 or more, 40 or more, 50 or more, 60 or more, 70 or more, 80 or more, 90 or more, 100 or more, 110 or more, 120 or more, 130 or more, 140 or more, 150 or more, 160 or more, 170 or more, 180 or more, 190 or more, 200 or more, 220 or more, 250 or more, 270 or more, 300 or more, 350 or more, 400 or more, 450 or more, 500 or more, 600 or more, 700 or more, 800 or more, 900 or more, 1000 or more or more, 2000 or more, or even up to all of the glycosylation sites listed in Tables 7, 8 and/or 10. In a particular embodiment, the plurality of standard peptides contains about 100 or more, about 110 or more, about 120 or more, about 130 or more, about 140 or more, about 150 or more, about 160 or more, about 170 or more, about 180 or more, about 190 or more, about 200 or more, about 220 or more, about 250 or more, about 270 or more, about 300 or more, about 350 or more, about 400 or more, about 450 or more, about 500 or more, about 600 or more, about 700 or more, about 800 or more, about 900 or more, or about 1000 or more peptides containing the glycosylation sites listed in Tables 7, 8 and/or 10. It is understood that when the plurality of standard peptides contains less than about 90 or about 80 peptides, the plurality of standard peptides specifically excludes peptides containing previously known glycosylation sites.

As disclosed herein, a number of N-linked glycosylated peptides have been identified in human plasma/serum, including those having the consensus N-X-S/T glycosylation motif (Table 7) and peptides that do not contain the consensus N-X-S/T motif (Table 8). It is understood that the sequences shown in Tables 7, 8 and 10 represent glycosylation sites and that the standard peptides referred to herein need only include the glycosylation sites but need not contain the exact sequences shown in Tables 7, 8 and/or 10, so long as the selected standard peptides correspond to peptides cleaved with the same cleavage reagent. For example, the first glycosylation site shown in Table 7 (SEQ ID NO:1) contains a glycosylated Asn at position 11 of the shown sequence. A standard peptide containing the glycosylation site referenced in SEQ ID NO: 1 can be, for example a peptide from Glu 2 to Lys 21 or Val 9 to Lys 21 if cleaved with trypsin (trypsin peptide), which cleaves on the carboxyl side of Lys or Arg. Both peptides are potential trypsin peptides since cleavage with proteases is not be 100% efficient at every cleavage site. Additionally, a standard peptide containing the glycosylation site referenced as SEQ ID NO: 1 can be, for example, Phe 2 to Glu 15 if cleaved with *Staphylococcus aureus* protease (sap peptide), which cleaves on the carboxyl side of Asp or Glu. Thus a plurality of standard peptides containing the glycosylation site of SEQ ID NO: 1 and corresponding to trypsin cleaved peptides can contain one or both of the trypsin peptides indicated above, whereas a plurality of standard paptides containing the glycosylation site of SEQ ID NO: and corresponding to *Staphylococcus αureus* protease can contain the sap peptide indicated above. Other proteases can also be used to generate protease specific peptides as desired and disclosed herein.

As used herein, a peptide that "corresponds" to a referenced condition means that the peptide has the same chemistry as if the referenced condition had been performed on the peptide. For example, if a sample peptide is derivatized, for example, by oxidation, cleaved with a particular cleavage reagent, and released from a solid support, the standard peptide is synthesized, either by the same process or using well known chemical synthesis methods, so that the standard peptide has identical chemistry except for any differential labeling due to the incorporation of an isotope tag. Because the standard and sample peptides are generally analyzed by MS and use identical chemistry except for any differential isotope labeling, the standard peptides are synthesized so that they have identical chemistry as the sample peptides to be analyzed.

In methods of the invention, the particular cleavage reagent used for the standard peptides can be selected by one skilled in the art based on a desired use. The standard peptides are synthesized so that the peptides incorporate the same resulting cleavage chemistry as selected for the sample peptides. In the case of generating the standard peptides, the peptides can be synthesized as longer peptides and cleaved with a desired reagent or can be synthesized as a desired sequence, so long as the resulting standard peptide would have the same product as though cleaved with the reagent used to cleave the sample glycopolypeptides. In methods of the invention in which the sample peptides are to be quantified, a predetermined amount of fhe standard peptide can be added for comparison and quantification (see, for example, Gygi et al., Nature Biotechnol. 17:994-999 (1999); WO 00/11208).

In a particular embodiment of a method of the invention, the solid support can comprise a hydrazide moiety. , In another embodiment of a method of the invention, the glycopeptides are released from the solid support using a glycosidase, for example, an N-glycosidase or an 0-glycosidase. In still another embodiment of a method of the invention, the glycopeptides can be released from the solid using sequential addition of N-glycosidase and O-glycosidasc. In yet another embodiment, the glycopeptides can be released from the solid support using chemical cleavage.

In one embodiment of a method of the invention, the glycopolypeptides can be oxidized with periodate. In still another embodiment of the invention, the glycopolypeptides can be cleaved with a protease, for example, trypsin.

In another embodiment, the invention provides a method for identifying glycopolypeptides in a serum sample. The method can include the steps of (a) immobilizing the sample glycopolypeptides to a solid support; (b) cleaving the immobilized sample glycopolypeptides, thereby releasing non-glycosylated peptide fragments and retaining immobilized sample glycopeptide fragments; (c) labeling the immobilized sample glycopeptide fragments with an isotope tag: (d) releasing the sample glycopeptide fragments from the solid support, thereby generating released sample glycopeptide fragments; (e) adding to the released sample glycopeptide fragments a plurality of standard peptides selected from peptides containing the glycosylation sites shown in Tables 7, 8 and/or 10 and referenced as SEQ ID NOS: 1-3482. wherein the standard peptides correspond to peptides cleaved as in step (b), and released as in step (d), and wherein the standard peptides are differentially labeled with a corresponding isotope tag as used in step (c); (1) analyzing the released: sample glycopeptide fragments using mass spectrometry; and (g) identifying released sample glycopeptide fragments that correspond to standard peptides added in step (e). Such a method can further comprise quantifying the amount of the sample glycopeptide fragments identified in step (g).

In yet another embodiment, the invention provides a method for identifying and quantifying glycopolypeptides in a control serum or plasma sample. The method can include the steps of (a) derivatizing glycopolypeptides in a control serum sample; (b) immobilizing the derivatized sample glycopolypeptides to a solid support; (c) cleaving the immobilized sample glycopolypeptides, thereby releasing non-glycosylated peptide fragments and retaining immobilized sample glycopeptide fragments; (d) labeling the immobilized sample glycopeptide fragments with an isotope tag; (e) releasing the sample glycopeptide fragments from the solid support, thereby generating released sample glycopeptide fragments; (f) adding to the released sample glycopeptide fragments a plurality of standard peptides selected from peptides containing the glycosylation sites shown in Tables 7, 8 and/or 10 and referenced as SEQ ID NOS: 1-3482, wherein the standard peptides correspond to peptides derivatized as in step (a), cleaved as in step (c), and released as in step (e), and wherein the standard peptides are differentially labeled with a corresponding isotope tag as used in step. (d); (g) analyzing the released sample glycopeptide fragments using mass spectrometry; (h) identifying released sample glycopeptide fragments that correspond to standard peptides added in step (f); and (i) quantifying the amount of the sample glycopeptide fragments identified in step (h). The control serum sample can be normal serum or plasma obtained from a healthy individual or individuals.

In an additional embodiment, the invention provides a method for identifying one or more diagnostic markers for a disease. The method can include the steps of (a) derivatizing glycopolypeptides in a serum sample from an individual having a disease; (b) immobilizing the derivatized sample glycopolypeptides to a solid support; (c) cleaving the immobilized sample glycopolypeptides, thereby releasing non-glycosylated peptide fragments and retaining immobilized sample glycopeptide fragments; (d) labeling the immobilized sample glycopeptide fragments with an isotope tag; (c) releasing the sample glycopeptide fragments from the solid support., thereby generating released sample glycopeptide fragments; (f) adding to the released sample glycopeptide fragments a predetermined amount of a plurality of standard peptides selected from peptides containing the glycosylation sites shown in Tables 7, 8 and/or 10 and referenced as SEQ ID NOS: 1-3482, wherein the standard peptides correspond to peptides derivatized as in step (a), cleaved as in step (c), and released as in step (e), and wherein the standard peptides are differentially labeled with a corresponding isotope tag as used in step (d); (g) analyzing the released sample glycopeptide fragments using mass spectrometry; (h) identifying released sample glycopeptide fragments that correspond to standard peptides added in step (f); (i) quantifying the amount of the sample glycopeptide fragments identified in step (h); and (j) comparing the amount of the sample glycopeptide fragments determined in step (i) to the amount of the same glycopeptide fragments determined in a normal serum sample. It is understood that the methods disclosed herein in which a glycopolypeptide sample is derivatized can also be performed in the absence of derivatization so long as the glycopolypeptides can be captured. An example of such a capture method includes lectin, antibody or affinity chromatography. In a particular embodiment, the disease is cancer.

In still another embodiment, the invention provides a method for identifying glycopeptides in a serum sample. The method can include the steps of (a) immobilizing glycopolypeptides from a serum sample to a solid support: (b) cleaving the immobilized sample glycopolypeptides, thereby releasing non-glycosylated peptide fragments and retaining immobilized sample glycopeptide fragments; (c) labeling the immobilized sample glycopeptide fragments with an isotope tag; (d) releasing the sample glycopeptide fragments from the solid support; (e) adding to the released sample glycopeptide fragments a plurality of standard peptides selected from peptides containing the glycosylation sites shown in Tables 7; 8 and/or 10 and referenced as SEQ ID NOS: 1-3482, wherein the standard peptides correspond to peptides cleaved as in step (b) and released as in step (d), and wherein the standard peptides are differentially labeled with a corresponding isotope tag as used in step (c); and-(f) analyzing the released sample glycopeptide fragments

In one embodiment, the cis-diol groups of carbohydrates in glycoproteins can be oxidized by periodate oxidation to give aldehydes, which are reactive to a hydrazide gel with an solid support to form covalent hydrazone bonds, The immobilized glycoproteins are subjected to protease digestion followed by extensive washing to remove the non-glycosylated peptides. The immobilized glycopeptide are released from beads by chemicals or glycosidases. The isolated peptides are analyzed by mass spectrometry (MS), and the glycopeptide sequence and corresponding proteins are identified by MS/MS combined with a database search. The glycopeptides can also be isotopically labeled, for example, at the amino or carboxyl termini to allow the quantities of glycopeptides from different biological samples to be compared.

The methods of the invention are based on selectively isolating glycosylated peptides, or peptides that were glycosylated in the original protein sample, from a complex sample. The sample consists of peptide fragments of proteins generated, for example, by enzymatic digestion or chemical cleavage. A stable isotope tag can be introduced into the isolated peptide fragments to facilitate mass spectrometric analysis and accurate quantification of the peptide fragments.

In one embodiment, a sample containing glycopolypeptides is chemically modified so that carbohydrates of the glycopolypeptides in the sample can be selectively bound to a solid support. For example, the glycopolypeptides can be bound covalently to a solid support by chemically modifying the carbohydrate so that the carbohydrate can covalently bind to a reactive group on a solid support. The carbohydrates of the sample glycopolypeptides are oxidized. The carbohydrate can be oxidized, for example, to aldehydes. The oxidized moiety, such as an aldehyde moiety, of the glycopolypeptides can react with a solid support containing hydrazide or amine moieties, allowing covalent attachment of glycosylated polypeptides to a solid support via hydrazine chemistry. The sample glycopolypeptides are immobilized through the chemically modified carbohydrate, for example, the aldehyde, allowing the removal of non-glycosylated sample proteins by washing of the solid support. If desired, the immobilized glycopolypeptides can be denatured and/or reduced. The immobilized glycopolypeptides are cleaved into fragments using either protease or chemical cleavage. Cleavage results in the release of peptide fragments that do not contain carbohydrate and are therefore not immobilized These released non-gtycosytated peptide fragments optionally can be further characterized, if desired.

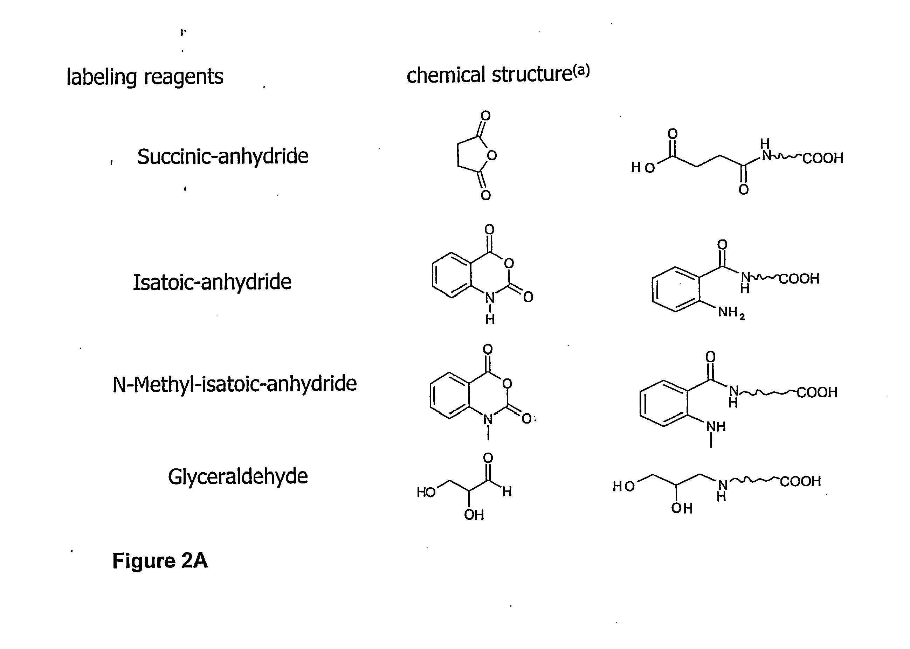

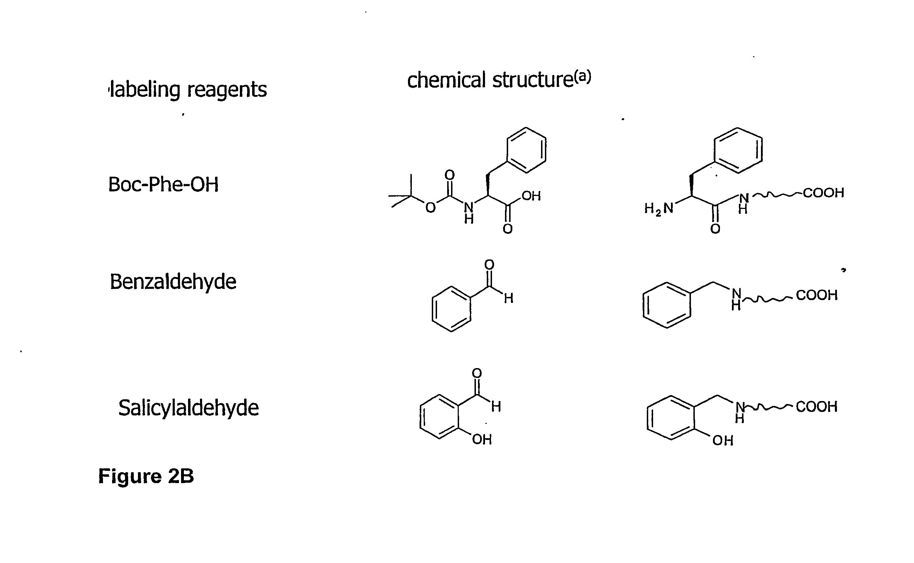

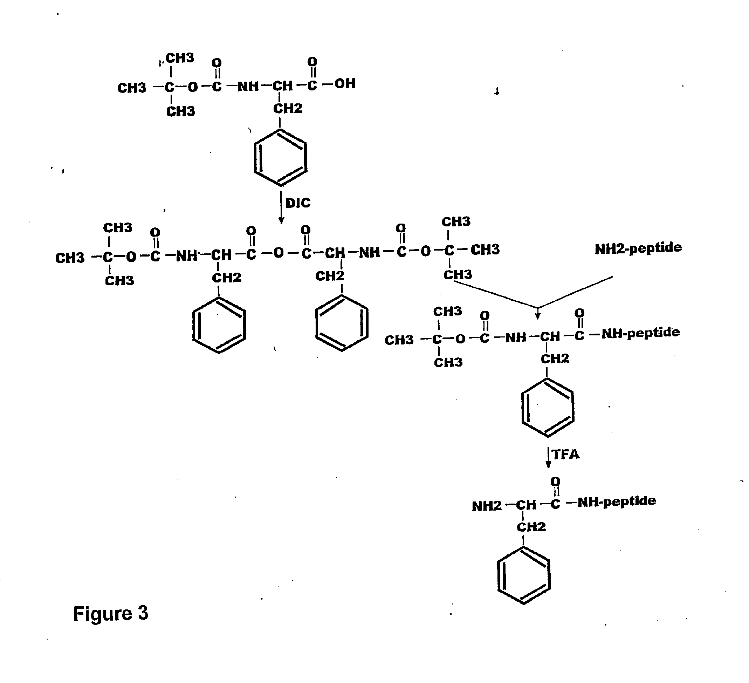

Following cleavage glycosylated peptide fragments (glycopeptide fragments) remain bound to the solid support. To facilitate quantitative mass spectrometry (MS) analysis, immobilized glycopeptide fragments can be isotopically labeled. If it is desired to characterize most or all of the immobilized glycopeptide fragments, the isotope tagging reagent contains an amino or carboxyl reactive group so that the N-terminus or C-terminus of the glycopeptide fragments can be labeled (see Figures 2 and 3). The immobilized glycopeptide fragments can be cleaved from the solid support chemically or enzymatically, for example, using glycosidases such as N-glycanase (N-glycosidase) or O-glycanase (O-glycosidase). The released glycopeptide fragments or their deglycosylated forms can be analyzed, for example, using MS.

As used herein, the term "polypeptide" refers to a peptide or polypeptide of two or more amino acids. A polypeptide can also be modified by naturally occurring modifications such as post-translational modifications, including phosphorylation, fatty acylation, prenylation, sulfation, hydroxylation, acetylation, addition of carbohydrate, addition of prosthetic groups or cofactors, formation of disulfide bonds, proteolysis, assembly into macromolecular complexes, and the like. A "peptide fragment" is a peptide of two or more amino acids, generally derived from a larger polypeptide.

As used herein, a "glycopolypeptide" or "glycoprotein" refers to a polypeptide that contains a covalently bound carbohydrate group. The carbohydrate can be a monosaccharide, oligosaccharide or polysaccharide. Proteoglycans are included within the meaning of "glycopolypeptide." A glycopolypeptide can additionally contain other post-translational modifications. A "glycopeptide" refers to a peptide that contains covalently bound carbohydrate. A "glycopeptide fragment" refers to a peptide fragment resulting from enzymatic or chemical cleavage of a larger polypeptide in which the peptide fragment retains covalent!y bound carbohydrate. It is understood that a glycopeptide fragment or peptide fragment refers to the peptides that result from a particular cleavage reaction, regardless of whether the resulting peptide was present before or after the cleavage reaction. Thus, a peptide that does not contain a cleavage site will be present after the cleavage reaction and is considered to be a peptide fragment resulting from that particular cleavage reaction. For example, if bound glycopeptides are cleaved, the resulting cleavage products retaining bound carbohydrate are considered to be glycopeptides fragments. The glycosylated fragments can remain bound to the solid support, and such bound glycopeptide fragments are considered to include those fragments that were not cleaved due to the absence of a cleavage site.

As disclosed herein, a glycopolypeptide or glycopeptide can be processed such that the carbohydrate is removed from the parent glycopolypeptide. It is understood that such an originally glycosylated polypeptide is still referred to herein as a glycopolypeptide or glycopeptide even if the carbohydrate is removed enzymatically and/or chemically. Thus, a glycopolypeptide or glycopeptide can refer to a glycosylated or de-glycosylated form of a polypeptide. A glycopolypeptide or glycopeptide from which the carbohydrate is removed is referred to as the de-glycosylated form of a polypeptide whereas a glycopolypeptide or glycopeptide which retains its carbohydrate is referred to as the glycosylated form of a polypeptide.

As used herein, the term "sample" is intended to mean any biological fluid, cell, tissue, organ or portion thereof, that includes one or more different molecules such as nucleic acids, polypeptides, or small molecules. A sample can be a tissue section obtained by biopsy, or cells that are placed in or adapted to tissue culture. A sample can also be a biological fluid specimen such as blood, serum or plasma, cerebrospinal fluid, urine, saliva, seminal plasma, pancreatic juice, breast milk, lung lavage, and the like. A sample can additionally be a cell extract from any species, including prokaryotic and eukaryotic cells as well as viruses. A tissue or biological fluid specimen can be further fractionated, if desired, to a fraction containing particular cell types. As used herein, a "serum sample" refers to the fluid portion of the blood obtained after removal of the fibrin clot and blood cells. As used herein, a plasma sample" refers to the fluid, non-cellular portion of the blood.

As used herein, a "polypeptide sample" refers to a sample containing two or more different polypeptides. A polypeptide sample can include tens, hundreds, or even thousands or more different polypeptides. A polypeptide sample can also include non-protein molecules so long as the sample contains polypeptides polypeptide sample can be a whole cell or tissue extract or can be a biological fluids. Furthermore, a polypeptide sample can be fractionated using well known methods, as disclosed herein, into partially or substantially purified protein fractions. In a particular embodiment, a polypeptide, sample can be a serum sample or plasma sample.

The use of biological fluids such as a body fluid as a sample source is particularly useful in methods of the invention. Biological fluid specimens are generally readily accessible and available in relatively large quantities for clinical analysis. Biological fluids can be used to analyze diagnostic and prognostic markers for various diseases. In addition to ready accessibility, body fluid specimens do not require any prior knowledge of the specific organ or the specific site in an organ that might be affected by disease. Because body fluids, in particular blood, are in contact with numerous body organs, body fluids "pick up" molecular signatures indicating pathology due to secretion or cell lysis associated with a pathological condition. Body fluids also pick up molecular signatures that are suitable for evaluating drug dosage, drug targets and/or toxic effects, as disclosed herein. The invention can advantageously be used with readily accessible samples such as blood, plasma or serum.

The methods of the invention utilize the selective isolation of glycopolypeptides coupled with chemical modification to facilitate MS analysis. Proteins are glycosylated by complex enzymatic mechanisms, typically at the side chains of serine or threonine residues (O-linked) or the side chains of asparagine residues (N-linlked). N-linked glycosylation sites generally fall into a sequence motif that can be described as N-X-S/T, where X can be any amino acid except proline. Glycosylation plays an important function in many biological processes (reviewed in Helenius and Aebi, Science 291:2364-2369 (2001); Rudd et al., Science 291:2370-2375 (2001)).

Protein glycosylation has long been recognized as a very common post-translational modification. As discussed above, carbohydrates are linked to serine or threonine residues (O-linked glycosylation) or to asparaginc residues (N-linked Glycosylation) (Varki et al. Essentials of Glycobiology Cold Spring Harbor Laboratory (1999)). Protein glycosylation, and in particular N-linked glycosylation, is prevalent in proteins destined for extracellular environments (Roth. Chem. Rev. 102:285-303 (2002)). These include proteins on the extracellular side of the plasma membrane, secreted proteins, and proteins contained in body fluids, for example blood serum, cerebrospinal fluid, urine, breast milk, saliva, lung lavage fluid, pancreatic juice, and the like. These also happen to be the proteins in the human body that are most easily accessible for diagnostic and therapeutic purposes.

Due to the ready accessibility of body fluids exposed to the extracellular surface of cells and the presence of secreted proteins in these fluids, many clinical biomarkers and therapeutic targets are glycoproteins. These include Her2/neu in breast cancer, human chorionic gonadotropin and $\alpha$-fetoprotein in germ cell tumors, prostate-specific antigen in prostate cancer, and CA125 in ovarian cancer. The Her2/neu receptor is also the target for a successful immunotherapy of breast cancer using the humanized monoclonal antibody Herceptin. (Shepard et al., J. Clin. Immunol. 11:117-127 (1991)). In addition, changes in the extent of glycosylation and the carbohydrate structure of proteins on the cell surface and in body fluids have been shown to correlate with cancer and other disease states, highlighting the clinical importance of this modification as an indicator or effector of pathologic mechanisms (Durand and Seta, Clin. Chem. 46:795-805. (2000); Freeze, Glycobiology 11:129R-143R (2001); Spiro, Glycobiology 12:43R-56R (2002)). Therefore, a method for the systematic and quantitative analysis of glycoproteins would be of significance for the detection of new potential diagnostic markers and therapeutic targets.

To selectively isolate glycopolypeptides, the methods utilize chemistry and/or binding interactions that are specific for carbohydrate moieties. Selective binding of glycopolypeptides refers to the preferential binding of glycopolypeptides over non-glycosylated peptides. The methods of the invention can utilize covalent coupling of glycopolypeptides, which is particularly useful for increasing the selective isolation of glycopolypeptides by allowing stringent washing to remove non-specifically bound, non-glycosylated polypeptides.

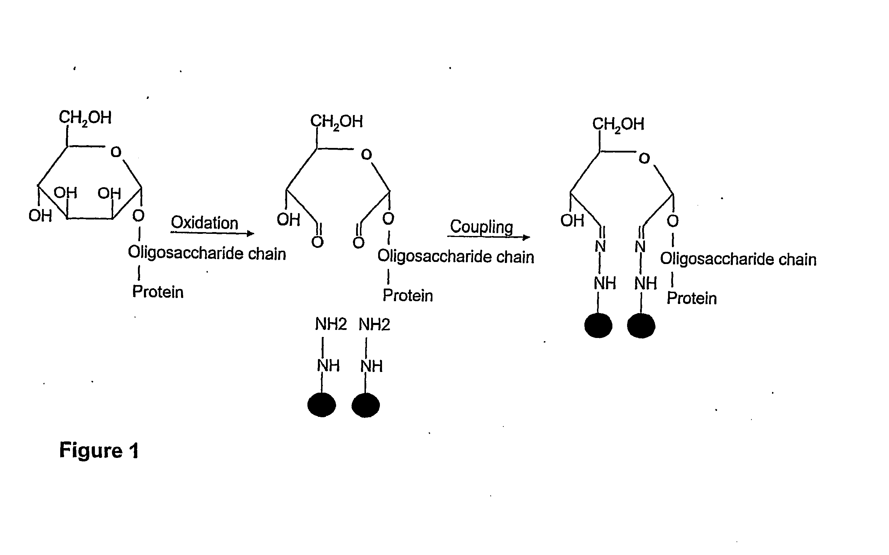

The carbohydrate moieties of a glycopolypeptide are chemically or enzymatically modified to generate a reactive group that can be selectively bound to a solid support having a corresponding reactive group. In the embodiment depicted in Figure 1. the carbohydrates of glycopolypeptides are oxidized to aldehydes. The oxidation call be performed, for example, with sodium periodate. The hydroxyl groups of a carbohydrate can also be derivatized by epoxides or oxiranes, alkyl halogen, carbonyldiimidazoles, N.N'-disuccinimidyl carbonates, N-hydroxycuccinimidyl chloroformates, and the like. The hydroxyl groups of a carbohydrate can also be oxidized by enzymes to create reactive groups such as aldehyde groups. For example, galactose oxidase oxidize terminal galactose or N-acetyl-D-galactose residues to form C-6 aldehyde groups. These derivatized groups can be conjugated to amine- or hydrazide- containing moieties.

The oxidation of hydroxyl groups to aldehyde using sodium periodate is specific for the carbohydrate of a glycopepide. Sodium periodate can oxidize hydroxyl groups on adjacent carbon atoms, forming aldehydes for coupling with amine- or hydrazide-containing molecules. Sodium periodate also reacts with hydroxylamine derivatives, compounds containing a primary amine and a secondary hydroxyl group on adjacent carbon atoms. This reaction is used to create reactive aldehydes on N-terminal serine residues of peptides. A serine residue is rare at the N-terminus of a protein. The oxidation to an aldehyde using sodium periodate is therefore specific for the carbohydrate groups of a glycopolypeptide.

Once the carbohydrate of a glycopolypeptide is modified, for example, by oxidition to aldehydes, the modified carbohydrates can bind to a solid support containing hydrazide or amine moieties, such as the hydrazide resin depicted in Figure 1. Although illustrated with oxidation chemistry and coupling to hydrazide, it is understood that any suitable chemical modifications and/or binding interactions that allows specific binding of the carbohydrate moieties of a glycopolypeptide can be used in methods of the invention. The binding interactions of the glycopolypeptides with the solid support are generally covalent, although non-covalent interactions can also be used so long as the glycopolypeptides or glycopeptide fragments remain bound during the digestion, washing and other steps of the methods.

The methods of the invention can also be used to select and characterize subgroups of carbohydrates. Chemical modifications or enzymatic modifications using, for example, glycosidases can be used to isolate subgroups of carbohydrates. For example, the concentration of sodium periodate can be modulated so that oxidation occurs on sialic acid groups of glycoproteins. In particular, a concentration of about 1 mM of sodium periodate at 00c can be used to modify sialic acid groups .

Glycopolypeptides containing specific monosaccharides can be targeted using a selective sugar oxidase to generate aldehyde functions, such as the galactose oxidase described above or other sugar oxidases. Furthermore, glycopolypeptides containing a subgroup of carbohydrates can be selected after the glycopolypeptides are bound to a solid support. For example, glycopeptides bound to a solid support can be selectively released using different glycosidases having specificity for particular monosaccharide structures.

The glycopolypeptides are isolated by binding to a solid support. The solid support can be, for example, a bead, resin, membrane or disk, or any solid support material suitable for methods of the invention. An advantage of using a solid support to bind the glycopolypeptides is that it allows extensive washing to remove non-glycosylated polypeptides. Thus, in the case of complex samples containing a multitude of polypeptides, the analysis can be simplified by isolating glycopolypeptides and removing the non-glycosylated polypeptides, thus reducing the number of polypeptides to be analyzed.

The glycopolypeptides can also be conjugated to an affinity tag through an amine group, such as biotin hydrazide. The glycopeptides can be cleaved by a protease. The affinity tagged glycopeptides can then be immobilized to the solid support, for example, an avidin or streptavidin solid support, and the non-glycosylated peptides are removed. The tagged glycopeptides can be released from the solid support by enzymatic or chemical cleavage. Alternatively, the tagged glycopeptides can be released from the solid support with the oligosaccharide and affinity tag attached.

Another advantage of binding the glycopolypeptides to the solid support is that it allows further manipulation of the sample molecules without the need for additional purification steps that can result in loss of sample molecules. For example, the methods of the invention can involve the steps of cleaving the bound glycopolypeptides as well as adding an isotope tag, or other desired modifications of the bound glycopolypeptides. Because the glycopolypeptides are bound, these steps can be carried out on solid phase while allowing excess reagents to be removed as well as extensive washing prior to subsequent manipulations.

The bound glycopolypeptides can be cleaved into peptide fragments to facilitate MS analysis. Thus, a polypeptide molecule can be enzymatically cleaved with one or more proteases into peptide fragments. Exemplary proteases useful for cleaving polypeptides include trypsin, chymotrypsin, pepsin, papain, Staphylococcus aureus (V8) protease. Submaxillaris protease, bromelain, thermolysin, and the like. In certain applications, proteases having cleavage specificities that cleave at fewer sites, such as sequence-specific proteases having specificity for a sequence rather than a single amino acid, can also be used, if desired. Polypeptides can also be cleaved chemically, for example, using CNBr, acid or other chemical reagents. A particularly useful cleavage reagent is the protease trypsin. One skilled in the art can readily determine appropriate conditions for cleavage to achieve a desired efficiency of peptide cleavage.

Cleavage of the bound glycopolypeptides is particularly useful for MS analysis in that one or a few peptides are generally sufficient to identify a parent polypeptide. However, it is understood that cleavage of the bound glycopolypeptides is not required, in particular where the bound glycopolypeptide is relatively small and contains a single glycosylation site. Furthermore, the cleavage reaction can be carried out after binding of glycopolypeptides to the solid support, allowing characterization of non-glycosylated peptide fragments derived from the bound glycopolypeptide. Alternatively, the cleavage reaction can be carried out prior to addition of the glycopeptides to the solid support. One skilled in the art can readily determine the desirability of cleaving the sample polypeptides and an appropriate point to perform the cleavage reaction, as needed for a particular application of the methods of the invention.

If desired, the bound glycopolypeptides can be denatured and optionally reduced. Denaturing and/or reducing the bound glycopolypeptides can be useful prior to cleavage of the glycopolypeptides, in particular protease cleavage, because this allows access to protease cleavage sites that can be masked in the native form of the glycopolypeptides. The bound glycopeptides can be denatured with detergents and/or chaotropic agents. Reducing agents such as β-mercaptoethanol, dithiothreitol, tris-carboxyethylphosphine (TCFp), and the like, can also be used, if desired. As discussed above, the binding of the glycopolypeptides to a solid support allows the denaturation step to be carried out followed by extensive washing to remove denaturants that could inhibit the enzymatic or chemical cleavage reactions. The use of denaturants and/or reducing agents can also be used to dissociate protein complexes in which non-glycosylated proteins form complexes with bound glycopolypeptides. Thus, the use of these agents can be used to increase the specificity for glycopolypeptides by washing away non-glyosylated polypeptides from the solid support.

Treatment of the bound glycopolypeptides with a cleavage reagent results in the generation of peptide fragments. Because the carbohydrate moiety is bound to the solid support, those peptide fragments that contain the glycosylated residue remain bound to the solid support. Following cleavage of the bound glycopolypeptides, glycopeptide fragments remain bound to the solid support via binding of the carbohydrate moiety. Peptide fragments that are not glycosylated are released from the solid support. If desired, the released non-glycosylated peptides can be analyzed, as described in more detail below.

The methods of the invention can be used to identify and/or quantify the amount of a glycopolypeptide present in a sample. A particularly useful method for identifying and quantifying a glycopolypeptide is mass spectrometry (MS). The methods of the invention can be used to identify a glycopolypeptide qualitatively, for example, using MS analysis. If desired, an isotope tag can be added to the bound glycopeptide fragments, in particular to facilitate quantitative analysis by MS.

As used herein an "isotope tag" refers to a chemical moiety having suitable chemical properties for incorporation of an isotope, allowing the generation of chemically identical reagents of different mass which can be used to differentially tag a polypeptide in two samples. The isotope tag also has an appropriate composition to allow incorporation of a stable isotope at one or more atoms. A particularly useful stable isotope pair is hydrogen and deuterium, which can be readily distinguished using mass spectrometry as light and heavy forms, respectively. Any of a number of isotopic atoms can be incorporated into the isotope tag so long as the heavy and light forms can be distinguished using mass spectrometry, for example, $^{13}C$, $^{15}N$ $^{17}O$, $^{18}O$ or $^{34}S$. Exemplary isotope tags include the 4,7, 10-trioxa-1,13-tridecanediamine based linker and its related deuterated form, 2,2',3,3',11,1 11',12,12'-octadeutero-4,7,t0-trioxa-1,1, 3-tridecanediamine, described by Gygi et al. (Nature Biotechnol. 17:994-999 (1999). Other exemplary isotope tags have also been described previously (see WO 00/1 1208, which is incorporated herein by reference).

In contrast to these previously described isotope tags related to an ICAT-type reagent, it is not required that an affinity tag be included in the reagent since the glycopolypeptides are already isolated. One skilled in the art can readily determine any of a number of appropriate isotope tags useful in methods of the invention. An isotope tag can be an alkyl, akenyl, alkynyl, alkoxy, aryl, and the like, and can be optionally substituted, for example, with O, S, N, and the like, and can contain an amine, carboxyl, sulfhydryl, and the like (see WO 00/11208). Exemplary isotope tags include succinic anhydride, isatoic-anhydride, N-methyl-isatoic-anhydride, glyceraldehyde, Boc-Phe_OH, benzaldehyde, salicylaldehyde, and the like (Figure 2). In addition to Phe, as shown in Figures 2 and 3, other amino acids similarly can be used as isotope tags. Furthermore, small organic,aldehydes, similar to those shown in Figure 2, can be used as isotope tags. These and other derivatives can be made in the same manner as that disclosed herein using methods well known to those skilled in the art. One skilled in the art will readily recognize that a number of suitable chemical groups can be used as an isotope tag so long as the isotope tag can be differentially isotopically labeled.

The bound glycopeptide fragments are tagged with an isotope tag to facilitate MS analysis. In order to tag the glycopeptide fragments, the isotope tag contains a reactive group that can react with a chemical group on the peptide portion of the glycopeptide fragments. A reactive group is reactive with and therefore can be covalently coupled to a molecule in a sample such as a polypeptide. Reactive groups are well known to those skilled in the art (see, for example, Hermanson, Bioconjugate Techniques, pp. 3-166, Academic Press, San Diego (1996); Glazer et al., Laboratory Techniques in Biochemistry and Molecular Biology: Chemical Modification of Proteins, Chapter 3, pp. 68-120, Elsevier Biomedical Press, New York (1975); Pierce Catalog (1994), Pierce, Rockford IL). Any of a variety of reactive groups can be incorporated into an isotope tag for use in methods of the invention so long as the reactive group can be covalently coupled to the immobilized polypeptide.

To analyze a large number or essentially all of the bound glycopolypeptides, it is desirable to use an isotope tag having a reactive group that will react with the majority of the glycopeptide fragments. For example, a reactive group that reacts with an amino group can react with the free amino group at the N-terminus of the bound glycopeptide fragments. If a cleavage reagent is chosen that leaves a free amino group of the cleaved peptides, such an amino group reactive agent can label a large fraction of the peptide fragments. Only those with a blocked N-terminus would not be labeled. Similarly, a. cleavage reagent that leaves a free carboxyl group on the cleaved peptides can be modified with a carboxyl reactive group resulting in the labeling of many if not all of the peptides. Thus, the inclusion of amino or carboxyl reactive groups in an isotope tag is particularly useful for methods of the invention in which most if not all of the bound glycopeptide fragments are desired to be analyzed.

In addition, a polypeptide can be tagged with an isotope tag via a sulfhydryl reactive group, which can react with free sulfhydryls of cysteine or reduced cystines in a polypeptide. An exemplary sulfhydryl reactive group includes an iodoacetamido group (see Gygi et al., supra, 1999). Other examplary sulfhydryl reactive groups include maleimides, alkyl and aryl halides, haloacetyls, $\alpha$-haloacyls, pyridyl disulfides, aziridines, acrylolyls, arylating agents and thiomethylsulfones.

In addition, a synthetic standard polypeptide can be tagged during the peptide synthesis process using heavy isotopic labeled residues as substitution. The heavy isotope labeled residues can be any amino acids present in the peptide sequence, such as heavy isotope tagged Leu, Val, Pro, Phe, and Asp (Underlined residues in Table 5 for synthesized stable isotope tagged standard peptides). Since the N-linked Asn residues are converted to Asp during the glycopeptide capture-and-release procedure. Asp instead of Asn was incorporated into peptide sequence in peptide synthesis of the stable isotope labeled peptides.

A reactive group can also react with amines such as the $\alpha$-amino group of a peptide or the $\epsilon$-amino group of the side chain of Lys, for example, imidoesters, N-hydroxysuccinimidyl esters (NHS), isothiocyanates, isocyanates, acyl azides, sulfonyl chlorides, aldehydes, ketones, glyoxals, epoxides (oxiranes), carbonates, arylating agents, carbodiimides, anhydrides, and the like. A reactive group can also react with carboxyl groups found in Asp or Glu or the C-terminus of a peptide, for example, diazoalkanes, diazoacetyls, carbonyldiimidazole, carbodiimides, and the like. A reactive group that reacts with a hydroxyl group includes, for example, epoxides, oxiranes, carbonyldiimidazoles, N,N'-disuccinimidyl carbonates, N-hydroxycuccinimidyl chloroformates, and the like. A reader group car also react with amino acids such as histidine, for example, $\alpha$-lialoacids and amides; tyrosine, for example nitration and iodination; arginine, for example, butanedione, phenylglyoxal, and nitromalondialdchyde; methionine, for example, iodoacetic acid and iodoacetamide: and tryptophan, for example, 2-(-2-nitronphenylsulfenyl-)3-bromoindolenine (BNPS-skatole), N-bromosuccinmide, formylation, and sulfenylation (Glazer et al., supra, 1975). In addition, a reactive group can also react with a phosphate group for selective labeling of phosphopeptides (Zhou et al., Nat. Biotechnol., 19:375-378 (2001)) or with other covalently modified peptides, including lipopeptides, or any of the known covalent polypeptide modifications. One skilled in the art can readily determine conditions for modifying sample molecules by using various reagents, incubation conditions and time of incubation to obtain conditions suitable for modification of a molecule with an isotope tag. The use of covalent-chemistry based isolation methods is particularly useful due to the highly specific nature of the binding of the glycopolypeptides.

The reactive groups described above can form a covalent bond with the target sample molecule. However, it is understood that an isotope tag can contain a reactive group that can non-covalently interact with a sample molecule so long as the interaction has high specificity and affinity.

Prior to further analysis, it is generally desirable to release the bound glycopeptide fragments. The glycopeptide fragments can be released by cleaving the fragments from the solid support, either enzymatically or chemically. For example, glycosidase such as N-glycosidases and O-glycosidases can be used to cleave an N-linked or O-linked carbohydrate moiety, respectively, and release the corresponding de-glycosylated peptide(s). If desired, N-glycosidases and O-glycosidases can be added together or sequentially, in either order. The sequential addition of an N-glycosidase and an O-glycosidase allows differential characterization of those released peptides that were N-linked versus those that were O-linked, providing additional information on the nature of the carbohydrate moiety and the modified amino acid residue. Thus, N-linked and O-linked glycosylation sites can be analyzed sequentially and separately on the same sample, increasing the information content of the experiment and simplifying the complexity of the samples being analyzed.

In addition to N-glycosidases and O-glycosidases other glycosidases can be used to release a bound glycopolypeptide. For example, exoglycosidases can be used. Exoglycosidases are anomeric, residue and linkage specific for terminal monnosaccharides and can he used to released peptides having the corresponding carbohydrate.

In addition to enzymatic cleavage, chemical cleavage can also be used to cleave a carbohydrate moiety to release a bound peptide. For example, O-linked oligosaccharides can be released specifically from a polypeptide via a β-elimination reaction catalyzed by alkali. The reaction can be carried out in about 50 mM NaOH containing about 1 M $NaBH_4$ at about 55°C for about 12 hours. The time, temperature and concentration of the reagents can be varied so long as a sufficient ÿ-elimination reaction is carried out for the needs of the experiment.

In one embodiment, N-linked oligosaccharides can be released from glycopolypeptides, for example, by hydrazinolysis. Glycopolypeptides can be dried in a desiccator over $P_2O_5$ and NaOH. Anhydrous hydrazine is added and heated at about 100°C for 10 hours, for example, using a dry heat block.

In addition to using enzymatic or chemical cleavage to release a bound glycopeptide, the solid support can be designed so that bound molecules can be released; regardless of the nature of the bound carbohydrate. The reactive group on the solid support, to which the glycopolypeptide binds, can be linked to the solid support with a cleavable linker. For example, the solid support reactive group can be covalently bound to the solid support via a cleavable linker such as a photocleavable linker. Exemplary photocleavable linkers include, for example, linkers containing o-nitrobenzyl, desyl, trans-o-cinnamoyl, m-nitrophenyl, benzylsulfonyl groups (see, for example, Dorman and Prestwich, Trends Biotech. 18:64-77 (2000); Greene and Wuts, Protective Groups in Organic Synthesis, 2nd ed., John Wiley & Sons, New York (1991); U.S. Patent Nos. 5,143,854; 5,986,076; 5,917,016; 5,489,678; 5,405,783). Similarly, the reactive group can be linked to the solid support via a chemically cleavable linker. Release of glycopeptide fragments with the intact carbohydrate is particularly useful if the carbohydrate moiety is to be characterized using well known -methods, including mass spectrometry. The use of glycosidases to release de-glycosylated peptide fragments also provides information on the nature of the carbohydrate moiety.

Glycopolypeptides from a sample are bound to a solid support via the carbohydrate moiety. The bound glycopolypeptides are generally cleaved, for example, using a protease, to generate glycopeptide fragments. As discussed above, a variety of methods can be used to release the bound glycopeptide fragments, thereby generating released glycopeptide fragments. As used herein, a "released glycopeptide fragment" refers to a peptide which was bound to a solid support via a covalently bound carbohydrate moiety and subsequently released from the solid support, regardless of whether the released peptide retains the carbohydrate. In some cases, the method by which the bound glycopeptide fragments are released results in cleavage and removal of the carbohydrate moiety, for example, using glycosidases or chemical cleavage of the carbohydrate moiety. If the solid support is designed so that the reactive group, for example, hydrazide, is attached to the solid support via a cleavable linker, the released glycopeptide fragment retains the carbohydrate moiety. It is understood that, regardless whether a carbohydrate moiety is retained or removed from the released peptide, such peptides are referred to as released glycopeptide fragments.

After isolating glycopolypeptides from a sample and cleaving the glycopolypeptide into fragments, the glycopeptide fragments released from the solid support and the released glycopeptide fragments are identified and/or quantitified. A particularly useful method for analysis of the released glycopeptide fragments is mass spectrometry. A variety of mass spectrometry systems can be employed in the methods of the invention for identifying and/or quantifying a sample molecule such as a released glycopolypeptide fragment. Mass analyzers with high mass accuracy, high sensitivity and high resolution include, but are not limited to, ion trap, triple quadrupole, and time-of-flight, quadrupole time-of-flight mass spectrometeres and Fourier transform ion cyclotron mass analyzers (FT-ICR-MS). Mass spectrometers are typically equipped with matrix-assisted laser desorption (MALDI) or electrospray ionization (ESI) ion sources, although other methods of peptide ionization can also be used. In ion trap MS, analytes are ionized by ESI or MALDI and then put into an ion trap. Trapped ions can then be separately analyzed by MS upon selective release from the ion trap. Fragments can also be generated in the ion trap and analyzed. Sample molecules such as released glycopeptide fragments can be analyzed, for example, by single stage mass spectrometry with a MALDI-TOF or ESI-TOF system. Methods of mass spectrometry analysis are well known to those skilled in the art (see, for example, Yates, J.Mass Spect. 33: 1-19 (1998): Kinter and Sherman, Protein Sequencing and Identification Using Tandem Mass Spectrometry, John Wiley & Sons, New York (2000): Aebersold and Goodlett, Chem. Rev. 101 :269-295 (2001)),

For high resolution polypeptide fragment separation, liquid chromatography ESI-MS/MS or automated LC-MS/MS, which utilizes capillary reverse phase chromatography as the separation method, can be used (Yates et al., Methods Mol. Biol. 112:553-.569 (1999)). Data dependent collision-induced dissociation (CID) with dynamic exclusion can also be used as the mass spectrometric method (Goodlett, et al., Anal. Chem. 72:1112-1118 (2000)).

Once a peptide is analyzed by MS/MS, the resulting CID spectrum can be compared to databases for the determination of the identity of the isolated glycopeptide. Methods for protein identification using single peptides has been described previously (Aebersold and Goodlett, Chem. Rev. 101:269-295 (2001); Yates, J. Mass Spec. 33:1-19 (1998)). In particular, it is possible that one or a few peptide fragments can be used to identify a parent polypeptide from which the fragments were derived if the peptides provide a unique signature for the parent polypeptide. Thus, identification of a single glycopeptide, alone or in combination with knowledge of the site of glycosylation, can be used to identify a parent glycopolypeptide from which the glycopeptide fragments were derived. Further information can be obtained by analyzing the nature of the attached tag and the presence of the consensus sequence motif for carbohydrate attachment. For example, if peptides are modified with an N-terminal tag, each released glycopeptide has the specific N-terminal tag, which can be recognized in the fragment ion series of the CID spectra. Furthermore, the presence of a known sequence motif that is found, for example, in N-linked carbohydrate-containing peptides, that is, the consensus sequence NXS/T, can be used as a constraint in database searching of N-glycosylated peptides.

In addition, the identity of the parent glycopolypeptide can be determined by analysis of various characteristics associated with the peptide, for example, its resolution on various chromatographic media or using various fractionation methods. These empirically determined characteristics can be compared to a database of characteristics that uniquely identify a parent polypeptide, which defines a peptide tag.

The use of a peptide tag and related database is used for identifying a polypeptide from a population of polypeptides by determining characteristics associated with a polypeptide, or a peptide fragment thereof, comparing the determined characteristics to a polypeptide identification index, and identifying one or more polypeptides in the polypeptide identification index having the same characteristics (see WO 02/052259). The methods are based on generating a polypeptide identification index, which is a database of characteristics associated with a polypeptide. The polypeptide identification index can be used for comparison of characteristics determined to be associated with a polypeptide from a sample for identification of the polypeptide. Furthermore, the methods can be applied not only to identify a polypeptide but also to quantitate the amount of specific proteins in the sample.

The incorporation of an isotope tag can be used to facilitate quantification of the sample glycopolypeptides. As disclosed previously, the incorporation of an isotope tag provides a method for quantifying the amount of a particular molecule in a sample (Gygi et al ., supra, 1999; WO 00/11208). In using an isotope tag, differential isotopes can be incorporated, which can be used to compare a known amount of a. standard labeled molecule having a differentially labeled isotope tag from that of a sample molecule, as described in more detail below. Thus, a standard peptide having a differential isotope can be added at a known concentration and analyzed in the same MS analysis or similar conditions in a parallel MS analysis. A specific, calibrated standard can be added with known absolute amounts to determine an absolute quantity of the glycopolypeptide in the sample. In addition, the standards can be added so that relative quantitation is performed, as described below.

Alternatively, parallel glycosylated sample molecules can be labeled with a different isotopic label and compared side-by-side (see Gygi et al., supra, 1999). This is particularly useful for qualitative analysis or quantitative analysis relative to a control sample. For example, a glycosylated sample derived from a disease state can be compared to a glycosylated sample from a non-disease state by differentially labeling the two sample, as described previously (Gygi et al., supra, 1999). Such an approach allows detection of differential states of glycosylation, which is facilitated by the use of differential isotope tags for the two samples, and can thus be used to correlate differences in glycosylation as a diagnostic marker for a disease.

The methods of the invention provide numerous advantages for the analysis of complex biological and clinical samples. From every glycoprotein present in a complex sample, only a few peptides will be isolated since only a few peptides of a glycoprotein are glycosylated. Therefore, by isolating glycopeptide fragments, the composition of the resulting peptide mixture is significantly simplified for mass spectrometric analysis. For example, every protein on average will produce dozens of tryptic peptides but only one to a few tryptic glycosylated peptides. For example, the number of glycopeptides is significantly lower than the number of tryptic peptides or Cys-containing peptides in the major plasma proteins. Thus, analysis of glycopolypeptides or glycopeptides reduces the complexity of complex biological samples, for example, serum.

Another advantage of the methods of the invention is the use for analysis of body fluids as a clinical specimen, in particular serum. Five major plasma proteins represent more than 80% of the total protein in plasma, albumin, $\alpha$1

antitrypsin, $\alpha$2 macroglobulin, transferrin, and $\gamma$-globutins. Of these, albumin is the most abundant protein in blood serum and other body fluids, constituting about 50% of the total protein in plasma. However, albumin is essentially transparent to the methods of the invention due to the lack of N-glycosylation. For example, no tryptic N glycosylated peptides from albumin were observed when the methods of the invention were applied and a N-glycosidase was used to release the N-linked glycopeptides. This is all the more significant because more than 50 different albumin species have been detected by 2D gel electrophoresis that collectively obscure a significant part of the gel pattern and the analysis of less abundant serum proteins having clinical significance. Therefore, the methods of the invention that allow analysis of glycosylated proteins compensate for the dominance of albumin in serum and allow the analysis of less abundant, glycosylated proteins present in serum. As disclosed herein, the methods of the invention allowed the identification of many more serum proteins compared to conventional methods. The methods of the invention also allow the analysis of less abundant serum proteins. These low abundance serum proteins are potential diagnostic markers. Such markers can be readily determined by comparing disease samples with healthy samples, as disclosed herein.

Additionally, the known sequence motif for N-glycosylation (N-X-S/T) serves as a powerful sequence database search contraint for the identification of the isolated peptides. This can be used to facilitate the-identifiication of the polypeptide from which the glycopeptide fragment was derived since a smaller number of possible peptides will contain the glycosylation motif.

The methods of the invention are also advantageous because they allow fast throughput and simplicity. Accordingly, the methods can be readily adapted for high throughput analysis of samples, which can be particularly advantageous for the analysis of clinical samples. Furthermore, the methods of the invention can be automated to facilitate the processing of multiple samples. As disclosed herein, a robotic workstation has been adapted for automated glycoprotein analysis.

As described above, non-glycosylated peptide fragments are released from the solid support after proteolytic or chemical cleavage. If desired, the released peptide fragments can be characterized to provide further information on the nature of the glycopolypeptides isolated from the sample. A particularly useful method is the use of the isotope-coded affinity tag (ICAT™) method (Gygi et al., Nature Biotechnol. 17:994-999 (1999) which is incorporated herein by reference). The ICAT™ type reagent method uses an affinity tag that can be differentially labeled with an isotope that is readily distinguished using mass spectrometry. The ICAT™ type affinity reagent consists of three elements, an affinity tag, a linker and a reactive group.

One element of the ICAT™ type affinity reagent is an affinity tag that allows isolation of peptides coupled to the affinity reagent by binding to a cognate binding partner of the affinity tag. A particularly useful affinity tag is biotin, which binds with high affinity to its cognate binding partner avidin, or related molecules such as streptavidin, and is therefore stable to further biochemical manipulations. Any affinity tag can be used so long as it provides sufficient binding affinity to its cognate binding partner to allow isolation of peptides coupled to the ICAT™ type affinity reagent. An affinity tag can also be used to isolate a tagged peptide with magnetic beads or other magnetic format suitable to isolate a magnetic affinity tag .In the ICAT™ type reagent methods, or any other method of affinity tagging a peptide, the use of covalent trapping, for example, using a cross-linking reagent, can be used to bind the ragged peptides to a solid support, if desired.

A second element of the ICAT™ type affinity reagent is a linker that can A second element of the ICAT™ type affinity reagent is a linker that can incorporate a stable isotope. The linker has a sufficient length to allow the reactive group to bind to a specimen polypeptide and the affinity tag to bind to its cognate binding partner. The linker also has an appropriate composition to allow incorporation of a stable isotope at one or more atoms. A particularly useful stable isotope pair is hydrogen and deuterium, which can be readily distinguished using mass spectrometry as light and heavy forms, respectively. Any of a number of isotopic atoms can be incorporated into the linker so long as the heavy and light forms can be distinguished using mass spectrometry. Exemplary linkers include the 4,7,10-trioxa-1,13-tridecanediamine based linker and its related deuterated form, 2,2',3,3',1 11, 11',12,12'-octadeutero-4,7,10-trioxa-1,13 tridecanediamine, described by Gygi et al. (supra, 1999). One skilled in the art can readily determine any of a number of appropriate linkers useful in an ICAT™ type affinity reagent that satisfy the above-described criteria, as described above for the isotope tag.

The third element of the ICAT™ type affinity reagent is a reactive group, which can be covalently coupled to a polypeptide in a specimen. Various reactive groups have been described above with respect to the isotope tag and can similarly be incorporated into an ICAT-type reagent.

The ICAT™ method or other similar methods can be applied to the analysis of the non-glycosylated peptide fragments released from the solid support. Alternatively, the ICAT™ method or other similar methods can be applied prior to cleavage of the bound glycopolypeptides, that is, while the intact glycopolypeptide is still bound to the solid support.

The method generally involves the steps of automated tandem mass spectrometry and sequence database searching for peptide/protein identification; stable isotope tagging for quantification by mass spectrometry based on stable isotope dilution theory; and the use of specific chemical reactions for the selective isolation of specific peptides. For example, the previously described ICAT™ reagent contained a sulfhydryl reactive group, and therefore an ICAT™-type reagent can be used to label cysteine-containing peptide fragments released from the solid support. Other reactive groups, as described above., can also be used.

The analysis of the non-glycosytated peptides, in conjunction with then methods of analyzing glycosylated peptides, provides additional information on the state of polypeptide expression in the sample. By analyzing both the glycopeptidc fragments as well as the non-glycosylated peptides, changes in glycoprotein abundance as well as changes in the state of-glycosylation at a particular glycosylation site can be readily determined.

If desired, the sample can be fractionated by a number of known fractionation techniques. Fractionation techniques can be applied at any of a number of suitable points in the methods of the invention. For example, a sample can be fractionated prior to oxidation and/or binding of glycopolypeptides to a solid support. Thus, if desired, a substantially purified fraction of glycopolypeptide(s) can be used for immobilization of sample glycopolypeptides. Furthermore, fractionation/purification steps can be applied to non-glycosylated peptides or glycopeptides after release from the solid support. One skilled in the art can readily determine appropriate steps for fractionating sample molecules based on the needs of the particular application of methods of the invention. In the case of a blood sample, one skilled in the art can readily use well known methods for processing the blood, for example, to obtain plasma or serum.

Methods for fractionating sample molecules are well known to those skilled in the art. Fractionation methods include but are not limited to subcellular fractionation or chromatographic techniques such as ion exchange, including strong and weak anion and cation exchange resins, hydrophobic and reverse phase, size exclusion, affinity, hydrophobic charge-induction chromatography, dye-binding, and the like (Ausubel et al., Current Protocols in Molecular Biology (Supplement 56), John Wiley & Sons, New York (2001); Scopes, Protein Purification: Principles and Practice, third edition. Springer-Verlag, New York (1993)). Other fractionation methods include, for example, centrifugation, electrophoresis, the use of salts, and the like (see Scopes, supra. 1993). In the case of analyzing membrane glycoproteins, well known solubilization conditions can be applied to extract membrane bound proteins, for example, the use of denaturing and/or non-denaturing detergents (Scopes, supra, 1993).

Affinity chromatography can also be used including, for example, dye-binding resins such as Cibacron blue, substrate analogs, including analogs of cofactors such as ATP, NAD, and the like, ligands, specific antibodies useful for immune-affinity isolation, either polyclonal or monoclonal, and the like. A subset of glycopolypeptides can be isolated using lectin affinity chromatography, if desired. An exemplary affinity resin includes affinity resins that bind to specific moieties that can be incorporated into a polypeptide such as an avidin resin that binds to a biotin tag on a sample molecule labeled with an ICAT™-type reagent. The resolution and capacity of particular chromatographic media are known in the art and can be determined by those skilled in the art. The usefulness of a particular chromatographic separation for a particular application can similarly be assessed by those skilled in the art.

Those of skill in the art will be able to determine the appropriate chromatography conditions for a particular sample size or composition and will know how to obtain reproducible results for chromatographic separations under defined buffer, column dimension, and flow rate conditions. The fractionation methods can optionally include the use of an internal standard for assessing the reproducibility of a particular chromatographic application or other fractionation method. Appropriate internal standards will vary depending on the chromatographic medium or the fractionation method used. Those skilled in the art will be able to determine an internal standard applicable to a method of fractionation such as chromatography. Furthermore, electrophoresis, including gel electrophoresis or capillary electrophoresis, can also be used to fractionate sample molecules.

The methods of the invention can be used in a wide range of applications in basic and clinical biology. The methods of the invention can be used for the detection of changes in the profile of proteins expressed in the plasma membrane, changes in the composition of proteins secreted by cells and tissues, changes in the protein composition of body fluids including blood and seminal plasma, cerebrospinal fluid, pancreatic juice, urine, breast milk, lung lavage, and the like. In a particular embodiment, the methods are used to identify and/or quantify glycopolypeptides in a blood, plasma or serum sample, in particular a human sample. Since many of the proteins in these samples are glycosylated, the methods of the invention allow the convenient analysis of glycoproteins in these samples. Detected changes observed in a disease state can be used as diagnostic or prognostic markers for a wide range of diseases, including congenital disorders of glycosylation or any disorder involving aberrant glycosylation; cancer, such as skin, prostate, breast, colon, lung and others; metabolic diseases or processes such as diabetes or changes in physiological state; inflammatory diseases such as rheumatoid arthritis: mental disorders or neurological processes; infectious disease; immune response to pathogens; and the like. Furthermore, the methods of the invention can be used for the identification of potential targets for a variety of therapies including antibody-dependent cell cytotoxicity directed against cell surface proteins and for detection of proteins accessible to drugs.

Thus, the methods of the invention can be used to identify diagnostic markers for a disease by comparing a sample from a patient having a disease to a sample from a healthy individual or group of individuals. By comparing disease and healthy samples, a diagnostic pattern can be determined with increases or decreases in expression of particular glycopolypeptides correlated with the disease, which can be used for subsequent analysis of samples for diagnostic purposes. The methods are based on analysis of glycopolypeptides, and such an analysis is sufficient for diagnostic purposes.

Thus, the invention provides a method for identifying diagnostic glycopolypeptide markers by using a method of the invention and comparing samples from diseased individual(s) to healthy individual(s) and identifying glycopolypeptides having differential expression between the two samples, whereby differences in expression indicates a correlation with the disease and thus can function as a diagnostic marker. The invention also provides the diagnostic markers identified using methods of the invention.

Furthermore, glycopolypeptides exhibiting differential expression are potential therapeutic targets. Because they are differentially expressed, modulating the activity of these glycopolypeptides can potentially be used to ameliorate a sign or symptom associated with the disease. Thus, the invention provides a method for identifying therapeutic glycopolypeptide targets of a disease. Once a glycopolypeptide is found to be differentially expressed, the potential target can be screened for potential therapeutic agents that modulate the activity of the therapeutic glycopolypeptide target. Methods of generating libraries and screening the libraries for potential therapeutic activity are well known to those skilled in the art. Methods tor producing pluralities of compounds, including chemical or biological molecules such as simple or complex organic molecules, metal-containing compounds, carbohydrates, peptides, proteins, peptidomimetics, glycoproteins, lipoproteins, nucleic acids, antibodies, and the like, are well known in the art (see, for example, in Huse, U.S. Patent No. 5,264,563; Francis et al., Curr. Opin. Chem. Biol. 2:422-428 (1998); Tietze et al., Curr. Biol., 2:363.-371 (1998); Sofia, Mol. Divers. 3:75-94 (1998); Eichler et al., Med. Res. Rev. 15:481-496 (1995); Gordon et al., J. Med. Chem. 37: 1233-1251 (1994); Gordon et al., J. Med. Chem. 37: 1385-1401 (1994); Gordon et al., Acc. Chem. Res. 29: 144-154 (1996); Wilson and Czarnik, eds., Combinatorial Chemistry: Syntheses and Application, John Wiley & Sons, New York (1997)). The invention additionally provides glycopolypeptide therapeutic targets identified by methods of the invention.

The methods can be used for a variety of clinical and diagnostic applications. Known therapeutic methods effected through glycopolypeptides can be characterized by methods of the invention. For example, therapies such as Enbrel™ and Herceptin function through glycoproteins. The methods of the invention allow characterization of individual patients with respect to glycoprotein expression, which can be used to determine likely efficacy of therapy involving glycoproteins.

Thus, the methods of the invention can be used in a variety of applications including, but not limited to, the following applications. The methods of the invention can be used, for example, for blood serum profiling for the detection of prognostic and diagnostic protein markers.

The methods of the invention are applicable in clinical and diagnostic medicine, veterinary medicine, agriculture, and the like. For example, the methods of the invention can be used to identify and/or validate drug targets and to evaluate drug efficacy, drug dosing, and/or drug toxicity. In such a case the blood proteome, that is serum, can be analyzed using the methods disclosed herein to look for changes in serum glycopolypeptide profiles associated with drug administration and correlated with the effects of drug efficacy, dosing and/or toxicity, and/or validation of drug targets. Such a correlation can be readily determined by collecting serum samples from one or more individuals administered various drug doses, experiencing drug toxicity, experiencing a desired efficacy, and the like. In addition, a plasma or serum profile can be generated in combination with the analysis of drug targets as a way to rapidly and efficiently validate a particular target with the administration of a drug or various drug doses, toxicity and the like. Thus, serum, plasma or blood samples provide a surrogate marker for the status of an individual and his or her ability to respond to a pharmacological intervention.

The methods of the invention can additionally be used for quantitative protein profiling in various body fluids in addition to blood plasma, including CSF, pancreatic juice, lung lavage fluid, seminal plasma, urine, breast milk, and the like. The methods of the invention can also be used for quantitative protein profiling of proteins secreted by cells or tissues for the detection of new protein and peptide hormones and other factors. Thus, the invention provides a method to generate quantitative profiles of glycoproteins. The invention also provides a method for quantifying a glycopolypeptide in a sample, as disclosed herein. The invention further provides a method for the detection of prognostic or diagnostic patterns in blood, serum or plasma and other body fluids. The invention additionally provides a method for the detection of secreted protein hormones and regulatory factors. Thus, the invention provides a method for profiling glycopolypeptides from body fluids.

The methods of the invention are also applicable to the detection of changes in the state of glycosylation of proteins based on the concurrent application of protein abundance measurement and measurement of protein glycosylation on the same sample. Thus, the invention provides a method to detect quantitative changes in the glycosylation pattern of specific proteins.

Although the methods disclosed herein have generally been described for the analysis of glycopolypeptides, similar methods are also applicable to the analysis of other carbohydrate-containing molecules. Because the methods are based on the specific binding of carbohydrate moieties, the methods of modification and/or isolation can similarly be applied to other carbohydrate-containing molecules. For example, method steps analogous to those disclosed herein can be applied to the identification and quantification of glycosylated molecules such as glycolipids, glycosphingolipids, and the like.

The invention also provides a composition comprising a plurality of peptides containing the glycosylation sites shown in Tables 7, 8 and/or 10 and referenced as SEQ ID NOS: 1-3482, wherein the peptides each correspond to peptide fragments derived by cleavage of polypeptides using the same cleavage reagent. In one embodiment, the cleavage reagent can be a protease, for example, trypsin.

The invention additionally provides a kit comprising a plurality of peptides containing the glycosylation sites shown in Tables 7, 8 and/or 10 and referenced as SEQ ID NOS: 1-3482, wherein the peptides each correspond to peptide fragments derived by cleavage of polypeptides using the same cleavage reagent. The kit can further comprise a pair of differentially labeled isotope tags. In addition, the kit can further comprise the cleavage reagent corresponding to the peptide fragments, for example, a protease such as trypsin or other proteases disclosed herein. Additionally, the kit can further comprise a hydrazide resin. Also, a kit of the invention can further comprise a glycosidase.

The contents of the kit of the invention, for example, any resins or labeling reagents, are contained in suitable packaging material, and, if desired, a sterile, contaminant-free environment. In addition, the packaging material contains instructions indicating how the materials within the kit can be employed to label sample molecules. The instructions for use typically include a tangible expression describing the reagent concentration or at least one assay method parameter, such as the relative amounts of reagent and sample to be admixed, maintenance time periods for reagent/sample admixtures, temperature, buffer conditions, and the like.

The test sample can be, for example, a specimen from an individual having a disease. The control sample can be, for example, a corresponding specimen obtained from a healthy individual, also referred to herein as a normal sample. The sample can be, for example, serum or a tissue biopsy, as described herein. Differential glycosylation can be a qualitative difference, for example, the presence or absence of a glycopolypeptide in the test sample compared to the control sample. Differential glycosylation can also be a quantitative difference. The determination of quantitative differences can be facilitated by the labeling with differential isotope tags such that the samples can be mixed and compared side-by-side, as disclosed herein and described in Gygi et al., supra. 1999. One or more glycopolypeptides exhibiting differential glycosylation are potential diagnostic markers for the respective disease. Such a method provides a glycopolypeptide disease profile, which can be used subsequently for diagnostic purposes. Accordingly, rather than using one or a few diagnostic markers, the methods of the invention allow the identification of a profile of diagnostic markers, which can provide more detailed information on the type of disease, the stage of disease, and/or the prognosis of a disease by determining profiles correlated with the type, stage and/or prognosis of a disease.

In yet another embodiment, the invention provides a method of diagnosing a disease. The method can include the steps of immobilizing glycopolypeptides from a test sample to a solid support; cleaving the immobilized glycopolypeptides, thereby releasing non-glycosylated peptides and retaining immobilized glycopeptides; releasing the glycopeptides from the solid support; analyzing the released glycopeptides; and identifying one or more diagnostic markers associated with a disease, for example, as determined by methods of the invention, as described above.

A test sample from an individual to be tested for a disease or suspected of having a disease can be processed as described for glycopeptide analysis by the methods disclosed herein. The resulting glycopeptide profile from the test sample can be compared to a control sample to determine if changes in glycosylation of diagnostic markers has occurred, as discussed above. Alternatively, the glycopeptide profile can be compared to a known set of diagnostic markers or a database containing information on diagnostic markers.

In another embodiment, the method of diagnosing a disease can include the step of generating a report on the results of the diagnostic test. For example, the report can indicate whether an individual is likely to have a disease or is likely to be disease free based on the presence of a sufficient number of diagnostic markers associated with a disease. The invention further provides a report of the outcome of a method of diagnosing a disease. Similar reports and preparation of such reports are provided for other methods of the invention.

It is understood that the methods of the invention can be performed in any order Suitable for glycopolypeptide analysis. One skilled in the art can readily determine an appropriate order of carrying out steps of methods of the invention suitable for qualitative and quantitative glycopeptide analysis.

As disclosed herein, serum proteins contain enormous information about the health of an individual while blood circulates in the body, and proteomic profiling of serum proteins by mass spectrometry can be a powerful approach for biomarker identification and disease detection. Conventional total tryptic peptide analysis of serum proteins is dominated by the appearance of the 22 most abundant proteins, which represent 99% of total plasma content and produce over one thousand peptides. The dominance of the most abundant proteins makes it extremely challenging to access the low abundance proteins and makes it difficult to identify biomarkers among the low abundance proteins.

Considering that most serum proteins are N-link glycosylated at one or a few tryptic peptides but the most abundant protein, albumin, is not, profiling sera using N-linked glycopeptides and liquid chromatography mass spectrometry (LC-MS) was chosen to achieve high sensitivity and throughput for low abundance serum proteins. As disclosed herein, using this method, over 4000 peptide peaks were detected using sera from normal and carcinogen induced skin cancer mice by two-hour LC-MS analysis (see Example 2). Peptide peaks from LC-MS analysis clearly separated sera of the cancer mice from the normal untreated mice using unsupervised clustering algorithms. The glycopeptides that were elevated in cancer mice were identified using tandem mass spectrometry after isotope labeling the glycopeptides at the amino termini. The combination of glycopeptide capture and LC-MS analysis (glyMS) greatly simplifies the complexity of serum profiling and increases the sensitivity and throughput for low abundance proteins over the total tryptic peptide analysis method.

Using this method, over 4000 peptide peaks were detected using sera from normal and carcinogen induced skin cancer mice by two-hour LC-MS analysis (see Example 2). Peptide peaks from LC-MS analysis clearly separated sera of the cancer mice from the normal untreated mice using unsupervised clustering algorithms. The glycopeptides that were elevated in cancer mice were identified using tandem mass spectrometry after labeling the glycopeptides with isotope at the amino termini. The combination of glycopeptide capture and LC-MS analysis (glyMS) greatly simplifies the complexity of serum profiling and increases the sensitivity and throughput for low abundance proteins over the total tryptic peptide analysis method.

It is expected that the composition of the serum proteome can provide valuable information about the state of the human body in health and disease, and that this information can be extracted via quantitative proteomic measurements. Suitable proteomic techniques need to be sensitive, reproducible and robust to detect potential biomarkers below the level of highly expressed proteins, to generate data sets that are comparable between experiments and laboratories, and have high throughput to support statistical studies. Disclosed herein is a method for high throughput quantitative analysis of serum proteins (see Example 2). It consists of the selective isolation of peptides that are *N*-linked glycosylated in the intact protein, the analysis of these, now de-glycosylated peptides by LC-ESI (electrospray ionization)-MS, and the comparative analysis of the resulting patterns. By focusing selectively on a few formerly *N*-linked glycopeptides per serum protein, the complexity of the analyte sample is significantly reduced and the sensitivity and throughput of serum proteome analysis are increased compared with the analysis of total tryptic peptides from unfractionated samples. Data are provided that document the performance of the method and show that sera from untreated normal mice and genetically identical mice with carcinogen induced skin cancer can be unambiguously discriminated using unsupervised clustering of the resulting peptide patterns. Some of the peptides that were consistently elevated in cancer mice compared to their control littermates were identified by tandem mass spectrometry.

There is growing interest in testing the hypothesis that the serum or plasma proteome contains protein biomarkers that are useful for classifying the physiological or pathological status of an individual. Such markers are expected to be useful for the prediction, detection and diagnosis of disease, as well as to follow the.efficacy, toxicology and side effects of drug treatment (Wulfkuhle et al., Nat. Rev. Cancer 3:267-275 (2003)). Reading diagnostic or prognostic signatures from human body fluids has been performed. Early attempts using high resolution two dimensional gel electrophoresis (2DE) were described more than 2 decades ago (Anderson and Anderson, Proc. Natl. Acad. Sci. USA 74: 5421-5425 (1977): Merril et al., Science 211:1437-1438 (1981); Merril et al., Proc. Natl. Acad. Sci. USA 76:4335-4339 (1979)). Renewed interest in this idea has emerged due to recent advances in proteomic technologies (Aebersold and Mann, Nature 422:198-207 (2003)), intriguing initial results from analyzing serum protein pattern using mass spectrometry (Wulfkuhle et al., Nat. Rev. Cancer 3:267-275 (2003)), and the clinical validation and use of a number of diagnostic disease markers, including CA 125 for ovarian cancer, prostate specific antigen (PSA) for prostate cancer and carcinoembryonic antigen (CEA) for colon, breast pancreatic and lung cancer (Diamandis, Mol. Cell. Proteomics 3:367-378 (2004).

A number of new approaches that differ from the traditional 2DE method for the discovery of protein biomarkers in serum have recently been described (Wulfkuhle et al., Nat. Rev. Cancer 3:267-275 (2003)). These include surface enhanced laser desorption ionization mass spectrometry (SELDI-MS)(Petricoin et al., Lancet 359:572-577 (2002)), liquid chromatography tandem mass spectrometry (LC-MS/MS) of serum proteome digests (Adkins et al., Mol. Cell. Proteomics 1:947-955 (2002); Tirumalai et al., Mol. Cell. Proteomics 2:1096-1103 (2003); Shen et al., Anal. Chem. 76:1134-1144 (2004), two or three dimensional (chromatography/gel electrophoresis) protein separation analyzed by differential fluorescent staining (Wang and Hanash, J. Chromatogr. B Analyt. Technol. Biomed. Life Sci. 787:11-18 (2003); Shin et al., J. Mammary Gland Biol. Neoplasia 7:407-413 (2002)), fractionation of the serum proteome on surface-modified magnetic beads followed by matrix-assisted laser desorption ionization mass spectrometry (MALDI-MS) (Villanueva et al., Anal. Chem. 76:1560-1570 (2004)), and combinations and variations of these approaches.

Any study of the serum proteome is confronted with the peculiar properties of serum samples. First, human blood serum is assumed to consist of minimally tens of thousands of different protein species that span a concentration range of an estimated 10 orders of magnitude (Anderson and Anderson, Mol. Cell. Proteomics 1:845-867 (2002)). Second, the serum proteome is dominated by a few highly abundant proteins, that is, the 22 most abundant human serum proteins combined constitute 99 % of total protein mass (Tirumalai et al., Mol. Cell. Proteomics 2:1096-1103 (2003)). Indeed, almost one half of total serum protein mass is represented by just one protein, albumin. Third, many of the serum proteins show complex 2D electrophoretic patterns, suggesting that they are extensively post-translationally modified, with glycosylation apparently being the major source of protein heterogeneity (Anderson and Anderson. Mol. Cell. Proteomics 1:845-867 (2002)). In fact, when protein spots from 2D electropherograms of serum were systematically identified by mass spectrometry, 5-7 protein spots on average were identified as products of the same gene (Pieper et al., R., Proteomics 3:1345-1364 (2003). Fourth, the serum proteome varies over time in an individual and among individuals in a population.

Useful platforms for serum proteome analysis should thus have minimally the following properties: first, sufficient analytical depth to reliably detect relatively low abundance proteins; second, quantitative accuracy to determine changes in the proteome pattern; third, reproducibility and robustness to detect disease-specific changes in a background of pattern changes unrelated to disease; fourth, the ability to identify distinct peptides for their cross-validation on different analytical platforms and comparison of results obtained from different research groups, studies and diseases; and fifth, high sample throughput to support studies with sufficient statistical power.

Disclosed herein (Example 2) is a method for quantitative serum proteome analysis. It is based on the selective isolation of those peptides from serum proteins that are *N*-linked glycosylated in the native protein, and the analysis of the complex peptide mixture representing the now de-glycosylated forms of these peptides by liquid chromatography mass spectrometry (LC-MS) and tandem mass spectrometry (MS/MS). By selectively isolating this subset of peptides, the procedure achieves a significant reduction in analyte complexity at two levels: first, a reduction of the total number of peptides due to the fact that every serum protein on average only contains a few *N*-linked glycosylation sites, and second, a reduction of pattern complexity by removing the oligosaccharides that contribute significantly to the peptide pattern heterogeneity. The method is reproducible, achieves increased analytical depth and higher throughput compared to the analysis of samples without selective analyte enrichment. Furthermore, in a controlled experiment, peptide patterns distinguishing the serum proteome of cancer-bearing mice from genetically identical untreated normal mice could be detected and discriminatory peptides could be subsequently identified. At present, this method affords one of the most comprehensive routine and high throughput analyses of the serum proteome. The methods are useful in a broad application in serum marker discovery research.

Mass spectrometry based proteomics is becoming one of the most important approaches for quantitative characterization of the function of biological systems. Due to the enormous complexity of the proteomes, the development of high throughput technologies capable of detecting and quantifying specific information-rich proteins is crucial for its applications in biotechnology, such as clinical diagnostics, drug metabolism studies, and improving the knowledge of fundamental biological processes. Disclosed herein is a novel approach for quantitative proteomics that builds on the extensive knowledge of proteomes, and a platform for the implementation of the concept (see Example 4). The disclosed analysis is related to serum analysis. The highly selective, high throughput platform is built based on a MALDI (matrix assisted laser desorption/ionization) TOF/TOF (time-of-flight) spectrometer and using stable isotope labeled peptides as internal standards. For each targeted protein, one (or more) peptide sequence that uniquely identifies the protein is selected, chemically synthesized and labeled with heavy stable isotope. The synthesized stable isotope labeled peptides were used as definitive signatures to represent the corresponding targeted proteins and spiked in the serum sample with known amounts. The detection and quantification of targeted proteins was accomplished using a complementary approach of specific mass matching, selective peptide sequencing and peptide quantification. The study has experimentally demonstrated the concept and feasibility of using mass spectrometry based proteomics as a screening technology for systematic detection and quantification of targeted proteins in a complex system at high throughput.

The comprehensive, quantitative analysis of proteomes is informative and challenging. It is informative because the comparative analysis of proteomes or fractions thereof identifies proteins that are present at different quantities in the samples compared. Such differences in turn have been used to identify cellular functions and pathways affected by perturbations and disease (Wright et al., Genome Biol. 5:R4 (2003); Flory and Aebersold, Prog. Cell Cycle Res. 5: 167-171 (2003); Guina et al., T., Wu, M., Miller, S. L, Purvine, S. O., Yi, E. C., Eng, J. et al. J. Am. Soc. Mass Spectrom. 14:742-751 (2003); Aebersold, Nature 422:115-116 (2003); Flory et al., M. R., Griffin, T. J., Martin, D. and Aebersold, Trends Biotechnol. 20:S23-29 (2002); Shiio, Y., Donohoe, S., Yi, E. C., Goodlett, D. R., Aebersold, R. and Eisenman, R. N. EMBO J. 21:5088-5096 (2002); Rabilloud et al., J. Biol. Chem. 277:19396-19401 (2002)), identify new components and changes in the composition of protein complexes and organelles (Brand et al., Nat. Struct. Mol. Biol. 11:73-80 (2004); Himeda et al., Mol. Cell. Biol.24:2132-2143 (2004); Ranish et al., Nat. Genet. 36:707-713 (2004); Ranish, J. A., Yi, E. C., Leslie. D. M., Purvine, S. O., Goodlett, D. R., Eng, J. et al. Nat. Genet. 33:349-355 (2003); Aebersold, J. Am. Soc. Mass Spectrom. 14:685-695 (2003); Aebersold, J. Infect. Dis. 187 Suppl 2:S315-320 (2003): Patterson and Aebersold, Nat. Genet. 33 Suppl, 311-323 (2003); Griffrin et al., Anal. Chem. 75,867-874 (2003)) and have led to the detection of putative disease biomarkers Hale et al., Brief Funct. Genomic Proteomic 2:185-193 (2003); Shau et al., Brief Funct Genomic Proteomic 2:147-158 (2003)). Comprehensive proteome analysis is challenging because of the enormous complexity of the proteome. In comparison to the number of open reading frames in a genome the number of unique protein species expressed by it is vastly expanded by the action of post transcriptional processing mechanisms including protein modifications, alternative splicing and proteolytic processing. Consequently, to date, neither the complexity of a proteome nor its actual composition has been determined for any species.

Over the last few years a number of mass spectrometry-based quantitative proteomics methods have been developed that identify the proteins contained in each sample and determine the relative abundance of each identified protein across samples (Flory et al., Trends Biotechnol. 20:S23-29 (2002); Aebersold, J. Am. Soc. Mass Spectrom. 14:

685-695 (2003); Aebersold, J. Infect. Dis. 187 Suppl 2:S315-320 (2003); Patterson and Aebersold, Nat. Genet. 33 Suppl, 311-323 (2003); Aebersold and Mann, Nature 422:198-207 (2003); Aebersold, R. and Cravatt, Trends Biotechnol. 20: S1-2 (2002); Aebersold and Goodlett, Chem. Rev. 101, 269-295 (2001); Tao and Aebersold, Curr. Opin. Biotechnol. 14: 110-118 (2003)). Generally, the proteins in each sample are labeled to acquire an isotopic signature that identifies their sample of origin and provides the basis for accurate mass spectrometric quantification. Samples with different isotopic signatures are then combined and analyzed, typically by multidimensional chromatography tandem mass spectrometry. The resulting collision induced dissociation (CID) spectra are then assigned to peptide sequences and the relative abundance of each detected protein in each sample is calculated based on the relative signal intensities for the differentially isotopically labeled peptides of identical sequence. Therefore, in a single operation the identity of the proteins contained in the samples and their relative abundance are determined. While the methods differ in the way the stable isotopes are incorporated into the polypetides and the precise analytical (separation; mass spectrometry; data processing) methods used, they have in common that in every experiment results are only obtained from those peptides for which in the tandem mass spectrometry (MS/MS) experiment precursor ions are selected, successfully fragmented and conclusively assigned to a peptide sequence. Therefore, in every proteomics experiment of this kind the proteome is rediscovered without the benefit of the data collected from prior experiments. Furthermore, it has previously been shown that this type of proteomic analysis is quite inefficient in that the number of successfully identified and quantified peptides is about an order of magnitude lower than the number of detectable peptides present in the sample (Li et al., Anal. Chem. 76:3856-3860 (2004)) and that it is biased towards the proteins of higher abundance Nesvizhskii and Aebersold, Drug Discov. Today 9:73-181 (2004); Nesvizhskii et al., Anal. Chem. 75:4646-4658 (2003); Keller et al., Anal. Chem. 74: 5383-5392 (2002)).

In many studies it is necessary to analyze a large number of proteomes and to compare the results obtained from each analysis. In biomarker discovery studies for example, large numbers of samples are required to detect protein patterns that consistently associate with a specific condition within a large background of proteins that may randomly fluctuate within the population tested (Aebersold, Nature 422:115-116 (2003); Domon and Broder, J. Proteome Res. 3: 253-260 (2004)). In the emerging field of systems biology, a key element is the quantitatively accurate and comprehensive measurement of the components that constitute the system in differentially perturbed states and the synthesis of these data into a model describing the system (Adv. Exp. Med. Biol. 547:21-30 (2004)). Therefore, it is essential that quantitative proteomics experiments can be carried out at high throughput.

Genomics-style biology can be separated into two distinct phases, a discovery phase in which all the possible elements of one type are discovered, and a browsing or screening phase, in which the list of all possible or known elements is searched for those that may be of interest in a particular study (Aebersold, Nature 422:115-116 (2003)). The transition from a discovery to a browsing mode of operation has been already implemented for genomic sequencing, gene expression array analysis and the analysis of single nucleotide polymorphisms (SNPs) (Aebersold, Nature 422: 115-116 (2004)). Disclosed herein (see Example 4) is a method and its implementation in a platform to also transform quantitative proteomic from a discovery into a browsing mode of operation. The performance of the system was demonstrated by analyzing proteins contained in human blood serum. Based on the characteristics of the method which include vastly simplified data analysis, high throughput, absolute quantification of proteins in complex samples, reduced redundancy, the ability to search for and quantify specific protein isoforms and the potential for standardization of results between laboratories, the method is expected to become widely applicable in quantitative proteomics studies:

Serum proteins have been the focus for biomarker identification and disease detection. Currently, most current serum proteomic analyses focus on discovery and annotation of serum proteins due to the enormous complexity of the serum proteome as well as individual variations over time and within a population. However, the serum proteins and peptides identified from discovery phased studies define the boundary of the serum proteome and can identify so-called proteotypic peptides which uniquely identify a given protein and are consistently observed by a mass spectrometer. These proteotypic peptides can be used to screen the proteome to reveal constellations associated with specific biological processes or physiological conditions. Since most serum proteins are *N*-linked glycosylated at one or several tryptic peptides, it was therefore proposed to identify proteotypic *N*-linked glycopeptides for serum proteome analysis using a recently developed solid-phase extraction of glycopeptides (SPEG) method (see Example 7). First, over three thousand unique *N*-linked glycosylation sites representing over two thousand unique serum proteins were experimentally identified. These identified glycopeptides were then used to calculate the frequency of each amino acid at each position surrounding the *N*-linked glycosylation sequon (NX(T/S) and physico-chemical properties of peptides that can be detected by mass spectrometry. The refined glycosylation motif and peptide properties were then used to predict all potential *N*-linked proteotypic glycopeptides from a database of candidate proteins. Quantitative analysis of serum proteins using these identified and predicted proteotypic N-linked glycopeptides increase the throughput and sensitivity of serum analysis for biomarker discovery research.

Physiologists believe that individual genetic backgrounds and pathological changes in organs affect serum protein composition. This allows for a systematic and quantitative analysis of serum proteins for identifying disease biomarker. This explains the current focus of numerous studies on serum proteome annotation for biomarker identification (Shen et al., Anal. Chem. 76:1134-1144 (2004); Anderson et al., Mol. Cell. Proteomics 3:311-326 (2004)). Two methods have been used preferentially to profile serum proteins. The first and most commonly used is protein/peptide patterns analysis. This is exemplified by two-dimensional gel electrophoresis (2DE), surface enhanced laser desorption ionization mass spectrometry (SELDI-MS), and liquid chromatography mass spectrometry (LC-MS). The limitations of this approach are that the molecules are not identified and that limited depth is achieved. The second is a more recently developed technique based on stable isotope tagging of proteins and automated peptide tandem mass spectrometry (MS/MS) (Shen et al., Anal. Chem. 76:1134-1144 (2004); Anderson et al., Mol. Cell. Proteomics 3:311-326 (2004); Pieper et al., Proteomics 3:422-432 (2003)). Due to the enormous complexity and high dynamic range of the plasma proteome, using the current abundance dependent proteomic approach, the MS/MS based method can only identify a small subset of the peptides, presumably the highly abundant peptides present in plasma proteome, and it is very difficult to access low-abundance proteins that represent new biomarkers.

In response to this challenge, some researchers have devised a "divide and conquer" strategy for analyzing subsets of the serum proteome to reduce complexity and to increase the detection limits of serum proteins by avoiding repetitive analyses of the most abundant proteins. Specifically, the most abundant serum proteins, for example, albumin and immunoglobulin, are removed by affinity depletion (Pieper et al., Proteomics 3:422-432 (2003); Pieper et al., Proteomics 3:1345-1364 (2003); Adkins et al., Mol. Cell. Proteomics 1:947-955 (2002)). In the second part of the "divide and conquer strategy," proteins or peptides are fractionated according to physico-chemical properties, for example, size, charge, or hydropathy, prior to mass spectrometric analysis. Specific implementations include two- or three-dimensional peptide chromatography (Shen et al., Anal. Chem. 76:1134-1144 (2004); Adkins et al., Mol. Cell. Proteomics 1:947-955 (2002); Tirumalai et al., Mol. Cell. Proteomics 2:1096-1103 (2003) : and size fractionation prior to protein digestion and analysis by LC-MS/MS 66. Tirumalai et al., Mol. Cell. Proteomics 2:1096-1 103 (2003). Alternatively, proteins that contain common distinguishing structural features in plasma proteins such as carbohydrate groups or cysteine residues (Pieper et al., Proteomics 3:422-432 (2003); Guppy et al., Oncologist 7:437-443 (2002) have been selectively enriched prior to MS analysis.

In every study, extensive efforts have been used to discover new serum proteins and annotate a serum protein database. This discovery phase of serum protein analysis normally does not contain quantitative information about individuals related to disease because it is not sufficiently reproducible, but it does define the boundary of the serum proteome. Analogous to trends seen in genomic studies; where a discovery phase marked by high-throughput DNA sequencing was followed by a scoring phase using microarrays, this extensive discovery based proteomic analysis of serum proteins is extremely useful to transverse this discovery phase of serum protein analysis to scoring phased analyses using the peptides and proteins identified in these data sets. This was demonstrated using synthetic stable isotope labeled peptides and ordered array as example 77. Pan et al., Mol. Cell. Proteomics 4:182-190 (2005). In that study, quantitative analysis of the serum proteome using prior identified proteotypic peptides was determined. The method included the selection and chemical synthesis of isotopically labeled reference peptides that uniquely identify a particular protein, and the addition of a panel of such peptides to the sample mixture consisting of tryptic peptides from the proteome in question. The combined peptide samples were then separated by chromatography to generate ordered peptide arrays on the sample plate of a matrix-assisted laser desorption/ionization (MALDI) mass spectrometer, and detected by MALDI-TOF/TOF mass spectrometer.

To identify the proteotypic peptides that are the basis for a high throughput plasma proteome screening, a large scale isolation of formerly N-linked glycopeptides was performed using the recently developed method, SPEG (Zhang et al., Nat. Biotechnol. 21:660-666 (2003)). The isolated peptides were fractionate by strong cation exchange (SCX) and identified by a combination of liquid chromatography, tandem mass spectrometry (LC-MS/MS), and a suite of software to determine the peptide sequence and statistical analysis of identification confidence (Eng et al., J. Am. Soc. Mass. Spectrom. 5:976-989 (1994); Keller et al., Anal. Chem. 74:5383-5392 (2002). With a minimum peptide probability score of 0.5, 3244 nonredundant N-linked glycosylation sites were identified, representing 2585 unique proteins. 2106 peptides are unique to single database entry, and selected as proteotypic peptides, representing-1671 proteins. Using-the identified N-linked-glycosytation sites,the amino acid composition surrounding the consensus N-linked glycosylation sites was further determined and generated a predictor for physico-chemical properties of peptide that were likely to be detected by mass spectrometry. The refined NXT/S motif and peptide properties were then used to predict potential N-linked glycopeptides as proteotypic peptide by scanning the human IPI protein database.

The experimentally identified and computationally predicted N-linked proteotypic peptides resulting from the database can be interrogated via a World Wide Web interface, UniPep, (db.systemsbiology.net/devPM/sbeams/cgi/PeptideAtlas/Glyco_prediction.cgi). This 5 is intended to provide a fast and accurate way to screen, the plasma proteome for biomarkers using proteotypic peptides as heavy isotopic standards in conjunction with mass spectrometry, and is expandable as more peptides are discovered and added.

It is understood that modifications which do not substantially affect the activity of the various embodiments of this invention are also provided within the definition of the invention provided herein. Accordingly, the following examples are intended to illustrate but not limit the present invention.

## EXAMPLE 1

**Isolation of Tryptic Peptides of Glycoproteins from Serum and N-linked Glycopeptides from Plasma**

The isolation method was described previously (Zhang et al., Nat. Biotechnol. 21:660-666 (2003)). In detail, proteins from 0.75 ml of serum or 1 ml of plasma were changed to buffer containing 100 mM NaAc, 150 mM NaCl, pH 5.5 (coupling buffer). Sodium periodate solution at 15 mM was added to the samples. The samples were rotated in dark at room temperature for I hour. The sodium periodate was removed from the samples using a desalting column (Bio-Rad; Herculed, CA). Eight ml of hydrazide resin (Bio-Rad; Hercules, CA) equilibrated in coupling buffer was added to the sample. The sample and resin were capped securely and rotated end-over-end for 18 hours at room temperature. The non-glycoproteins were removed, and resin was washed 3 times with 20 ml of 8M urea/0.4M $NH_4HCO_3$. The proteins on the resin were denatured in 20 ml of 8M urea/0.4M $NH_1HCO_3$ at 37°C for 30 min, followed by 3 washes with the urea solution. After the last wash and removal of the urea buffer, the resin was diluted 4 times with water. 200 $\mu$g of trypsin in 24 ml of water was added to digest the bound proteins at 37°C overnight. Peptides were reduced by adding 8 mM TCEP (Pierce, Rockford IL) at room temperature for 30 min, and alkylated by adding 10 mM iodoacetamide at room temperature for 30 min. For serum sample, the trypsin released peptides were collected and further analysed by mass spectrometry. The resin was washed with 20 ml of 1.5 M NaCl 3 times, 80% acetonitrile 3 times, 100% methanol 3 times, and 0.1 M $NH_4HCO_3$ 6 times. N-linked glycopeptides were released from the resin by digestion with 6 $\mu$l of peptide-N-glycosidase F (New England Biolabs; Beverly, MA) overnight. The peptides were dried and resuspended in 0.4% acetic acid for LC-MS/MS analysis.

For separation of peptide by chromatography and analysis of peptides by mass spectrometry, the resulting peptide mixture was fractionated by two-demensional chromatography (Han et al., Nat. Biotechnol. 19:946-951 (2001): (1) caption-exchange chromatography using a 2.1 mm 20 cm Polysulfoethyl A column (Poly LC Inc., Columbia, MD) at a flow rate of 200 $\mu$l/min using 1-hour gradient from buffer A.(20 mM $KH_2PO_4$, 25% acetonitrile, pH 3.0) to buffer B (20 mM $KH_2PO_4$, 350 mM KCl, 25% acetonitrile, pH 3.0); and (2) reverse-phase capillary chromatography using a 75 $\mu$m 10 cm self-packed C18 column at a flow rate of 250 nl/min using -hour gradient from buffer A (5% acetonitrile and 0.1% formic acid) to buffer B (35 % acetonitrile). The peptide identification by collision-induced-dissociation (CID) was carried out in an automated fashion using the dynamic-exclusion option on Finnigan LCQ ion trap mass spectrometer (Finnigan, San Jose, CA) or ESI-QqTOF (Macromass, Beverly, MS).

For data analysis, CID spectra was searched using SEQUEST (Eng et al., J. Am. Soc. Mass Spectrom. 5: 976-989 (1994)) against the human International Protein Index sequence database (version 2.21, downloaded from the European Bioinformatics Institute ftp.ebi.ac.uk/pub/databases/tPJ/current/ipi.HUMAN.fasta.gz). The database search results were analysed by a suit of software tools including INTERACT (Han et al., *supra,* 2001), peptide probability (Keller et al., Anal. Chem. 74:5383-5392 (2002)), and protein prophet (Nesvizhskii et al., Anal. Chem. 75:4646-4658 (2003)) to sign the probability and confidence score for each identified peptides and proteins.

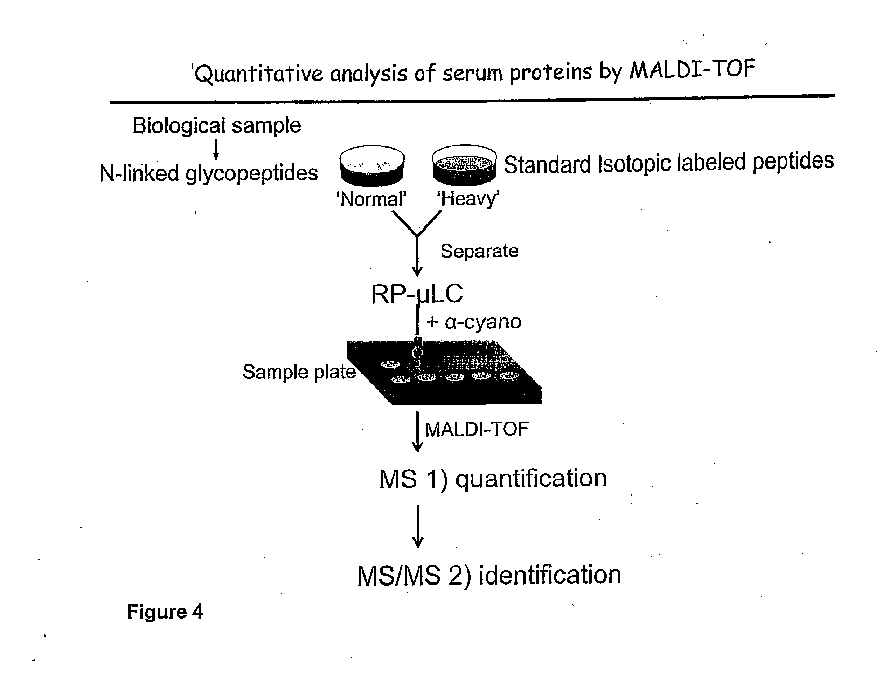

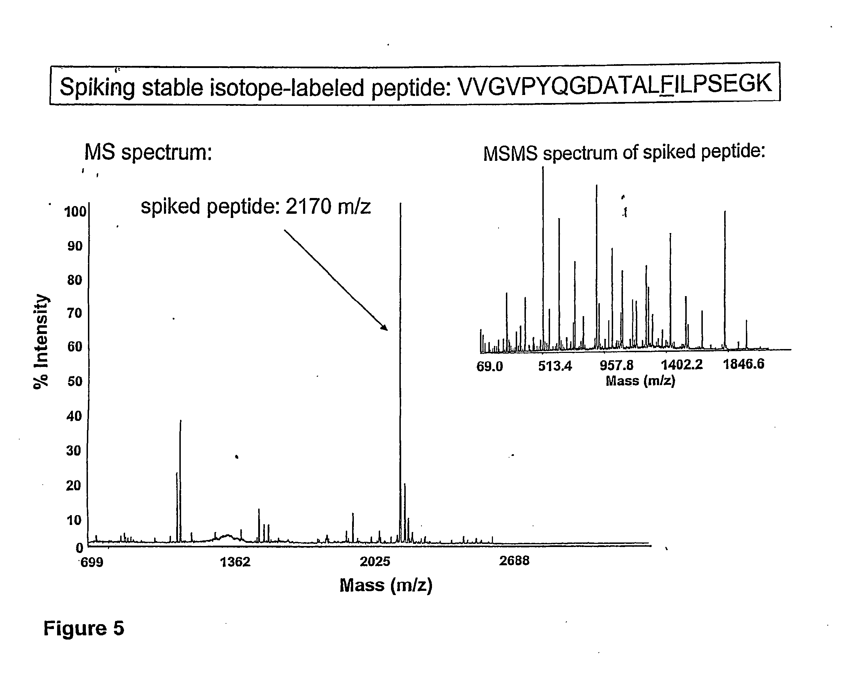

The-profocor is illustrated in Figures 4 and 5: Figure 4 shows a schematic of quantitative analysis of serum proteins. Figure 5 shows an exemplary analysis with the addition of a standard peptide.

## EXAMPLE 2

**High Throughput Quantitative Analysis of Serum Proteins Using Glycopeptide Capture and LC-MS**

This example describes analysis of serum proteins by capturing glycopeptides and analyzing by mass spectrometry. The analysis was performed on normal and cancer mice to identify differentially expressed glycopeptides associated with a cancer condition.

It is expected that the composition of the serum proteome can provide valuable information about the state of the human body in health and disease, and this information can be extracted via quantitative proteomic measurements. Suitable proteomic techniques need to be sensitive and capable of detecting potential biomarkers below the level of highly expressed proteins, to be reproducible and robust, to generate data sets that are comparable between experiments and laboratories, and to have high throughput to support studies with sufficient statistical power. High throughput quantitative analysis of serum proteins has been performed: Peptides that are N-glycosylated in the intact protein were selectively isolated and analyzed by LC-ESI-MS. A comparative analysis was performed to determine any resulting patterns indicating differential expression of glycopeptides between normal and cancer mice as potential biomarkers for the cancer condition. By focusing selectively on the few N-linked glycopeptides per serum protein, the complexity of the analyte sample is significantly reduced and the sensitivity and throughput of serum proteome analysis are increased. The results show that sera from normal mice and genetically identical mice with carcinogen induced skin cancer can be unambiguously discriminated using unsupervised clustering of the resulting peptide pattern. It was further determined by tandem mass spectrometry that some of the glycopeptides were consistently elevated in cancer mice compared to their healthy littermates.

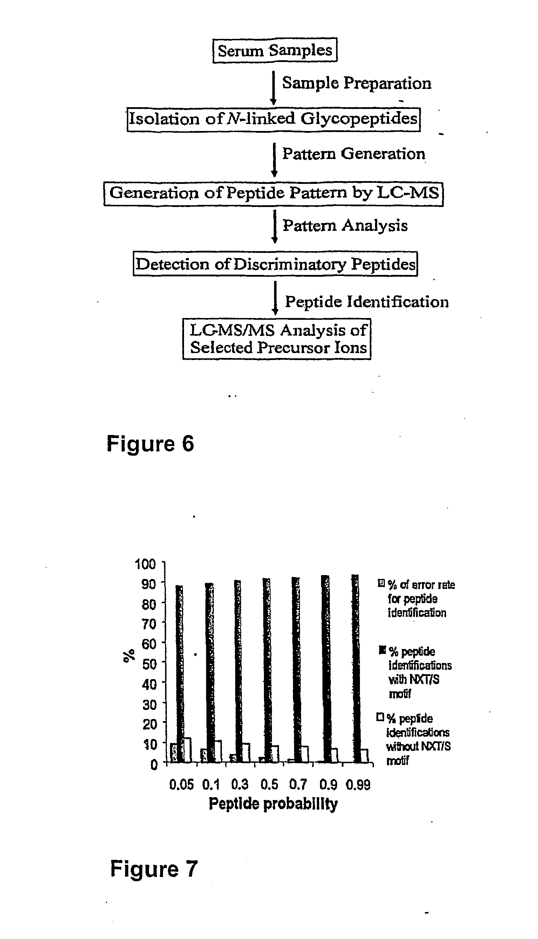

Serum from normal mice and mice: with carcinogen induced-skin-cancer were analyzed essentially as described previously (Zhang et al., Nat. Biotechnol. 21:660-666 (2003) and essentially as described in Example 1. Figure 6 shows a schematic outline of the procedure for glycopeptide profiling of serum proteins using LC-MS. Serum samples were obtained from normal mice and mice having carcinogen induced skin, and N-linked glycopeptides were isolated essentially as described in Example 1 . Peptides were analyzed by LC-MS, and peptides that discriminated between normal and cancer mice were determined. LC-MS/MS analysis was then performed on selected precursor ions.

Table 1 shows that the glycopeptide capture-and-release method reduces the number 5 of peptides to be analyzed from each serum protein and reduces sample complexity for serum profiling.

Table 1. Reduction of sample complexity for serum profiling using glycopeptide capture and release.

|  | A | B | C | D |
|---|---|---|---|---|
| Total number of peptides | 2889 | 355 | 338 | 166 |
| Number of peptides for each protein | 29.8 | 3.66 | 3.48 |  |
|  |  |  |  |  |
| A: Number of tryptic peptides |  |  |  |  |
| B: Number of glycopeptides |  |  |  |  |
| C: Number of identified glycopeptides |  |  |  |  |
| D: Number of N-linked Glycosylation sites |  |  |  |  |

The use of the glycopeptide capture method greatly reduces sample complexity, thereby increasing the sensitivity of analysis, particularly of less abundant serum proteins. A comparison of the analysis of glycopeptides from 5 $\mu$1 of serum (left panel) and tryptic peptides from 0.05 $\mu$1 of serum was performed. Proteins were analyzed in 100 min LC-MS. It was found that 100 times the amount of serum can be analyzed due to the reduction in complexity from isolating glycopeptides and omitting analysis of abundant non-glycosylated proteins, thus allowing the analysis of less abundant serum proteins.

The high throughput serum analysis method was highly reproducible-. The distribution of CV (coefficient of variance) from 9 repeated LC-MS analysis of the same sample was determined. The distribution of CV from 4 repeated sample preparations using the glycopeptide capture method and LC-MS analysis was also determined. The distribution of CV obtained from 5 normal male mice of the same litter was additionally determined.

Unsupervised hierarchical clustering analysis of N-linked glycopeptides can separate carcinogen induced cancer mice from normal mice. Both increased and decreased abundance was observed for various peptides in comparison of cancer mice with normal mice. In some cases, peptide abundance was higher than the mean 5 peptide intensity of normal mouse sera. In other cases, peptide abundance was lower compared to the mean of this peptide in different, that is, normal mouse sera.

The method of glycopeptide capture allows the identification of peptides that are elevated in carcinogen induced cancer mice. The abundance of an identified peptide from serum amyloid P-component with m/z value of 709.7 in sera of normal 10 and cancer mice was determined by highly reproducible LC-MS analysis.

These results demonstrate that selectively isolating those peptides that are N-glycosylated serum proteins has a number of favorable consequences for the analysis of the serum proteome. Together with the high reproducibility of the method, the unprecedented serum proteome coverage achieved at a moderate throughput indicates that the method is useful for the detection of proteins or protein patterns that distinguish individuals in different physiological states. These studies were extended and are described below.

Materials and Reagents. For all chromatographic steps, HPLC grade reagents were purchased from Fisher Scientific (Pittsburgh, PA, USA). PNGase F was from New England Biolabs (Beverly, MA). Hydrazide resin was from Bio-Rad (Hercules CA). All other chemicals and the human serum sample used in this study were purchased from Sigma (St. Louis, MO, USA).

Chemical induction of mouse skin tumors. Male mice of strain NIHO1a were subjected to the two-stage skin carcinogenesis protocol (Kemp et al., Cell 74:813-822 (1993)). Five littermates were used; 2 untreated and 3 treated with carcinogen. The shaved backs of three 8-week old mice were treated with a single dose of the carcinogen 7,12 dimethylbenz[a]anthracene (DMBA) (Sigma: 25mg in 200ml acetone). Initiated cells were promoted with 12-O-tetradecanoylphorbol-13-acetate (TPA) twice a week for 15 weeks, giving rise to papillomas that were hyperplastic, well-differentiated, benign lesions consisting of keratinocytes together with stromal tissue. Papillomas appeared as early as 8 weeks after DMBA initiation and continued to grow for the next several months. A small percentage of these benign papillomas progressed to squamous cell carcinomas (SCC). At week 22 after DMBA initiation, all mice were sacrificed and whole blood collected by heart puncture with a 21G needle and 1cc syringe. Blood was allowed to clot for 1 hr at room temperature. Sera were collected by centrifugation at 3000 rpm. The untreated mice contained no tumors, while the DMBA/TPA treated mice each had at least one carcinoma as confirmed by histological analysis.

Preparation of peptide samples for mass spectrometry analysis. Formerly *N*-linked glycosylated peptides were isolated and labeled using N-linked glycopeptide capture procedure as described previously (Zhang et al., Nat. Biotechnol. 21:660-666 (2003)). Proteins from 100 $\mu$l of serum were used in isolation and isotope labeling of formerly *N*-linked glycopeptides, and peptides from 5 $\mu$l of original serum were used in each mass spectrometry analysis.

To prepare tryptic peptides from serum proteins, proteins from 1 $\mu$l (80 $\mu$g) of mouse serum were denatured in 20 $\mu$l of 8M urea/0.4M $NH_4HCO_3$ for 30 min at room temperature. The proteins were diluted 4 times with water, after which I $\mu$g of trypsin was added and the proteins were digested at 37°C overnight. The peptides were then reduced by adding 8 mM Tris(2-carboxyethyl)phosphine (TCEP)(Pierce, Rockford IL) at room temperature for 30 min and alkylated by adding 10 mM iodoacetamide at room temperature for 30 min. The peptides were dried and resuspended in 0.4% acetic acid. Peptides from 0.05 $\mu$l of original serum (4 $\mu$g original serum proteins) were used for each LC-MS analysis.

Analysis of peptides by mass spectrometry. The peptides and proteins were identified using MS/MS analysis using an LCQ ion trap mass spectrometer (Thermo Finnigan, San Jose, CA) as described previously (Gygi et al., Nat. Biotechnol. 17:994-999 (1999)). For quantitative analysis of peptides using LC-MS, an ESI-QTOF (liquid chromatography elcctrospray ionization quadrupole-time-of-flight) mass spectrometer (Waters, Beverly, MA) was used. In both systems, peptides isolates from 5 $\mu$l of serum samples using the glycopeptide capture method were injected into a homemade peptide cartridge packed with Magic C18 resin (Michrome Bioresources, Auburn, CA) using a FAMOS autosampler (DIONEX, Sunnyva!e, CA), and then passed through a 10 cm x 75 $\mu$m inner diameter microcapillary HPLC ($\mu$-LC) column packed with Magic C18 resin (Michrome Bioresources, Auburn. CA). The effluent from the $\mu$-LC column entered a homebuilt electrospray ionization source in which peptides were ionized and passed directly into the respective mass spectrometer. The C18 peptide trap cartridge, $\mu$-ESI-emitter/$\mu$-LC pulled tip column combination, a high voltage line for ESI and the waste line were each connected to separate ports of a four port union (Upchurch Scientific, Oak Harbor, WA) constructed entirely out of polyetheretherketone (PEEK)(Yi et al., Rapid Commun. Mass Spectrom. 17:2093-2098 (2003)). A linear gradient of acetonitrile from 5%-32% over 100 min at flow rate of~300 nL/min was applied. During the LC-MS mode, data was acquired with a profile mode in the mass range scan between *m/z,* 400 and 2000 with 3.0 sec scan duration and 0.1 sec interscan. After completion of the LC/MS runs, inclusion peptide mass lists were created from data analysis software. The inclusion lists were then used for targeted LC/MS/MS analysis for peptide/protein identifications with the remaining of samples.

ESI-QTOF data analysis: A suite of software tools were developed or optimized in house to analyze LC-MS data for this project and will be published separately (Li et al. manuscript in preparation). The software tools use LC-MS data generated by ESI-QTOF analysis of formerly *N*-linked glycopeptides from serum samples and sequentially perform the following tasks to determine peptides that are of different abundance in cancer and normal mice, respectively.

1. Peptide list: A list of peptide peaks was generated from each LC-ESt-MS run. The tool performing this operation was a straightforward extension of previous tool for the analysis of LC-MALDI-MS data (Griffin et al., Anal. Chem. 75:867-874 (2003)). That tool was modified to take into account the fact that, in ESI-MS, peptides are observed in different charge states. Peaks were selected if the signal to noise ratio exceeded 2.

2. peptide alignment: Peptides detected in individual LC-MS patterns were aligned mainly based on peptide mass. The retention time was then used to align peptides with the same *m/z* value. The software tool accounted for shifts in the retention time in different LC-MS analyses during peptide alignment. Peptide alignment was facilitated by the following factors: i) the, glycopeptide capture procedure significantly simplifies the sample complexity, ii) the high mass accuracy achieved in ESI-QTOF instrument, and iii) the optimized HPLC system that produced highly consistent and reproducible peptide patterns. In the mouse studies, peptides that appeared at least in two of three analyses in either group were selected for further quantitative analysis.

3. Peptide abundance ratio: An abundance ratio of matched peptides in different samples was determined for each peptide peak using the same method as described in the ASAPRatio software tool developed for LC-ESI-MS/MS data (Li et al., Anal. Chem. 75:6648-6657 (2003)). Briefly, the software uses spectra from multiple LC-MS analyses of a peptide peak (with same mass-to-charge ratio *(m/z),* charge state, and close retention time) and calculates one ratio for each peptide peak. In the present study, ratios calculated for different charge states of the same peptide were not combined. The algorithm also estimated a noise background level in each spectrum and subtracts that value from the signal intensities when calculating the peak area.

4. Clustering analysis: The lists of matched peptides with their relative signal intensities were subjected to unsupervised hierarchical clustering (Eisen et al., Proc. Natl. Acad. Sci. USA 95:14863-14868 (1998)) to identify peptides distinguishing cancer samples from normal samples. Prior to clustering, the data was transformed to log value and the mean intensity of each peptide cross all samples was normalized; Peptides present at least in 50% of the total samples were used for clustering analysis.

The objective of the method is the generation of reproducible peptide patterns representing the serum proteome, leading to the detection of peptides that discriminate between related groups of proteomes and the subsequent identification of these discriminatory peptides. The method is schematically illustrated in Figure 6 and consists of four steps. (1) Sample preparation. Peptides that contain *N*-linked carbohydrates in the native protein were isolated in their de-glycosylated form using a recently described solid-phase capture-and-release method (Zhang et al.. Nat. Biotechnol. 2: 660-666-(2003)) (2)-Pattern generation. Isolated peptides were analyzed by LC-MS to generate three-dimensional (retention time, *m/z, and* intensity) patterns. (3) Pattern analysis. Peptide patterns obtained from different samples were compared and the discriminatory peptides determined. (4) Peptide identification. Discriminatory peptides and the proteins from which they originated were identified by tandem mass spectrometry and sequence database searching.

To determine the selectivity of the glycopeptide capture method for serum protein analysis, serum samples from four genetically identical mouse littermates were individually processed using the *N*-linked glycopeptide capture-and-release method and the isolated peptides were analyzed by LC-MS/MS. The resulting collision induced dissociation (CID) spectra were searched against the mouse International Protein Index sequence database (version 1.24) and the database search results were further statistically analyzed using the PeptideProphet software tool (Keller et al., Anal. Chem. 74:5383-5392 (2002)). From four LC-MS/MS analyses of the mouse sera, 1722 CID spectra resulted in peptide identifications from database search with peptide probability scores of at least 0.99 (corresponding to a false positive error rate of 0.0007 (Keller et al., Anal. Chem. 74:5383-5392 (2002)). The identified sequences were then examined for the presence of the known consensus N-linked glycosylation motif (N-X-T/S, where X=any amino acid except proline). The number of proteins represented by the selected peptides were determined using INTERACT (Han et al., Nat. Biotechnol. 19:946-951 (2001)). The number of identified proteins and peptides are summarized in Table 2. A total of 319 unique peptides were identified, representing 93 unique proteins. 93.6 % of the identifications, 81.8% of unique peptides, and 93.5% of identified proteins contained the consensus *N*-linked glycosylation motif (Table 2).

Table 2. Total number of peptide identifications. unique peptides, and unique proteins, and the proportion of each that contain N-X-T/S motif.

|  | Total peptides | Peptides containing N-X-T/S motif | Percentage of motif containing peptides |
|---|---|---|---|
| Number of identifications | 1722 | 1611 | 93.6%. |
| Number of unique peptides identified | 319 | 261 | 81.8% |
| Number of unique proteins identified | 93 | 87 | 93.5% |

The peptide identified as not containing the consensus *N*-linked glycosylation motif can be grouped into two pools. The first contains peptides that are correctly identified and the second is peptides that are incorrectly identified by SEQEST search (false positives). In the present analysis, the false positive error rate was estimated by the Peptide-Prophet statistical model. To further estimate the selectivity of the isolation method, the fraction of peptides identified without consensus *N*-linked glycosylation motif was calculated as a function of the PeptideProiphet probability values. The data are shown in Figure 7. It is apparent that the fraction of peptides without N-X-S/T motif decreases as the stringency of the identification criteria increases. Concurrently, as expected, the number of false positive peptide identifications also decreases. Significantly, and consistent with the data in Table 2, the percentage of peptides without N-X-S/T motif plateaus out at approximately 6.4%, as the false positive error rate approaches 0. It is therefore concluded that the peptide isolation method used has a selectivity that is not lower than 93.6%.

Reduction in the complexity of serum-derived peptides mixtures obtained via the glycopeptide capture-and-release method. The data described above was used to estimate the reduction in sample complexity achieved via the glycopeptide capture-and-release method. A total of 93 proteins were identified collectively from the four serum samples analyzed. Disregarding the complexity caused by protein post-translational modifications, the 93 identified proteins were expected to generate an average of 28.8 of tryptic peptides per protein. Of these, 3.6 peptides on average contained the N-X-S/T motif and were therefore designated potentially *N*-linked glycosylated peptides. Among the 93 identified proteins in this study, an average of 3.6 peptides representing 1.8 unique *N*-linked glycosylation sites per protein were actually identified. By comparing the number of unique *N*-linked glycosylation sites identified with the number of predicted peptides containing consensus *N*-linked glycosylation motif, it was found that 50% of the predicted glycosylated peptides had been detected. Interestingly, an analysis of the actual occupancy rate of potential *N*-linked glycosylation sites in glycoproteins in the crystallographic database showed approximately 65% site occupancy(petrescu et al; Glycobiology14: 103-114(2004). Collectively, these data indicate that the glycopeptide capture-and-release from serum proteins significantly reduces sample complexity and that the method captured a significant fraction of the potentially available *N*-linked glycosylated peptides.

To determine whether the increased sensitivity achieved by reducing sample complexity was sufficient to detect serum protein biomarkers of clinical relevant concentration, we related data obtained in this study to the concentrations of human serum marker proteins (Putnam, The plasma proteins: Structure, Function, and Genetic Control, 2nd ed. Academic Press, New York, NY (1975); Lum and Gambino, Am. J. Clin. Pathol. 61:108-113 (1974)). A direct comparison of the protein compositions between the human and mouse serum proteomes has not previously been determined. However, the serum two-dimensional (2-D) maps of human and mouse are sufficiently similar to allow an approximate comparison of the concentrations of the proteins identified in this study between human and mouse (Duan et al., Electrophoresis 25:3055-3065 (2004)). From the 93 proteins identified above, several proteins are known to be present in human serum at low $\mu$g/ml concentration (Table 3). These include carboxypeptidase N and coagulation factors II, V, XII, and XIII. Except for epidennal growth factor receptor and serum amyloid P-component, none of the other proteins listed in Table 3 have been identified in previous mouse 2-D map, suggesting that they are present at low abundance in mouse serum (Duan et al., Electrophoresis 25:3055-3065 (2004)). To estimate the detection sensitivity, the peak intensities of the identified peptides from these proteins were calculated using the intensities of chromatographic peaks at the charge states used for peptide identification. Examination of the peak intensities indicated an average peptide peak intensity of $2.7 \times 10^7$, which is ~900 times greater than the observed background signal for these experiments (Table 3). This indicates that even without multidimensional separation, serum proteins at concentrations on the order of ng/ml may be detected by LC-MS of formerly *N*-linked glycopeptides. Table 3. Peak intensities of formerly *N*-linked glycopeptides identified from mouse sera and the reported concentration of their corresponding proteins in human serum.

Table 3. Peak intensities of formerly *N*-linked glycopeptides identified from mouse sera and the reported concentration of their corresponding proteins in human serum.

| Protein name | IPI Number | Peptide sequences | $\mu$g/ml | Intensity |
|---|---|---|---|---|
| kallikrein B, plasma 1 | IP100113057 | R.IVGGTN#ASLGEWPWQVSLQWWVK.L | 50 | $1.50 \times 10^7$ |
| | | K.LQTPLN#YTEFQKPICLPSK.A | | $3.30 \times 10^7$ |
| coagulation factor 11 | IP100114206 | RCAMDLGVNYLGTVN#VTHTGIOCOLWR.S | 20 | $1 30 \times 10^7$ |
| | | R.WVLTAAHCILYPPWDKN#FTENDLLVR.I | | $290 \times 10^7$ |
| coagulation factor V | IP100117064 | K.SN#ETALSPDLN#QTSPSMSTDR.S | 20 | $1 50 \times 10^6$ ! |
| Similar to carboxypeptidase N | IP100119522 | E.ITGSPVSN#LSAHIFSN#LSSLEK.L | 35 | $1.10 \times 10^8$ |
| | | R.DGSDSAAM*VYN#SSQEWGLR.S | | $3.20 \times 10$ |
| Epidemial growth factor receptor | IP100121190 | R DCVSCQN#VSR G | | $8.30 \times 10^6$ |
| | | R DIVQNVFM*SN#M⁻SM*DLQSHPSSCPKC | | $180 \times 10^7$ |
| | | K.DTLSIN#ATNIK.H | | $1.10 \times 10^7$ |
| coagulation factor XIII, beta subunit | IP100122117 | K.EQETCLAPELEHGN#YSTTQR.T | 10 | $5.30 \times 10^6$ |
| | | R.TYEN#GSSVEYR.C | | $8.40 \times 10^6$ |
| coagulation factor XII (Hageman factor) | IP100125393 | R.HN#QSCEWCQTLAVR.S | 30 | $3.30 \times 10^7$ |
| interferon (alpha and beta) receptor 2 | IP100132817 | K.SGPPAN#YTLWYTVM*SK.D | | $1.70 \times 10^7$ |

(continued)

| Protein name | IPI Number | Peptide sequences | μg/ml | Intensity |
|---|---|---|---|---|
| serum amyloid P-component | IP100267939 | K.LIPHLEKPLQN#FTLCFR.T | 20 | $7.00 \times 10^7$ |
| | | | Average | $2.70 \times 10^7$ |
| | | | Background | $3.00 \times 10^4$ |
| | | | SNR | $8.99 \times 10^2$ |
| N# indicates the *N*-linked glycosylation site. M*=oxidized methionine SNR=signal to noise ratio | | | | |

<u>Assessment of reproducibility of LC-MS patterns following glycopeptide capture-and-release of serum proteins.</u> Out of the 319 peptides and 93 proteins identified by four LC-MS/MS analyses, 109 unique peptides and 52 unique proteins were identified from all four analyses. The number of peptides identified in all four LC-MS/MS runs is low compared to the total number of unique peptides identified (34.2%). The Pep3D software tool was used (Li et al., Anal. Chem. 76:3856-3860 (2004)) to determine whether these observations were due to peptide under sampling in the LC-MS/MS experiment or whether they indicated poor pattern reproducibility. The results show that, first, as expected, the LC-MS patterns of the peptides from individual mouse serum were consistent. Second, due to the complexity of the sample, not all peptides in a given analysis were selected for MS/MS analyses and subsequently identified. Third, as far as could be determined from the difference between the number of identified peptides from MS/MS analysis and total peptides present in a sample from MS analysis, only a small portion of peptides, predominantly the high abundance peptides from each sample were selectively identified by MS/MS analyses. Fourth, the differences between peptide/protein identifications by MS/MS analyses between different samples were caused mainly by the fact that only a fraction of total peptides was identified by MS/MS analysis in the data dependant mode of operation. Collectively, these results suggest that LC-MS analyses of glycopeptides isolated from genetically identical mice are reproducible. However, peptide/protein identifications using MS/MS analyses, due to peptide under sampling, results in a relatively small number of peptide identifications and a seemingly poor reproducibility of the method.

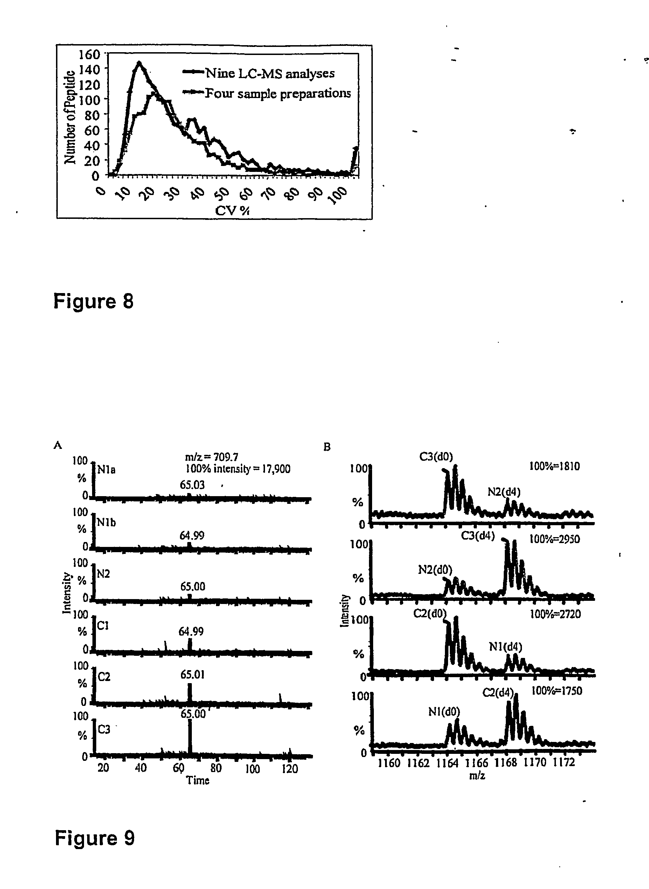

The reproducibility of the peptide patterns obtained by LC-MS was examined. Four 50 μl aliquots from a single serum sample were processed in parallel to generate four isolates and then analyzed by LC-MS. First, to assess LC-MS reproducibility, equal amounts of each isolate were combined and analyzed the combined sample 9 times by LC-MS using a 100 min reverse phase gradient. In house developed software tools were used to detect peaks in the resulting patterns, to measure peak intensity, and to align corresponding peptide peaks between multiple patterns (Li et al. manuscript in preparation). From these data, the average intensity, standard deviation of intensity, and coefficient of variance (CV) was caculated for each peptide. A histogram of CV from the 9 repeat analyses of identical samples by LC-MS is shown in Figure 8 (rectangles). The mean and median CVs observed in the 9 repeat LC-MS analyses of the same sample were 28.3% and 21.8%, respectively. Next glycopeptides were analyzed from the four individual isolates as described above to determine reproducibility with respect to peptide isolation. This data is shown in Figure 8 (squares). The mean and median CVs for the four replicate sample preparations were 25.7% and 21.6%, respectively, and therefore comparable to the analogous values from repeat LC-MS analysis of identical samples. These results indicate that sample preparation does not significantly contribute to the variability of observed peptide patterns.

<u>Application of the method to distinguish sera from normal and skin cancer-bearing mice.</u> To test the hypothesis that the serum proteome profiles from individuals in different physiological states can be differentiated, the glycopeptide capture-and-release method was applied to serum samples from mice in which skin tumors had been induced and from normal untreated littermates. Skin tumors were induced in a well established skin carcinoma model via topical treatment of the skin with a single dose of DMBA followed by repeated treatments with the tumor promoter TPA (Kemp et al., Cell 74:813-822 (1993)). This treatment gives rise to papillomas that are hyperplastic and well differentiated benign lesions of the skin, each one originating from a single initiated cell (Brown et al., K.. Cell 46:447-456 (1986); Quintanilla et al., Nature 322:78-80 (1986)). After a latency period of several months, a small percentage of these lesions progress to squamous cell carcinomas.

From the sera of three cancer-bearing male mice (C I. C2, and C3) and two untreated normal male mice (N1 and N2) from the same litter, glycopeptides were isolated and analyzed by LC-MS as described above. The sample from N1 was analyzed by LC-MS twice (N 1 a, N 1b), thus a total of six LC-MS patterns were generated. After aligning peptide peaks from all six patterns, over 3000 peptide peaks were found to occur in at least 2 of the 3 analyses from either normal or cancer-bearing mice. The six LC-.MS patterns consisting of the peptide peaks matched between the samples and their associated intensities were next subjected to unsupervised hierarchical clustering (Eisen et al., Proc. Natl. Acad. Sci. USA 95:14863-14868 (1998)). Neither predefined reference vectors nor prior knowledge about the nature of each pattern (untreated normal versus cancer-bearing) was used. The results of this unsupervised hierarchical clustering analysis are represented by a tree structure. The lengths of the branches among different samples are proportional to the similarity of the obtained peptide patterns. From this clustering, it is apparent that the cancer-bearing mice (C1, C2, and C3) were clustered together and clearly differentiated from the patterns obtained from their sex and litter matched normal mice (N I a, N1b, and N2) which were also clustered together.

To test whether the same serum samples could be equally differentiated without applying the glycopeptide capture-and-release enrichment method, tryptic peptides from 50 nl of each unprocessed serum sample were subjected to the same LC-MS and pattern analysis procedure. Peptide peaks were aligned from the resulting patterns, and a similar number of peptide peaks were detected as for the glycopeptide enriched samples. In contrast to the glycopeptide enriched samples, unsupervised clustering of the total serum peptide patterns did not differentiate the cancer group from normal group. These results indicate that the larger number of proteins and/or the deeper penetration into the serum proteome achieved by the glycopeptide selection chemistry is critical to the successful differentiation between serum samples according to the clinical state of the individuals.

The glycopeptide enriched samples were then further analyzed by MS/MS to identify peptides that increase in abundance in cancer-bearing mice from untreated normal animals. The $m/z$ and retention time coordinates of these peptides were added to the inclusion list on a tandem mass spectrometer and identified by LC-MS/MS and sequence database searching. Figure 9A shows a peptide at $m/z$ of 709.7 (eluted at ~65 min) that. while showing variation between individual, also clearly showed consistently increased abundance in cancer-bearing mice (C1. C2, C3) compared with normal animals (N1a, N1b, N2). The signal at $m/z$ of 709.7 was subsequently identified as a peptide with the amino acid sequence LIPHLEKPLQN#FTLCFR (in which N# indicates the formerly *N*-linked glycosylation site; SEQ ID NO:) derived from serum amyloid P component in mouse. This is an acute-phase protein whose expression is known to be elevated during inflammation (Mole et al., J. Immunol. 141:3642-3646 (1988).

The differential abundance of the identified peptides was verified by applying accurate quantitative analysis using stable isotope labeling. In these experiments, the amino groups of the glycopeptides were isotopically labeled with d0 and d4 succinic anhydride, respectively, while the peptides were still attached to the solid support during their isolation (Zhang et al., Nat. Biotechnol. 21:660-666 (2003)). Equal aliquots of samples from two cancer-bearing mice (C2 and C3) and two normal mice (N1 and N2) were reverse labeled with either the d0- and d4- succinic anhydride and the released peptides were combined in the following way: sample N1 (d0) was paired with sample C2 (d4); sample C2 (d0) was paired with sample N1 (d4); sample N2 (d0) was paired with sample C3 (d4); and sample C3 (d0) was paired with sample N2 (d0). The combined samples were analyzed by LC-MS/MS. The $m/z$ of peptides identified with higher abundance in cancer-bearing mice using LC-MS analysis and pattern matching were selected and the corresponding mass for light and heavy succinic anhydride labeled peptides were included in the mass inclusion list (with a 100 Dalton addition for the light form of succin ic anhydride and a 104 Dalton addition for the heavy succinic anhydride labeling) and then sequenced by MS/MS analysis using ESI-QTOF and identified by database searching. Table 4 lists the identified peptides and proteins with elevated protein level in the cancer-bearing mouse group detected by LC-MS analysis and verified by reverse stable isotope labeling. The LC-MS spectrum obtained for the same peptide from scrum amyloid P-component is shown in Figure 9B. The increased level of this peptide in cancer-bearing mice quantified by isotopic labeling was consistent with that determined by LC-MS analysis (Figure 9A).

Table 4. Identification of peptides and proteins with elevated abundance in treated cancer-bearing mice

| Protein name | IPI number | peptide sequences | CID spectrum number given in Supplementary |
|---|---|---|---|
| | | | **Figure 1 online** |
| Ig gamma-1 chain C region secreted form | IP100109911 | R EEQFN#STFR S | 332 |
| serum amyloid P-component | IP100267939 | K LIPHLEKPLQN#FTLCFR T | 333 |
| haptoglobin | IP100274017 | K NLFLN#HSETASAK.D | 334 |
| | | K.N#LTSPVGVQPILNIHTPCAGLTK.Y | 335 |
| leucine-rich alpha-2-glycoprotein | IP100129250 | R.SLPPGLFSTSAN#LSTLVLR.E | 336 |

(continued)

| | | | |
|---|---|---|---|
| complement component factor h | IP100130010 | K DNSCVDPPHVPN#ATIVTR.T | 337 |
| fetuin beta | IP100134837 | R VLYLPAYN#CTLRPVSK R | 338 |
| | | R RVLYLPAYN#CTLRPVSK.R | 339 |

Collectively these data indicate that the LC-MS-based analysis of isolated, formerly *N*-linked glycosylated peptides reproducibly detected peptides of different abundance in serum samples of cancer and normal mice and that the discriminatory peptides could be identified by MS/MS analysis.

Described above is a method for high throughput quantitative analysis of serum proteins using glycopeptide capture and LC-MS. It consists of the selective and reproducible isolation of those peptides from the serum proteome that are modified by *N*-linked glycosylation in the intact protein. The complex mixture of the de-glycosylated forms of these peptides was then analyzed by LC-MS. The mass of discriminatory peptides was determined using pattern matching software, and these peptides were subsequently identified by MS/MS. These results indicate that the glycopeptide capture-and-release method is specific for the isolation of *N*-linked glycopeptides. On average, 3.6 peptides were isolated per protein representing an average of 1.8 glycosylation sites per protein. This is contrasted with a predicted 28.8 unique tryptic peptides per protein calculated from the pool of identified proteins. The data also indicates that this reduced sample complexity resulted in an increase in sensitivity compared to the analysis of non-selected serum digests using an identical analytical platform. To test its suitability for analysis of disease, the method was applied to the differentiation of sera from genetically identical mice that were either untreated normal or cancer-bearing. The resulting peptide patterns could clearly and correcly-be differentiated into two groups via unsupervised clustering. Some of the discriminatory peptides were further identified by MS/MS and their differential abundance in cancer versus control mice was verified by accurate quantification using stable isotope labeling.

Ideally, for the detection and validation of protein biomarkers in serum the complete serum proteomes of multiple individuals representing different clinical states would be completely and quantitatively analyzed. Due to the enormous complexity of the serum proteome and technical limitations, all the current proteomic technologies for such analyses can only sample a small part of the proteome, predominantly the most abundant proteins (Anderson and Anderson, Mol. Cell. Proteomics 1:845-867 (2002); Zhang et al., Curr. Opin. Chem. Biol. 8:66-75 (2004)). For example, 2DE-based studies have identified about 300 serum proteins collectively (Anderson and Anderson, Mol..Cell. Proteomics 1:845-867 (2002); Pieper et al., R., Proteomics 3:1345-1364 (2003); Anderson et al., Mol. Cell. Proteomics 3:311-326 (2004). It has also been estimated that SELDI-TOF approaches have limited detection of low abundant proteins due to the high dynamic range of serum proteins and the limited binding capacity of the SELDI chip (Diamandis, Mol. Cell. Proteomics 3:3.67-378 (2004)). In the method described above, the selective isolation of the *N*-linked glycosylated peptides resulted in a substantial improvement in the concentration limit of protein detected due to the reduction in sample complexity.

A number of factors contribute to this effect. First, the number of peptides per protein isolated after applying the glycopeptide capture-and-release method is significantly reduced. The 93 proteins identified in this study are predicted to generate an average of 28.8 tryptic peptides per protein. Of these, only 3.6 on average contain the *N*-linked glycosylation consensus motif and can be potentially glycosylated, and an average of 3.6 peptides representing 1.8 unique *N*-linked glycosylation sites per protein were actually identified. By comparison, a similar number of *N*-linked glycosylation sites identified per protein was reported by Kaji and colleagues (1.8 sites per protein) in a study in which *N*-linked glycopeptides were isolated from *C. elegans* proteins using lectin enrichment (Kaji et al., Nat. Biotechnol. 21:66 7-672 (2003)). Second, the most abundant serum protein, albumin, does not contain *N*-linked glycosylation motifs and therefore is effectively transparent to the analysis. Since albumin itself comprises almost 50% of total serum protein content, exclusion of albumin climinates numerous peptides that otherwise dominate serum peptide samples. Indeed, quantitative removal of albumin, a goal that is normally attempted by use of costly affinity depletion methods (Pieper et al., Proteomics 3: 422-432 (2003) is an automatic by-product of the glycopeptide capture method. Third, the method only selects peptides from the constant region of immunoglobulins and thus dramatically reduces the number of immunoglobulin-derived peptides. This is important since immunoglobulins constitute approximately 20% of total protein mass in serum (Putnam, The plasma proteins: Structure, Function, and Genetic Control, 2nd ed. Academic Press, New York, NY (1975)) and comprise a population of an estimated 10 million different molecules (Anderson and Anderson, Mol. Cell. Proteomics 1: 845-867 (2002)). The difficulty of penetrating the population of immunoglobulins in unbiased serum proteome analyses was recently illustrated in a study in which a tryptic digest of serum was analyzed by ultra-high-efficiency strong cation exchange LC/RPLC/MS/MS. Of the 1061 plasma protein identifications reported, 38% were immunoglobulins (Shen et al., Anal. Chem. 76:1134-1114 (2004)). It is also likely that an even more significant fraction of peptides observed in LC-MS patterns of unbiased serum protein digests are derived from immunoglobulins since nucleic acid and protein sequence databases dramatically underreport the contribution of somatic combinatorial gene rearrangement to immunoglobulin diversity. Fourth, many serum proteins are post-translationally altered by phosphorylation, glycosylation, acetylation, methionine oxidation, protease processing and other mechanisms, resulting in multiple forms for each protein. It has been estimated that one protein may generate on the order of 100 species (Anderson and Anderson, Mol. Cell. Proteomics 1:845-867 (2002)). In the case of glycosylation, the oligosaccharide structures attached at each site are typically diverse, compounding the complexity of the peptide mixture. The peptides isolated by the glycopeptide capture method remove the heterogeneous oligosaccharides, and thus by isolating a few peptides per protein only; also eliminate other significant sources of pattern heterogeneity.

The cumulative effect of these factors is the generation of a peptide sample from the serum proteome with a moderate redundancy of an average of 3.6 unique peptides per protein. Theoretically, an average of 3.6 potential *N*-linked glycopeptides (containing an N-X-T/S motif) is predicted for the 93 identified serum proteins. However, not all of these potential *N*-linked glycosylation sites were observed. Some of these potential *N*-linked glycosylation sites may not actually be occupied (Petrescu et al., Glycobiology 14:103-114 (2004), or the peptides from certain sites may not be detectable by mass spectrometry, or protein digestion may be hindered by the protein post-translational modifications such as oligosaccharide attachment and/or disulfide bond formation. On the other hand, the number of peptides from each glycosylation site was increased due to other types of protein modifications (that is, methionine oxidation, protease processing) in the glycosylaton region. It is expected that the same factors would also lead to an inflation of the number of peptides observed if digests of non-selected serum samples were analyzed. In the analyses of peptides generated from 5 μl of mouse serum using glycopeptide capture-and-release method, over 3000 peptide peaks were detected and quantified that were present at least at 2 of 3 samples in either group with intensity at least at 2-folds above background noise level. In MS/MS analysis, only a small fraction of peptides (319 unique peptides) were identified. This was due to the complexity of the sample and the fact that the mass spectrometer only had time to sequence a small portion of the peptides, predominantly the highly abundant peptides in each sample. The same under sampling factor was also the major cause of the inconsistency of protein identifications using LC-.MS/MS. In this study, reproducible LC-MS was used for quantitative analyses, and this allowed analysis of all the peptide ions in each sample, including those from proteins of low abundance.

While the reduction of peptide redundancy is beneficial for achieving higher coverage of the proteome per analysis, it is also apparent that it leads to the loss of some, potentially important information. First, non-glycosylated proteins are transparent in this system. While it is believed that the majority of serum-specific proteins are in fact glycosylated (Durand and Seta, Clin. Chem. 46:795-805 (2000), intracellular proteins (typically non-glycosylated) that may represent a rich source of biomarkers if leaked into serum might go undetected. Second, the availability of fewer peptides per protein increases the challenge of identifying the corresponding protein. Third, this approach will reveal differences in protein level, or glycosylation level (glycosylation site occupancy). Disease markers that alter other protein post-translational modifications including proteolytic processing will not be detected on a glycopeptide level. Finally, collapsing peptides modified by different oligosaccharide strcture into a single signal will obscure potential disease markers that are due to oligosaccharide structure alteration (Durand and Seta, Clin. Chem. 46:795-805 (2000).

In this study, the glycopeptide capture and LC-MS analysis platforms was used to differentiate serum from mice with chemically induced skin cancer from that of non-treated littermates. In this experiment, the mice with skin cancer and their untreated littermates had the same genetic background and lived in the same environment. The study therefore represents a controlled experiment with chemically induced skin cancer being the sole variable. The sera were clearly distinguished by numerous distinct peptides, the abundance of which was consistently increased or decreased between the cancer and control sera. While in this controlled experiment, the low number of samples was sufficient to detect disease-associated signatures, the application of the method to identification of potential biomarkers in much more variable human samples will require the analysis of a larger sample numbers in order to facilitate statistical validation of the data. The current method, at present, has sufficient throughput to perform studies involving a few hundred samples, a number that appears sufficient to generate statistically significant result within a reasonable time frame (Sullivan Pepe et al., J. Natl. Cancer Inst. 93:1054-1061 (2001); Adam et al., Cancer Res. 62:3609-3614 (2002)). By developing a robotic procedure to allow automated sample preparation, and by further optimizing LC-MS analysis procedures and the development of a robust, automated data analysis platform, the performance of the system can be further increased.

In contrast to the widely used SELDI-TOF and similar polypeptide profiling methods, the signals detected in the present method are defined molecular species, mostly peptides ranging in length between 7 and 30 amino acids. These peptides, if selected for CID in a tandem mass spectrometer, are readily sequenced. By adding the coordinates of selected discriminatory peptides to an inclusion list, several serum proteins were identified for which the abundance is increased in correlation with the chemical induction of skin cancer in mice (Table 4). While these proteins are indicators of interesting biology and have been reported to change the abundance in different types of cancer (Vejda et al., Mol. Cell. Proteomics 1:387-393 (2002), they are likely not markers for the specific diagnosis of skin cancer. Proteins useful for cancer detection, diagnosis or stratification might be proteins released in small amounts from the primary lesion, indicators of a specific response of the system-to,the lesion or other subtle changes in the serum proteome. For the reliable detection of such proteins or patterns of proteins, it is imperative that a large number of candidate molecules are identified, so that potential markers or signatures observed in different diseases, studies and laboratories can be validated, correlated and compared. This will allow the proteomics biomarker discovery community to establish defined molecular signatures as the currency of communication and to distinguish between true biomarkers and coincidental changes.

The identification of discriminatory peptides in this study furthermore indicates that at least some of the proteins changing in abundance in the skin cancer model are moderately to highly expressed. In contrast, serum cancer markers currently in clinical use have concentrations in the ng/ml range. Diamandis has argued that the SELDI-TOF method and by implication similar methods, are about 3 orders of magnitude too insensitive from the sensitivity required to detect such proteins (Diamandis, Mol. Cell. Proteomics 3:367-378 (2004)). The method presented here has the potential to reach ng/ml sensitivity levels and even lower concentration limits if high performance Fourier transform ion cyclotron resonance mass spectrometry (FTICR-MS) instruments are used. For example, at a concentration of 4ng/ml, 5 $\mu$l of serum sample contains approximately 20 picogram (~700 attomole) of PSA, an amount that is readily detected in a modern mass spectrometer. In comparison, if non-biased serum digests are analyzed on the same capillary LC-MS system, the total amount of serum that can be applied to the system would be 50 nl and therefore the concentration limit of detection would be 100 fold reduced, compared with the glycopeptide selected sample. Thus PSA would be well outside the detection limit of such an analysis. If further increases in the concentration limit of detection were required, the glycopeptide capture-and-release method could easily be combined with other peptide fractionation methods, including electophoresis (gel based or free flow electrophoresis) chromatography or affinity depletion.

In summary, selectively isolating peptides from *N*-linked glycosylated serum proteins has been found to be a powerful method for the analysis of the serum proteome. Together with the high reproducibility of this method, the high level of serum proteome coverage achieved at a moderate throughput suggests that this method will be most useful for the detection of proteins or protein patterns that distinguish individuals in different physiological states.

## EXAMPLE 3

Development of High-throughput LC-MALDI MS/MS Method Using Stable Isotope-labeled Peptides for Biomarker Identification and Quantification

In the past few years MS-based proteomics as a "discovery science tool" has been quickly emerging into an informative quantitative technology for studying systems biology. Quantitative proteomics has demonstrated its potential applications in detection and quantification of diagnostic or prognostics disease markers and therapeutic proteins. The combination of off-line LC separation/spotting and MALDI MS/MS provides several conceptual advantages for such applications: more complete peptide coverage, the ability for repeat or multiple analysis on the same sample, selective MS/MS analysis based on MS information, high mass range, higher contamination tolerance, and easy to interpret data structure.

A straightforward, high-throughput screening technique was developed, which can be applied for clinical diagnostics, using LC-MALDI MS/MS combined with isotope-labeled peptide spiking. The isotope-labeled peptides were synthesized and spiked in the samples with appropriate concentration. The complex peptide mixture were separated and spotted on MALDI plates using HPLC/probot system (LC Packing). The spotted MALDI plate was analyzed in a MALDI TOF/TOF instrument (Applied Biosystems 4700 Proteomics Analyzer) in MS mode. The selected peptides were further analyzed in MS/MS mode for peptide/protein identification and confirmation. The CID fragmentation information of the peptides was searched against a human sequence database for peptide/protein identification and confirmation using a suite of software tools essentially as described in Examples I and 2. Quantification was achieved using the abundance ratio of the native peptide and the corresponding spiked peptide.

The isotope-labeled peptides were synthesized and characterized with HPLC and mass spectrometry. The elution properties of the isotope-labeled peptides and the effects of competitive ionization in a complex system were further evaluated by LC-MALDI TOF/TOF. The glycopeptides were captured from human serum proteins using hydrazide chemistry. The glycopeptide mixtures were spiked with isotope-labeled peptides and analyzed by L.C-MALDI TOF/TOF. The study has demonstrated that the approach using LC-MALDI TOF/TOF and isotope-labeled peptide spiking can specifically target interesting peptide/protein identifications and quantification, therefore, significantly reducing the time intensive MS/MS analysis and database searching.

In these studies, a novel approach to facilitate the detection and quantification of specific proteins in a complex sample was developed. The highly selective, high throughput platform is built based on a MALDI TOF/TOF spectrometer and using stable isotope labeled peptides as internal standards. The detection and quantification of targeted proteins was accomplished using a complementary approach of specific mass matching, peptide sequencing and peptide quantification. The system demonstrated the capability to detect, selectively identify and quantify proteins of interest in a complex serum sample. These studies were extended and are described in more detail in Example 4.

**Example 4**

**High-throughput Proteome Screening for Biomarker Detection**

This example describes the use of TOF-TOF analysis on an array as an example of qualitative and/or quantitative analysis of serum glycoproteins.

<u>Preparation of formerly N-linked glycosylated peptides from serum</u>. Serum glycoproteins in coupling buffer 100 mM NaAc and 150 mM NaCl, pH 5.5) were oxidized by adding 15 mM of sodium periodate at room temperature for 1 hour. After removal of sodium periodate, the sample was conjugated to the hydrazide resin at room temperature for 10-24 hours. Non-glycoproteins were then removed by washing the resin 6 times with an equal volume of urea solution (8M. urea/0.4M $NH_4HCO_3$. pH 8.3). After the last wash and removal of the urea solution, the resin was diluted with 3 bed volumes of water. Trypsin was added at a concentra.tion of 1 mg of trypsin/200 mg of serum protein and digested at 37°C overnight. The peptides were reduced by adding 8 mM TCEP (PIERCE, Rockford, IL) at room temperature for 30 min, and alkylated by adding 10 mM iodoacetamide at room temperature for 30 min. The trypsin-released peptides were removed by washing the resin three times with 1.5 M NaCl, 80% Acetonitrile/0.1% trifluoroacetic acid (TFA), 100% methanol, and six times with 0.1 M $NH_4HCO_3$ *N*-linked glycopeptides were released from the resin by addition of pertide-N-glycosidase F (PNGase F) (at a concentration of 1 ml of PNGase F/40 mg of serum protein)(New England Biolabs: Beverly MA) overnight. The released peptides were dried and resuspended in 0.4% acetic acid for mass spectrometry analysis.

<u>Synthesis of stable isotope labeled peptides.</u> Fluorenylmethoxycarbonyl-derivatized phosphoamino acid monomers were from AnaSpec, Inc (San Jose, CA). Fmoc-derivatized stable-isotope monomers containing one $^{15}$N and five to nine $^{13}$C atoms were from Cambridge Isotope Laboratories (Andover, MA). Pre-loaded Wang resins were from Applied Biosystems. Synthesis scale was 5 $\mu$mol. Amino acids activated *in situ* with 1-H-benzotriazolium, 1-[bis(dimethylamino) methylene]-hexafluorophosphate(1-),3-oxide:1-hydroxybenzotriazole hydrate were coupled at a 5-fold molar excess over peptide. Each coupling cycle was followed by capping with acetic anhydride to avoid accumulation of one-residue deletion peptide byproducts. After synthesis, peptide-resins were treated with a standard scavenger-containing trifluoroacetic acid-water cleavage solution, and the peptides were precipitated by addition to cold ether. Peptides were purified by reversed-phase C 18 HPLC using standard TFA/acetonitrile gradients and characterized by matrix-assisted laser desorption ionization-time of flight (Biflex III, Bruker Daltonics, Billerica, MA) and ion-trap (ThermoFinnigan, LCQ DecaXP) MS.

<u>LC/Probot fractionation and MALDI TOF/TOF analysis.</u> The glycopeptide mixture was separated by reverse phase C 18 column and spotted on a MALDI. plate. The separation was performed using an Ultimate HPLC system (LC Packing/Dionex, Sunnyvale, CA) coupled with a Famos micro autosampler (LC Packing/Dionex, Sunnyvale**,** CA). A 100 minute gradient was used with liquid chromatography (LC) for peptide separation using a house packed C18 column. The eluent from the capillary column was mixed with the $\alpha$-cyano-4-hydroxycinnapinic acid matrix solution (Agilent Technologies, Palo Alto, California) in a mixing tee before spotting onto the MALDI plate. The matrix solution was delivered with a syringe pump. The fractions were automatically collected with 30 second intervals and spotted on a 192-well MALDI plate (Applied Biosystems, Foster City, CA) using a Probot Micro Fraction collector (LC Packing/Dionex, sunnyvala,CA). The samples were analyzed by a MALDI TOF/TOF tandem mass speetrometer (ABI 4700 protemics Analyzer, Applied Biosystems Foster City, CA). Both MS and MS/MS data were acquired with a Nd:YAG laser with 200Hz sampling rate. For MS spectra, 1000 laser shots per spot were used. MS acquisition for the entire plates took 16 minutes with a total of 192000 laser shots per plate. MS/MS mode was operated with LKeV collision energy. The CID was performed using air as the collision gas. A typical 2000 laser shots was used for MS/MS acquisition. Both MS and MS/MS data were acquired using the instrument default calibration.

<u>Database searching of MS/MS data.</u> MS/MS data were searched against the human protein database from NCBI and a standard peptide database containing the spiked peptides. The mass tolerance of the precursor peptide was set at $\pm$0.4 Daltons (Da), and the database search was set to expect the stable isotope labeling and the following modifications: carbonxymethylated cysteins, oxidized methionine and an enzyme-catalyzed conversion of asparagine to aspartic acid at the site of carbonhydrate attachment. No other constrains were include in the SEQUEST search. All of the MS/MS spectra were manually checked to verify the validity of the results.

<u>Quantification.</u> Binary files of MS survey scans were exported using 4700 Explorer software. Each file is corresponding to a single MS spectrum. The peak information, including spot number, mass and intensity, was extracted from the binary files and converted to text files. The individual files were then combined into a single text files, which contains the peak information from all the spots. The file was scanned for peptides that had been eluted across more than one sample spot. The signal intensities of these peptides from each adjacent spots were summed together to determine an accurate intensity over the entire peptide elution profile. The quantification of targeted peptides was achieved using the abundance ratio of a native peptide to the corresponding spiked stable isotope labeled peptide, which the amount is known. The quantification of each identified peptide was manually checked to verify the validity of the results.

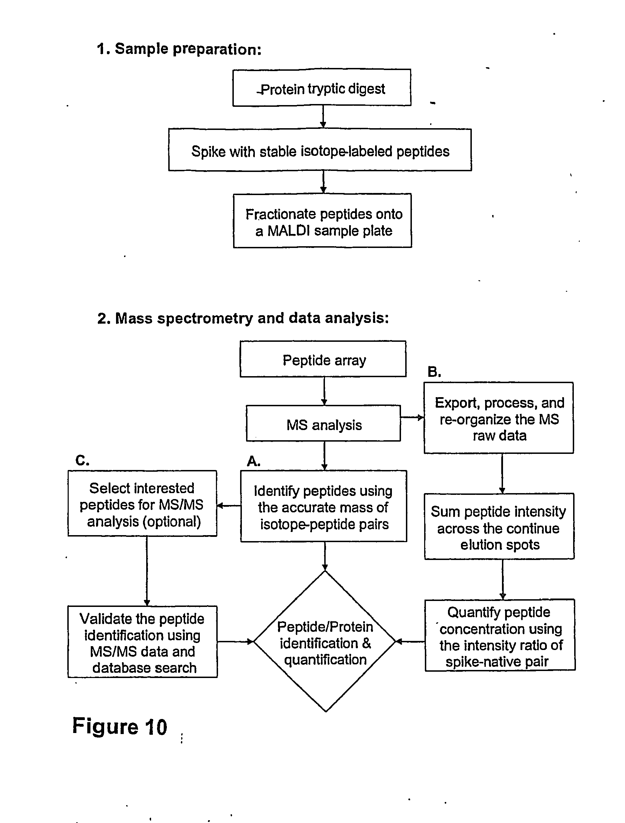

The method used is schematically outlined in Figure 10. It is conceptually simple and consists of two main steps, the production of ordered peptide arrays and their interrogation by MALDI-MS and MS/MS. For the production of ordered peptide arrays, protein samples (untagged proteins or proteins labeled with specific stable isotope tags) were subjected to tryptic digestion and combined with a cocktail of defined amounts of isotopically labeled reference peptides, each of which uniquely identified a particular protein or protein isoform (proteotypic peptides). The reference peptides were generated by chemical synthesis and contained heavy stable isotopes. The combined peptide mixture was separated by capillary reverse phase chromatography ($\mu$LC) and the eluting peptides are deposited on a sample MALDI plate to form an ordered peptide array in which each array element contains peptides that are derived from the digested sample proteins and/or from the cocktail of reference peptides. For the detection and quantification of the target polypeptides, that is, those proteins for which a reference peptide was added to the sample, the sample was analyzed using a matrix assisted laser desorption/ionization (MALDI) tandem time-of flight (TOF-TOF) mass spectrometer that operated under a data-driven instrument control protocol, carrying out the following sequential steps A-C.

Step A) High speed MS scanning. MALDI-MS spectra were acquired from each array element, generating two types of signals, one representing the signals of the peptides for which no reference peptide was added, appearing as single peaks, and the other representing the signals for those peptides for which a reference peptide was added, appearing as paired signals with a mass difference that precisely corresponded to the mass differential encoded in the stable isotope tag. B) Peptide quantification. The signal intensities of the isotopically heavy and light forms of a signal pair were determined and used to calculate the absolute abundance of the peptide derived from the protein sample. As reverse-phase chromatography could split a specific pair of isotopic peptides across several consecutive spots on the MALDI plate, it was necessary to process the data prior to quantification. A specifically developed software tool scanned the MS data files for peptides (pairs) that eluted across more than one sample spot, summed the signal intensities of the corresponding signals from adjacent spots and used the integrated value for quantification, thus ensuring higher quantitative accuracy. C) Optional confirmation of peptide identity by MS/MS. In this method, proteins are primarily identified by correlating the array position and the accurately measured mass of each isotopically labeled peptide pair in the array with a list of added reference peptides with known mass. Optionally, peptide sequences could be confirmed by subjecting selected peptides to CID and sequence database searching of the resulting spectra (Eng ct al., J. Am. Soc. Mass Spect. 5:976-989 (1994)).

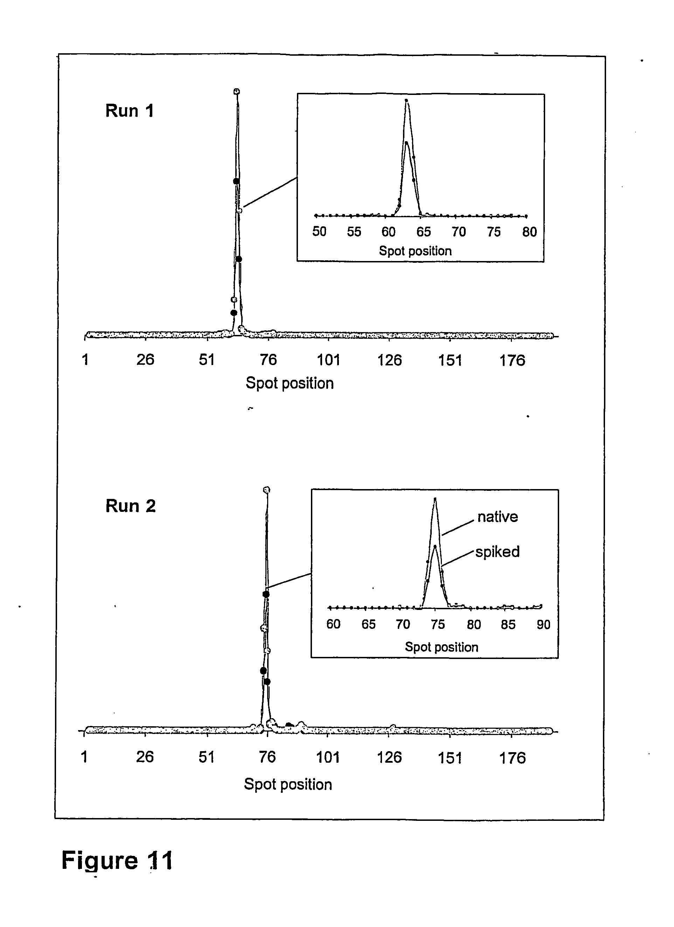

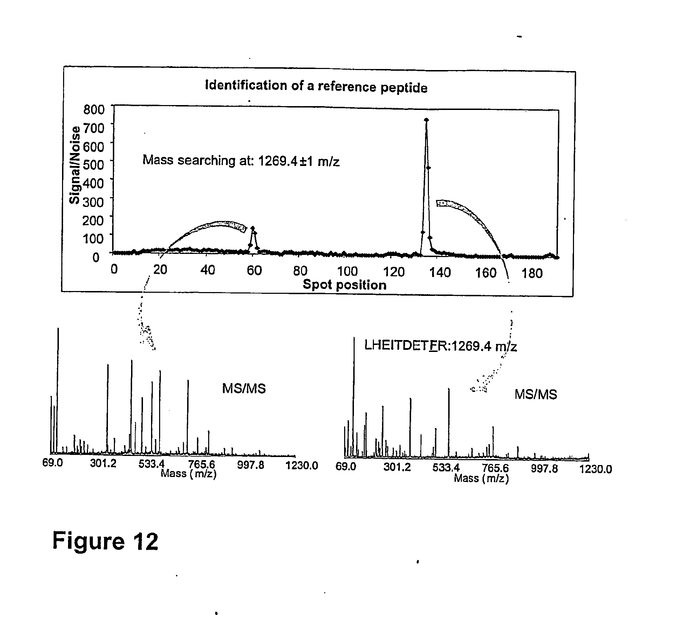

To test the robustness of peptide identification reference peptides were added to a complex glycopeptide mixture extracted from human serum (Zhang et al., Nat. Biotechnol. 21:660-666 (2003))and spotted onto the sample plate under slightly different chromatography conditions. The plates were then analyzed and the peptides were identified in the sample mixture based on their accurate mass, the paired nature of the signal and the location on the peptide array. Figure 11 shows the extracted ion trace over the chromatographic separation range for two consecutive runs. It is apparent that peptide LADLTQGEDQYYLR (1683.8 Da, derived from Clusterin precursor; SEQ ID NO:) was unambiguously identified in the complex background even though the targeted peptide pair was found in different spot positions in the two runs. The accurate mass, together with the paired nature of the signal, were sufficient for the identification of the target peptide. With increasing complexity of the analyzed sample, the chance that these criteria are insufficient for unambiguous peptide identification also increases. In these cases, peptide identities were confirmed by the fragment ion spectra of the precursors that are isobaric to the targeted peptide. An example of peptide confirmation by CID is illustrated in Figure 12. Two peaks that corresponded to the mass of the stable isotope labeled reference peptide LHEITDETFR (1269.4 Da, from proteins similar to RIKEN cDNA 2610528G05 gene (Fragment); SEQ ID NO:) were detected within the mass search window. The expected signal was discriminated from the unexpected one based on the CID spectrum. The SEQUEST search results (Eng et al., *supra,* 1994) of the obtained spectra indicated that the precursor ion with higher intensity, eluting across spot 133 to spot 138, was the target peptide. Using this approach that limits the number of sequencing operations, the platform not only provided the high confidence for peptide identification, but also operated in a high throughput mode. For instance, with a laser sampling rate at 200Hz available in the 4700 MALDI TOF/TOF instrument, a 192-well sample plate can be analyzed in less than 1 hour by MS scan of 192 spots followed by 200 MS/MS scans for selected peptide sequence validation.

To assess the performance of the system for rapid profiling of selected proteins in complex mixtures, N-glycoproteins were analyzed in human serum. The serum-derived peptides were generated from scrum proteins by using a solid-phase glycopeptide capture and release method as described above (Zhang et al., Nat. Biotechnol. 21:660-666 (2003)). In brief, serum glycoproteins were immobilized on a solid phase via their glycostructure. Immobilized glycoproteins were trypsinized and the non-glycosylated peptides were washed to waste. The peptides that carried an *N*-linked carbohydrate on the native protein were isolated in their de-glyscosylated form using the enzyme PNGA'se F that cleaves between the carbohydrate and the peptide, converting the carbohydrate anchoring Asn into an Asp residue. The serum derived sample was added with a cocktail of iostopically labeled reference peptides. The composition of the reference peptide sample is summarized in Table 5.

Table 5. List of reference peptides labeled with heavy stable isotope.

| Swiss-Prot/TrEMBL accession No. | Protein annotation | Synthesized stable isotope labeled peptide sequences |
|---|---|---|
| P03952 | Plasma kallikrein precursor | IVGGTDSSWGEWPWQVSLQ VK |
| P08185 | Corticosteroid -binding globulin precursor | AQLLQGLGFD LTER |
| P55058 | Phaspholipid transfer protein precursor | IYSDHSALESLALIPLOAP LK |
| P10909 | Clusterin precursor | LADLTQGEDQYY LR |
| P51884 | Lumican precursor | LGSFEGLVDLTFIH LQHNR |
| P19652 | Alpha-1 -acid glycoprotein 2 precursor | SVQEIQATFFYFTPDKTEDTIF LR |
| P02750 | Leucine-rich alpha-2-glycoprotein precursor | LPPGLLADFTL LR |
| Q9H4M1 | Glycosylphosphatidylinositol -specific phospholipase D precursor, Phosphatidylinositol-glycan-specific phospholipase D 1 precursor | FHDVSESTHWTPFLDAS VHYIR |
| P04004 | Vitronectin precursor | DGSLFA FR |
| P04004 | Vitronectin precursor | DNATVHEQVGGPSLTSD LQAQS K |
| NA | Prenylcysteine lyase precursor | GELDTSIFSSR |
| Q9UK55 | Protein Z -dependent protease inhibitor precursor | LPYQGDATmLW LmEK |
| P04180 | Phosphatidylcholine -sterol acyltransferase precursor | mAWPEDHVFISTPS FDYTGR |
| Q13201 | Endothelial cell multimerin precursor | FNPGAESWLSDST LK |
| P40197 | Platelet glycoprotein V precursor | ISALGLPTDLTHILL FGmGR |
| Q04756 | Hepatocyte growth factor activator precursor | CFLGDGTG YR |
| P41222 | Prostaglandin -H2 D-isomerase precursor | SWAPATDGGLDLTSTF LR |
| P41222 | Prostaglandin -H2 D-isomerase precursor | WFSAGLASDSSW LR |
| P11597 | Cholesteryl ester transfer protein precursor | GHFIYKDVSEDLPLPTFSPTL LGD SR |
| P33151 | Vascular endothelial -cadherin precursor | EVYPWYDLT VEAK |
| P02786 | Transfernin receptor protein 1 | KDFEDLYTPVDGSIVI VR |
| P04278 | Sex hormone -binding globulin precursor | LDVDQALDR |
| P06681 | Complement C2 precursor | TmFPDLTD VR |
| Q96KN2 | Glutamate carboxypeptidase -like protein 2 precursor | LVPHmDVSA VEK |
| Q9UGM5 | Fetuin-B precursor | GCDDSOVLAVAGFA LR |

(continued)

| Swiss-Prot/TrEMBL accession No. | Protein annotation | Synthesized stable isotope labeled peptide sequences |
|---|---|---|
| Q9UGM5 | Fetuin-B precursor | VLYLAAYDCTLRP VSK |
| P06276 | Cholinesterase precursor | DDYTKAEELSR |
| P07333 | Macrophage colony stimulating factor 1 receptor precursor | HTDYSFSPWHGFTIHR |
| P03952 | Plasma kallikrein precursor | LQAPLDYTEFQKPICIPSK |
| P05156 | Complement factor 1 precursor | DGTAVCATNR |
| P04114 | Apolipoprotein B-100 precursor | YDFDSSmLYSTAK |
| P80188 | Neutrophilgelatinase-associated lipocalin precursor | SYDVTSVLFR |
| P54289 | Dihydropyridine-sensitive L-type, calcium channel alpha-2/delta subunits precursor | IDVNSWIEDFTK |
| P40225 | Megakaryocyte stimulating factor | DGTLVAFR |
| Q13876 | Quiescin,Bone-derived growth factor (Fragment) | DGSGAVFPVAGADVQTLR |
| Q16769 | Glutaminyl-peptide cyclotransferase precursor | NYHQPAILDSSALR |
| P40189 | Interleukin-6 receptor beta chain precursor | ETHLETDFTLK |
| NA | Nectin-like protein 2,Hypothetical protein HEMBA1001879 | FQLLDFSSSELK |
| NA | hypothetical protein XP_174441 | SHAASDAPEDLTLLAETADAR |
| P13473 | lysosomal-associated membrane protein 2 precursor, Lysosome-associated membrane glycoprotein 2 precursor | IAVQFGPGFSWIADFTK |
| P13473 | lysosomal-associated membrane protein 2 precursor,Lysosome-associated membrane glycoprotein 2 precursor | WQMDFTVR |
| Q96CX1 | Similar to RIKEN cDNA 2610528G05 gene (Fragment) | LHEITDETFR |
| Q07954 | Low-density lipoprotein receptorrelated protein 1 precursor | FDSTEYQVVTR |
| P01009 | Alpha-1-antitrypsin precursor | QLAHQSDSTNIFFSPVSIATAFAmLSLGTK |
| Q86SU4, Q8N5V4 | Similar to RIKEN cDNA 1300018K11 gene (Fragment) | QGSLGLQYDASQEWDLR |
| Q16853 | Membrane copper amineoxidase | IQmLSFAGEPLPQDSSmAR |
| P23470 | Protein-tyrosine phosphatase gamma precursor | SDFSQTmLFQADTTR |
|  | Metalloproteinase inhibitor 1 precursor | FVGTPEVDQTTLYQR |

(continued)

| Swiss-Prot/TrEMBL accession No. | Protein annotation | Synthesized stable isotope labeled peptide sequences |
|---|---|---|
| Q92859 | Neogenin precursor | TLSDVPSAAPQDLSLEVR |

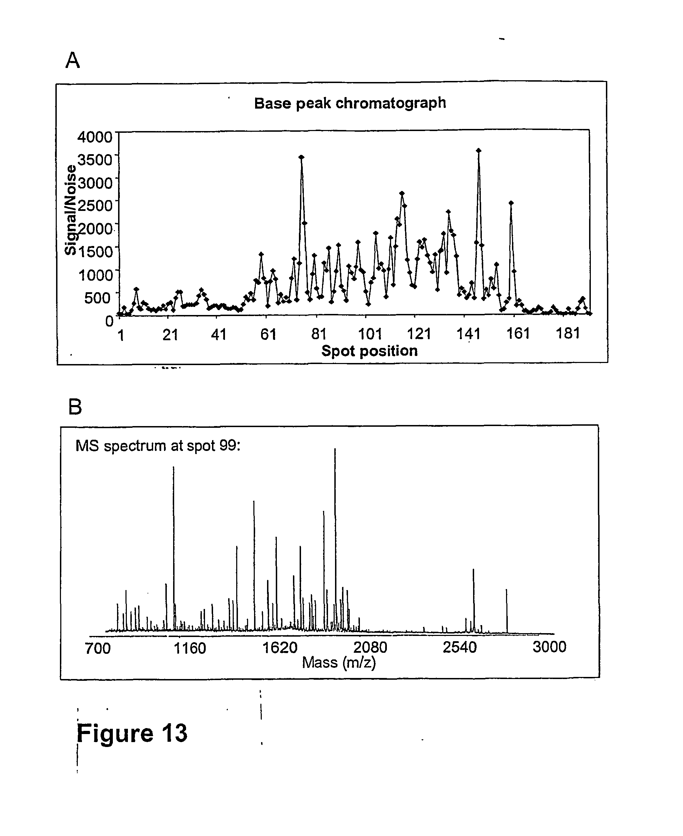

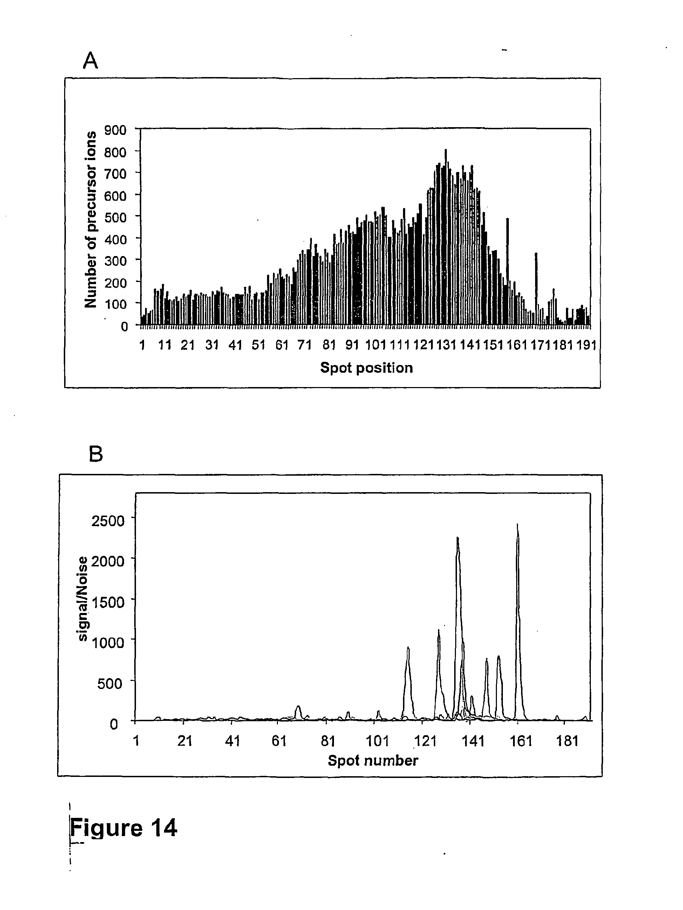

The combined sample was separated by capillary reverse phase chromatography and spotted onto the sample plate in 192 spots and analyzed by MALDI-MS. Results from this analysis are shown in Figure 13. Figure 13A shows the base peak display of the detected peptides, indicating that peptides were detected over the whole separation range, with the majority of peptide signals concentrated between fractions 45 and 165. Figure 13B shows the mass spectrum of a representative spot, indicating the complexity of the sample analyzed. In total, more than 2500 unique precursor ions were detected in MS mode. To identify and quantify the target peptides, the computer driven selective peptide analysis method described above was used. Figure 14 indicates that the added reference peptides could be detected and identified over a broad range of the chromatographic separation range in a very complex sample. Figure 14A shows the number of precursor ions detected in each spot in MS mode; and Figure 14B shows the distribution of spike-in peptides over the chromatographic separation range. The distribution profile of the spike-in peptides were extracted from the very complex background.

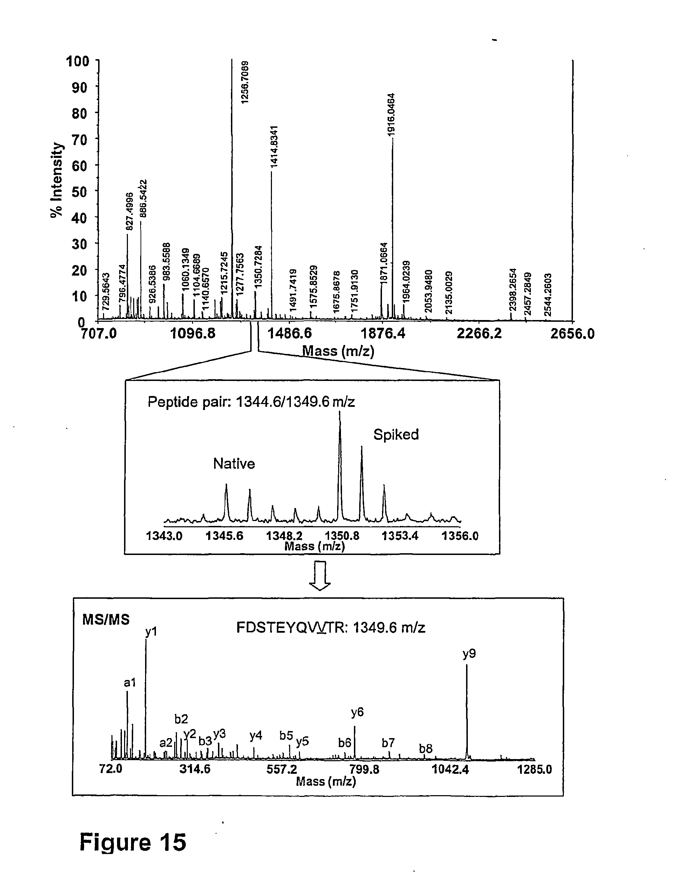

Figure 15 shows that the peptides could be identified and quantified even though they represented relatively minor peaks in a complex spectrum. Data for peptide FDSTEYQVVTR (SEQ ID NO:), which was derived from low-density lipoprotein receptor-related protein 1 precursor and $^{13}$C labeled on residue valine 9, are shown. Using the specific mass matching to search the MS data, the spot (or spots) containing the peptide pairs was located. By examining the MS spectrum, the paired peaks (spiked and native) were identified. The mass of the spike-in peptide and native peptide were 1349.6 Da and 1344.6 Da, respectively. The identification of the peptides was further confirmed by MS/MS analysis and sequence database searching. Since the amount of the spike-in peptide was known, the concentration of the native peptide could be calculated based on the signal intensity ratio of the paired peptide signals. Consequently, the identification and quantification of the related proteins in a complex serum sample was accomplished. The concentration of the protein in a serum sample can be calculated according to equation 1 :

$$C = \frac{(A_n/A_s)*M_s}{V_a} *(V_b/V) \qquad \text{Equation 1}$$

where $A_n$ and $A_s$ are the integrated peak area of the native and spike-in peptide in the MS spectrum, respectively. $M_s$ is the amount of stable isotope labeled peptide spiked in the glycolpeptide mixture. $V_a$ is the volume of the glycopeptide mixture used for MALDI TOF/TOF analysis. $V_b$ is the total volume of the glycopeptide mixture extracted from the scrum sample. V is the total volume of the serum used for glycoprotein extraction. It is important to note that the accuracy of the result estimated from the above formula depends on many factors, including data processing, sample purification, and glycopeptide extraction efficiency, and these factors can be readily determined.

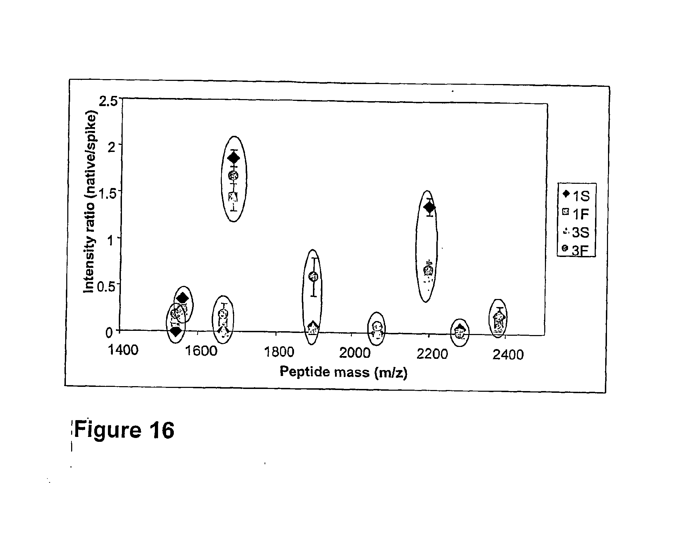

To demonstrate the capacity of the system to rapidly and quantitatively profile selected serum proteins, isolates from four human serum samples using the glycopeptide capture and release method described above were spiked with reference peptides and analyzed by offline LC-MALDI TOF/TOF platform. The proteins and the corresponding signature peptides for which both spike-in and native signals are detected by the platform are listed in Table 6. The results are presented in the form of a peptide map in Figure 16. The x axis represents the mass of the targeted native peptides and the y axis indicates the abundance ratio of a native peptide to the corresponding isotope-labeled peptide, providing the quantitative information describing the corresponding protein. The result demonstrates that even in very complex samples with an enormous number of proteins that may fluctuate within a population, the key elements that indicate the state of a specific biological condition can be effectively extracted and expressed quantitatively by this approach.

Table 6. List of proteins and the corresponding signature peptides used in Figure 16.

| Protein | Peptide sequence | Mass (m/z) |
|---|---|---|
| Apolipoprotein B-100 precursor | YDFN*SSM#LYSTAK | 1542.7 |
| Corticosteroid -binding globulin precursor | AQLLQGLGFN*LTER | 1559.8 |
| Endothelial cell multimerin precursor | FNPGAESVVLSN*ST LK | 1662.9 |

(continued)

| Protein | Peptide sequence | Mass (m/z) |
|---|---|---|
| Clusterin precursor | LAN*LTQGEDQYY LR | 1683.8 |
| Neogenin precursor | TLSDVPSAAPQN*LSLE VR | 1897.1 |
| Transferrin receptor protein 1 | KDFEDLYTPVN*GSIVI VR | 2065.4 |
| Lumican precursor | LGSFEGLVN*LTFIH LQHNR | 2195.5 |
| Phospholipid transfer protein precursor | IYSN*HSALESLALIPLQAP LK | 2278.7 |
| Vitronectin precursor | N*NATVHEQVGGPSLTSD LQAQSK | 2331.5 |
| * enzyme-catalyzed conversion of asparagine to aspartic acid at the site of carbonhydrate attachment.<br># methionnine oxidation.<br>_ amino acid labeled with $^{15}$N and $^{13}$C. | | |

These results demonstrate a method for proteome screening and an experimental platform that supports the method. The method has the potential to reach very high throughput because the redundancy common to LC-MS/MS based proteomics experiments is eliminated and the analysis is focused on specific, information rich analytes. The offline LC-MALDI TOF/TOF based platform provides several advantages for such an approach. These advantages include more complete peptide coverage, low redundancy, the option to perform repeated or multiple analyses on the same sample, high mass range and accuracy, selective MS/MS analysis based on MS information, higher contamination tolerance, and easy to interpret data structure. The generation of predominantly singly charged peptides by MALDI simplifies the quantitative analysis. Global identification can be performed on the same MALDI plate afterwards, if the information is needed. The ability to reexamine and verify the same sample set can be very beneficial for quantitative applications.

It noteworthy that not all of the spike-in peptides behaved the same in a complex sample. In selection of reference peptides, criteria, such as biological significance, sensitivity for mass analysis, good mass range, and without potential mass overlap with other peptides, and the like, need to be satisfied. The development of proteome-screening technology indicates an important transition of quantitative proteomics from a sole discovery mode into a multi-phase technology. The implementation of the browsing/screening mode allows the utilization of the extensive genomic and proteomic knowledge that has been accumulated by biology and medicine, and focus on analyzing the key elements that uniquely represent a specific biological condition, as was demonstrated in this study. Technically, since the identification and quantification of targeted proteins is based on searching and identifying the corresponding signature peptide pairs directly, the approach significantly reduces sample complexity, therefore improving the throughput and identification confidence. It provides a greater analytical dynamic range and facilitates the detection of low abundance proteins. The ability to describe specific protein patterns associated with certain biological conditions within a complex background in an absolute quantitative way provides the feasibility for data standardization. The proteome-screening technology described in this example opens new opportunities for quantitative proteomic analysis and can be developed into a high throughput technology for clinical diagnostic at proteome level.

**Example 5**

**Identification of Serum Glycoproteins**

Thousands of N-linked glycosylation sites have been isolated and identified using the methods described above. Sixty peptides have been synthesized according to the identified N-linked glycosylation sites, and one N-linked glycopeptide with heavy isotope labeling was spiked to human sera of different persons to quantify the abundance of the glycopeptide. Table 7 shows the N-linked glycosylation sites of glycopeptides (SEQ ID NOS: 1-3244) identified from human serum/plasma using the above-described methods. Table 8 shows N-linked glycopeptides (SEQ ID NOS: 3245-3369) identified from human serum/plasma using the above-described methods and which do not contain the consensus (N-X-T/S) glycosylation motif. Asparagines modified in the peptide sequence are marked (*).

Table 7. N-linked glycosylation sites identified from human serum/plasma.

| Protein IPI # (VERSION 2.28) | Identified Peptide Sequences | Peptide Probability |
|---|---|---|
| IPI00000001 | R.EFVMQVKVGN#HTAEGTGTNK.K | 0.7332 |
| IPI00000013 | Y.RPENSVAN#DTGFTVVAPGKEK.A | 0.6348 |
| IPI00000070 | M.SDEVGCVN#VTLCEGPNK.F | 0.6201 |
| IPI00000075 | R.LASPPSQGEVPPGPLPEAVLALYN#STR.D | 0.9994 |
| IPI00000087 | K.QFSLN#WTYQECNN#CSEEMFLQFR.M | 0.6626 |
| IPI00000124 | R.N#LSFLDLCFTTSIIPQM*L.V | 0.55 |
| IPI00000137 | K.YEFCPFHN#VTQHEQTFR.W | 1 |
| IPI00000151 | R.SEVELEVLGDTEGLN#LSFTAIC | 0.8574 |
| IPI00000160 | R.N#SSSSGSSGAGQK.R | 0.7791 |
| IPI00000213 | M.GAAVTLKN#LTGLNQRR.- | 0.9553 |
| IPI00000213 | R.AM*LAAIYN#TTEIVMM*QDSSPDFED.T | 0.7446 |
| IPI00000324 | E.RFASSN#SSQSLAPLMMEVPMLSSLGVTNSKS | 0.7342 |
| IPI00000330 | R.KMAAN#SSGQGFQVK.N | 0.9623 |
| IPI00000350 | L.M*QN#QSSTNHPGASLALSRPSLNKDFR.D | 0.7219 |
| IPI00000352 | L.DTCPSSSTASSISSSGGSSGSSSDN#RTYR.Y | 0.6171 |
| IPI00000375 | Q.RNAENTKSN#VTHK | 1 |
| IPI00000458 | K.NNYGLLENEN#ESLFLM*VVLWK.L | 0.9232 |
| IPI00000691 | K.VFDSLLN#LSSTLQATR.A | 0.9897 |
| IPI00000758 | I.N#STLKGRWR.V | 0.514 |
| IPI00000764 | R.AQPLNLQMVN#ASLYEHVER.M | 0.5611 |

| | | |
|---|---|---|
| IPI00000775 | R.NN#ISKLTDGAFWGI.SKMHVLH.L | 0.5702 |
| IPI00000792 | K.GIDIIEMLANVN#LSKDL S | 0.6397 |
| IPI00000812 | S.MISNMN#ASR.A | 0.5828 |
| IPI00000828 | M.KKDAEEDDSLAN#SSDLLKELLETGDNR E | 0.6778 |
| IPI00000837 | R.AN#ASFTWVASDGWGAQESIIK.G | 0.5568 |
| IPI00000839 | R.NVN#FSGIAGNPVTFNENGDAPGR.Y | 0.7319 |
| IPI00000845 | R.ENALNNLDPNTELN#VSR.L | 0.9806 |
| IPI00000877 | R.KDIN#TTAQ.N | 0.7877 |
| IPI00000877 | R.LSALDNLLN#HSSM*FLK G | 0.9941 |
| IPI00000877 | K.VIN#ETWAWK.N | 0.9549 |
| IPI00000899 | M VDKYIPN#ISM*CLKDSDPFIR.K | 0.6638 |
| IPI00001091 | K RGRGNFGGQSEQENTLNQLLVEMDGFN#T.T | 0.6573 |
| IPI00001120 | L.TSLSVTNTN#LSTVPFLAFK.H | 0.7904 |
| IPI00001120 | R.VLN#VSQNLLET L | 0.9643 |
| IPI00001120 | K.LVPLGVFTGLSN#LTK.L | 0.7469 |
| IPI00001152 | S YQPWGNVPDAN#YTSDEEEEK Q | 0.6651 |
| IPI00001451 | S EEN#STFR.N | 0.8398 |
| IPI00001458 | K LFKEVASLQENFEVFLSFEDYSN#SSLVADLR E | 0.9428 |
| IPI00001461 | G.FVN#STM*EEAGLCGLREK.A | 0.6389 |
| IPI00001497 | E.NAPN#GTLVVTVN#ATDLDEGVNK D | 0.5032 |
| IPI00001510 | K.EKGN#STTDNSDQ.- | 0.723 |
| IPI00001522 | A.SSFKGYIEN#CSTPNTYICMQR.T | 0.5919 |
| IPI00001586 | K.KAADTAVGWALGYM*I.N#LTNLIPADPPGLR.K | 0.9623 |
| IPI00001592 | R.VSVNTAN#VTLGPQLM*EVTVYRR.H | 0.9272 |
| IPI00001592 | K.NDRN#SSDETFLKDLPIMFD.V | 0.6647 |
| IPI00001593 | K.NGGSILFYTGNEGDIIWFCN#NTGFM*WDVAEELK.A | 0.6036 |
| IPI00001651 | Y.KPLN#DSVRAQYSNWLLAGNLAI.SPTGNAKK P | 0.7263 |
| IPI00001654 | K.KMNMM*N#RSYN#VTLKR.Q | 0.7934 |
| IPI00001662 | K GRM*STLTFFN#VSEK D | 0.7988 |
| IPI00001672 | K KSLELNPN#NSTAM*LR.K | 0.7527 |
| IPI00001759 | K.IN#STADLDFIQQAISYSSFPFWM*GLSRR.N | 0.8974 |
| IPI00001866 | R.GSDDGDGESFN#GSPTGSIN#I.SL.D | 0.6138 |
| IPI00001872 | R.ETVPEYN#LSITAR.D | 0.5406 |
| IPI00002054 | K.GTVLPVATIQN#ASTAM*LM*AASVAR K | 0.5668 |
| IPI00002070 | K.TLEELHLTGN#LSAENNR.Y | 0.6831 |
| IPI00002103 | M TVKVDGVAQDGTTMYIINKVHN#RTR T | 0.7155 |
| IPI00002159 | R.KFSFYGN#LSPRR.S | 0.6247 |
| IPI00002159 | K SCSSIISSSNTLSSN#TSSNSDDK.H | 0.7344 |
| IPI00002185 | K N#WSALLTAVVIITIAGNILVIM*AVSLEKK.L | 0.6377 |
| IPI00002197 | K.FLMSN#ETVLLAKHNIFTLALMIV N | 0.9243 |
| IPI00002224 | R.RGDN#DSIIQGDLEPILEASVI.SSHHK.K | 0.6188 |
| IPI00002232 | K.LRKEQI KEELNEN#QSTPKKEK Q | 0.5897 |
| IPI00002251 | R.VPPTLN#SSPCGGFTLCK.A | 0.5339 |
| IPI00002272 | R KKVTAQN#LSDGDIKI.I VNIVRAYDIPVR.K | 0.6656 |
| IPI00002283 | R ETPPLEDLAAN#QSEDPRNQRI.SK.N | 0.807 |
| IPI00002283 | K.NGRYQPSIPPHAAVAAN#QSRARR.G | 0.8831 |
| IPI00002293 | K CGSAYEPEN#QSK.D | 0.5995 |
| IPI00002320 | K.ELFVIIITIFPPNGM*NLYKNN#HSESSSN#R.S | 0.8932 |
| IPI00002335 | M NSETN#ISLLAPCI.SI GMSEISGGQK S | 0.6653 |
| IPI00002354 | R.AFDMLSECGIIM*VACN#SSVTASFINQYT D | 0.6123 |
| IPI00002366 | - MSTLSN#ITQTLEDVFK R | 0.5344 |

| | | |
|---|---|---|
| IPI00002374 | K.TNLDDDVPILLFESN#GSLIYTPTIEIN#SSHHSAMEK.R | 0.63 |
| IPI00002526 | A.N#ASGYM*YETSYR.R | 0.6113 |
| IPI00002541 | K.AFN#STLPTMAQMEK A | 0.8872 |
| IPI00002547 | R LFCDPTFLPEN#DSLFYNRLLPGK V | 0.7198 |
| IPI00002580 | K.YEIYLN#SSLVQFLLS R | 0.7748 |
| IPI00002632 | K.RNHM*LLLYPREILILDLEVN#QTVGVIAIERTGV.P | 0.5125 |
| IPI00002647 | K.LLKGDIDIGSQN#GTDLFGFGNTHEYPDLQMIL S | 0.8714 |
| IPI00002666 | N.GPDTNHQNPQN#KTSPFSVSPTGPSTK I | 0.6038 |
| IPI00002689 | K.AQHEFTEFVGATM*FGTYNVISLVVLLNMLIAMMN#NS.Y | 0.5671 |
| IPI00002707 | K.TN#RTNKPSTPTTATRKKKDLKNFR N | 0.6502 |
| IPI00002707 | R.NVDSNLANLIMNEIVDN#GTAVK.F | 0.6768 |
| IPI00002790 | K M*YSEGSDIVPQSN#ETALHYFK K | 0.5645 |
| IPI00002806 | -.M*NQFGPSALIN#LSN#FSSIKPEPASTPPQGSM*AN.S | 0.9191 |
| IPI00002816 | F.KNFVITYN#RTYESKEEARWR L | 0.7172 |
| IPI00002876 | H.SFLVHLIGLLVWQCDISVSPVAAIVTDIFN#TSDGGR.F | 0.6888 |
| IPI00002984 | K.NPELSGSLMTLSN#VSCLSNTPARK.I | 0.5173 |
| IPI00003048 | P.DN#ASGCGEQINYGR.V | 0.9521 |
| IPI00003057 | R.DVNICN#MTSHLPAAASAS P | 0.8908 |
| IPI00003096 | R.RLSLSQSITDDDLEAIAN#DSEEEIIKPR S | 0.5042 |
| IPI00003323 | K.TSKSEENSAGIPEDN#GSQRIEDTQK.L | 0.9714 |
| IPI00003323 | K.SDSSKSESDSSDSDSDSKSDSSDSN#SSDSSDNS D | 0.957 |
| IPI00003325 | R DLAELKSSLVN#ESEGAAGSAGIPGVPGAGAGAR.G | 0.5809 |
| IPI00003351 | K HIPGLIHN#MTAR C | 0.9991 |
| IPI00003365 | R.KVHLM*GYNCN#ATTK.C | 0.9023 |
| IPI00003370 | K.SIEQSIEQEEGLN#RSSADLR.I | 0.7622 |
| IPI00003384 | R.N#LSVDGKNVDMAGFIANN#GTREG.C | 0.6318 |
| IPI00003451 | R.FSSFVPVTIPHATTAN#TSV.L | 0.6909 |
| IPI00003478 | K.CEFLANLHITALLN#VSRR.T | 0.5036 |
| IPI00003480 | K.DIDVSPKHVGFATIPRN#YTMSFLPR - | 0.6035 |
| IPI00003515 | K AKSDQLLSSNEN#FTNK.V | 0.8642 |
| IPI00003515 | K.N#ISLTKQIDQLSK.D | 0.6204 |
| IPI00003515 | K LM*SLAN#SSEGKVDKVLM*R.N | 0.6075 |
| IPI00003562 | K.TEPMDADDSNN#CT.G | 0.5459 |
| IPI00003590 | K.N#GSGAVFPVAGADVQTLR E | 0.9966 |
| IPI00003706 | K KPYVSLAQQMAPPSPSN#STPN#SSSGSNGNDQLSK.T | 0.6332 |
| IPI00003834 | R DAGGEIAN#LSQAELVDLVQWTDLILFDYLTANFDR.L | 0.8774 |
| IPI00003897 | R.DIIGSPTLSAN#VSLRVLVGDRNDNAPR V | 0.9405 |
| IPI00003919 | K.NYHQPAILN#SSALR Q | 0.9994 |
| IPI00003932 | I.N#CTTEASMAIRPK T | 0.5913 |
| IPI00003965 | K VLKN#SSLAEFVQSI.SQTM*GFPQDQIR L | 0.6703 |
| IPI00004022 | T N#QTPPTYN#K TNK.F | 0.8329 |
| IPI00004022 | R.IDDLQM*VI.N#QTEDHRQR.V | 0.9878 |
| IPI00004047 | K.M*PGDIKNWVDAHM*NCEDIAMNFLVAN#VTGK A | 0.5867 |
| IPI00004047 | K.CTN#LSEGVI SVRK R | 0.783 |
| IPI00004067 | K.SPDTFM*IPM*ALPNDN#GSVSG.V | 0.7678 |
| IPI00004084 | K.MSLVM*PAM*APN#ETLSGR G | 0.5375 |
| IPI00004121 | R YDGAVQVMATQDGAN#FTAARQGYR R | 0.6431 |
| IPI00004237 | K.TNQGIPELN#ASSVGM*AK A | 0.7276 |
| IPI00004247 | R.CSAEEATEGLM*N#LSPSAM*K N | 0.5785 |
| IPI00004362 | S LPSWKSLLNVPMEDVN#LSSGHIAR V | 0.6084 |
| IPI00004368 | - MAHSQNSLELPININ#ATQTTAYGHR A | 0.7548 |

| | | |
|---|---|---|
| IPI00004388 | K.EN#NTGYIN#ASHIK.V | 0.5666 |
| IPI00004399 | E.FKNNFLNIDPITMAYSLN#SSAQER.L | 0.8111 |
| IPI00004413 | R.ECTCPPGMFQSN#ATCAPHTVCPVGWGVRKK.G | 0.545 |
| IPI00004416 | L.KSN#NSM*AQ.A | 0.5174 |
| IPI00004457 | R.IQM*LSFAGEPLPQN#SSM*AR.G | 1 |
| IPI00004457 | R.KEEEPSSSSVFNQNDPWAPTVDFSDFINN#ETIAGK.D | 0.999 |
| IPI00004462 | R.GGLN#LTAVTVAAENN#HTVAFLGTSDGRILK.V | 0.6774 |
| IPI00004462 | R.EAESLQPM*TVVGTDYVFHN#DTK.V | 0.9936 |
| IPI00004462 | R.SFASGGRSIN#VTGQGFSLIQR.F | 0.8388 |
| IPI00004480 | K.EHAVFTSNQEEQDPAN#HTCGVK.S | 0.9335 |
| IPI00004494 | F.GLFN#TTSNIFR.G | 0.734 |
| IPI00004503 | M.FMVKNGN#GTACIM*AN#FSAAFSVNYDTK.S | 0.9952 |
| IPI00004503 | R.GHTLTLN#FTR.N | 0.9941 |
| IPI00004527 | K.GVSFN#ESAADNLK.L | 0.7928 |
| IPI00004529 | R.IDWDDDKYYN#TSLETR.L | 0.9999 |
| IPI00004534 | K.FCDN#SSAIQGKEVRFLR.P | 0.5374 |
| IPI00004557 | M.SN#YSSSSLLSGAGK.D | 0.7134 |
| IPI00004560 | T.KNVNPN#WSVNVK.T | 0.5555 |
| IPI00004560 | K.IKKHFNTGPKPN#STAAGVSVIATTALDK.E | 0.9054 |
| IPI00004565 | -.M*ALNN#VSLSSGDQRSR.V | 0.8782 |
| IPI00004573 | R.AN#LTNFPEN#GTFVVNIAQLSQDDSGR.Y | 0.9994 |
| IPI00004573 | K.VPGN#VTAVLGETLK.V | 1 |
| IPI00004573 | R.LSLLEEPGN#GTFTVILNQLTSR.D | 1 |
| IPI00004573 | K.WN#NTGCQALPSQDEGPSK.A | 0.99 |
| IPI00004576 | M.PVSSSSPLSSLTFNAINRYTN#TSK.T | 0.6661 |
| IPI00004617 | R.EQQFN#STFR.V | 1 |
| IPI00004618 | R.EEQFN#STYR.V | 1 |
| IPI00004641 | W.SESGQN#VTAR.N | 0.7299 |
| IPI00004641 | K.TPLTAN#ITK.S | 1 |
| IPI00004641 | K.HYTN#PSQDVTVPCPVPPPPPCCHPR.L | 1 |
| IPI00004641 | R.LSLHRPALEDLLLGSEAN#LTCTLTGLR.D | 1 |
| IPI00004641 | R.LAGKPTHVN#VSVVMAEVDGTCY.- | 1 |
| IPI00004670 | R.IAEN#YTAVVSPDIASIDLNTFFFNK.P | 0.5166 |
| IPI00004671 | K.KNADN#NSSAFTALSEER.D | 0.6348 |
| IPI00004712 | K.SVVEKM*KN#ISNHLVIEANLDGELNLK.I | 0.5179 |
| IPI00004758 | K.VSPRGIIL.TDN#LTNQLIEN#VSIYR.I | 0.98 |
| IPI00004758 | K.APLSTVSAN#TTNMDEVPRP.Q | 0.9034 |
| IPI00004901 | T.ANSQVM*GSAN#STLR.A | 0.8959 |
| IPI00004951 | R.YN#VSQQALDLQNLR.F | 0.7112 |
| IPI00004957 | R.IDGSQNFN#ETWENYK.Y | 0.9995 |
| IPI00004957 | K.NEFVKN#M*SLELNSK.L | 0.9674 |
| IPI00004970 | C.FYNLELGDM*SLSDN#ASMCLM*SHK.K | 0.5294 |
| IPI00004977 | R.DGVFLEQELHN#LSPGSHDEK.D | 0.9554 |
| IPI00005037 | K.TN#VTHEEHTAVEK.I | 0.5072 |
| IPI00005084 | I.WEKAN#LTLPK.G | 0.5219 |
| IPI00005089 | K.VN#KTLTSLNESNFITGTGILALVEALK.E | 0.6373 |
| IPI00005101 | K.KN#IIYYDSM*GGINNEACR.I | 0.7313 |
| IPI00005107 | K.DGYDLVQEICPGFFFGN#VSLCLDVR.Q | 0.5078 |
| IPI00005118 | R.RQAVELNVVAIVN#DTVG.T | 0.8452 |
| IPI00005118 | R.RQGAYNIDVVAVVN#DTVGTM*M*.G | 0.8535 |
| IPI00005146 | K.LN#VSDLYKLTDTVAIR.E | 0.8929 |

| IPI00005188 | S.VGAAPN#ASDGLAHSGK.V | 0.5798 |
|---|---|---|
| IPI00005258 | K.YEYLMTLHGVVN#ESTVCLM*GHER.R | 0.8617 |
| IPI00005264 | K.ANGLLDFDIFYN#VTGCLRN#MSSAGADGRK.A | 0.9686 |
| IPI00005270 | M.M*SVQANTGPPWESKN#STAVWR.G | 0.7247 |
| IPI00005439 | R.VLYLAAYN#CTLRPVSK.K | 1 |
| IPI00005439 | R.GCN#DSDVLAVAGFALR.D | 1 |
| IPI00005485 | K.GPGEVIPGGN#HSLYSLK.G | 0.5225 |
| IPI00005512 | K.EFYLTPNSPAEMLHN#VTLALELLK.D | 0.5241 |
| IPI00005543 | K.N#WTFGPQDVDELIFMLSDSPGVMCR.P | 0.7216 |
| IPI00005549 | K.EDGSGSAYDKESM*AIIKLN#NTTVLYLK.E | 0.8451 |
| IPI00005565 | T.ILLDAHEAGSAEN#DTADAEPPK.I | 0.8401 |
| IPI00005607 | R.LQNSQCYN#WTLLLGNR.W | 0.9011 |
| IPI00005613 | Q.N#SSQSADGLR.C | 0.5344 |
| IPI00005638 | R.LAM*AYGLN#VSFLER.L | 0.7107 |
| IPI00005667 | R.EFSAGTVYPETN#KTK.N | 0.7715 |
| IPI00005675 | K.HWTNFVITENANDAIGILN#NSASFNK.M | 0.5914 |
| IPI00005683 | K.ALWNLRSN#DTGLLGNVVNIQTGHWVGK.Q | 0.5878 |
| IPI00005683 | M.GN#SSEFQKAVKLVINTVSFDK.D | 0.7686 |
| IPI00005700 | K.KTLDEERN#SSSRSGITGTTNK.K | 0.702 |
| IPI00005704 | A.LCDQEGWDTPIN#YSK.T | 0.839 |
| IPI00005704 | K.GAEIEVDEN#GTLDLSMKK.N | 0.5705 |
| IPI00005750 | M.TGVADN#GSVLEITPDVAEVYLVRK.N | 0.8014 |
| IPI00005791 | K.ELLN#ETEEEINKALNK.K | 0.7699 |
| IPI00005792 | K.QMN#MSPPPGNAGPVIM.S | 0.6751 |
| IPI00005808 | M.LAQEGM*LANLVEQN#ISVRR.R | 0.6502 |
| IPI00005826 | K.SLN#VSSSVNQASR.L | 0.657 |
| IPI00005858 | G.FDEDM*VIQALQKTNN#RSIEAAIEFISK.M | 0.9909 |
| IPI00005858 | R.REQMAAAAARPIN#ASMKPGNVQQSVNR.K | 0.7193 |
| IPI00005858 | R.QPPPPYPLTAANGQSPSALQTGGSAAPSSYTN#GSIP.Q | 0.5362 |
| IPI00006011 | K.N#LSCTNVLQSN#STK.K | 0.9003 |
| IPI00006011 | R.M*KSDSFLQFMPN#VTN.I | 0.7042 |
| IPI00006011 | R.NCQAIQQN#HSCSK.S | 0.6602 |
| IPI00006035 | R.LPLAN#MSYYVSPQAVDAVHRGLGLPLPR.T | 0.908 |
| IPI00006038 | R.ALSMYEEAFQN#TSDSDR.Y | 0.5732 |
| IPI00006038 | K.ITNFKGECIVSDFTIGRKGYGSIYFEGDVN#LT.N | 0.6621 |
| IPI00006065 | M.RPRGQPADIRQQPGM*M*PHGQLTTIN#QSQLSAQLG.L | 0.5097 |
| IPI00006079 | Q.KFN#DSEGDDTEETEDYRQFRK.S | 0.5259 |
| IPI00006093 | K.VVNPQEYSSN#CTEPFPN#STNLLPT.E | 0.6183 |
| IPI00006096 | S.LN#GTSRGSSDITSAR.N | 0.9003 |
| IPI00006096 | K.LQTTN#TTRSVLK.D | 0.8793 |
| IPI00006097 | Q.PQAVPPYASEN#QTCR.D | 0.7754 |
| IPI00006114 | K.VTGN#LTLIEESLTSEFIHDIDR.F | 1 |
| IPI00006154 | R.EQNRESN#ISCVER.G | 1 |
| IPI00006158 | K.CPGPTSGPSPGTN#LSGCIR.M | 0.7724 |
| IPI00006165 | K.NIFVN#GTTGEGLSLSV.S | 0.797 |
| IPI00006173 | K.GVVVN#SSVM*VK.F | 0.9573 |
| IPI00006173 | K.GIIFIYKN#VSEDLPLPTFSPTLLGDSR.M | 1 |
| IPI00006173 | K.TVSN#LTESSSTSIQSFLQSM*ITAVGIPEVM*SR.L | 0.9999 |
| IPI00006191 | R.NLAMEATYINIIN#FSQQCLR.M | 0.8303 |
| IPI00006195 | K.NN#YSPTAAGTERR.K | 0.6855 |
| IPI00006195 | R.VESN#SSAHPWGLVGK.S | 0.9732 |

| IPI00006197 | Y.PDPQSANHMN#SSLLSLYR.K | 0.6374 |
|---|---|---|
| IPI00006213 | M.QINTN#KSKDASTSPPNR.E | 0.683 |
| IPI00006213 | M.NDQDLPN#WSNENVDDR.L | 0.859 |
| IPI00006266 | R.GRGASPRGGGPLILLDLNDENSN#QSFHSEGSLPKGTEP.S | 0.6518 |
| IPI00006278 | R.SFCKDQQGDHNGEN#SSK.C | 0.7131 |
| IPI00006280 | R.IVAARL.N#GSLDFF.S | 0.5099 |
| IPI00006288 | K.ESYLQIPSAKVRPQTN#ITLQIATDEDSGILLYK.G | 0.5946 |
| IPI00006314 | R.N#TTLFIDQVEAK.W | 0.6619 |
| IPI00006374 | K.SSRMETVGN#ASSSSN#PSSPGRIKGR.L | 0.9833 |
| IPI00006496 | A.AEGGN#TSDTQSSSSVNIVMGPSAR.A | 0.7315 |
| IPI00006515 | K.RYN#GSDPASGPSVQDKYVTALYFTFSSLTSV.G | 0.7263 |
| IPI00006543 | R.EQFCPPPPQIPNAQN#M*TTTVNYQDGEK.V | 0.9848 |
| IPI00006552 | K.FN#LTEDM*YAQDSIELLTTSGIQFKK.H | 0.5749 |
| IPI00006612 | A.TN#ETNVNIP.Q | 0.6571 |
| IPI00006631 | R.FIN#STFLEQK.E | 0.5296 |
| IPI00006662 | R.CIQAN#YSLMENGK.I | 0.9993 |
| IPI00006662 | R.ADGTVNQIEGEATPVN#LTEPAK.L | 1 |
| IPI00006663 | R.KTFPTVN#PSTGEVICQVAEGDKEDVDK.A | 0.5661 |
| IPI00006665 | K.NGDPELNVIQNYNEGIIDN#LSK.D | 0.5882 |
| IPI00006669 | R.QSKSESDYSDGDN#DSIN#STSNSN#DTIN#CSSESSSR.D | 0.5434 |
| IPI00006674 | R.QN#NTSLRLGVYAALGILQGFLVMLAAMAMAAGGIQAAR.V | 0.8225 |
| IPI00006675 | K.M*DTELAESGSN#FSVGQR.Q | 0.7018 |
| IPI00006680 | K.LSELHDNQDGLVNMESLN#STR.S | 0.848 |
| IPI00006735 | L.QVEQQLAN#ITV.S | 0.6736 |
| IPI00006746 | R.KLQGNM*LLN#SSMEDKM*LKENPEEK.L | 0.894 |
| IPI00006803 | R.DDALKN#LSHTPVSKFVLDR.I | 0.9538 |
| IPI00006854 | R.TKSQSKLDRN#TSFR.L | 0.6814 |
| IPI00007002 | R.GIEAALGTRASASSFLN#MSRCCIR.A | 0.5876 |
| IPI00007032 | K.TNEISVIQSGGVPTLPVSLGATSVVNN#ATVSK.M | 0.5945 |
| IPI00007063 | K.EEEN#KSSSEGGDAGN#DTR.N | 0.7125 |
| IPI00007096 | C.YPDNPAN#RSLVI.PWSFPLEWAPQN#LTR.W | 0.6644 |
| IPI00007124 | M.DGM*N#SSGVYASPTCSNM*AHHALSFR.G | 0.7767 |
| IPI00007160 | R.EVTNKN#GTNVFQEESR.K | 0.7667 |
| IPI00007178 | R.TGLLKQTHIAPKPAAHLAAPAN#GSAP.S | 0.7895 |
| IPI00007182 | R.ARAGHTM*N#TSPGTVGSDPVILATAGYDHTVR.F | 0.7311 |
| IPI00007193 | K.NN#RSDM*M*SALGI.GQEEDIESPWDSESISENFPQK.Y | 0.7689 |
| IPI00007193 | K.M*N#RTALIILACANGHPEVVT.L | 0.6784 |
| IPI00007199 | K.ETFFN#LSK.R | 0.9904 |
| IPI00007199 | K.LPYQGN#ATM*LVVLM*EK.M | 1 |
| IPI00007202 | G.SPPGFN#NTER.T | 0.9431 |
| IPI00007205 | K.VM*VVLTDGGIFEDPLN#LTTVI.N | 0.5793 |
| IPI00007210 | K.YKSVYVGEETN#ITLNDLKPAM*DYHAKVQAEYNSIK.G | 0.5686 |
| IPI00007224 | R.VVGVPYQGN#ATALFIEPSEGK.M | 1 |
| IPI00007221 | K.VI.PSLGISNVFTSHADLSGISN#HSNIQVSEMVHK.A | 1 |
| IPI00007221 | R.EDQYHYLLDRN#LSCR.V | 0.9996 |
| IPI00007240 | K.EHETCLAPFLYNGN#YSTTQK.T | 1 |
| IPI00007240 | K.HGVHSSTVDIYEN#GSSVEYR.C | 1 |
| IPI00007243 | R.RN#ASGLTNGLSSQFR.P | 0.5707 |
| IPI00007249 | R.LNN#ITMWLN#NSNPPV.T | 0.7009 |
| IPI00007250 | K.QVI.LFN#NSHI.TYVSFDFHEHCR.G | 0.9651 |
| IPI00007253 | L.LLSN#CSK.A | 0.7067 |

| | | |
|---|---|---|
| IPI00007273 | M.FFMNHQHSTAQLN#LSNMK.I | 0.8387 |
| IPI00007296 | G.RVPVN#VTSTALLSVLDIFPTVVALAQASLPQGR R | 0.9365 |
| IPI00007321 | G NN#M*STPLPAIVPAARK.A | 0.844 |
| IPI00007362 | K.NGLSN#SSILLDK.C | 0.7844 |
| IPI00007367 | K.GN#M*TLSPENGYWVVIM*MK.E | 0.7557 |
| IPI00007404 | R GFN#M*SIPMPGHPVN#FSSVTLEQAR.R | 0.7404 |
| IPI00007612 | R.MKRGYDNPNFILSEVN#ETDDTKM.- | 0.5981 |
| IPI00007614 | K.RN#ETLVFSHNAVIAMR.D | 0.8212 |
| IPI00007632 | S.LERFIHGGAN#VTGFQLVDFNTPMVTK.L | 0.5962 |
| IPI00007672 | S.KKEHISAEN#MSLETLR N | 0.5996 |
| IPI00007682 | R.TALVAN#TSNM*PVAAR E | 0.6446 |
| IPI00007765 | K.NAVITVPAYFN#DSQR.Q | 0.9757 |
| IPI00007775 | S.FMN#VSESHFVSALTVVFINSK.S | 0.6851 |
| IPI00007775 | K.QPKVGFYSSLN#QT.H | 0.622 |
| IPI00007778 | K.QIN#SSISGNLWDKDQR A | 0.9991 |
| IPI00007798 | R LLN#LSLNSEVVLDQDAIDVIIHVAR N | 0.9597 |
| IPI00007798 | K.NNFN#GSLVQASYQHEELR.R | 0.9406 |
| IPI00007818 | R.NFNYHILSPCDLSN#YTDLAMSTVK.Q | 0.9536 |
| IPI00007818 | M.VVLEWLAN#PSNDMYADTVTTVILEVQSNPKIR.K | 0.6691 |
| IPI00007834 | T.VAPQGQDM*ASIAPDN#RSK.S | 0.5314 |
| IPI00007843 | R.NPDM*EVDEN#GTLDLSMNKQR.P | 0.8337 |
| IPI00007858 | C.LIPN#ETKTPGVMDHYLVM*HQLRCNGVLEGIR.I | 0.6604 |
| IPI00007927 | K.YLINGVNAN#NTRVQDLFCSV.G | 0.533 |
| IPI00007941 | R YHTESLQN#M*S.K | 0.7632 |
| IPI00007979 | M.AILFNNMLSGQWTMTN#TTNQYSSLM*IMM*AMAMK L | 0.5334 |
| IPI00008052 | E.EADVDM*EPN#VSVYSGLK.E | 0.6963 |
| IPI00008085 | A.HNHHGEN#KTVLR.K | 0.6903 |
| IPI00008091 | K.NKISIEDLLQSSM*GSTQQAQN#TTSSLMNLVM*QFR.K | 0.9063 |
| IPI00008129 | R.N#M*SQLM*ETGEVSDDLASQLIYQLVAELAK.A | 0.896 |
| IPI00008129 | L.FN#GSLLLQN#VSLENEGTYVCIATNALGK.A | 0.5277 |
| IPI00008135 | R.DYKQTGDN#LSSMLLEN#LTDN#ESENTNLKKK.V | 0.9339 |
| IPI00008135 | K.INFENAN#LSALNLK I | 0.6945 |
| IPI00008161 | R QDAVVAVTGDGVN#DSPALKK.A | 0.7642 |
| IPI00008198 | K LLYNLRASLNKN#QSSR II | 0.5161 |
| IPI00008226 | K LPGLAN#TTLSTPNPDTQASASPDPR P | 0.5447 |
| IPI00008274 | K KWRVENQEN#VSNLVIEDTELK Q | 0.5781 |
| IPI00008283 | K.TLDLQSGLKDITGN#KSEM*IEK.P | 0.6912 |
| IPI00008334 | Y.DLKEGLLVSPGSVIM*N#GSNMAN#TSPSVKSK.E | 0.6222 |
| IPI00008372 | K.EASLADN#NTDVRLIGEKLFHGVSM*SER C | 0.9182 |
| IPI00008372 | R LDKSNFQQPYITN#RTFML.A | 0.5419 |
| IPI00008454 | M.APQN#LSTFCLLLLYLIGAVIAGR.D | 0.6872 |
| IPI00008490 | K MLM*GIN#VTPIAALLYTPVLIR F | 0.6937 |
| IPI00008491 | R LN#PTVTYGN#DSFSAK.A | 0.9995 |
| IPI00008494 | R.AN#LTVVLLR.G | 0.9973 |
| IPI00008522 | G IDITSLHSHN#GSPLTSK N | 0.5483 |
| IPI00008522 | K IIGNSVGALGN#LTIILAIIVFVFALVGK Q | 0.9389 |
| IPI00008556 | K LEITVN#YTDSQRPICIPSK.G | 1 |
| IPI00008556 | R VYSGILN#QSEIK.E | 1 |
| IPI00008556 | K GINYN#SSVAK S | 0.9987 |
| IPI00008558 | R.GVNFN#VSK.V | 0.9985 |
| IPI00008558 | R IVGGTN#SSWGFWPWQVSLQVK L | 1 |

| | | |
|---|---|---|
| IPI00008558 | R.IYSGILN#LSDITK.D | 1 |
| IPI00008558 | K.IYPGVDFGGEELN#VTFVK G | 1 |
| IPI00008558 | K.LQAPLN#YTEFQK.P | 0 9983 |
| IPI00008569 | K.EQDYLCHVYVRN#DSLAGVVIADNEYPS.R | 0.5379 |
| IPI00008588 | K.TN#DTYMKFSWLTVPEESLDKEHR C | 0.5907 |
| IPI00008632 | T.M*NPLIYN#ITR.V | 0.6089 |
| IPI00008787 | R.VFPQVN#VTK.M | 0 9774 |
| IPI00008822 | M.ATYSATCAN#NSPAQGINMANSIANLRLK A | 0.8428 |
| IPI00008829 | R.AYYGNINFFGGPSN#TSV.K | 0.5929 |
| IPI00008868 | R DVMSDETNNEETESPSQEFVN#ITK.Y | 0 813 |
| IPI00008884 | D.DDLIISQDTDIIQDMVAGEN#TSEAGSEDEGEVSLPEQPK.V | 0.9406 |
| IPI00008887 | K.MDIEN#LTISNAQ.M | 0.5802 |
| IPI00008905 | M.ISN#MSEESANM*IASALAQIPQKVLWR.F | 0 914 |
| IPI00008909 | R.ILSN#M*TFLFVSLSYTAESAIVTAFITFI.P | 0.7843 |
| IPI00008913 | R.NRHDLLN#VSQGTVFIFWGPSSYMR.R | 0.7151 |
| IPI00008918 | A.VSKQSSSTN#YTNELK A | 0.8421 |
| IPI00008918 | R.SNTEN#LSQHFR.K | 0.6229 |
| IPI00008942 | R.TCYYPTTVCLPGCLN#QSCGSS C | 0.8511 |
| IPI00008982 | T.KSRVGMGGMEAKVKAALWALQGGTSVVIAN#GTHPK.V | 0 7814 |
| IPI00008982 | R.NLN#GTLHELLRM*NIVPIVNTNDAVV.P | 0.6147 |
| IPI00008993 | K.SVNKMQEATPSAQATN#ETQM*CYASLDHSVK G | 0.6581 |
| IPI00009009 | M.ENILSGNPLLN#LTGPSQPQANFK V | 0.6902 |
| IPI00009030 | R.VQPFN#VTQGK.Y | 0.8397 |
| IPI00009030 | K.IAVQFGPGFSWIAN#FTK.A | 1 |
| IPI00009030 | K VASVININPN#TTHSTGSCR.S | 0.9999 |
| IPI00009030 | K.WQMN#FTVR.Y | 0.9569 |
| IPI00009054 | R.KLYKCPACGETLQDSTGN#FSSPEYPNGYSAHM H | 0.728 |
| IPI00009101 | R.AETQGAN#HTPVISAHQTR.S | 0.8619 |
| IPI00009135 | K.ANQQLN#FTEAK E | 0.9998 |
| IPI00009137 | R.TPQVIGVMQSQN#SSAGNR.G | 0.6672 |
| IPI00009143 | K.YMISTSETIIDIN#GTVMN#YSGWSHR.D | 0 5276 |
| IPI00009149 | K.RAM*N#KSFM*ESGGTVLSTN#WSDVGKRK.V | 0.9192 |
| IPI00009243 | K.FCVVLLHWEFIYVITAFN#LSYPITPWR.F | 0.9082 |
| IPI00009268 | K DM*N#LTLEPEIM*PAATDNRYIR A | 0 7641 |
| IPI00009291 | R.GLN#SSFETSPKK.V | 0.7305 |
| IPI00009329 | R IPRADELN#QTGQILVEQMGK.E | 0.7349 |
| IPI00009477 | . K.HYLVSN#ISHDIVLQCH.F | 0.9946 |
| IPI00009477 | R GN#ETLHYETFGK.A | 0.9998 |
| IPI00009477 | K.AAPAPQEATATFN#STADR E | 0.9997 |
| IPI00009499 | R ESLASYLSLTSFDN#TSFDRKK.K | 0 8712 |
| IPI00009504 | R.KGIIDVNLYN#ETVETLMAG E | 0.5421 |
| IPI00009521 | R.VDN#ITQNPGM*FR I | 0.9997 |
| IPI00009604 | R-SSN#SSVSGTKKFDSTAKIH.A | 0.7484 |
| IPI00009612 | R NFDN#SSQN#TTASVSSKGPM*ILLQAT K | 0.7958 |
| IPI00009618 | R.LM*LPDDITN#HSN#SSK E | 0 7359 |
| IPI00009631 | R N#SSLGDAINKYDVVIRLNNAPVAG Y | 0 6899 |
| IPI00009646 | R RLRELAGN#SSTPPPVSPGRGNPM*HRLLNP.F | 0.7324 |
| IPI00009655 | C FPTLSDFLTEIN#STVDK.D | 0 8633 |
| IPI00009703 | M.SQHYQSGPVPGTAIN#GTLPLS H | 0.8971 |
| IPI00009704 | R VVSN#SSVLASQSVGITNVRT V | 0 7275 |
| IPI00009791 | K.KGIXGLQLP.AADGAAASNAADSAN#ASLVNGK M | 0 8429 |

46

| | | |
|---|---|---|
| IPI00009791 | K.QN#SSPPSSLNKN#NSAIDSGIN#LTTDTSK.S | 0.6347 |
| IPI00009793 | R.KN#QSVNVFLGHTAIDEMLK.L | 0.9367 |
| IPI00009793 | K.GFLALYQTVAVN#YSQPISEASR.G | 1 |
| IPI00009793 | R.QDGEEVLQCM*PVCGRPVTPIAQN#QTTLGSSR.A | 0.9998 |
| IPI00009793 | N.VLPVCLPDN#ETLYR.S | 0.827 |
| IPI00009802 | K.N#SSTAEIN#ETTTSSTDFLARAYGFEMAKE.F | 0.9241 |
| IPI00009803 | K.M*N#LTFHVINTGNSMAPN#VSVEIM*VPNSFSPQTDK.L | 0.8668 |
| IPI00009803 | K.TLMLN#VSLFNAGDDAYETTLHVK.L | 0.6288 |
| IPI00009804 | K.EHSEMSNN#VSDPKGPPAKIAR.L | 0.687 |
| IPI00009804 | R.NGKPEN#NTMNIN#ASIYDEIQQEMK.R | 0.6373 |
| IPI00009822 | K.GLFKGGDMSKN#VSQSQMAK.L | 0.5094 |
| IPI00009841 | M.GVYGQESGGFSGPGEN#RSMSGPDNRGR.G | 0.6221 |
| IPI00009861 | -.MDPN#CSCAAGVSCTCASSCKCKECK.C | 0.644 |
| IPI00009865 | K.TIDDLKNQILN#LTTDNANILLQIDNAR.L | 0.9999 |
| IPI00009896 | M.ALVLSN#FSTLTLLLGQR.F | 0.9324 |
| IPI00009906 | I.VLNN#LSVNAEN.Q | 1 |
| IPI00009910 | K.HLDLSSNLLKTIN#KSALETK.T | 0.79 |
| IPI00009910 | K.KQN#DSVIAECSNRR.L | 0.6285 |
| IPI00009913 | M.FSLITWNIDGLDLNN#LSER.A | 0.5047 |
| IPI00009920 | K.VLN#FTTK.A | 0.9934 |
| IPI00009920 | R.TRLSSN#STK.K | 0.9678 |
| IPI00009961 | K.EGEHDLVQGSGQQPQAGLSQAN#FTLGPVSR.S | 0.9394 |
| IPI00009992 | K.IGHPHGLQVTYLKDN#STR.N | 0.6302 |
| IPI00009995 | K.GVARVVN#ITSPGHDASSR.S | 0.6514 |
| IPI00009997 | R.VAQPGINYALGTN#VSYPNNLLR.N | 0.9618 |
| IPI00010037 | K.HTGPGILSM*ANAGPNAN#GSQFFM*CPA.K | 0.7553 |
| IPI00010065 | R.NPFHHSLPFSIPVHFIN#GTYHVVGFDGSSTVDEFLQR.L | 0.623 |
| IPI00010088 | M.PIASEFAPDVVLVSSGFDAVEGHPTPLGGYN#LSAR.C | 0.8827 |
| IPI00010134 | N.#CTCVGIAASKSGN#SSGIVGRCQK.D | 0.6131 |
| IPI00010141 | R.IIKEALPDGVN#ISK.E | 0.5035 |
| IPI00010193 | G.N#MSGN#FTYIIDK.L | 0.9081 |
| IPI00010196 | L.LSN#KTNAVEENK.A | 0.5128 |
| IPI00010196 | R.SPYNSHM*GNN#ASRPHSANGEVYGLLGSVLTIKK.E | 0.8051 |
| IPI00010213 | M.EVCNN#ETISVSSYK.I | 0.8865 |
| IPI00010221 | R.N#CTTLQGLAPGTAYLVTVTAAFRSGR.E | 0.6559 |
| IPI00010250 | K.GTN#SSASSNFRCR.S | 0.8811 |
| IPI00010272 | R.NSKN#CTEPALHFFPNDIFTNEDRR.Q | 0.9345 |
| IPI00010281 | K.TN#GTLLRNGGIPGGPNKIPNGDICCIPNSNLDK.A | 0.7153 |
| IPI00010286 | F.NEHMIN#STMSPGTVGQSLK.S | 0.8811 |
| IPI00010286 | K.QLNVQMN#MSNVMGN#TTWTTSGLK.S | 0.7521 |
| IPI00010381 | R.NKEVN#ISAVVWPS.Q | 0.5495 |
| IPI00010421 | K.KQIN#DSANLR.E | 0.5543 |
| IPI00010433 | R.SM*NPN#VSAIVSSTSSSPSSSK.T | 0.6833 |
| IPI00010448 | K.ATMGLLQNKLNN#NTKDSPSR.Q | 0.6608 |
| IPI00010463 | K.LLQTTN#NSPM*NSKP.Q | 0.5738 |
| IPI00010487 | K.RVN#QSMVTAEIQR.L | 0.5103 |
| IPI00010540 | R.LDN#ITQVM*SLHFQYLESFLR.S | 0.6334 |
| IPI00010540 | K.FRM*VVYN#LTYNTM*ATHCDVDTTMI.R.R | 0.9082 |
| IPI00010625 | M.NN#NSGAPATAPDSAGQPPALGPVFELVSK.E | 0.5206 |
| IPI00010676 | R.GPM*NQCLVATGTHEPKN#QSYMVR.G | 0.9334 |
| IPI00010700 | K.ETPPNGN#LSPAPRLR.R | 0.6319 |

47

| | | |
|---|---|---|
| IPI00010728 | R.GVSGDRDENSFSLN#SSISSSAR.R | 0.6981 |
| IPI00010790 | M.IEN#GSLSFLPTLR.E | 0.9074 |
| IPI00010807 | Y.N#RTDLTTAAPSPPR.R | 0.9763 |
| IPI00010862 | K.GTGSWTQLYLITDYHEN#GSLYDYLK.S | 0.5774 |
| IPI00010903 | R.LINLYIIQN#NSFS.Q | 0.5246 |
| IPI00011031 | M.GINECQYQFRFGRWN#CSALGEK.T | 0.7317 |
| IPI00011041 | -.MDGDN#QSENSQFLLLGISESPEQQR.I | 0.5781 |
| IPI00011092 | M.TSGN#ISVSWPATK.E | 0.7217 |
| IPI00011092 | M.LSSSSEMNEEFLKEN#NSVEYKKSK.A | 0.5895 |
| IPI00011155 | R.SLKEAFSN#FSSSTLTEVQAISTHGGSVGDK.I | 0.6585 |
| IPI00011155 | R.FVACQM*ELLHSN#GSQR.T | 0.9947 |
| IPI00011168 | R.GISARVWGHFPKWLN#GSLLRIG.P | 0.6584 |
| IPI00011177 | Y.LAN#LTQSQIALNEKR.V | 0.7331 |
| IPI00011180 | K.AYTDFQNN#HSSPK.P | 0.7463 |
| IPI00011218 | K.VLTLNLDQVDFQHAGN#YSCVASNVQGK.II | 0.9379 |
| IPI00011218 | R.HTN#YSFSPWHGFTIHR.A | 0.9994 |
| IPI00011218 | K.VM*VEAYPGLQGFN#WTYLGPFSDHQPEPK.L | 0.9985 |
| IPI00011219 | R.HSSTDSNKASSGDISPYDN#NSPVLSER.S | 0.9822 |
| IPI00011229 | K.GSLSYLN#VTR.K | 0.9947 |
| IPI00011252 | R.GGSSGWSGGLAQN#R.S | 0.9998 |
| IPI00011255 | K.VASHLEVNCDKRN#LTALPPDLPK.D | 0.9977 |
| IPI00011264 | R.SPYEM*FGDEEVMCLNGN#WTEPPQCK.D | 0.9927 |
| IPI00011285 | K.RDFFLAN#ASRARSEQFINLR.E | 0.5852 |
| IPI00011374 | R.LECN#GTISAHCNLHLPGSSDSPASSSRVAGITGIK.T | 0.892 |
| IPI00011528 | K.SAMPIEVMMN#ETAQQNMENHPVIR.F | 0.9028 |
| IPI00011538 | R.KKN#LTLALEALVQLR.G | 0.8122 |
| IPI00011578 | C.N#ATNAIGSASVVTVLR.V | 0.6967 |
| IPI00011601 | K.AN#MTLTSGIMFIVSGLCAIA. | 0.981 |
| IPI00011609 | K.GASSAYLENSKGAPN#NSCSEIKM*NK.K | 0.8334 |
| IPI00011651 | R.SDFSQTM*LFQAN#TTR.I | 0.9999 |
| IPI00011651 | K.VEFHWGIISN#GSAGSEHSINGR.R | 0.9516 |
| IPI00011651 | S.GVTHAAEERN#QTEPSPTPSSPN#R.T | 0.7968 |
| IPI00011651 | K.NRN#SSVVPSERARVGL.A | 0.5831 |
| IPI00011665 | K.DFLN#VTTEANIL.P | 0.5409 |
| IPI00011730 | K.VNLNSVSKSLTGLSDSVSQYSDAFLAAN#TSLDER.E | 0.6698 |
| IPI00011756 | R.SAN#LTDQPSW.N | 0.7055 |
| IPI00011757 | R.KLN#PSQN#ATGTSRS.E | 0.51 |
| IPI00011798 | K.RN#ASSSSHSSTEGLQELK.R | 0.526 |
| IPI00011836 | R.YVKQPI.PDEFGSSPLLPGACN#GS.R | 0.748 |
| IPI00011879 | K.VN#GSHEANMLSQVHR.- | 0.6038 |
| IPI00011989 | Q.VGIYN#GTHVIPNDR.K | 0.9222 |
| IPI00012009 | M.N#LSWDCQFN#TTFSKCFLTDK.K | 0.5996 |
| IPI00012033 | R.RPLVLQLVN#ATTEYAEFLHCK.G | 0.5378 |
| IPI00012058 | E.EYKNYLDAAN#MSMRVR.R | 0.6311 |
| IPI00012113 | R.TLPLILILIALLSPGAADFN#ISSLSGLLSPALT.E | 0.5196 |
| IPI00012136 | R.RRGRPRGNN#LSTISDTSPMKR.S | 0.6504 |
| IPI00012136 | M.GN#STDPGPM*IAIPAMATNPQNAASR.R | 0.8807 |
| IPI00012165 | R.VVLLDPKPVAN#VTCVNK.H | 0.5881 |
| IPI00012221 | F.QTYHMALNAATFFKN#ATWIGPLW.- | 0.797 |
| IPI00012269 | K.FNPGALSVVLSN#STI.K.F | 0.1 |
| IPI00012269 | K.LQN#LTLPTN#ASIK.F | 0.9972 |

| IPI00012318 | K.CRLDVNTELN#SSIEDLLEASMPSSD.T | 0.9028 |
|---|---|---|
| IPI00012363 | K.SLM*DQLQGVVSN#FSTAIPDFHAVLAGPGGPGNGLR.S | 0.8632 |
| IPI00012390 | M.KKVHVNSVNPN#YTGGEPK.R | 0.9153 |
| IPI00012391 | R.QM*SQQN#LTK.Q | 0.7042 |
| IPI00012471 | K.MSHPPNIPKEQTPAGTSN#TTSVSV.K | 0.5742 |
| IPI00012488 | K.VTGSGGPFKSDPHWESMLN#ATTR.R | 0.8033 |
| IPI00012503 | R.TN#STFVQALVEHVKEECDR.L | 0.9923 |
| IPI00012503 | R.NLEKN#STKQEILAALEK.G | 0.6514 |
| IPI00012508 | R.YLINSYDFVN#DTLSLK.H | 0.678 |
| IPI00012519 | V.YYM*VVCLVAFTIVMVLN#ITR.L | 0.917 |
| IPI00012545 | K.DGSN#KSGAEEQGPIDGPSKSGAEEQTSK.D | 0.6523 |
| IPI00012574 | A.MVN#TTQQQGLSN#ASTEGPVADAFN#NSSISIK.E | 0.9076 |
| IPI00012578 | R.VQN#TSLEAIVQN#ASSDNQGIQLSAVQAAR.K | 0.5783 |
| IPI00012585 | K.LDSFGPIN#PTLN#TTYSFLTTFFK.E | 0.9903 |
| IPI00012728 | K.RKEAELRSGIIRN#NSLWDR.L | 0.5083 |
| IPI00012730 | G.AASYFLILDSTNTVPDSAGSGN#VTR.C | 0.562 |
| IPI00012773 | K.AGVVN#GTGAP.G | 0.7917 |
| IPI00012773 | K.VAPVINN#GSPTILGKR.S | 0.5426 |
| IPI00012792 | R.EVYPWYN#LTVEAK.E | 0.9992 |
| IPI00012792 | R.LDREN#ISEYHLTAVIVDK.D | 1 |
| IPI00012792 | R.AQVIIN#ITDVDEPPIFQQPFYHFQLK.E | 1 |
| IPI00012828 | K.DGSTTAGN#SSQVSDGAAAILLARR.S | 0.7344 |
| IPI00012843 | L.SSLSPVN#SSNHGPVSTGSLTN#R.S | 0.8791 |
| IPI00012876 | R.N#RSFQPGLDNIIFVVETGPL.P | 0.718 |
| IPI00012885 | M.ADLIDGYCRLVN#GTSQSFIIRPQK.E | 0.56 |
| IPI00012887 | K.YSVAN#DTGFVDIPK.Q | 0.5022 |
| IPI00012891 | R.CEGSQPWN#LTPR.Q | 0.9283 |
| IPI00012990 | L.IAAGVAHAITAACTHGN#LSDCGCDKEK.Q | 0.6049 |
| IPI00013010 | D.LGNVPN#GSALTDGSQLPSR.D | 0.5082 |
| IPI00013049 | K.VSRIPQGTFSNLEN#LTLLDLQNNK.L | 0.879 |
| IPI00013096 | N.#ISRLDPQTN#SSQIKDEFQTLNIVTPR.V | 0.5466 |
| IPI00013174 | M.SQGAVANAN#STPPPYERTR.L | 0.7707 |
| IPI00013177 | K.RVYSLMEN#NSYPRFLCSEFYQDLCK.K | 0.8289 |
| IPI00013179 | R.WFSAGLASN#SSWLR.E | 1 |
| IPI00013179 | K.SVVAPATDGGLN#LTSTFLR.K | 1 |
| IPI00013226 | R.RGASVN#RTTRTN#STPLR.A | 0.7364 |
| IPI00013234 | K.LKLFLN#ETQTQEITEDIPVKTLNM*K.T | 0.6948 |
| IPI00013299 | K.SAWCEAKN#ITQIVGHSGCEAK.S | 0.838 |
| IPI00013303 | K.ISN#ISSDVTVN#EGSN#VTLVCMANGR.P | 0.5254 |
| IPI00013409 | R.YTVSN#LSMQTIIAARFK.T | 0.5953 |
| IPI00013414 | K.LQDIFYPN#TSNCAK.G | 0.716 |
| IPI00013436 | K.LQINN#LTMNLIELEN.- | 0.725 |
| IPI00013437 | K.AFSN#SSTLANIIKITHTEEKPYKCK.E | 0.5749 |
| IPI00013441 | R.THKM*N#VSPVPPLR.R | 0.6658 |
| IPI00013452 | N.#ISSN#SSASILESK.S | 0.5918 |
| IPI00013492 | R.SGTN#IYSTSSCTPPAN#GTDSIMANR.G | 0.5103 |
| IPI00013624 | K.EYPN#LSTSLDDATLLR.F | 0.9721 |
| IPI00013712 | R.LLQMPSVVN#YSGLRK.R | 0.5647 |
| IPI00013743 | R.RARHDSPDLAPN#VTYSLPRTK.S | 0.7072 |
| IPI00013744 | V.AIVYN#ITLDADGTSSR.V | 0.7322 |
| IPI00013877 | R.MGM*GNN#YSGGYGTPDGLGGYGRGIGGSGGYYGQGGMSGGGWR.G | 0.9604 |

| | | |
|---|---|---|
| IPI00013880 | W.TPVN#ISDNGDHYEQR.F | 0.5364 |
| IPI00013892 | K.GEGLN#KTVIGDYLGERDEFNIK.V | 0.7954 |
| IPI00013928 | M.N#NSESHFVPNSLIGMGVLSCVFNSLAGK.I | 0.6737 |
| IPI00013967 | R.LN#M*TTEQFTGDHTQHFLDGGE.M | 0.527 |
| IPI00013970 | T.DCGHTWNSPN#CTDPKLLN#GSVLGN#HTK.Y | 0.5064 |
| IPI00013972 | M.SEEVTGQFSVHPETPKPSISSN#NSNPVEDK.D | 0.7948 |
| IPI00014011 | K.DLYRSN#ISPLTSEKDLDDFR.R | 0.9479 |
| IPI00014053 | K.GLSNHFQVN#HTVALSTIGESNYHFG.V | 0.8905 |
| IPI00014072 | K.RRPLNN#NSEIALS.L | 0.7144 |
| IPI00014147 | K.VLGSSTSATN#STSVSSR.K | 0.971 |
| IPI00014186 | K.ASASPGEN#DSGTGGEEPQRDK.R | 0.601 |
| IPI00014194 | M.CVKN#STGVEIK.R | 0.9649 |
| IPI00014202 | V.N#LTGLDLSQNN#LSSVTNINVKK.M | 0.9364 |
| IPI00014211 | G.KSVLVN#GTKER.D | 0.6008 |
| IPI00014312 | R.DM*SISN#TTM*DEFR.Q | 0.8411 |
| IPI00014319 | G.HKQISSSSTGCLSSPN#ATVQSPK.H | 0.6699 |
| IPI00014335 | R.DATGNMN#DTIISGM*NCN#GSAACGLGYD.F | 0.9396 |
| IPI00014456 | R.HIKFYDN#NTGK.L | 0.8422 |
| IPI00014502 | E.PCYVSASEIKFDSQEGSVDQN#HSWLGRKRR.N | 0.6144 |
| IPI00014544 | T.NN#ISLMATLK.A | 0.6331 |
| IPI00014553 | M.QSQAGGN#NTGSTPLR.K | 0.75 |
| IPI00014802 | A.VSQN#WTFHGPGASGQAAANWLAGFGR.G | 0.847 |
| IPI00014829 | K.N#GTIFTISPVLLLDTISTTR.F | 0.8234 |
| IPI00014845 | M.ITVVQTYSTLSN#STIEGIDIMAIKFR.N | 0.8202 |
| IPI00014898 | M.NEILTDPSDDTKGFFDPNTEEN#LTYLQLM*ER.C | 0.6611 |
| IPI00015102 | R.TVNSLN#VSAISIPEHDEADEISDENR.E | 0.7966 |
| IPI00015102 | N.#LSEN#YTLSISNARISDEK.R | 0.9391 |
| IPI00015102 | K.NAIKEGDN#ITLK.C | 0.8636 |
| IPI00015115 | R.VEIISN#NSIQAVFN#PTGVYAPSGYSYR.C | 0.5105 |
| IPI00015283 | K.N#CTNN#CTFVYAAEQPPEAPGK.I | 0.7401 |
| IPI00015286 | K.WRSN#TSLLQQNLR.Q | 0.5223 |
| IPI00015309 | -.MDLSN#NTM*SLSVRTPGLSR.R | 0.5004 |
| IPI00015345 | P.GAYN#NTALFEESGLIR.I | 0.9034 |
| IPI00015467 | K.N#QSASPPPKDRSSSPATEQSWTQ.N | 0.6481 |
| IPI00015488 | R.DLDGFLAQASIVLN#ETATSLDNVLRTMLRR.F | 0.9121 |
| IPI00015508 | V.QDSVN#ISGHTNTNTLK.V | 0.748 |
| IPI00015525 | F.GNFQGLMEAN#VSLDLGK.L | 0.9741 |
| IPI00015525 | K.FN#TTYINIGSSYFPEHGYFR.A | 1 |
| IPI00015525 | R.SFN#QSLHSLTQAIR.N | 0.7485 |
| IPI00015553 | V.YN#PSGLN#LSIK.G | 0.833 |
| IPI00015573 | R.QLCGLLLGGGGN#RSIISTPYCGLR.R | 0.7096 |
| IPI00015688 | C.CTSEMLENLAN#R.S | 0.8297 |
| IPI00015745 | K.TGTTLN#TSIIFGPN#LS.- | 0.6054 |
| IPI00015749 | K.NFAQNRGAGN#TSSLNPLAVGFVQTPPVISSAHQDER.V | 0.9563 |
| IPI00015782 | R.SLSTPNALSFGSPTSSDDM*TLTSPSM*DN#SSAE.L | 0.8927 |
| IPI00015830 | K.FCHSQLSN#NSVSFFLYNLDHSHAN*YFCN.I | 0.5405 |
| IPI00015902 | D.AYYVYRLQVSSIN#VSVNAVQTVVR.Q | 0.5157 |
| IPI00015902 | R.SHHIPSAELEDSGTYTCN#VT.E | 0.5395 |
| IPI00015911 | A.LLN#NSHYYHM*AHGTDFASR.G | 0.8476 |
| IPI00015952 | R.DDNSAAN#NSANEKER.H | 0.5125 |
| IPI00015963 | K.LMISSYSGSVDIVN#TFDGCH.I | 0.6129 |

| | | |
|---|---|---|
| IPI00015980 | R.LM*QGDQILM*VNGEDVRN#ATQEAVAALLK.C | 0.5682 |
| IPI00015990 | K.GQNLNN#YSFSTNGFSGSGGGSGSHGS.S | 0.695 |
| IPI00015994 | K.QQN#HTLDYNLAPGPLGR.G | 0.7563 |
| IPI00016006 | R.EAPGM*ALAMLMGSLN#VTP.L | 0.7394 |
| IPI00016053 | R.RLSLN#QSR.G | 0.9005 |
| IPI00016095 | K.ELVNAGCN#LSTLN#ITLLSWSKK.R | 0.9813 |
| IPI00016339 | K.LPKNEPQN#ATGAPGR.N | 0.5492 |
| IPI00016371 | K.MEDGLLTCHGPGPDN#CTKCSHFK.D | 0.6301 |
| IPI00016422 | R.EGDNRERALN#TTQPGSLQLTVGNLK.P | 0.7916 |
| IPI00016454 | R.VMVHGRN#HTPFLGHHS.F | 0.5467 |
| IPI00016475 | R.LQQLAEPQSDLEELKHEN#K.S | 0.515 |
| IPI00016480 | K.ENLPEN#VTASESDAEVER.S | 0.5715 |
| IPI00016488 | D.YNVQTSN#WTR.T | 0.6482 |
| IPI00016542 | R.LKN#ISENADFFASLQLSESAARLREM.I | 0.9726 |
| IPI00016553 | K.ASLCLPTTSAPASAPSNGN#CS.S | 0.9822 |
| IPI00016589 | K.EN#STASEVLDSLSQSVHVKPENLR.L | 0.5232 |
| IPI00016590 | R.SIQKLGELNIGM*DGLGNEVSALNQQCN#GSK.G | 0.5157 |
| IPI00016633 | R.HVLATILAQLSDMDLIN#VSK.V | 0.7999 |
| IPI00016637 | R.FIGLTNSFGFGGTN#A.T | 0.5093 |
| IPI00016645 | R.PPSAPQNLIFNIN#QTTVSLEWSPPADNGGR.N | 0.5488 |
| IPI00016645 | K.DN#FTAAGYNSLES.V | 0.5271 |
| IPI00016665 | K.HQVEALKNMQHQN#QSLSM*LDEILEDVRK.A | 0.6939 |
| IPI00016677 | K.SEM*AQIQQNAVQN#HTATMLEIGTSLLSQTAEQTRK.L | 0.5277 |
| IPI00016677 | R.DCADVYQAGFN#KSGIYTIYINNM*PEPK.K | 0.8654 |
| IPI00016701 | K.RGN#TTLESTD.T | 0.5684 |
| IPI00016709 | K.RLVSMNM*PLNSDGTVMFN#ATLFALVRTALRIK.T | 0.9595 |
| IPI00016709 | K.CAPESEPSN#STEGETP.C | 0.5736 |
| IPI00016709 | K.LM*GSAGN#ATISTVSSTQRKR.Q | 0.55 |
| IPI00016780 | -.MNQN#TTEPVAATETLAEVPEHVLR.G | 0.5736 |
| IPI00016783 | R.SQSSSQSPASHRN#PTGAHSSSGHQSQSPN#TSPPPKRHK.K | 0.5254 |
| IPI00016890 | K.EPHLN#YSPTCLEPPVLSIHPGAID.- | 0.9034 |
| IPI00016906 | R.GATAAVLAPDSSN#ASSEPSS.- | 0.8257 |
| IPI00016906 | K.VKM*VVSREEVELAYQEAMFNMATLN#RTAAGLMIIT.F | 0.7829 |
| IPI00016949 | R.M*FTNPDN#GSPAM*TIIRN.L | 0.6304 |
| IPI00017025 | M.ANAGPNTN#GSQCFICTAK.T | 0.6715 |
| IPI00017070 | R.DPNIEALNGN#CSDTEIHEK.E | 0.571 |
| IPI00017094 | M.VISPSGFTASPYEGEN#SSNIIPQQM*AAIIMLR.S | 0.606 |
| IPI00017094 | M.N#STDIQWSAILSWGYADNILRLK.S | 0.6979 |
| IPI00017163 | R.STHSN#TTNPIWHR.E | 0.8535 |
| IPI00017174 | R.PQSN#SSAVTGTSGSIM*FNGVSSSNTADK.S | 0.5115 |
| IPI00017203 | R.LIGEPDLVVSVIPN#NSNENIPR.V | 0.6294 |
| IPI00017234 | R.RNAEWIIVHM*M*EYYAAENMNN#WSHGM*NEAER.F | 0.5073 |
| IPI00017373 | K.M*FILSDGEGKN#GTIELMEP.L | 0.9304 |
| IPI00017381 | R.VLELN#ASDFRGIQVVREK.V | 0.9423 |
| IPI00017390 | R.WEYCN#LTR.C | 0.9997 |
| IPI00017405 | K.VLLN#SSVPPAGAEELSSAMANPPPKR.P | 0.7221 |
| IPI00017480 | R.LLLTAAHLLFVAPHN#DSATGEPEASSGSGPPSGGALGPR.A | 0.9963 |
| IPI00017522 | M.ILAVVFGN#VTAIIQR.M | 0.7185 |
| IPI00017562 | K.GDYN#DSVQVVDCGLSLN#DIAFFK.M | 0.5841 |
| IPI00017601 | K.LEFALLFLVFDEN#ESWYLDDNIK.T | 0.9879 |
| IPI00017601 | K.AGLQAFFQVQECN#K.S | 1 |

51

| | | |
|---|---|---|
| IPI00017601 | K.EN#LTAPGSDSAVFFEQGTTR.I | 1 |
| IPI00017601 | K.EHEGAIYPDN#TTDFQR.A | 1 |
| IPI00017601 | K.ELHHLQEQN#VSNAFLDK.G | 1 |
| IPI00017601 | R.QKDVDKEFYLFPTVFDEN#ESLLLEDNIR.M | 0.9993 |
| IPI00017603 | R.NLFLTNLDNLHEN#NTHNQEK.K | 0.829 |
| IPI00017617 | R.LM*EEIMSEKEN#KTIVFVETK.R | 0.6564 |
| IPI00017640 | R.HLTLIDLSN#NSISMLTN#YTFSN#M*S.H | 0.5467 |
| IPI00017640 | R.HLTLIDLSN#NSISM*LT.N | 0.7484 |
| IPI00017648 | K.FGLYHVDFN#NTNRPR.T | 0.9931 |
| IPI00017696 | K.TM*QEN#STPRED.- | 1 |
| IPI00017696 | K.NCGVN#CSGDVFTALIGEIASPNYPK.P | 0.9996 |
| IPI00017696 | H.CAGN#GSWVNEVLGPELPK.C | 0.7099 |
| IPI00017734 | K.GLGAQTGVLRM*KGVN#LS.C | 0.6495 |
| IPI00017818 | A.EESPFVGNPGN#ITGAR.G | 0.9891 |
| IPI00017841 | K.VQN#M*SQSIEVLDR.R | 0.9999 |
| IPI00017919 | R.ESNAPSVPTVSLLPGAPGGN#ASS.R | 0.6685 |
| IPI00017940 | M.EEVRKVN#ESIK.Y | 0.55 |
| IPI00017964 | M.SN#ITVTYR.D | 0.9826 |
| IPI00018071 | R.EALN#ISSSISESGGLNWKM*.T | 0.7432 |
| IPI00018073 | R.DEDTLQDPAPLETPM*N#ASSSHS.C | 0.7424 |
| IPI00018098 | K.N#ESKEKSNK.R | 0.7036 |
| IPI00018198 | M.AAANPWDPASAPNGAGLVLGHFIASGMVNQEMLN#MSKK.T | 0.5016 |
| IPI00018214 | K.N#HTHQQDIDDLKRQNALLEQQVR.A | 0.5199 |
| IPI00018251 | M.MRGQGLN#M*TPSMVAPSGM*PATMSNPR.I | 0.8896 |
| IPI00018287 | K.ALWN#SSVPVCEQIFCPNPPAILNGRH.T | 0.5438 |
| IPI00018305 | R.AYLLPAPPAPGN#ASESEEDR.S | 1 |
| IPI00018305 | K.VDYESQSTDTQN#FSSESK.R | 1 |
| IPI00018305 | R.GLCVN#ASAVSR.L | 1 |
| IPI00018313 | R.FLIEDINDNAPLFPATVIN#ISIPENSAINSK.Y | 0.6066 |
| IPI00018313 | R.YIVNPVN#DTVVLSENIPLNTKL.A | 0.6323 |
| IPI00018672 | R.VSGAVATAVLWVLAALLAMPVMVLRTTGDLEN#TTK.V | 0.8758 |
| IPI00018678 | F.VQN#CTSLNSLNEVIPTDLQSK.F | 0.7695 |
| IPI00018810 | K.VGSFGN#GTVLR.S | 0.7229 |
| IPI00018860 | K.FFLIIYDCGN#KTVTPVSPLGKK.L | 0.9597 |
| IPI00018953 | R.IPN#NTQWVTWSPVGHK.L | 0.6221 |
| IPI00018953 | K.KLDFIILN#ETK.F | 0.9992 |
| IPI00018956 | K.NSDFYMGAGGPLEIIVM*ETLDN#ESFYSK.A | 0.824 |
| IPI00019006 | M.KYLN#LSSTR.I | 0.7613 |
| IPI00019020 | L.FQN#ITLEDAGSYILR.T | 0.5141 |
| IPI00019056 | R.DN#LSETASTM*ALAGASITGSLSGSAM*VNCFNR.L | 0.9179 |
| IPI00019148 | K.TMN#NSAEN#HTAN#SSMAYPSLVAM*ASQR.Q | 0.7877 |
| IPI00019157 | R.YVHDGSFLTDSFVLMAN#ASEMDR.Q | 0.7313 |
| IPI00019157 | R.GVN#ASAVVN#VTVRALLHVWAGGPWPQGATLR.L | 0.6896 |
| IPI00019223 | K.IISRIVELLN#ETEKYK.L | 0.64 |
| IPI00019226 | A.YLM*EPLCISSN#ESSEGCCPPSGIR.Q | 0.5861 |
| IPI00019226 | M.FQNAVMYN#SSDHDVYHMAVLM*QK.D | 0.7918 |
| IPI00019243 | P.RMSVLRSAETM*QSALAAMQQFYGIN#M*TGK.V | 0.8088 |
| IPI00019398 | A.IM*NN#MSLIIIR.S | 0.7765 |
| IPI00019311 | R.CFPWIN#ITPPALPGIFN#DTTIQQGISGLIDSLNAR.D | 0.9139 |
| IPI00019359 | K.N#YSPYYNTIDDLKDQIVDLTVGNNIK.F | 0.6174 |
| IPI00019391 | F.IIGLYEEKN#LSPGFNTR.I | 0.6242 |

| | | |
|---|---|---|
| IPI00019399 | R.VYLQGLIDYYLFGN#SSTVLEDSK.S | 0.9998 |
| IPI00019449 | R.CKNQNTFLLTTFANVVNVCGNPN#M*TCPSN#K.T | 0.6502 |
| IPI00019450 | T.PADVFIVFTDN#ETFAGGVHPAIALR.E | 0.6459 |
| IPI00019450 | R.VLGSILN#ASTVAAAMCM*VVTR.T | 0.5794 |
| IPI00019464 | K.DRN#ASNDGFEM*CSLSDFSANEQK.S | 0.5948 |
| IPI00019491 | K.QDYNMDLELDEYYN#KTLATEN#N.T | 0.7281 |
| IPI00019537 | R.SSINSVDGESPN#GSSDR.G | 0.576 |
| IPI00019568 | R.SEGSSVN#LSPPLEQCVPDR.G | 0.8766 |
| IPI00019568 | R.YPHKPEIN#STTHPGADLQENFCR.N | 1 |
| IPI00019568 | K.N#FTENDLLVR.I | 0.9816 |
| IPI00019568 | R.GHVN#ITR.S | 0.7855 |
| IPI00019571 | K.NLFLN#HSEN#ATAK.D | 1 |
| IPI00019571 | K.VVLHPN#YSQVDIGLIK.L | 1 |
| IPI00019571 | K.MVSHHN#LTTGATLINEQWLLTTAK.N | 1 |
| IPI00019580 | R.GNVAVTVSGHTCQHWSAQTPHTHN#R.T | 0.9931 |
| IPI00019581 | R.N#HSCEPCQTLAVR.S | 0.9997 |
| IPI00019581 | R.N#VTAEQAR.N | 0.9524 |
| IPI00019591 | R.SPYYN#VSDEISFHCYDGYTLR.G | 1 |
| IPI00019591 | K.IVLDPSGSMNIYLVLDGSDSIGASN#FTGAK.K | 1 |
| IPI00019591 | K.ALQAVYSM*M*SWPDDVPPEGWN#R.T | 0.9768 |
| IPI00019591 | R.GSAN#RTCQVNGR.W | 0.5636 |
| IPI00019600 | K.INMNGIN#NSSGMVDAR.S | 0.5128 |
| IPI00019729 | S.RGCN#VSR.K | 0.6694 |
| IPI00019772 | R.HRAGMQN#LTEFIGSEPSKKRKR.R | 0.57 |
| IPI00019943 | F.TTCCTLSEEFACVDNLADLVFGELCGVNEN#R.T | 0.7473 |
| IPI00019943 | R.DIENFN#STQK.F | 1 |
| IPI00019943 | R.YAEDKFN#ETTEK.S | 1 |
| IPI00019943 | K.HN#FSHCCSK.V | 0.9818 |
| IPI00019981 | K.QNSDHSN#GSFNLKA.L | 0.9833 |
| IPI00019983 | R.TEAPEGTESFMETPSAINGN#PSWHLAD.S | 0.6169 |
| IPI00019989 | R.ALQWNAGSGGLPEN#ETTFARL.Q | 0.6498 |
| IPI00020003 | R.RN#NSIRRN#NSSLMVPK.V | 0.8358 |
| IPI00020036 | K.LRWDPADYEN#VTSIR.I | 0.9655 |
| IPI00020078 | R.QFN#QTVQSSGN#MTDK.S | 0.5929 |
| IPI00020091 | V.PITN#ATLDR.I | 0.9855 |
| IPI00020091 | R.QNQCFYN#SSYLNVQR.E | 1 |
| IPI00020091 | K.SVQFIQATFFYFTPN#K.T | 1 |
| IPI00020091 | R.EN#GTVSR.Y | 0.9899 |
| IPI00020091 | R.NEEYN#K.S | 0.9747 |
| IPI00020094 | R.N#STELSEM*FPVLPGSH.L | 0.9587 |
| IPI00020122 | -.M*LVNGENFGVSLNIFPSVAIN#KSSGAPRR.V | 0.547 |
| IPI00020124 | K.DN#KSPLHLVQMPPVIVET.A | 0.8207 |
| IPI00020134 | R.QN#SSPHLPK.L | 0.9677 |
| IPI00020354 | R.N#TSPDTN#YTLYYWHR.S | 0.5808 |
| IPI00020366 | A.FNCPPN#STM*NR.G | 0.8296 |
| IPI00020368 | R.QFGKAPN#FSVN#WTVGDSAIEVIN#A.ITGK.D | 0.8377 |
| IPI00020396 | K.TGFFQLGTSCITN#HTCSNADETFCEMVK.S | 0.5834 |
| IPI00020407 | K.VDNLVVN#GTGTN#STN#STTAVPSLVALEK.I | 0.5666 |
| IPI00020416 | R.KQEEFDVANN#GSSQANK.I | 0.5453 |
| IPI00020426 | K.SPTSPTQNLFPASKTSPVNLPN#KSSI.P | 0.7398 |
| IPI00020501 | K.EIFN#LTQQYELK.A | 0.8717 |

| | | |
|---|---|---|
| IPI00020546 | R.MIEQYHN#HSDHYCLNLDSGM*VIDSYR.M | 0.5458 |
| IPI00020557 | R.INNGGCQDLCLLTHQGHVN#CSCR.G | 0.9991 |
| IPI00020557 | R.FN#STEYQVVTR.V | 0.9997 |
| IPI00020557 | R.M*HLN#GSNVQVLHR.T | 0.997 |
| IPI00020557 | R.ELQGN#CSRLGCQHHCVPTLDGPTCY.C | 0.8785 |
| IPI00020557 | K.DN#ATDSVPLRTGIGVQLKDIKVFNRDR.Q | 0.8689 |
| IPI00020557 | K.SDALVPVSGTSLAVGIDFHAEN#DTIYWVDMGLSTISRAK.R | 0.6213 |
| IPI00020586 | T.ELQLAAVETTANSLM*WILYN#LS.R | 0.6761 |
| IPI00020598 | R.ILKVAEFFN#YSKNR.I | 0.7997 |
| IPI00020692 | R.NSN#VSQASMSSR.M | 0.54 |
| IPI00020772 | T.GN#YTACQKDLCCHLTYKM*SEKR.T | 0.792 |
| IPI00020873 | K.WSPTGPATSNPN#SSIMLASASFDSTVR.L | 0.5122 |
| IPI00020903 | R.RRN#VSGNNGPFGQDKNIAM*TGQITSTKPKR.T | 0.5652 |
| IPI00020918 | K.AAHN#NSENIPLHK.S | 0.8707 |
| IPI00020966 | Y.N#QSTATTLFHSLPLLRYIFVRER.V | 0.9881 |
| IPI00020985 | M.GMNTGTNAGM*NPGMLAAGNGQGIMPNQVMN#GSIGAGR.G | 0.7024 |
| IPI00020986 | R.LSHNELADSGIPGNSFN#VSSLVELDLSYNK.L | 1 |
| IPI00020986 | K.LGSFEGLVN#LTFIHLQHNR.L | 1 |
| IPI00020986 | K.LHINHNN#LTESVGPLPK.S | 1 |
| IPI00020986 | K.AFEN#VTDLQWLILDHNLLENSK.I | 1 |
| IPI00020996 | R.YLSLRN#NSLR.T | 0.7943 |
| IPI00020996 | K.AGAFLGLTNVAVMN#LSGNCLR.N | 1 |
| IPI00020996 | R.FVQAICEGDDCQPPAYTYNN#ITCASPPEVVGLDLR.D | 0.9999 |
| IPI00020996 | K.ALRDFALQN#PSAVPR.F | 0.9955 |
| IPI00021089 | R.WLSS1GPECN#CSLGNFDSQVGACGFNSR.I | 0.8905 |
| IPI00021106 | K.AMAETFYLSNIVPQDFDN#NSGYWNR.I | 0.7649 |
| IPI00021131 | L.DLSM*NN#ISQLLPNPLPSLRFLEELRLAGNALTYIPK.G | 0.8889 |
| IPI00021131 | K.IHHIPDYAFGN#LSSLVVLHLHNNRIHSLGK.K | 0.5879 |
| IPI00021175 | K.EN#SSVEAKDSGLESK.K | 0.7359 |
| IPI00021175 | M.KTQEPAGSLEEN#NSDK.N | 0.6109 |
| IPI00021176 | K.TTTRQLSSPN#HSPSQSPN#QSPR.I | 0.6246 |
| IPI00021187 | R.AQTEGIN#ISEEALNHLGEIGTKTTLR.Y | 0.9365 |
| IPI00021250 | K.EIQHPNVITLHEVYEN#K.T | 0.5461 |
| IPI00021302 | K.VSM*M*EKSELVN#ETRWQYYGTAN#TSGN#LSLTWIIVK.S | 0.6375 |
| IPI00021304 | R.FGGFGGPGGVGGLGGPGGFGPGGYPGGIHEVSVN#QSLLQPLNVK.V | 0.999 |
| IPI00021304 | R.M*SGDLSSN#VTVSVTSSTISSNVASK.A | 0.882 |
| IPI00021305 | K.VDEFN#VSSPQF.V | 0.7039 |
| IPI00021319 | M.QNNWCFPACSFN#GTSAQEWFM*AQDCPYRK.R | 0.5829 |
| IPI00021364 | K.N#VTFWGRPLPR.C | 0.9279 |
| IPI00021388 | R.CTCGFSAVM*NRKFGN#NSGLFLE.D | 0.6283 |
| IPI00021426 | R.M*LWEIIN#STIIVMLFK.L | 0.641 |
| IPI00021426 | R.KVEVFPLN#STAVIIVYWK.L | 1 |
| IPI00021477 | R.QNVYIPGSN#ATLTNAAGK.R | 0.8796 |
| IPI00021531 | R.N#PSASTFLHLSTNSFR.I | 0.9423 |
| IPI00021556 | R.MQSPQNLHGQQDDDSAAFSFNGN#IET.L | 0.8236 |
| IPI00021557 | K.M*FLN#NTTTNRHTSGIGPGSKTGDKEE.K | 0.9548 |
| IPI00021578 | K.LGYNAN#TSVLSFQAVCR.E | 1 |
| IPI00021612 | K.TLPDSAGYVEGLQCM*SVEN#ATTIR.I | 0.5723 |
| IPI00021689 | K.EGKFNTRITN#LTVNTGLDCSEK.T | 0.643 |
| IPI00021695 | K.EASDHLTDDN#FTSIVK.A | 0.8332 |
| IPI00021711 | R.SN#IIIQATNDPPEVTVFPK.E | 0.5239 |

54

| | | |
|---|---|---|
| IPI00021727 | K.DQYVEPEN#VTIQCDSGYGVVGPQSITCSGN#R.T | 0.9884 |
| IPI00021727 | R.FSLLGHASISCTVEN#ETIGVWR.P | 1 |
| IPI00021731 | M.KLGTEALSTN#HSVIVNSPVITAAINKEFSNK.V | 0.6088 |
| IPI00021731 | K.QSESSFITGDIN#SSASLNREGLL.N | 0.909 |
| IPI00021753 | K.NEN#SSEQLDVDGDSSSEVSSEVNFNYEYAQM*EVTMK.A | 0.5242 |
| IPI00021786 | R.M*IEDAIRSHSESASPSALSSSPNN#LSPTGWSQPK.T | 0.5917 |
| IPI00021807 | R.M*ELSM*GPIQAN#HTGTGLLLTLQPEQK.F | 0.6119 |
| IPI00021817 | R.EVSFLN#CSLDNGGCTHYCLEEVGWR.R | 1 |
| IPI00021834 | R.YFYNN#QTK.Q | 0.9342 |
| IPI00021846 | P.ASSDFSDLNTQTN#WTK.S | 0.8431 |
| IPI00021885 | R.M*DGSLNFN#R.T | 0.7193 |
| IPI00021888 | R.VSGYLNLAADLAHN#FTDGLAIGASFRGGR.G | 0.7351 |
| IPI00021891 | K.DLQSLEDILHQVEN#K.T | 1 |
| IPI00021903 | D.NTLQQN#SSSN#ISYSNAMQK.E | 0.5195 |
| IPI00021935 | R.NGIALEILQN#TSYLPVLEGQALR.L | 0.7079 |
| IPI00021968 | G.GQSPASGN#VTGNSN#STFISSGQVMNFK.G | 0.8734 |
| IPI00021970 | R.SEHTGACNPCTEGVDYTN#ASNNEPSCFPCTVCK.S | 0.7994 |
| IPI00021997 | K.LN#ITNIWVLDYFGGPK.I | 0.9916 |
| IPI00021998 | K.CTLHFLTPGVN#NSGSYICRPKMIK.S | 0.8551 |
| IPI00021998 | R.RKFVCFVQNSIGN#TTQSVQLKEK.R | 0.9146 |
| IPI00022072 | K.SEHN#PSTSGCSSDQSSK.V | 0.5193 |
| IPI00022080 | M.N#RSQFEELCAELLQK.I | 0.7362 |
| IPI00022080 | K.ELN#NTCEPVVTQPK.P | 0.9299 |
| IPI00022080 | K.NQQITHAN#NTVSNFKR.F | 0.616 |
| IPI00022200 | R.VAVVQHAPSESVDN#ASM*PPVK.V | 0.9999 |
| IPI00022215 | K.EHKAEKVPAVANYIMKIHN#FTSK.C | 0.5359 |
| IPI00022229 | K.FVEGSHN#STVSLTTK.N | 1 |
| IPI00022229 | R.FN#SSYLQGTNQITGR.Y | 1 |
| IPI00022229 | K.N#LTDFAEQYSIQDWAK.R | 1 |
| IPI00022229 | R.VNQNLVYESGSLN#FSK.L | 1 |
| IPI00022229 | K.QVLFLDTVYGN#CSTHFTVK.T | 1 |
| IPI00022229 | R.FEVDSPVYN#ATWSASLK.N | 1 |
| IPI00022229 | K.YNQN#FSAGNNENIM*EAH.V | 0.9997 |
| IPI00022229 | K.SSVITLNTNAELFN#QSDIVAHLLSSSSSVIDALQYK.L | 0.9997 |
| IPI00022229 | K.TIHDLHLFIENIDFN#K.S | 0.9993 |
| IPI00022229 | K.IQSPLFTLDANADIGN#GTTSANEAGLAASITAK.G | 0.9989 |
| IPI00022229 | K.YDFN#SSMLYSTAK.G | 0.9986 |
| IPI00022229 | K.SYN#ETK.I | 0.9971 |
| IPI00022229 | K.QVFPGLNYCTSGAYSN#ASSTDSASYYPLTGDTR.L | 0.9936 |
| IPI00022229 | K.DFHSEYIVSASN#FTSQLSSQVEQFLHR.N | 0.9921 |
| IPI00022229 | A.ELEM*LEN#VSLVCPK.D | 0.985 |
| IPI00022229 | G.GN#TSTDIIFSLR.A | 0.9798 |
| IPI00022229 | K.VHN#GSEILFSYFQDLVTTLPFELR.K | 0.9723 |
| IPI00022229 | K.IYQLQVPLLGVLDLSTNVYSNLYN#WSASYS.G | 0.5051 |
| IPI00022250 | R.ALKGETVN#TTISFSFKGIKFSKGK.Y | 0.6772 |
| IPI00022255 | K.LN#DTTLQVLNTWYTK.Q | 0.8462 |
| IPI00022286 | A.HAASTEEREAGVGN#GTCAPVR.L | 0.8239 |
| IPI00022296 | R.SLYGKEDN#DTLVR.C | 0.9917 |
| IPI00022296 | R.TFTDKWEDYPKSEN#ESNIR.Y | 1 |
| IPI00022296 | K.QISESTNHYSNLAN#CSPNRQK.P | 0.8465 |
| IPI00022314 | K.FNGGGHIN#HSIFWTN#LSPN.G | 0.6848 |

| | | |
|---|---|---|
| IPI00022325 | K.VQGLVPAGGSSSN#STR.E | 0.9971 |
| IPI00022325 | R.QNM*CPAHQN#RSL.A | 0.8074 |
| IPI00022331 | R.M*AWPEDHVFISTPSFN#YTGR.D | 1 |
| IPI00022331 | R.QPQPVHLLPLHGIQHLNMVFSN#LTLEHINAILLGAYR.Q | 0.9557 |
| IPI00022331 | K.AELSN#HTRPVILVPGCLGNQLEAK.L | 0.5521 |
| IPI00022371 | R.VEN#TTVYYLVLDVQESDCSVLSR.K | 1 |
| IPI00022371 | R.VIDFN#CTTSSVSSALANTK.D | 1 |
| IPI00022371 | R.HSHNN#NSSDLHPHK.H | 0.9976 |
| IPI00022375 | M.LGGYGHISSSIDIN#SSR.K | 0.8028 |
| IPI00022391 | R.ESVTDHVNLITPLEKPLQN#FTLCFR.A | 1 |
| IPI00022392 | R.NPPM*GGNVVIFDTVITNQEEPYQN#HSGR.F | 1 |
| IPI00022395 | R.FSYSKN#ETYQLFLSYSSK.K | 1 |
| IPI00022395 | R.AVN#ITSENLIDDVVSLIR.G | 1 |
| IPI00022417 | A.VEFFN#LTHLPANLLQGASK.L | 0.997 |
| IPI00022417 | K.LPPGLLAN#FTLLR.T | 1 |
| IPI00022417 | R.DGFDISGNPWICDQN#LSDLYR.W | 1 |
| IPI00022417 | K.MFSQN#DTR.C | 0.9952 |
| IPI00022417 | R.QLDMLDLSN#NSLASVPEGLWASLGQPNWDMR.D | 0.6558 |
| IPI00022418 | F.LYNNHN#YTDCTSEGR.R | 0.9808 |
| IPI00022418 | K.I.DAPTNLQFVN#ETDSTVLVR.W | 1 |
| IPI00022418 | R.DQCIVDDITYNVN#DTFHK.R | 1 |
| IPI00022418 | S.PGLEYN#VSVYTVK.D | 0.9975 |
| IPI00022418 | R.HEEGHMLN#CTCFGQGR.G | 0.9938 |
| IPI00022426 | T.PPDNIQVQENFN#ISR.I | 1 |
| IPI00022426 | R.YFYN#GTSMACETFQYGGCM*GNGNNFVTEK.E | 0.9996 |
| IPI00022426 | K.WN#ITM*ESYVVHTNYDEYAIFLTK.K | 0.9955 |
| IPI00022429 | I.TN#ATLDQITGK.W | 0.5341 |
| IPI00022429 | R.QDQCIYN#TTYLNVQR.E | 1 |
| IPI00022429 | R.EN#GTISR.Y | 0.887 |
| IPI00022430 | M.FVMGVNENDYNPGSM*NIVSN#ASCTTNCLAPLAK.V | 0.6785 |
| IPI00022431 | K.VCQDCPLLAPLN#DTR.V | 1 |
| IPI00022431 | K.AALAAFNAQNN#GSNFQLEEISR.A | 1 |
| IPI00022432 | K.ALGISPFHEHAEVVFTAN#DSGPR.R | 1 |
| IPI00022447 | K.IILNALVAQQKN#GSPAGGDAKELDSKSK.G | 0.824 |
| IPI00022447 | K.KNNLPFLTN#VTLPR.S | 0.5774 |
| IPI00022461 | W.NLNN#DTEVPTASVAIEGASALNRVR.W | 0.5787 |
| IPI00022462 | K.LAVDFEENADN#NTKAV#VTK.P | 0.5547 |
| IPI00022462 | K.DFFDLYTPVN#GSIVIVR.A | 1 |
| IPI00022462 | R.KQNNGAFN#LTLFK.N | 0.9965 |
| IPI00022463 | K.CGI.VPVLAENYN#K.S | 1 |
| IPI00022463 | R.QQQHI.FGSN#VTDCSGNFCLFR.S | 1 |
| IPI00022471 | R.EDGDGDEDGPAQQLSGFNTN#QSNNVLQAPLPPMR.L | 0.5492 |
| IPI00022479 | R.QSLTSPDSQSARPAN#RTAISDPSSR.I | 0.5326 |
| IPI00022479 | M.CQELETGIVDLLIPSPN#ATAEVGYNR.D | 0.804 |
| IPI00022488 | R.CSDGWSFDATTLDDN#GTMLFFK.G | 0.9981 |
| IPI00022488 | K.ALPQPQN#VTSLLGCTH.- | 1 |
| IPI00022488 | R.SWPAVGN#CSSALR.W | 1 |
| IPI00022488 | R.N#GTGHGN#STHHGPFYM*R.C | 0.9993 |
| IPI00022525 | A.MNFPQCFYN#ESIAFFYN#R.S | 0.9212 |
| IPI00022529 | K.N#LSDVNILHR.L | 0.5671 |
| IPI00022557 | R.GYPGQVCAN#DSDTLLLPDSSRALLLGWVPTR.I | 0.8556 |

| | | |
|---|---|---|
| IPI00022579 | R.AVFIQGAEEHPAAFCYQVN#GSCPR.T | 0.5777 |
| IPI00022608 | R.VEGLQGVYIATLIN#GSMNEENM*RSVITFDK.G | 0.7377 |
| IPI00022608 | S.LLCLPKAN#NSR.S | 0.6448 |
| IPI00022609 | L.KRKWNSLSVIPVLN#SSSYTK.E | 0.5925 |
| IPI00022643 | K.ELLLTLDDSFNDVGSDNSN#QSSPRLRLPSPSMDK.I | 0.7513 |
| IPI00022674 | R.SVNILFN#LTHR.V | 0.999 |
| IPI00022674 | F.EN#LTYNQAASDSGSCGHVPVSPK.A | 0.9785 |
| IPI00022674 | Q.N#FTTLEAAPSEAPDVWR.I | 0.9323 |
| IPI00022731 | K.ELLETVVN#R.T | 0.9883 |
| IPI00022733 | K.EGHFYYN#ISEVK.V | 1 |
| IPI00022733 | R.IYSN#HSALESLALIPLQAPLK.T | 1 |
| IPI00022733 | K.VSN#VSCQASVSR.M | 0.9999 |
| IPI00022733 | R.GAFFPLTERN#WSLPNR.A | 0.999 |
| IPI00022733 | K.VTELQLTSSELDFQPQQELM*LQITN#ASLGLR.F | 0.9888 |
| IPI00022792 | R.VDLEDFEN#NTAYAK.Y | 0.8906 |
| IPI00022808 | R.VGSSPKIN#VSPFYQN#QTSTQR.S | 0.837 |
| IPI00022850 | K.VWKKIGIWNSNSGLN#MTDSNK.D | 0.7854 |
| IPI00022892 | K.DEGTYTCALHHSGHSPPISSQN#VTVLR.D | 0.8764 |
| IPI00022895 | H.N#ISVADSAN#YSCVYVDLKPPFGGSAPSER.L | 1 |
| IPI00022895 | R.EGDHEFLEVPEAQEDVEATFPVHQPGN#YSCSYR.T | 1 |
| IPI00022895 | R.FQSPAGTEALFELIN#ISVAD.S | 0.9998 |
| IPI00022895 | L.AN#VTLTCQAR.L | 0.961 |
| IPI00022933 | K.M*RMATPLLMQALPM*GALPQGPMQN#ATKYGN#MTE.D | 0.9428 |
| IPI00022933 | R.MATPLLMQALPMGALPQGPM*QN#ATK.Y | 0.9613 |
| IPI00022937 | R.QFYVAAQGISWSYRPEPTN#SSLN#LSVTSFK.K | 0.9497 |
| IPI00022937 | K.VSAITLVSATSTTAN#M*TVGPEGK.W | 1 |
| IPI00022937 | K.NSVLN#SSTAEHSSPYSEDPIEDPLQPDVTGIR.L | 1 |
| IPI00022937 | K.NM*ASRPYSIYPHGVTFSPYEDEVN#SSFTSGR.N | 1 |
| IPI00022937 | R.TNIN#SSRDPDNIAAWYLR.S | 1 |
| IPI00022937 | K.TYEDDSPEWFKEDNAVQPN#SSYTYVWHATER.S | 0.9214 |
| IPI00022937 | M.DNVGTWMLTSMN#SSPR.S | 0.7819 |
| IPI00023014 | R.M*EACM*LN#GTVIGPGK.T | 0.9997 |
| IPI00023014 | R.TEPM*QVALIICTN#GSVVYHEVLNAM*ECK.C | 1 |
| IPI00023014 | R.HCDGN#VSSCGDHPSEGCFCPPDK.V | 0.9999 |
| IPI00023014 | K.TTCNPCPLGYKEEN#NTGECCGR.C | 0.9991 |
| IPI00023014 | K.GQVYLQCGFPCN#LTCR.S | 0.9986 |
| IPI00023014 | R.GLQPTLTNPGECRPN#FTCACR.K | 0.9829 |
| IPI00023014 | K.WN#CTDHVCDATCSTIGM*AH.Y | 0.8999 |
| IPI00023019 | R.LDVDQALN#R.S | 0.9997 |
| IPI00023019 | R.SHFIWTHSCPQSPGN#GTDASH.- | 1 |
| IPI00023100 | R.TGGIGDSRPPSFHPNVASSRDGMDN#ETGTESMVSHR.R | 0.6243 |
| IPI00023109 | R.TILEN#NSGRSNSNPFNKEELTAII.K.F | 0.7956 |
| IPI00023118 | -.MGLN#TSASTFQLTGFPGMEK.A | 0.9007 |
| IPI00023183 | K.N#WTAALFTGNLLLAR.D | 0.5026 |
| IPI00023186 | K.EATN#FTTSEPSAPSQDLLDLSPSPR.M | 0.8169 |
| IPI00023212 | K.AMILLN#SSMYPLVTA?Q.D | 0.7868 |
| IPI00023217 | K.DM*VVM*LLSM*LEGNVVN#GTIGK.Q | 0.9428 |
| IPI00023217 | Q.KAMFDHLSYLI.EN#SSVGLASPAM*R.G | 0.5504 |
| IPI00023217 | -.MSYQLYNYPN#KTLLFSK.H | 0.7534 |
| IPI00023246 | K.VAGSEEN#GTAETEEVEDESASGELDLEAQFHLIF.F | 0.5677 |
| IPI00023258 | K.AENSAAVQIN#LSPTM*LENVK.K | 0.5821 |

57

| | | |
|---|---|---|
| IPI00023312 | R.EDFHYN#DTAGYFIIGGSRYVAGIEGFFGPLK.Y | 0.6552 |
| IPI00023314 | R.EQLCEIISFAETGLSTIN#QTR.L | 0.9485 |
| IPI00023315 | R.YDPFPAGDPEPRAAPN#NSADPR.V R | 0.9586 |
| IPI00023339 | K.KKPSMPN#VSNDLSQKLYATM*EKHK.E | 0.52 |
| IPI00023339 | R.NQQTILGSPASGIQNTIGSVGTGQQN#AT.S | 0.5983 |
| IPI00023340 | R.MTQPMM*N#SSYHSNPAYMN#QTAQYPMQM.Q | 0.5611 |
| IPI00023412 | N.N#YTAVFLGTVNGR.L | 0.5886 |
| IPI00023502 | R.YTSAGISVTVKELFPAPVLN#ASVTSP.L | 0.6911 |
| IPI00023586 | K.VADRTKSENGLQN#ESLSSTHHTDGLSK.I | 0.6943 |
| IPI00023586 | R.NHETTN#LSIQQK.R | 0.6117 |
| IPI00023648 | R.FQAFAN#GSLLIPDFGK.L | 0.9988 |
| JPI00023648 | K.SLDLSHNLISDFAWSDLHN#LSALQLLK.M | 0.9995 |
| IPI00023673 | K.GLN#LTEDTYKPR.I | 0.9999 |
| IPI00023673 | K.EPGSN#VTM*SVDAECVPM*VR.D | 1 |
| IPI00023673 | R.TVIRPFYLTN#SSGVD.- | 1 |
| IPI00023673 | R.ALGFEN#ATQALGR.A | 1 |
| IPI00023673 | K.AAIPSALDTN#SSK.S | 0.9996 |
| IPI00023673 | R.DAGVVCTN#ETR.S | 0.9987 |
| IPI00023722 | R.LLPILSQQSTIN#LSHNPLDCTCSNIHFLTWYK.E | 0.798 |
| IPI00023722 | T.FSRLM*N#LTFLDLTR.C | 0.7961 |
| IPI00023768 | K.FLALVTMN#QSGWGTSGR.R | 0.5767 |
| IPI00023785 | R.GLDVEDVKFVINYDYPN#SSEDYVHRIGR.T | 0.9016 |
| IPI00023807 | K.NLLIFN#LSEGDSGVYQ.C | 0.989 |
| IPI00023814 | R.TLSDVPSAAPQN#LSLEVR.N | 0.9998 |
| IPI00024036 | M.APMN#QSQVLM*SGSPLELNSLGFEQR.I | 0.5572 |
| IPI00024046 | K.ANYNLPIM*VTDSGKPPM*1N#ITDLR.V | 0.9979 |
| IPI00024046 | K.IN#NTHALVSLLQNLNK.A | 0.6016 |
| IPI00024067 | K.FDVN#TSAVQVLIEHIGNLDR.A | 0.8201 |
| IPI00024089 | -.MACLM*AAFSVGTAMN#ASSYSAEMTEPK.S | 0.6233 |
| IPI00024151 | M.AVRGLIRPMN#KSPM*LITGIR.C | 0.5572 |
| IPI00024163 | R.FRGN#LSGKRVDFSGR.T | 0.8261 |
| IPI00024214 | R.KDEN#ESSAPADGEGGSFLQPK.N | 0.5864 |
| IPI00024278 | K.SSLLLAILGEMQTLEGKVHWSNVN#ESEPSFEATR.S | 0.674 |
| IPI00024282 | K.M*N#DSNSAGAGGPVKITEN#RSK.K | 0.7826 |
| IPI00024284 | R.NLHQSN#TSR.A | 0.9647 |
| IPI00024289 | I.WCEDFLVRSFYLKNLQTN#ETR.T | 0.76 |
| IPI00024289 | K.AENSHSHSDYIN#ASPIMDIIDPR.N | 0.7796 |
| IPI00024292 | R.CIPQSWVCDGDVDCTDGYDENQN#CTRR.T | 0.5367 |
| IPI00024292 | K.YDGSNRQTLVN#TTHRPFD.I | 0.8456 |
| IPI00024316 | R.IIN#LSLHSKFIKVIINFATGK.S | 0.6199 |
| IPI00024316 | R.NAWGN#QSYAELISQAIE.S | 0.6386 |
| IPI00024330 | R.DGTLEYAPVDITVNLDASGSQCGLHSPLQSDN#ATDSPK.S | 0.6181 |
| IPI00024343 | K.TIKSLQKN#GSVVAM*TGDGVNDAVALKAADIGVAMGQT.G | 0.9981 |
| IPI00024357 | M.ALYHN#ISGVGLFLIIPVGLELLLDHR.A | 0.7441 |
| IPI00024382 | R.NTGN#GTQSSM*GSPLTR.P | 0.6092 |
| IPI00024403 | -.M*AAQCVTKVALN#VSCANLLDKDIGSK.S | 0.5908 |
| IPI00024425 | V.HSDFTAAATRGAM*AVIDGNVM*AIN#PSEETK.M | 0.8108 |
| IPI00024467 | K.IKGIVENMGINANN#M*SDFIM*KVDALMSSVPK.R | 0.71 |
| IPI00024519 | K.MLAQKSGNIIN#M*SSVASSVK.G | 0.7651 |
| IPI00024617 | K.YLCIPAADSPSQN#LIR.H | 0.9154 |
| IPI00024619 | K.CLKNLYVEN#RTK.S | 0.7714 |

| | | |
|---|---|---|
| IPI00024684 | M.FNQDIEKLVEGEEVVREN#ETR.L | 0 7901 |
| IPI00024684 | K.HFGEFFNLN#QTVQSTIEDIK.V | 0 8074 |
| IPI00024726 | K.LENSKN#GTAGLIPSPELQEWR V | 0.7954 |
| IPI00024769 | R.RTEKLFFTILSPN#QSK P | 0 8668 |
| IPI00024787 | K.YN#VTVIQYIGELLR.Y | 0.6148 |
| IPI00024802 | R LMPLEAGIPDPPN#MSAELIQLKAKER.H | 0.8143 |
| IPI00024816 | P HM*LPEDGAN#LSSARGILSLIQSSTR R | 0.6406 |
| IPI00024825 | R N#GTLVAFR.G | 0 992 |
| IPI00024825 | K.MTSTMPELN#PTSRIAEAM*LQTTTR.P | 0 7371 |
| IPI00024842 | K.DN#SSLNPLDRLISEDKKEK M | 0.8154 |
| IPI00024887 | R.RQQSRN#R S | 0.8613 |
| IPI00024896 | K.IKNM*N#STLTFVTLSGELRAR.R | 0 7423 |
| IPI00024911 | R.QALLKQGQDN#LSSVKETQK.K | 0.5629 |
| IPI00024933 | K.NIKHSGN#ITFDEIVNIAR.Q | 0.9525 |
| IPI00024970 | A KEAAGASKALN#V T | 0 8369 |
| IPI00024970 | R.VLAPILPDN#FSTPTGSR.T | 0.5501 |
| IPI00024975 | K.TFTMMGPSESDN#FSHNLR.G | 0 5064 |
| IPI00025054 | M.EPGN#GSLDLGGDSAGR.S | 0.6398 |
| IPI00025073 | K.ENEEFLIGFN#ITSKGRQLPKR.R | 0.6998 |
| IPI00025076 | M.DNPFEFNPEDPIPVSFSPVDTN#STSGDPVEKK.D | 0.9351 |
| IPI00025092 | E.APM*FTQPLVNTYAIAGYN#ATLN#CSVR.G | 0.6144 |
| IPI00025110 | R.KWN#VTSLETLK A | 0.7974 |
| IPI00025158 | M.KM*YSDAFLN#DSYLK.Y | 0 5533 |
| IPI00025193 | R.LQEM*GFIIYGNEN#ASVVPLLLYMPGK.V | 0 8126 |
| IPI00025264 | R.EKTSLSANN#ATLEKQLIELTRTNELLKSK.F | 0 7759 |
| IPI00025276 | R.GLRGPN#LTSPASITFTTGLEAPR.D | 1 |
| IPI00025310 | K.NCRN#KSLLRSRR T | 0.7196 |
| IPI00025310 | K.RPETKLKPLPVAPSQPTLGSSNIN#GSIDYPAK N | 0.5894 |
| IPI00025333 | R.KACKN#CTCGLAEELEK.E | 0.6207 |
| IPI00025426 | N.YLN#ETQQLTQEIK.A | 0.9924 |
| IPI00025426 | K.TFSSM*TCASGAN#VSEQLSLK.L | 1 |
| IPI00025426 | K.KGCVLLSHLN#ETVTVSASLESGR.E | 1 |
| IPI00025426 | K.GCVLLSHLN#ETVTVSASLESGREN#R.S | 0 5814 |
| IPI00025465 | R.VIHLQFNNIASITDDTFCKAN#DTSYIR.D | 0.971 |
| IPI00025468 | -.M*ENLQTN#FSLVQGSTK K · | 0.5035 |
| IPI00025477 | M IFDFYKQN#KTTR D | 0 5184 |
| IPI00025477 | K TAN#SSPIHFAGAQTSLPAFSPGR.L | 0 7326 |
| IPI00025489 | K TLIPN#ASNEAIQLM*TEM*LNWDPK.K | 0.5307 |
| IPI00025489 | -.M*NRYTTM*RQLGDGTYGSVLMGKSN#ESGELVAIKR M | 0 5123 |
| IPI00025616 | R FLGTSGQN#VSDIFR.Y | 0 9973 |
| IPI00025700 | R SLIIN#LSTPEVPASVQTVTIESSVTVKIENKESR.E | 0.9517 |
| IPI00025753 | M.ATFAGQIEENSNANTLVM*ILN#ATDADEPNNLNSK I | 0.7385 |
| IPI00025736 | K.QFN#SSN#SSPAPFPNSAVPSDGTEAK.V | 0 793 |
| IPI00025788 | K.RKN#STSSTSN#SSAGNNAN#STGSKK.K | 0 5861 |
| IPI00025815 | M MGM*LASQQN#QSGPSGNNQNQGNMQR.E | 0 6898 |
| IPI00025852 | K ELYEPIWQN#FTDPQLR R | 0 9959 |
| IPI00025861 | L TFPN#SSPGLRR Q | 0 666 |
| IPI00025862 | K TLFCN#ASK E | 0 984 |
| IPI00025862 | R I GHCPDPVLVNGEFSSSGPVN#VSDK.I | 1 |
| IPI00025862 | K EWDN#TTTFCR.L | 0 9096 |
| IPI00025862 | R DCDPPGNPVHGYFEGNN#FTLGSTISYYCEDR Y | 0 9962 |

| IPI00025864 | R.GM*N#LTVFGGTVTAFLGIPYAQPPLGR.L | 1 |
|---|---|---|
| IPI00025864 | K.YGNPN#ETQN#NSTSWPVFK.S | 1 |
| IPI00025864 | R.DN#YTKAEEIL.SR.S | 1 |
| IPI00025864 | K.WSDIWN#ATK.Y | 0.9996 |
| IPI00025864 | K.DN#NSIITR.K | 0.9939 |
| IPI00025864 | K.N#ATVLIWIYGGGFQTGTSSLHVYDGK.F | 0.7544 |
| IPI00025864 | R.EN#ETEIIK.C | 0.6817 |
| IPI00025879 | K.VKN#LTEEMAGLDETIAKLTK.E | 0.7899 |
| IPI00025879 | R.QAEEAEEQSNVN#LSK.F | 0.9989 |
| IPI00025879 | R.EN#QSILITGESGAGK.T | 0.9959 |
| IPI00026029 | R.SVRKN#LTYSCRSNQDCIINK.H | 0.9277 |
| IPI00026089 | K.LLLKIKN#GTPPM*R.K | 0.561 |
| IPI00026108 | M.KN#VSHNPLLLLTPQKVK.R | 0.643 |
| IPI00026157 | R.IRTYN#FTQDRVSDHRIAYEVR.D | 0.747 |
| IPI00026157 | N.RNCILHLLSKN#WSR.R | 0.6247 |
| IPI00026201 | A.ETTLTQSPAFM*SATPGDKVN#ISCK.A | 0.9996 |
| IPI00026240 | K.DSSGVIHVMLN#GSEPTGAYPIK.G | 0.7287 |
| IPI00026270 | M.HGDETVGRELLLHLIDYLVTSDGKDPEITNLIN#STR.I | 0.6956 |
| IPI00026327 | K.NQELKAGTSIMGSHLTSAETVTLDSLKAVEVVN#LSVS.C | 0.5498 |
| IPI00026466 | K.LSTLLNHNN#DTEEEER.L | 0.6877 |
| IPI00026631 | D.EAKNN#ITIFTRILDRLLDGYDNR.L | 0.6508 |
| IPI00026638 | R.STEEPTAPASPQPPN#DSR.L | 0.5892 |
| IPI00026638 | A.N#RTGSVEAQTALKK.R | 0.6591 |
| IPI00026639 | R.LSYNVIPLN#LTLDNRVADQLWVP.D | 0.8409 |
| IPI00026647 | R.FLITHN#PTN#ATLSTFIEDLK.K | 0.9108 |
| IPI00026659 | M.YFFLAN#LSLADACFVSTFVPK.M | 0.8187 |
| IPI00026673 | R.EQLSSAN#HSLQLASQIQK.A | 0.6035 |
| IPI00026813 | Q.RYFVISN#TTGYNDR.A | 0.7041 |
| IPI00026828 | L.EECCTHN#NSATI.SWK.Q | 0.8658 |
| IPI00026885 | M.ILN#SSTEDGIKR.I | 0.7889 |
| IPI00026885 | K.SHSN#LSTKMSTLSYRPSDN#VSSSTK.K | 0.5691 |
| IPI00026975 | L.DCPSSIIGMGLGN#ASTGYGK.I | 0.8383 |
| IPI00026987 | M.QRLNIGYVIN#VTTHLPLYHYEK.G | 0.9563 |
| IPI00026993 | G.DSSHCSN#ASTHSNQEAGPSNKR.T | 0.7712 |
| IPI00027035 | F.EAFQDALNQETTYVSN#LTR.S | 0.914 |
| IPI00027086 | M.SACN#ISIQGPSIYNK.E | 0.7837 |
| IPI00027087 | R.YFCLAANDQNN#VTIM*AN.L | 0.6509 |
| IPI00027146 | K.INPKN#YTENELEKITR.R | 0.6551 |
| IPI00027174 | R.M*DKPAN#CTHDLYMIM*R.E | 0.5864 |
| IPI00027178 | R.EGVQLN#LSFIR.P | 0.783 |
| IPI00027195 | V.NALN#FSVN#YSEDFVELNAAR.Y | 0.9366 |
| IPI00027200 | R.GSHAGN#LTVAVVLPLAN#TSYPWSWARVGPAVELALAQVK.A | 0.9747 |
| IPI00027220 | K.INQGTLTITSLN#SSLLGYYQCLAN#NSIGAIVS.G | 0.8917 |
| IPI00027235 | K.AATCIN#PLN#GSVCERPAN#HSAK.Q | 0.9999 |
| IPI00027235 | R.YLHTAVIVSGTMLVFGGNTHN#DTSM*SHGAK.C | 1 |
| IPI00027235 | K.AATCIN#PLN#GSVCER.P | 1 |
| IPI00027235 | K.M*PSQAPTGNFYPQPLLN#SSM*CLEDSR.Y | 1 |
| IPI00027235 | K.ISN#SSDTVECECSENWK.G | 1 |
| IPI00027235 | R.VFSHN#FSWVLLTPK.A | 1 |
| IPI00027235 | K.IDSTGN#VTNELK.V | 1 |
| IPI00027235 | R.N#HSCSECGQISITR.Y | 0.9999 |

| IPI00027235 | R.YN#WSFIHCPACQCNGHSK.C | 0 9999 |
| IPI00027235 | R GICN#SSDVR G | 0.9996 |
| IPI00027235 | R.NHPN#ITFFVYVSN#FTWPIK.I | 0.9982 |
| IPI00027235 | K.CIN#QSICEK.C | 0.9913 |
| IPI00027235 | R.GCSCFSDWQGPGCSVPVPAN#QSFWTR.E | 0.9717 |
| IPI00027235 | K.AVVNGNIMWVVGGYMFN#HSDYNMVLAYDLASR.E | 0.9159 |
| IPI00027235 | K.CIN#QSICEKCEN#LTTGK.H | 0.6417 |
| IPI00027242 | Q RHAAEIAN#MSLDILSAVGTFRMRHMPEVPVR I | 0.9484 |
| IPI00027259 | K N#NSPGTAEGCGGGGGGGGGGGSGGSGGGGGGGGGGDK.S | 0 9968 |
| IPI00027269 | R.LGSTFSLDTSMSMN#SSPLVGPECDHPK.I | 0.7599 |
| IPI00027310 | R VN#STELFHVDR.H | 0.9983 |
| IPI00027310 | R.ALLTN#VSSVALGSR.R | 0.9989 |
| IPI00027341 | R.EVQGN#ESDLFM*SYFPR.G | 0 6737 |
| IPI00027377 | R TVYLYPN#QTGLPDPLSR.H | 1 |
| IPI00027410 | R.ISALGLPTN#LTHILLFGM*GR.G | 1 |
| IPI00027410 | R N#LSSLESVQLDHNQLETLPGDVFGALPR.L | 1 |
| IPI00027410 | K.LLDLSGNN#LTHLPK.G | 0.9931 |
| IPI00027412 | R LQLSNGN#MTLTLLSVKR.N | 0.7565 |
| IPI00027422 | G.QTCN#CSTGSLSDIQPCLR E | 0.5268 |
| IPI00027444 | K.LEESYTLNSDLARLGVQDLFN#SSK.A | 0 976 |
| IPI00027473 | L WLALDYVVSN#ASVMNLLIISFDR.Y | 0 526 |
| IPI00027474 | M AN#FTPVN#GSSGN#QSVR.L | 0 9558 |
| IPI00027482 | K.AVLQLNEEGVDTAGSTGVTLN#LTSK.P | 0.9994 |
| IPI00027482 | R AQLLQGLGFN#LTER.S | 1 |
| IPI00027482 | D.PNAAYVN#M*SNHHR.G | 1 |
| IPI00027482 | K.VTISGVYDLGDVLEEMGIADLFTNQAN#FSR.I | 0.9995 |
| IPI00027483 | N YVGN#GTVFFILPDK.G | 0.9813 |
| IPI00027493 | K.DASSFLAEWQN#ITK.G | 1 |
| IPI00027493 | K.SLVTQYLN#ATGNR.W | 1 |
| IPI00027504 | R.AFGSNPN#LTK.V | 0.9987 |
| IPI00027504 | K.LYLGSNN#LTALHPALFQN#LSK.L | 1 |
| IPI00027504 | R.NAITHLPLSIFASLGN#LTF L | 1 |
| IPI00027504 | R.QGSLGLQYN#ASQEWDLR.R | 0.9999 |
| IPI00027504 | N.IFSN#LTSLGK.L | 0 9416 |
| IPI00027507 | K LGYNAN#TSILSFQAVCR.E | 0.9999 |
| IPI00027507 | K FVQGN#STEVACHPGYGLPK A | 1 |
| IPI00027508 | R STLITVLN#ISEIESR.F | 0 75 |
| IPI00027508 | K.WN#GSVIDEDDPVLGEDYYSVENPANKR.R | 0 5759 |
| IPI00027534 | K.SISWDEN#GTCIVINE.E | 0 8155 |
| IPI00027569 | - MASN#VTNKM*DPHSM*NSR.V | 0 9216 |
| IPI00027642 | R.NTEASSEEESSASRMQVEQN#LSDII.I | 0 8298 |
| IPI00027666 | R SN#SSAANLMAKK R | 0.7236 |
| IPI00027682 | A.ASSIWSPASISPGSAPASVSVPEITAAPSN#TSCM*QR.S | 0 8377 |
| IPI00027701 | R GCASTGVIM*SVN#NSLYLGPILKFGSK.E | 0.7524 |
| IPI00027709 | R N#GSANRN#SSHRTAAQPAITPIDVPGSLDDGADCEA.V | 0.962 |
| IPI00027803 | K MLSLNN#YSVPQSTR E | 0 5599 |
| IPI00027828 | - M*NN#PSETSKPSM*ESGDGNTGTQTNGLDFOK Q | 0 768 |
| IPI00027968 | K.KQPFSSASSQN#GSLSPHVLSSVIK Q | 0.5436 |
| IPI00028030 | R YRCN#DTIPEDYETHQLR.Q | 1 |
| IPI00028030 | K CGPCPAGFTGN#GSHCTDVNECNAHPCFPR.V | 0 9891 |
| IPI00028119 | K IYN#VTYTEPSLR.I | 0 5842 |

| | | |
|---|---|---|
| IPI00028210 | K.GTGN#DTVLNVALLNVISNQECNIK.H | 1. |
| IPI00028277 | R.AAYN#VTLLNFMDPQK.M | 0.993 |
| IPI00028338 | R.VFYFMVGTAFAN#STCQLIVCQM*SSTR.C | 0.7299 |
| IPI00028382 | R.NGESMLN#ASLVN#ASSLSEAEQLQR.E | 0.9011 |
| IPI00028413 | K.TAFITN#FTLTIDGVTYPGNVK.E | 0.9995 |
| IPI00028413 | K.NAHGEEKEN#LTAR.A | 0.9994 |
| IPI00028448 | K.NLDLILPTLRN#YTVINSKUVVTIR.P | 0.625 |
| IPI00028448 | R.FRDIPN#TSSMENPAPNK.N | 0.8805 |
| IPI00028448 | K.KDLSCSN#FSLLAYQFDHFSHEKIK.D | 0.8152 |
| IPI00028448 | M.FPKN#FTN#CTWTLENPDPTK.Y | 0.6661 |
| IPI00028490 | N.FGHN#DSTSQM*SLNSAAVTK.T | 0.6305 |
| IPI00028492 | R.MCQAGN#ATVKQSRYR.I | 0.5036 |
| IPI00028514 | K.KFLYN#FTQIPHLAGTEQNFQL.A | 0.5133 |
| IPI00028541 | R.KM*PSNQN#VSPSQR.D | 0.6196 |
| IPI00028570 | I.QAAASTPTN#ATAASDANTGDR.G | 0.9839 |
| IPI00028588 | K.NFHSMQNLCPPQTN#GTPEGR.Q | 0.6444 |
| IPI00028610 | K.SCVSNIESTLSALQYVSSIVVSLEN#R.S | 0.5546 |
| IPI00028642 | I.LESLM*CN#ESSMQSLRQR.K | 0.5424 |
| IPI00028928 | K.DGKN#KTDKKDHSNIGN#DSKKTDGTKQRS.H | 0.7479 |
| IPI00028931 | R.ERESFLAPSSGVQPTLAMPNIAVGQN#VTVTER.V | 0.7219 |
| IPI00028952 | D.KMIENHN#ISTPFSCQFCK.E | 0.5917 |
| IPI00028957 | M.SNVEQALFARLLLQDPGNHLIN#MTSSTTLN#LSADR.D | 0.7777 |
| IPI00028987 | K.LIPFSPAVN#TSVSTVASTVAPMYAGDLR.T | 0.6633 |
| IPI00028987 | K.TRGRGAAN#DSTQFTVAGRMVKK.G | 0.6108 |
| IPI00029011 | C.HQMQGN#STSIINPK.N | 0.5849 |
| IPI00029019 | R.RGGRFSAQGM*GTTNPADYAEPANTDDNYGN#SSGN.T | 0.5824 |
| IPI00029048 | R.FTLTQSEADADILFN#FSHFK.D | 0.7949 |
| IPI00029061 | K.EGYSN#ISYIVVNIIQGISSR.L | 1 |
| IPI00029061 | K.VSEHIPVYQQFEN#QTDVWTLLN#GSK.D | 1 |
| IPI00029061 | K.CGN#CSLTTLK.D | 0.9955 |
| IPI00029061 | R.DQDPM*LNSN#GSVTVVALLQASCY.L | 0.6691 |
| IPI00029166 | R.FQGN#DTSPESFLLHNALAR.K | 0.6772 |
| IPI00029172 | K.WYLENVYPEM*RVYN#NTLTYGEVRNSK.A | 0.5181 |
| IPI00029178 | K.CEQSYGTN#SSDESGSFSEADSESCPVQDR.G | 0.6131 |
| IPI00029193 | R.CFLGN#GTGYR.G | 0.9999 |
| IPI00029193 | K.YIPYTLYSVFN#PSDHDLVLIR.L | 1 |
| IPI00029193 | R.DSVSVVLGQHFFN#R.T | 1 |
| IPI00029260 | R.LRN#VSWATGR.S | 0.9999 |
| IPI00029260 | R.CMWSSALNSLN#LSFAGLEQVPK.G | 0.9515 |
| IPI00029268 | K.SPM*QWDN#SSNAGFSEASNTWLPFNSDYHTVNVDV.Q | 0.9545 |
| IPI00029273 | M.VSN#ESVDYRATFPEDQFPN#SSQN#GSCR.Q | 0.8522 |
| IPI00029273 | R.HVFPIN#IITADIQSFVH.C | 0.9993 |
| IPI00029324 | K.RNTLETLLAKEIQTCQDVEVN#ASK.Q | 0.8552 |
| IPI00029343 | R.NMANGQPHSVN#IITR.H | 0.6786 |
| IPI00029411 | R.IDSQLHTPMYFFLAN#LSFVDVCN#STTITPK.M | 0.7805 |
| IPI00029449 | K.SVTIQAPGEPLLDN#FSTR.G | 0.617 |
| IPI00029468 | M.ESYDVIANQPVVIDN#GSGVIK.A | 0.6352 |
| IPI00029533 | R.N#VTSNDEVLFN#VTVTMKK.C | 0.7091 |
| IPI00029591 | M.LRN#SIDTTPLFGPGTPFSTTVEPAARR.S | 0.906 |
| IPI00029643 | M.NKAGAVM*HSQM*QINM*QAKQN#SSKTTSKRR.G | 0.6042 |
| IPI00029643 | R.FHN#HTTHM*SLVGTFPWMAPEVIQSLPVSETC.D | 0.6596 |

62

| | | |
|---|---|---|
| IPI00029728 | K.AVVVFDEAHNIDNVCIDSM*SVN#LTRR.T | 0.9764 |
| IPI00029739 | K.IPCSQPPQIEHGTIN#SSR.S | 1 |
| IPI00029739 | R.ISEEN#ETTCYMGK.W | 1 |
| IPI00029739 | K.SPDVIN#GSPISQK.I | 0.9999 |
| IPI00029739 | K.MDGASN#VTCINSR.W | 0.9995 |
| IPI00029739 | R.WDPEVN#CSM*AQIQLCPPPPQIPNSHN#MTTTLNYR.D | 0.9978 |
| IPI00029739 | R.SPYEMFGDEEVMCLNGN#WTEPPQCK.D | 0.9941 |
| IPI00029739 | K.LN#DTLDYECH.D | 0.99 |
| IPI00029739 | Y.YYGDSVEFN#CSESFTM*IGHR.S | 0.941 |
| IPI00029751 | K.GTEWLVN#SSR.I | 0.7866 |
| IPI00029751 | K.GTEWLVN#SSRILIWEDGSLEINN#ITR.N | 0.8616 |
| IPI00029768 | K.IN#NSTNEGM*NVK.K | 0.6256 |
| IPI00029778 | R.RSN#VSSPATPTASSSSSTTPTR.K | 0.5124 |
| IPI00029863 | H.LALGAQN#HTLQR.L | 0.9987 |
| IPI00029954 | K.VSKN#DTEEESN#K.S | 0.5904 |
| IPI00030075 | R.LHVGNYN#GTAGDALRFNK.H | 0.6391 |
| IPI00030099 | R.GQGTASSGN#VSDLAQTVKTFDNLK.T | 0.6284 |
| IPI00030101 | R.GPSHPLDLGTSSPN#TSQIHWTPYR.A | 0.8037 |
| IPI00030153 | K.EQQPNDLLSVQFIDYGN#VSVVHTNK.I | 0.7133 |
| IPI00030241 | T.TDFCSVSTATPVPTAN#STAKPTVQPSPSTTSK.T | 0.6144 |
| IPI00030250 | K.TNLIVNYI.PQN#MTQEELK.S | 0.7865 |
| IPI00030360 | R.EITASSAVSILIKPEQETDPLPVVSRN#VSADAK.C | 0.6648 |
| IPI00030380 | K.LQESIEYEDLGKN#NSVK.T | 0.9004 |
| IPI00030380 | R.LNVN#ATDSSSTSNHK.Q | 0.5921 |
| IPI00030393 | M.RSFLQQDVN#KTKSR.L | 0.6779 |
| IPI00030414 | R.EPSDPTSN#RSTFHPGDSQKPVK.R | 0.9579 |
| IPI00030418 | K.QTDVIN#ASWWVMSN#KTRDELER.S | 0.6331 |
| IPI00030536 | K.RN#SSSSSTDSETLRYNIINFEPK.S | 0.6821 |
| IPI00030572 | K.LPLSHSALPSQALGGIASGLGMQNLN#SS.R | 0.7686 |
| IPI00030572 | R.NNPVIQSSTTTN#TTTTTTTITSN#TTHR.V | 0.6237 |
| IPI00030648 | R.GNEATEGSGLLLN#STGDI.M*K.K | 0.6517 |
| IPI00030739 | R.FLLYN#R.S | 0.7531 |
| IPI00030739 | K.TELFSSSCPGGIMLN#ETGQGYQR.F | 0.9999 |
| IPI00030746 | K.FQDLLSEEN#ESTALPQVLAQPSTSRKR.P | 0.5888 |
| IPI00030790 | K.LSSQGN#VSGKRK.N | 0.5281 |
| IPI00030828 | R.DVLQNIILTEVLTLVAMELPIIN#VSSAEAVLR.H | 0.9971 |
| IPI00030851 | K.KLN#CSPDSFR.C | 0.7295 |
| IPI00030868 | K.EDGKTLYANTIN#GSGLAID.R | 0.8024 |
| IPI00030871 | K.LLLSQLDSHPSHSAVVN#WTSYASSIEALSSGNK.E | 0.9997 |
| IPI00030871 | K.LTGVAGN#YTVCQK.D | 0.9998 |
| IPI00030875 | L.MYPIAVMGN#ITIILMSR.L | 0.6124 |
| IPI00030882 | K.VAKNAQNIN#PSSSQNSQNFATYK.E | 0.5715 |
| IPI00030882 | M.VVTETEEPSGN#DTSGIENFKFVVVTTHEESPYVMMKK.N | 0.9147 |
| IPI00030907 | R.IVAECNAVRQALQDLLSEY\IN#NTGRK.E | 0.5831 |
| IPI00030907 | K.KN#ATM*LYTASQAFLRHPDVAATRANR.D | 0.557 |
| IPI00030930 | K.FIENIGYVLYGVYN#VTM*VVVI.NM*LIAM*IN#N.S | 0.5581 |
| IPI00030930 | K.TRYQAGMRNSFN#LTAN#NTLSKPTR.Y | 0.8356 |
| IPI00030940 | R.LVKVKNEGDDFGWGVVXN#FSKK.S | 0.8263 |
| IPI00030986 | K.LEDFMQLSPQEI.ISVISN#DSLNVEK.E | 0.7113 |
| IPI00031002 | K.N#VSQENMCSASAAFK.S | 0.885 |
| IPI00031002 | M.VAGLLNSGNSN#KTIHTSSSIK.L | 0.6624 |

| | | |
|---|---|---|
| IPI00031023 | K.NM*LVLN#LSHNSIDTIPNQLFIN#LTDLLYLDLSENR.L | 0.6059 |
| IPI00031046 | M.M*N#NTDFLMLNNPWNK.L | 0.8844 |
| IPI00031064 | E.N#ITIVDISR.K | 0.5226 |
| IPI00031076 | R.IVN#DTYR.T | 0.9162 |
| IPI00031131 | R.AGPN#GTLFVADAYK.G | 0.9997 |
| IPI00031138 | R.RSLEQHGLPWAIISIPVN#VTSIPTFELLQPPWTFW.- | 0.8046 |
| IPI00031171 | R.SVQLHDSGN#YSCYR.A | 0.9963 |
| IPI00031282 | A.VAAGTPN#TSGSIHENPPK.A | 0.542 |
| IPI00031411 | R.FAN#LTPEEFVGDYWR.N | 0.9062 |
| IPI00031509 | V.ENLIILAN#NSLSSNGN#VTESGCK.E | 0.5445 |
| IPI00031522 | K.STKPIVAAIN#GSCLGGGLEVAISCQYR.I | 0.5098 |
| IPI00031556 | K.N#ATLVSPPAQTIN#QTPVTLQVPG.L | 0.9944 |
| IPI00031589 | R.CN#SSLSNHQR.I | 0.6205 |
| IPI00031595 | R.TASIWVPPLQERN#SSWDR.I | 0.5407 |
| IPI00031620 | L.GDQM*LN#ATVM*NHGDTLTATATATAR.A | 0.7552 |
| IPI00031620 | R.EN#LTVFSFLGPIVN#LSEPTAHEGSTVTVSC.M | 0.6788 |
| IPI00031658 | R.YVKTTGN#ATVDHLSK.Y | 0.5665 |
| IPI00031696 | K.LNYLPPN#ASALFR.K | 0.9459 |
| IPI00031710 | K.CVPVTLWHLGYWLCYVN#STVNPICYALCN#R.T | 0.6615 |
| IPI00031773 | M.SAAAM*GSGSGN#MSAGSMN#MSSYVGAGMSPSLAGM*S.P | 0.9983 |
| IPI00031789 | R.PTLLN#DTGN#YTCM*LR.N | 0.9637 |
| IPI00031801 | E.GEKGAEAAN#VTGPDGVPVEGSRYAADR.R | 0.5246 |
| IPI00031907 | K.IFQIYKGN#FTGSVEPEPSTLTPR.T | 0.6701 |
| IPI00032034 | K.DFLDVYYN#LTLKTM*MGIEW.V | 0.8857 |
| IPI00032038 | K.IN#RTLETANCMSSQTK.N | 0.5753 |
| IPI00032162 | A.M*IQFAIN#STERKR.M | 0.5121 |
| IPI00032179 | K.LGACN#DTLQQLMEVFK.F | 1 |
| IPI00032179 | K.SLTFN#ETYQDISFLVYGAK.L | 1 |
| IPI00032179 | K.WVSN#KTEGR.I | 0.9915 |
| IPI00032190 | R.LALTPAHLLFTADN#HTEPAAR.F | 0.9997 |
| IPI00032215 | K.KLINDYVKN#GTR.G | 1 |
| IPI00032215 | N.SPLDEEN#LTQFNQDR.G | 1 |
| IPI00032215 | K.YTGN#ASALFILPDQDK.M | 1 |
| IPI00032215 | K.ALDKNVIFSPLSISTALAFLSLGAHN#TTLTEILK.A | 0.9562 |
| IPI00032220 | R.VYIHPFHLVIIN#ESTCEQLAK.A | 1 |
| IPI00032220 | R.LQAILGVPWKDKN#CTSR.L | 1 |
| IPI00032220 | K.GFSLLAEPQEFWVDN#STSVSVPMLSGM*GTFQH.W | 0.8079 |
| IPI00032256 | R.GNEANYYSN#ATTDEHGLVQFSIN#TTNVM*GTSLTVR.V | 1 |
| IPI00032256 | K.VSN#QTLSLFFTVLQDVPVR.D | 1 |
| IPI00032256 | K.SLGNVN#FTVSAEALESQELCGTEVPSVPEHGR.K | 1 |
| IPI00032256 | K.GCVLLSYLN#ETVTVSASLESVR.G | 1 |
| IPI00032256 | K.IITLEEEMN#VSVCGLYTYGK.P | 1 |
| IPI00032256 | Y.VLDYLN#FTQQLTPEVK.S | 0.9998 |
| IPI00032256 | K.GCVLLSYLN#ETVTVSASLESVRGN#R.S | 0.9739 |
| IPI00032258 | R.N#PSDPM*PQAPALWIETTAYALLHLLLHEGK.A | 0.9655 |
| IPI00032258 | R.GLN#VTLSSTGR.N | 1 |
| IPI00032258 | R.FFQLEIRPVLYNYLDKN#LTVSVII.V | 1 |
| IPI00032258 | K.FSDGLESN#SSTQFEVK.K | 1 |
| IPI00032258 | K.N#ATTCQDLQIEVIVK.G | 0.9998 |
| IPI00032291 | K.VEGSSSHLVTFTVLPLEIGLHNIN#FSLETWFGK.F | 0.9986 |
| IPI00032291 | R.AN#ISHKDM*QLGR.L | 0.9982 |

EP 2 302 394 A1

| | | |
|---|---|---|
| IPI00032291 | K.YN#FSFR.Y | 0.9944 |
| IPI00032292 | K.FVGTPEVN#QTTLYQR.Y | 1 |
| IPI00032299 | R.ILTN#FTGVM*PPQFK.K | 0.7638 |
| IPI00032328 | R.ITYSIVQTN#CSK.E | 1 |
| IPI00032328 | K.LNAENN#ATFYFK.I | 1 |
| IPI00032328 | K.YNSQN#QSNNQFVLYR.I | 1 |
| IPI00032328 | R.HGIQYFNN#NTQHSSLFMLNEVK.R | 0.9985 |
| IPI00032334 | R.VYVN#ISHPDMVDFARGK.T | 0.602 |
| IPI00032388 | R.DDNMN#TSEDEDMFPIEMSSDEAMELLESSR.T | 0.7408 |
| IPI00032402 | K.LM*QN#STSPPLK.L | 0.5598 |
| IPI00032402 | M.IQTAHVGVGISGNEGLQAAN#SSDYSIAQFK.Y | 0.5178 |
| IPI00032406 | R.YGEQGLREGSGGGGGMDDIFSHIFGGGLFGFMGN#QSR.S | 0.839 |
| IPI00032449 | K.NAKSSGN#SSSSGSGSGSTSAGSSSPGAR.R | 0.8221 |
| IPI00032461 | K.N#YTSVYDKNNLLTN#KTVMAHGCY.L | 0.5331 |
| IPI00032466 | K.LREQVNSMVDISKMHM*ILYDLQQN#LSSSHR.A | 0.9741 |
| IPI00032680 | R.CDKDSMPDGN#LSEEEK.L | 0.8587 |
| IPI00033017 | M.NWVVGSADLEIIN#ATTGR.R | 0.7931 |
| IPI00033102 | A.QPIEPITAAPSGSGN#GSGSSSSGGSSGGSGFCAVR.A | 0.6723 |
| IPI00033419 | R.VKN#ISDADVHNAM*DNYE.C | 0.8216 |
| IPI00033486 | R.KFAMSPSN#FSSSDCQDEEGR.K | 0.7204 |
| IPI00033486 | W.GKELIETLWNLGDHELLHMYPGN#VSK.L | 0.8142 |
| IPI00033583 | K.M*VAWSSSEN#MSEESVVLSFPR.F | 0.9582 |
| IPI00033583 | K.TFVEVDEN#GTQAAAATGAVVSERSLR.S | 0.5917 |
| IPI00033798 | K.SEVNEM*ENN#LTRR.R | 0.7871 |
| IPI00033946 | A.EAYLGYPVTNAVITVPAYFN#DSQR.Q | 0.7137 |
| IPI00034003 | K.SLTN#LSQEEQITKLLILK.L | 0.9128 |
| IPI00034277 | K.ATFVKVVPTPNN#GSTELVALHR.N | 0.5215 |
| IPI00034283 | M.ASTFIGN#STAIQELFK.R | 0.933 |
| IPI00034309 | L.ENSGRSKN#FSYNLQSATQPKN#K.T | 0.9674 |
| IPI00034317 | K.ALKSN#SSLTKGLRTMVEQNLMEK.L | 0.575 |
| IPI00034378 | K.EGGVFTFGAGGYGQLGHN#STSHEINPRK.V | 0.8953 |
| IPI00034558 | K.N#RSTASIQPTSDDLVSSAEC.S | 0.8906 |
| IPI00035165 | K.AYIHAQAEN#CSHTAELVSWK.R | 0.9374 |
| IPI00035691 | R.RDMGN#FSWGSE.C | 0.7735 |
| IPI00036554 | M.FRM*LN#SSFEDDPFFSESILAHRENM*R.Q | 0.6512 |
| IPI00037319 | K.SIMEN#ASAGVEHLLLGNK.C | 0.7305 |
| IPI00040730 | K.EKPPNENCNN#NSPESSLLPR.A | 0.9121 |
| IPI00043469 | R.EN#M*SLPSNLQLNDLTPDSR.A | 0.7873 |
| IPI00043550 | M.PN#SSGLM*NRR.D | 0.5396 |
| IPI00043654 | M.LASNHM*N#GSNGESPLA.- | 0.6334 |
| IPI00043705 | R.YFNPVIXQENALIAAIAN#WSELASMPVGR.S | 0.7932 |
| IPI00043716 | K.KLRLPDIGLYN#MTDSGTGSCKN.S | 0.6021 |
| IPI00043716 | K.N#GTVDGTSENTEDGLDRK.D | 0.8943 |
| IPI00043724 | R.LSQNQNNYQISGN#LTVPVITGCSR.K | 0.7428 |
| IPI00043744 | R.QGN#LSLPLNRELVEKVTNEYN#ESLLYSPEEPK.I | 0.8137 |
| IPI00043745 | R.VKN#GSRVVSTALLSSYHKGI.A | 0.92 |
| IPI00043992 | S.LKDN#SSCSVMSEEPEGR.S | 0.5533 |
| IPI00044283 | R.TPRPASTHN#GSVDTEN#DSCLQQTH.- | 0.9394 |
| IPI00044315 | -.MSAGN#GTPWGSAAGEEVWAG | 0.5879 |
| IPI00044369 | R.VN#LSFDFPTYGHFLR.E | 0.9099 |
| IPI00044369 | R.SFTDLLLDDGQDN#NTQIEEDTDHNYYISR.I | 1 |

65

| | | |
|---|---|---|
| IPI00044369 | K.ITN#ISAVEM*TPL.P | 0.6299 |
| IPI00044456 | R.FPGVM*EN#LTISAAHWLTAPAPRPRPR.R | 0.544 |
| IPI00044461 | M.DRWN#ETVGLEWELERQLALMNSQFNRR.V | 0.9317 |
| IPI00044461 | R.NFDKNGNMMDWWSN#FSTQHFR.E | 0.5094 |
| IPI00044529 | R.KSIDEM*NNAWENLN#KTW.K | 0.6338 |
| IPI00044631 | R.N#ITPLLLDMVVHNDR.L | 0.5685 |
| IPI00044650 | K.SKSDLAVSN#ISPPSPDSK.S | 0.8147 |
| IPI00044683 | G.DQLSN#LSNLLQQYK.T | 0.633 |
| IPI00044714 | D.QYGKN#FSQ.S | 0.6101 |
| IPI00044726 | R.N#NSKGYM*KLENKEDPM*DRLL.V | 0.6866 |
| IPI00045438 | N.N#ATN#ESYVDTAAMEAER.L | 0.5244 |
| IPI00045486 | R.TEDVMFISDN#ESFN#PSLWEEQR.K | 0.975 |
| IPI00045512 | R.GSVIGNINDVEFGIAFLN#ATITDSPN.S | 0.5086 |
| IPI00045512 | R.YLQINNADLGDTAN#YTCVASNIAGK.T | 0.7655 |
| IPI00045512 | R.QLGN#GSLAIYGTVNEDAGDYTC.V | 0.6632 |
| IPI00045512 | R.VRASSYSAN#GTIE.Y | 0.5949 |
| IPI00045856 | R.KAVPM*APAPASPGSSN#DSSAR.S | 0.5172 |
| IPI00045914 | S.SSREEN#WSFLDWDSR.F | 0.8475 |
| IPI00045914 | K.N#DTAAVQLHFVSGNNVLAHRS.L | 0.676 |
| IPI00045928 | K.SVGIFLGIFSGSFTMGAVTGVNAN#VTK.F | 0.5903 |
| IPI00045942 | R.LKTEYN#ITLR.V | 0.7003 |
| IPI00045953 | -.NN#FSTEIN#TTSILVGPLVSNLEITHTSN#LTR.V | 0.5945 |
| IPI00046047 | R.HN#FTLAFSTAEKLADCAQLLD.V | 0.5968 |
| IPI00046260 | K.VHTGTHM*WN#STPVXQGRQLSGDGPMTFLGGNPIK.F | 0.6128 |
| IPI00046366 | K.GTENHLLAIVN#GTKGSR.W | 0.5746 |
| IPI00046793 | K.QPSSPLAN#TTYNIFIM*DGK.T | 0.9425 |
| IPI00047137 | K.MENGQQAADNILSAVPPGLIN#TSEAGIPAMSTND.L | 0.7651 |
| IPI00047437 | R.KMFLFGTYLTKN#GSEIPSTM*QDAK.D | 0.7148 |
| IPI00047620 | Y.HGN#GTHSESLEHHGYIGN#GTDR.E | 0.8103 |
| IPI00047620 | R.GYHGN#GTHSESLEHR.G | 0.6073 |
| IPI00047620 | R.VQN#TSLEHRGYHGSGTDGESSGRR.G | 0.5039 |
| IPI00049891 | D.DRGSYTASIYQNYMGNSFSGYSHSPPLLQVN#R.S | 0.6228 |
| IPI00050342 | -.MENRNN#M*TEFVLLGLTENPKM*QK.I | 0.6014 |
| IPI00050486 | R.TN#FTLAELGICEPSPHRSGYCSDMGILHQ.G | 0.7133 |
| IPI00051170 | K.YKELTLTRNQGICGKN#NSYLE | 0.7528 |
| IPI00051926 | N.QGN#FSVVGTVLAATQAEKAVANFDR.T | 0.8374 |
| IPI00053761 | R.TQN#LSQPSTGIPSGEPGHSAGGAAGSRCTRSMFR.K | 0.6955 |
| IPI00054085 | M.AN#RTDNTN#RTGDATVIKQEM*LTGQEM*PR.E | 0.6436 |
| IPI00054853 | K.VTCDIDVN#SSLN#ISAVGKSTEK.E | 0.6021 |
| IPI00054874 | K.IILFEDSQNKLGAEM*VIN#TSGKYGYK.S | 0.6513 |
| IPI00055218 | R.SKGAIAPPEVIVPAQN#TSLGPK.K | 0.9359 |
| IPI00055405 | K.KVLAPRVN#LTFR.K | 0.5407 |
| IPI00056324 | K.TERGLIFYIN#HSLYENLDEELNEELAAK.V | 0.5595 |
| IPI00056499 | K.WMLKTGMKNN#ATK.Q | 0.9557 |
| IPI00056506 | R.IFVGGIDFKTN#ESDLR.K | 0.541 |
| IPI00056511 | K.YNLEKDLKDKFVALTIDDICFSLNN#NSPNIR.Y | 0.5393 |
| IPI00056521 | -.MHRLMGVN#STAAA.A | 0.6667 |
| IPI00057386 | R.QMGGNTNTGAALN#FTISLLQKAK.K | 0.5167 |
| IPI00058265 | -.M*M*GHQN#HIF.S | 0.6264 |
| IPI00058344 | K.SSN#LSEHQTLHTGQR.P | 0.7346 |
| IPI00058949 | R.YGN#TTQNVPHNPR.R | 0.566 |

| IPI00059144 | R.VVN#ESTVCLMNHERR.Q | 0.7988 |
| IPI00059279 | R.ISESGIKKMCRNIFVLQQN#LTN#ITMSR.E | 0.9055 |
| IPI00059434 | K.N#NSMNSNMGTGTFGPVGNGVHTGPESR.E | 0.8325 |
| IPI00059632 | K.DHPVSCCLGLLLESLVPFIVNDN#ITNNFFR.F | 0.8635 |
| IPI00060143 | R.GLNIALVN#GTTGAVLGQKAFDMYSGDVM*HLVK.F | 0.7846 |
| IPI00061178 | R.GPPSRGGHMDDGGYSMNFN#MSSSRGPLPVK.R | 0.589 |
| IPI00061245 | R.RLWQGLGN#FSVN#TSKGNTAKNGGLLLSTNM*K.W | 0.6969 |
| IPI00061280 | F.SYATAAQN#NTVTDPK.N | 0.8079 |
| IPI00061780 | R.VSTN#GSDDPEDAGAGENRR.V | 0.7468 |
| IPI00061876 | K.KYLWEN#ETVGAQDDPL.A | 0.8062 |
| IPI00062751 | K.ILPISLEPSSSTEPTQSN#LSVTAK.I | 0.944 |
| IPI00063106 | K.N#DSDCGVFVLQY.C | 0.6791 |
| IPI00063106 | K.FNVATQN#VSTLSSK.V | 0.8109 |
| IPI00063120 | K.WIHTLTSLI.QN#ISSYYTSLPR.F | 0.5428 |
| IPI00063217 | R.N#DSIYEASSLYGISAMDGVPFTLHPR.F | 0.5123 |
| IPI00063408 | M.LPN#PSHLEAVNPVAVGKTRGR.Q | 0.5408 |
| IPI00063523 | R.ESEELECNTGSN#ITNMHQDK.E | 0.6076 |
| IPI00063523 | K.QN#KSPDTEKINYAGPLEETGISDITKKEK.E | 0.8635 |
| IPI00063523 | K.AN#LTDM*ESGSSNAMNMNVQHER.E | 0.7769 |
| IPI00063590 | K.VSN#LSLFGGLPANHVLVNQYLP.G | 0.8335 |
| IPI00063780 | -.MVDLLSM*SQN#ISPYKNPM*R.F | 0.6475 |
| IPI00063800 | R.SPPGEN#PSPQGELPSPESSRRL.F | 0.8459 |
| IPI00064174 | A.GFGNN#FTTVDN#K.S | 0.5261 |
| IPI00064201 | K.SSVTPAIISAALQQVVHN#K.S | 0.7279 |
| IPI00064219 | K.N#VTLEEDGTRAVRAAGYAHGLVFSTK.E | 0.6342 |
| IPI00064667 | R.LVPHM*N#VSAVEK.Q | 0.9997 |
| IPI00064667 | K.AIHLDLEEYRN#SSR.V | 0.9958 |
| IPI00064743 | K.AQNGIAIMVYTN#SSNTLYWELNQAVR.T | 0.9689 |
| IPI00065253 | K.ADN#HTAIIRIADQTALRVPSQAESSIFSQATN.G | 0.5338 |
| IPI00065348 | K.INLI.N#LTFCLFVWLTFNLPFLK.N | 0.8832 |
| IPI00065383 | K.YITVN#ISYVNIF.R | 0.9461 |
| IPI00065390 | K.CPECDQN#FSDHSYLVLIIQK.I | 0.5167 |
| IPI00065457 | K.LEVEDLDENFLN#SSYQTVFK.T | 0.9197 |
| IPI00065553 | R.VLTN#M*THEDDVPIN#CTMVLLHIVSK.C | 0.542 |
| IPI00066511 | K.VSSPLENEKLKSM*TIN#FSSLNR.K | 0.5623 |
| IPI00066511 | R.SYSVSGVCQPAIPN#SSLHIPHN.A | 0.7931 |
| IPI00067421 | -.MLTGVLLAN#GTLN#ASILNSLYNFNLVK.E | 0.6772 |
| IPI00067744 | F.FLTTPAIIM*NTIDMYN#VTRPIEK.L | 0.9394 |
| IPI00068174 | K.GKRMLSEYLSPN#LSLR.A | 0.9293 |
| IPI00069084 | D.SDMDPN#SSGEGVNSVSSS.I | 0.7917 |
| IPI00069084 | D.KAKKEHERSN#ASPAIFPEYQLWEDHWIR.C | 0.9279 |
| IPI00069126 | F.APFLN#NSPQQNPAAQIPAR.Q | 0.5403 |
| IPI00069232 | Q.IVIKMFQN#ISNIIKSGK.M | 0.6518 |
| IPI00069817 | K.N#ASMNTQHGTATEVAVETTTPK.Q | 0.9321 |
| IPI00070643 | R.EYNLN#FSGSSTIQEVK.R | 0.7141 |
| IPI00071171 | R.VGLCSCQVSLMLQN#SSAR.A | 0.9243 |
| IPI00071509 | V.VRSGASLLSN#M*SR.H | 0.7027 |
| IPI00072656 | K.LIKTDESVVDRAKAN#ASLWFAR.L | 0.6179 |
| IPI00073264 | V.SWEILSN#LSFLVTIQR.A | 0.5504 |
| IPI00073289 | N.DNFGIGGN#ISGLGGTGGSR.G | 0.5598 |
| IPI00073577 | R.RLSIGLDN#GTISEFILSLDYN.K | 0.5822 |

| | | |
|---|---|---|
| IPI00073730 | -.MNGGN#ESSGADRA.G | 0.6388 |
| IPI00075272 | M.N#GTSSQPKKEEYGS.- | 0.786 |
| IPI00080897 | N.KM*GQLGLGN#QTDAVPSPAQIMYNGQPITK.M | 0.9485 |
| IPI00081089 | K.LKLEAELGNM*QGLVSGM*QN#MSIHTK.T | 0.8603 |
| IPI00083235 | G.KVFN#DSGN#LSNHK.R | 0.8681 |
| IPI00083281 | K.VTRDALTEPLAIVEGYNSYFSFSRN#R.S | 0.8071 |
| IPI00083708 | K.SNEVVAVPTN#GTVNNVAQEPVNTL.G | 0.5435 |
| IPI00084434 | R.AQIFANTVDN#SSIALQTDNTHLAADDLR.V | 0.9349 |
| IPI00084684 | K.KSFACSSCN#YTFAKKEQF.D | 0.8025 |
| IPI00085314 | M.ESLALSN#ATGLSADGGAKR.Q | 0.7372 |
| IPI00090720 | K.NGQSLGDLDGIPIAVKDN#FSTSGIETTCASNMLK.G | 0.9304 |
| IPI00090972 | R.VKAISDSDGVSYPWYGN#TTETV.T | 0.9788 |
| IPI00091258 | R.FAN#GSAVIQSGDTAVMATAVSK.T | 0.5183 |
| IPI00091258 | N.GNSVALSLSDILWNGPVGTVXIGMTDGECVVN#PTRK.E | 0.6853 |
| IPI00092641 | K.FRNPPLVN#GSLALAFQGTAPPPNWRR.P | 0.6463 |
| IPI00097839 | V.YN#GSVDEGSKPGTYVMTVTANDADDSTTANGM*VR.Y | 0.691 |
| IPI00098769 | R.RVYDFVGLLVSPEMEQFALN#MT.S | 0.7711 |
| IPI00098827 | R.FIIVSAFDHFASVHSVSAEGTVVSN#LSS.- | 0.721 |
| IPI00099004 | K.YRETKSQESEELVVTGGGGLRRFKTIELN#ST.I | 0.708 |
| IPI00099111 | K.MSGGSTMSSGGGNTN#NSNSKK.K | 0.5752 |
| IPI00099433 | V.TQQMSN#ISGSCSM*LQQTSISSPPTCSVK.S | 0.6188 |
| IPI00099433 | R.RRIN#SSVTTETISETTEVLNEPFD.N | 0.7223 |
| IPI00099650 | K.RRKPGSHTHSASEDN#TTNNVR.E | 0.6515 |
| IPI00099688 | R.LGKPSVM*TPTEGLDTGEMSN#STSSLK.R | 0.9425 |
| IPI00099863 | E.YDTIPIITN#RTILK.E | 0.9414 |
| IPI00099890 | L.GLN#LSEGDGEEVYHF.- | 0.616 |
| IPI00100099 | K.YSTTTAQN#SSSSSSQSK.M | 0.6061 |
| IPI00100099 | R.GVN#GSPRISVTVGNIPKNSVSS.S | 0.7602 |
| IPI00100151 | R.M*QN#NSSPSISPN#TSFTSDGSPSPLGGIKR.K | 0.555 |
| IPI00100291 | K.EVTQATQPEAIPQGTN#ITEEKPGR.K | 0.9988 |
| IPI00100402 | K.THMNVLGVLGPLDPQWLVENN#FTGC.P | 0.6885 |
| IPI00100453 | K.KSSLDSN#SSEM*AIMMGADAK.I | 0.6373 |
| IPI00100715 | R.SSSGHLFTLPGATPGGDPNSN#NSNNK.L | 0.6021 |
| IPI00100984 | C.QAEAAAAN#GTGGEEDDGPAAELLEK.L | 0.9125 |
| IPI00101172 | R.WEALGNTLSSQPN#LTVSWDPR.I | 0.8018 |
| IPI00101261 | R.GM*GPM*GTPIMPSPADSFN#SSDNIYTM*TGGR.S | 0.5278 |
| IPI00101462 | K.THTN#ISFSHPN#ATFSAVGEASICEDDWNSGER.F | 1 |
| IPI00101462 | R.GLTFQQN#ASSM*CVPDQDTAIR.V | 1 |
| IPI00101462 | K.N#NSDISSTR.G | 1 |
| IPI00101952 | R.NPVTSTN#VLGMMTAILGVFLYN#KTK.Y | 0.9204 |
| IPI00102329 | K.DDWIRPALLSGPVAANVLN#FSDIHVIPM*PLLK.G | 0.7592 |
| IPI00102378 | K.LEILPEEIGQM*QKLRVLN#LSDNRL.K | 0.5894 |
| IPI00102543 | K.MNCNNRN#VSSLADLKPK.L | 0.7013 |
| IPI00102677 | R.LFEFFGGGGGFGN#VSQVGRVWPSSYR.A | 0.9512 |
| IPI00102678 | R.LVSN#DSFISIQPSLSSCGQDLPR.D | 0.7196 |
| IPI00102752 | R.SKKLGGSGGSN#GSSSGKTDSGGGSRR.S | 0.6252 |
| IPI00102829 | T.DGTHFESSN#LSEIESR.L | 0.6275 |
| IPI00102856 | M.MNYGQSMSGGNGQAAN#QTLSPQMWK.- | 0.5956 |
| IPI00103026 | F.SYPNGLSEN#FSVVEKLK.H | 0.7284 |
| IPI00103055 | K.HAAFFADAEGYFVACTFDTTMN#SSLSLPLYVPVK.F | 0.8229 |
| IPI00103277 | K.N#GTAVCATNR.R | 0.9995 |

| | | |
|---|---|---|
| IPI00103277 | K.LSDLSIN#STECLHVHCR.G | 1 |
| IPI00103277 | K.FLNN#GTCTAEGK.F | 1 |
| IPI00103288 | R.ASVVWM*AYM*N#ISFHVGNHVLSELGETGVFGRSSTLK.R | 0.6024 |
| IPI00103335 | E.DGLYGAPEPN#GSWTGM*VGELINR.K | 0.6009 |
| IPI00103380 | D.DSGATLLSAN#QTLRRLHNRR.T | 0.6553 |
| IPI00103419 | K.MEQKAKQNQVASPQPPHPGEITNAHN#SSCISNK.F | 0.5721 |
| IPI00103451 | K.DRCNVEKVPSNSQLEIEGN#SSGR.Q | 0.5878 |
| IPI00103487 | K.KLAEILVN#TSSENWIR.N | 0.7167 |
| IPI00103552 | K.VPTGTITEVSSTGVN#SSSK.I | 0.8545 |
| IPI00103552 | T.QHFYLN#FTITNLPYSQDKAQPGTTNYQRNKR.N | 0.9423 |
| IPI00103552 | K.FN#TTEXVLQGLLXPX.F | 0.7668 |
| IPI00103552 | R.KTNELPSDSSSSSDLIN#TSIAS.S | 0.7492 |
| IPI00103552 | K.N#TSVGPLYSGSRLT.L | 0.7448 |
| IPI00103552 | M.AAGPLLVPFTLN#FTITNLQYGEDMGHPGSRK.F | 0.5561 |
| IPI00103552 | R.EPGTSSTSN#LSSTSHER.L | 0.5329 |
| IPI00103577 | -.MERAPPDGPLN#ASGALAGDAAAAGGAR.G | 0.6536 |
| IPI00103606 | V.QVCSLPACGGNHQN#STVR.A | 0.5192 |
| IPI00103647 | R.MIVEIHLEEYNN#ISKKPM*NLVLFR.F | 0.5834 |
| IPI00103723 | K.GEISEKAKLEN#STQAEEGFDVPDCK.K | 0.7861 |
| IPI00103752 | K.RRTTN#RTIPSVDDFQNYLRVAFQEVNSGCTGK.T | 0.5825 |
| IPI00103755 | K.M*PYIQN#LSSLPTRTELR.T | 0.5618 |
| IPI00103772 | P.MQNFM*AGTAGVYQTQGLVGSSN#GSSHKK.S | 0.7419 |
| IPI00103871 | R.IQLEN#VTLLNPDPAEGPKPR.P | 0.9983 |
| IPI00103879 | R.TLQQLYEAYASKSN#NTAYLIYNDGVPK.P | 0.9018 |
| IPI00104074 | R.WGHSECGHKEDAAVN#CTDISVQK.T | 0.9993 |
| IPI00105353 | W.LNAQFDGNN#ETIK.W | 0.7496 |
| IPI00105532 | R.CTLHPN#DSLAMEGPLSRVKSLKKS.L | 0.7472 |
| IPI00106786 | R.VGVDPDQDPPPNN#DSFQVLQGDSPDSAR.G | 0.5136 |
| IPI00106795 | V.KVVMDIPYELWN#FTSAEVADLK.K | 0.6752 |
| IPI00107463 | K.LN#PTPGSNAISDAYLN#ASETTTLSPS.G | 0.7419 |
| IPI00107463 | K.WKNIETFTCDTQN#ITYR.F | 0.7143 |
| IPI00107617 | K.HSSGSSN#TSTANRR.A | 0.6024 |
| IPI00107642 | R.KFKTNVLPFRQN#DSSSHCQKSGSPISSEERR.R | 0.6554 |
| IPI00107728 | K.VDSN#DSLYGGDSKFL.A | 0.6038 |
| IPI00107838 | R.ASSPN#STVSN#TSTEGFGGIM*SFASSLYR.N | 0.6865 |
| IPI00140246 | M.DTRN#LSLAHNR.I | 0.8804 |
| IPI00140449 | I.SNPLHCN#MTMTPGTCR.V | 0.5482 |
| IPI00141118 | M.N#NSCLTNAVHLNN#VSVVSPVNVHINTR.T | 0.7235 |
| IPI00141559 | M.LFLSMN#LTISAGPASTLPTATPAAGELTMR.S | 0.7546 |
| IPI00142487 | R.RSLN#SSSSSSPPSSPTM*MPR.L | 0.6373 |
| IPI00142538 | M.KNSCNVTHPQSPN#NSNR.Q | 0.8927 |
| IPI00142768 | S.ISHDNNN#ISSTSELGTDLANTK.V | 0.8639 |
| IPI00142919 | R.ILVN#LSMVENKLVFLEHTLLSK.G | 0.5 |
| IPI00142289 | R.VRRTDDTPVVLVGN#KSDLKQLRQVTK.E | 0.5634 |
| IPI00146438 | G.IKVKN#HSGGGMSLTHNKNFRK.L | 0.6838 |
| IPI00147583 | M.LGSEM*XGQN#VSNPAPSPSLSGVSWPDNVPK.I | 0.6936 |
| IPI00147583 | R.VWQN#LSEPIR.P | 0.5571 |
| IPI00147633 | R.KN#ASALYEKIR.G | 0.5294 |
| IPI00147702 | K.HTGSGILSMANAGPN#NGSQFFICTAK.T | 0.6152 |
| IPI00148050 | K.GACN#GSVDCEDFTNHNILQAR.D | 0.7687 |
| IPI00149695 | K.QVGFKAM*N#ASAN#ITSDGVEVLGKMVR.S | 0.9047 |

| | | |
|---|---|---|
| IPI00151777 | M SSHFYINDVN#FTRKMLLM*FFEVSAHE.S | 0.6298 |
| IPI00151888 | M.N#GSGQSPSVLKGILHEAAMQYPK.Q | 0.9394 |
| IPI00151982 | M.KENPAKEQLWALEQDN#CSLANLVCK V | 0.6234 |
| IPI00152048 | M VVLCASTLPDWRNAAADN#RSLDDR.S | 0 7416 |
| IPI00152075 | R.FVRLGTASMLTSPDGPFIN#LSR.L | 0.911 |
| IPI00152101 | K.KDGEN#VSM*KDPPDLLDRQK C | 0.6088 |
| IPI00152101 | K N#ETVSSN#SSN#NTGN.S | 0 7436 |
| IPI00152101 | K.VLQAMGYPTGFDADIECMSSDEKSDN#ESKN#ET.V | 0.7046 |
| IPI00152254 | R.QRN#ASRDQ.V | 0.6481 |
| IPI00152295 | K.VRRPSPN#RSKLSNVARK.A | 0.7394 |
| IPI00152316 | M.TWSFGWN#SSLPVYYIR.E | 0.5125 |
| IPI00152391 | K.VKFGMN#VTSSEK.V | 0.5162 |
| IPI00152410 | K LKTN#VTFPLDILLLSFK.A | 0.6682 |
| IPI00152418 | K.TLSTKTPSAAQNPMMTN#ASATQATLTAQK.F | 0.5389 |
| IPI00152427 | R CDKAFN#QSAN#LTK.H | 0 9383 |
| IPI00152440 | K.EKDSN#SSSGSFNGEQEPIIGFQPMDSTR.A | 0.828 |
| IPI00152468 | L.YN#DSTYNQQLIIPSIGLPLK.T | 0.9822 |
| IPI00152474 | K.N#TSNKEISRDTLLTIENNP.C | 0.7336 |
| IPI00152510 | R.SRATIFEIN#ASSRDLCSQVMRAKR Q | 0.6619 |
| IPI00152513 | R GNIYPGN#DTFDIDPEIE.T | 0.9361 |
| IPI00152524 | M.NRRNILVMKHN#YSQDAADACDIDEIEEVPTTSIIR.L | 0.9451 |
| IPI00152527 | A IYFENLQN#SSNDLGDIISMKER.D | 0 8129 |
| IPI00152540 | K.FDILMTSNEIN#ATGHQQTLLVPSEDGA.T | 0.6607 |
| IPI00152540 | M.EAVQKIN#YTVPQSGTFK.I | 0.7268 |
| IPI00152542 | R M*DDVPSHSKALSDGN#GSSGIEWS.N | 0.5941 |
| IPI00152542 | L.IHALATN#SSSELFRLAAHPLNNR.M | 0 791 |
| IPI00152581 | Q RN#ISLQLM*SNM*N#ISNKIR.N | 0.9822 |
| IPI00152602 | K.MFFETNENN#DTTYQNLWDA.F | 0 597 |
| IPI00152602 | K N#LTQSHSTTWKLNNLLLNDYWVHNEMK A | 0.9032 |
| IPI00152615 | L KLGVVPVYYGSPSITDWI.PSN#KSAILVSEFSHPR.E | 0.756 |
| IPI00152627 | A.HN#MSGPN#SSSEWSIEGRR L | 0.5616 |
| IPI00152627 | K ALATSMLTGEAGSLPSTHMVVAGMAN#STPQ Q | 0 5357 |
| IPI00152642 | R.VQPAQN#IISSLSN#VSQAVASTTPL.PPPK P | 0.5464 |
| IPI00152647 | K.N#LTAVKSGGTSDSFVKGYLLP.D | 0 7705 |
| IPI00152661 | M.DFGDSSGVEM*RLHN#M*SEAMAVTAYHQYSK G | 0.627 |
| IPI00152696 | K.LPHN#GSTGSTPLLR.N | 0 7825 |
| IPI00152720 | P FPGN#M*SSM IPSSPGM*SQQGGPGMGPPMPI VNR.K | 0.741 |
| IPI00152788 | L.VISGLSAAEGGN#TSDTQSSSSVNIVMGPSAR A | 0.7899 |
| IPI00152797 | K.DNKYTLN#QTSAVFDSIPEVVHYYSNEK.L | 0.7721 |
| IPI00152818 | K LSDSN#QTLKVIGEFILER N | 0 7604 |
| IPI00152849 | K.N#PTTEFTVLTK T | 0 3801 |
| IPI00152944 | R LM*AFGCVSGSVQVYTIDN#STGAMLLSHK I | 0.837 |
| IPI00152985 | K M*IGLEDTVADN#YSK.I | 0 9678 |
| IPI00154162 | K ELFGDDSEDEGASHHSGSDN#HSER S | 0.9915 |
| IPI00154451 | K.SGN#YTVI QVVEALGSSLENPFPRTR.A | 0 7456 |
| IPI00154325 | M RGIETVLLIKN#NSVARAVM*QSQK P | 0.963 |
| IPI00154585 | R.IAQKGGAEAM I.VVN#NSVLFPPSGN#R S | 0.5979 |
| IPI00154813 | K EANIN#STSISDDNSASI R C | 0 5682 |
| IPI00155237 | K.AKAGFSEWI AVDGLGSPSN#NSKE D | 0.5323 |
| IPI00155637 | M.VDAASYAAN#LTDSAEAPKGSPGSWWKK E | 0 7446 |
| IPI00155729 | R LVVGDFSDYN#NSYVGAFADAR S | 0 5716 |

| | | |
|---|---|---|
| IPI00156651 | R YQQLAVALDSSYVTNKQLN#ITIEK.L | 0.6814 |
| IPI00157364 | K.NYEDEPNNYRTMHGRAVN#GSQLGK.D | 0.687 |
| IPI00157589 | R KN#ITNDIR.T | 0 6462 |
| IPI00157790 | R.YIRTLMSSGQMAPSSSN#K.S | 0 5034 |
| IPI00157790 | R.QN#SSSAQGSSSNSGGGSGIPQP.P | 0.6873 |
| IPI00158615 | T.PKGN#SSNGNSGSNSNK A | 0 7669 |
| IPI00158615 | K.LYDQCHDTLVQFGGFLASN#LSTEDY.I | 0.589 |
| IPI00159049 | S.PARQN#VSSASNPEN#DSSHVR.I | 0.6941 |
| IPI00159322 | K GNKNGDN#NSNHNGEGNGQSGHS.A | 0 5747 |
| IPI00160130 | R.FQFCGRN#ASAVPVFYSSM*STAMVIFKSGVVNR N | 0.794 |
| IPI00160130 | K.LCSSVN#VSNEIK.S | 0 7188 |
| IPI00160131 | K.ISNVALDSMHWQN#DSVQ I | 0.8062 |
| IPI00160131 | V.ERPSSLLSLN#TSNK.G | 0.6142 |
| IPI00160265 | R.N#ITIMASGNTGGEK D | 0.5234 |
| IPI00160290 | R TNSRLSHMPPLPLN#PSSN#PTS.L | 0 6013 |
| IPI00160316 | N #LTQNLMQN#LTQSLSQKENR E | 0.7429 |
| IPI00160348 | T N#ASPEKTTYDSAEEENKENLYAGK.N | 0.5938 |
| IPI00160395 | K AIIN#STVTPN.M | 0.5471 |
| IPI00160432 | K.VDPETNKN#ITRGQSLDNLIK.V | 0.5003 |
| IPI00160566 | K.AFSQN#ISLVQHLR.T | 0.7411 |
| IPI00160901 | K SSEFASIPAN#SSRPLSN#ISKSGR.M | 0.7207 |
| IPI00162732 | R.EQQN#DTSSELQNR.E | 0.5727 |
| IPI00163147 | R.DNGPDGMEPECVIESNWNEIVDSFDDMN#LSESLLR.D | 0.8212 |
| IPI00163207 | R.LYIIFLLGAWSLN#ATELDPCPLSPEI LGLTK E | 1 |
| IPI00163207 | R.GFGVAIVGN#YTAALPTEAALR T | 1 |
| IPI00163207 | R.LEPVHLQLQCMSQEQLAQVAAN#ATK E | 1 |
| IPI00163328 | T PVPGYMN#NTVNTM*R L | 0.5923 |
| IPI00163446 | R.EVN#TSGFAPARPPPQPGSTTFWAWSVLR.V | 1 |
| IPI00163446 | R.TLLN#ASR.S | 0.9762 |
| IPI00163504 | K.VLEAN#ATPLDRGDGVLRTCALR P | 0.9855 |
| IPI00163507 | R.TRSTSSAGSN#NSAEGAGLTDNGCR.R | 0.7505 |
| IPI00163644 | K GILYGTMTLELGGTVN#ITCQK.T | 0.5468 |
| IPI00163749 | R KQAGPLLSGDPHLLPPAASPKGASVSINVN#TSLEDMRS.N | 0.5218 |
| IPI00163782 | K.RPLEDGDQPDAKKVAPQN#DSFGTQLPPMH.Q | 0.9462 |
| IPI00163866 | K.QKN#SSDQEGNN#ISSSSGHRVR.L | 0 6176 |
| IPI00163904 | K.NTNQN#SSAHPPIILNMDDTVN#QSNIELKNVNR.N | 0.7566 |
| IPI00164104 | I QIRN#VSQLPATWRM*K E | 0.7807 |
| IPI00164104 | F TQNLLLEYTN#QTTQAR P | 0.5026 |
| IPI00164246 | R.DGEQSPN#VSLM*QRMSDM*LSR W | 0.7974 |
| IPI00164345 | R KQSESSFISGDIN#STSTLNQGLTSHGLR A | 0 7627 |
| IPI00164356 | K SFLNAFSFEIN#NSMIILSLSPTTFK N | 0 5919 |
| IPI00164356 | M DGN#DSDYDPK.K | 0.5836 |
| IPI00164623 | K TVLTPATNIIMGN#VTFTIPANR E | 1 |
| IPI00164623 | K HYLMWGLSSDFWGEKPN#I SYIIGK D | 1 |
| IPI00164623 | K.VVPEGIRM*N#K.T | 0 8373 |
| IPI00164755 | K GEPGAFGEN#GTPGQTGAR G | 0 8205 |
| IPI00164831 | K RYEDGTISSN#ATHVEHPI CPPK P | 0 6156 |
| IPI00164930 | R HIANSIRTHGTGIMN#FTKWAFFAWRGG.P | 0 6579 |
| IPI00164898 | N #KTTSLGQMENNNLDELN#KSKIPVKKK P | 0 6713 |
| IPI00165021 | M IQNTFN#FSLK Q | 0 9781 |
| IPI00165024 | K SKEQN#VSDDPFSTGFLYPYNDLLVWAVLM*KR Q | 0.5517 |

| | | |
|---|---|---|
| IPI00165064 | R.RYTN#SSADNEECRV.P | 0.6205 |
| IPI00165171 | R.VGGFGFVATPSPAPGVN#ESPM*MTWGEVENTPLR.V | 0.5058 |
| IPI00165210 | K.LAKIRDNLAISLDN#QSSPSPPVL.I | 0.5037 |
| IPI00165246 | R.FM*GPASGM*N#M*SGMGGLGSLGDVSK.N | 0.8641 |
| IPI00165250 | L.GNTKDFIISFDLKFLTN#GSVSVVLETTEK.N | 0.6943 |
| IPI00165319 | K.TSIAQSVLQSLPSSQWSVLVVN#MSAQ.T | 0.869 |
| IPI00165357 | K.CSVALLN#ETESVLSYLDKE.D | 0.9629 |
| IPI00165438 | R.GPECSQN#YTTPSGVIK.S | 0.9998 |
| IPI00165598 | R.N#PSQLPALSSSPAHSGM*MGINSYGSQLGVSI.S | 0.6384 |
| IPI00165934 | R.EPAQGLFGTVTVQFIVTEVN#SSN#ESK.D | 0.6157 |
| IPI00165934 | M.TSWISPAVN#NSDFWTYRK.N | 0.6995 |
| IPI00165934 | F.QLM*N#ITAGTSHVMISR.R | 0.5155 |
| IPI00165934 | N.DQLSEIEEFFYIN#LTSVEIRGLQK.F | 0.5067 |
| IPI00165979 | V.KPYVN#GTSPVYSR.E | 0.9069 |
| IPI00165981 | R.HDQEN#DTR.W | 0.6783 |
| IPI00166010 | K.DVPPSIN#TTNIDTLLVAT.D | 0.5707 |
| IPI00166010 | M.LLN#GTPFAFVIDLAALASRR.E | 0.513 |
| IPI00166010 | R.N#LTAGMAMSTCR.E | 0.5019 |
| IPI00166031 | K.TRPQN#GSM*ILYNRK.K | 0.8987 |
| IPI00166078 | K.HPENNQKSENNQKLLTGAN#SSR.F | 0.7187 |
| IPI00166078 | L.RTTNGRLNIDNLN#LSFRK.E | 0.604 |
| IPI00166086 | L.NRIPGVPGSM*PN#ASWTGNLR.A | 0.9053 |
| IPI00166121 | K.LVGLN#LSPPMSPVQLPLR.A | 0.7296 |
| IPI00166145 | R.LLN#LSFCGGISDAGLLHLSHM*GSLRSLNLR.S | 0.8326 |
| IPI00166161 | R.QKEN#DTQIFN#DSAVDN#HSK.C | 0.816 |
| IPI00166161 | K.SIEN#DSDEVEERAENFPR.T | 0.8327 |
| IPI00166201 | M.PTFCIPENHCGTHAPVWLN#GSHPLEGDGIVQR.Q | 0.7812 |
| IPI00166283 | R.RGAIKHQVN#FSSGGVAPLGGSWHR.L | 0.8876 |
| IPI00166301 | S.PQRSSM*NN#GSPTALSGSKTNSPK.N | 0.6801 |
| IPI00166323 | K.WLPN#STTTCSLSPDSAI.L | 0.7488 |
| IPI00166392 | R.FQLLN#FSSSELK.V | 0.9999 |
| IPI00166500 | S.VN#GSGALGSTGGGGPVGSM*FNGK.P | 0.6749 |
| IPI00166533 | R.FLNN#DSSGAEANSEK.Y | 0.772 |
| IPI00166652 | K.TDEKLN#VSDENTASCPLSPIK.M | 0.8775 |
| IPI00166705 | R.GM*YLVFDGSVDLHYN#CSAKCK.S | 0.9101 |
| IPI00166713 | K.AYAGRKQHYIAGN#CSSNGR.G | 0.5749 |
| IPI00166729 | R.FGCEIENN#R.S | 0.9998 |
| IPI00166729 | K.DIVEYYN#DSN#GSHVLQGR.F | 1 |
| IPI00166729 | R.GDVLHNGN#GTYQSW.V | 0.8111 |
| IPI00166842 | S.TSGLLN#STWPLPSATQR.C | 0.787 |
| IPI00166861 | K.VIQM*DVALFEMN#QSDSK.E | 0.5371 |
| IPI00166863 | K.FEALKEENMDLNNM*N#QSLTL.F | 0.9357 |
| IPI00166930 | R.LEDLEVTGSSFLN#LSTNIFSN#LTSLGK.L | 1 |
| IPI00166930 | K.LYLGSNN#LTALHPALFQN#LSK.L | 1 |
| IPI00166930 | K.LGSLQFLFLDSNN#ISELPPQVFSQLFCLER.L | 0.9989 |
| IPI00166930 | R.AFGSNPN#LTK.V | 0.9987 |
| IPI00166930 | R.WLNVQLSPWQGSLGLQYN#ASQEWDLR.S | 0.9721 |
| IPI00166930 | R.NAIHHLPLSIFASLGN#LTFLSLQWNM*LR.V | 0.7729 |
| IPI00166972 | M.SGRFETEKVN#TSPSVNTKTTTESK.A | 0.6357 |
| IPI00166979 | K.WN#FSSGHEAVFK.H | 0.5225 |
| IPI00167009 | M.KN#ATSSKQLPLEPESPSGQVGPRPAP.P | 0.3009 |

| | | |
|---|---|---|
| IPI00167036 | M.RKLGHLNN#FTK.L | 0.7118 |
| IPI00167074 | K.RSVLPPDGN#GSPVLPDKR.N | 0.9653 |
| IPI00167103 | R.SAPSGGGASFN#LSLTEEHSGN#YSCEANNGLGAQR.S | 0.6969 |
| IPI00167131 | R.NLDPEN#GSGMALQPLQAAPEPGAQGQR.E | 0.7197 |
| IPI00167172 | R.KLFQEILN#TSR.E | 0.657 |
| IPI00167196 | K.HLSDYCIGPN#ASINVIM*QPLEK.M | 0.5303 |
| IPI00167238 | K.EIKGIQIGREEVN#LSLLADDMILYLENPVVSAQR.P | 0.8465 |
| IPI00167254 | D.PLTFNFISSLKAICTEIAN#CSLK.V | 0.703 |
| IPI00167513 | R.DCYYDN#STTCPKCARLSLR.K | 0.5354 |
| IPI00167549 | T.QIIN#GSVDVDTEDRQK.R | 0.5016 |
| IPI00167560 | K.N#ESNLGDLLLGFLK.Y | 0.5532 |
| IPI00167574 | M.LFNVEN#GTPASR.E | 0.9511 |
| IPI00167574 | V.PTVFAFQDPTQQVRENTDPASERGN#ASSSQK.E | 0.8038 |
| IPI00167706 | G.CNHN#STQILVNCLR.A | 0.5296 |
| IPI00167778 | M.QTTDLEQTSPPVNQAPN#QTKLEVK.A | 0.8726 |
| IPI00167801 | K.M*PPGIN#SSQSLPVDNHE.K | 0.7964 |
| IPI00167830 | R.TSNGQPVKTAGEITQHN#VTELLR.D | 0.8741 |
| IPI00167841 | R.VFTEEAKDSLN#TSEN#DSEHQ.T | 0.944 |
| IPI00167841 | M.LISVESPN#LTTPITSN#PTDTR.K | 0.667 |
| IPI00167860 | R.TSHGEPKSAVPFNQYLPN#KSN#QTAYVPAPLRK.K | 0.8829 |
| IPI00167867 | D.SSPEHN#LTKIANGVPNSK.G | 0.7867 |
| IPI00167908 | Q.QTTN#TTSTQMTNIGVYVSN#M*TDK.L | 0.7188 |
| IPI00167910 | R.NINGLFLPPSSN#ITLQK.E | 0.6072 |
| IPI00167941 | K.ILQPN#TTDEFVIPLDPR.W | 0.528 |
| IPI00168043 | M.GTPSQTSQDTSLETGQGYEDEQDGWN#SSSKTTR.V | 0.8964 |
| IPI00168056 | K.SYRN#SSYENARENSQMN#ESAPGTYVVQNPH.S | 0.8354 |
| IPI00168060 | M.KDFLTDRSN#QSHLVGVPKPGVPQTPVNK.I | 0.5068 |
| IPI00168154 | M.RANGN#TTSNKNSAAM*DAEIVLR.S | 0.9437 |
| IPI00168255 | R.FGSGAAGGSGSSN#SSGDALVTRISILLR.D | 0.6684 |
| IPI00168280 | R.SLTYLSIN#CTSISLNMFSLLHDILHE.P | 0.8199 |
| IPI00168352 | M.TN#GTLEPAAEWSVLLGVIISQDGPLDGAHTR.A | 0.575 |
| IPI00168406 | K.N#NSYSLAFLAGKLNSKVFRS.Q | 0.8794 |
| IPI00168431 | K.DYPSN#TTSSTSNSGN#ETSGSSTIGETSKKK.R | 0.924 |
| IPI00168442 | R.HM*DM*LTAADRLPTQAPLSTSQSVSGKN#M*TASQGP.C | 0.6251 |
| IPI00168475 | P.DRN#CSWALGPPGAALELTFR.L | 0.5633 |
| IPI00168525 | K.NKAQN#ITAPESEAICWQ.L | 0.7609 |
| IPI00168526 | R.KATM*AGGLANLQDLEN#TTPAQPK.N | 0.5775 |
| IPI00168526 | R.SPTQGYRVTPDAVHSVGGN#SSQSSS.P | 0.7164 |
| IPI00168627 | R.GRDGGEGCSWM*FQPM*N#NSKM*R.V | 0.9536 |
| IPI00168627 | K.VDTN#ENSVNTMN#R.S | 0.961 |
| IPI00168632 | K.QHLQIN#WTGLTNLLDAPGINDVSDS.L | 0.9894 |
| IPI00168728 | R.EEQFN#STFR.V | 1 |
| IPI00168745 | K.M*DFLLFN#YSAPSYLRLL.- | 0.6143 |
| IPI00168759 | M.N#ISLAFFLYDLLSLM*DR.G | 0.7068 |
| IPI00168839 | E.ALAAMQDPEVMVAFQDVAQNPAN#MSK.Y | 0.9109 |
| IPI00168868 | R.AFPTPSPANAHVN#GSADAPENAIHLAEAGGLCGESR.A | 0.8508 |
| IPI00168931 | K.VANN#VTEHFLGLSQDSGM*R.W | 0.8801 |
| IPI00168954 | M.GDVN#QSVASDFI.L | 0.7879 |
| IPI00169020 | -.MTLVSFFSFLSKPIIMLLSN#SSWR.L | 0.8454 |
| IPI00169030 | S.N#LSIIDVCYISSTVPK.M | 0.7937 |
| IPI00169113 | R.YPIIM*NKVVYVLLTSVSWLSGGIN#STVQTSLAM*R.W | 0.718 |

| | | |
|---|---|---|
| IPI00169156 | - MSFLN#GTSLTPASFILNGIPGLE.D | 0 8803 |
| IPI00169179 | R CPQCDCITLQN#VSAGLNHHQTF.S | 0 7436 |
| IPI00169288 | R AN#NSDFGLVAAVFTNDINK A | 0 5559 |
| IPI00169303 | R.FRTVN#STSWMEVNFAKNRK.D | 0.7314 |
| IPI00169325 | K.FN#KSLGHGLINKK.R | 0.8613 |
| IPI00169385 | K GNCEDYLMISCN#NSDGIENR.N | 0.8012 |
| IPI00169401 | K.ERPISMINEASNYN#VTSDYAVHPM*SPVGR.T | 0 9183 |
| IPI00169420 | R.GVEGPQGSPRPPAPIQQLN#RS.S | 0.5758 |
| IPI00169440 | V.FNILFVTSEN#GSR.N | 0.8134 |
| IPI00170428 | E DDFQHSSN#STYR.T | 0.7862 |
| IPI00170549 | K.ERIINYAN#SSDPTSGVSKRK.S | 0.9595 |
| IPI00170594 | K.SM*ADVLGDGGN#SSLTISEGP.I | 0.8441 |
| IPI00170605 | R.WQALVQVQPSVDPTN#ATGLDGR.E | 0.7801 |
| IPI00170667 | K.ASPSENNAGGGSPSSGSGGN#PTN#TS G | 0.5378 |
| IPI00170675 | M.RKLHLGSSLDSSN#ASVSSSLSLASQK.D | 0.9167 |
| IPI00170730 | R.SSSFGSVSTSSN#SSK.G | 0.6301 |
| IPI00170766 | K DPRNLLAN#QTLVYSQDLGEMTK.L | 0.6412 |
| IPI00170778 | K.TGTDSN#STESSETSTG.S | 0.9363 |
| IPI00171002 | K RTTYLN#ITNTLN#ITNNNYYSVE.V | 0.8805 |
| IPI00171015 | K.AGHQVMVFVHARN#ATVRTAMSLIER A | 0 619 |
| IPI00171052 | R.VKVQDLVLEPTQN#ITTKGVSVRRKR.Q | 0.9171 |
| IPI00171111 | M NSNLPAEN#LTIAVN#MTK T | 0 6397 |
| IPI00171111 | W EDTQN#ASQNKIKIVGLGLLR.V | 0.7109 |
| IPI00171134 | K.RRKELGAMAFSTTAIN#FSTVN#SSAGFR.S | 0.7412 |
| IPI00171134 | K.KLCGENDRLN#HTYSQLL K | 0.6624 |
| IPI00171134 | M.NQNAQLLIQQSSLENEN#ESVIKER.E | 0.5445 |
| IPI00171176 | K.N#FSSLHTVFCATGGGAYK F | 0.6267 |
| IPI00171183 | C LN#LSN#TTITN#R T | 0.8748 |
| IPI00171206 | R.YM*IYEFWEN#SSVWNSHLQTN#YS K | 0.533 |
| IPI00171211 | M.ASFLKN#VSATVSIN#GSGISGNTAINYK.H | 0 6028 |
| IPI00171312 | K.N#SSEFPLFSYNNGVVMTSCR.E | 0.8799 |
| IPI00171509 | K.KRQEENSQN#SSEKVM*FQSTHILPDEEKMVK.E | 0.5659 |
| IPI00171537 | R.NMYTVQN#NSGPYFNPR S | 0 555 |
| IPI00171636 | K.AKAVALDSDN#ISLK.S | 0 5793 |
| IPI00171636 | R QN#SSDSISSLNSITSHSSIGSSKDADAK.K | 0.7472 |
| IPI00171636 | K QKSLTN#LSFLTDSEKK.L | 0.7166 |
| IPI00171678 | R LEVHYHNPLVIEGRN#DSSGIR L | 0 999 |
| IPI00171678 | R.SLEAIN#GSGLQM*GLQR V | 1 |
| IPI00171678 | K.ALYSFAPISMHCN#K.S | 0.9956 |
| IPI00171716 | K MNM*NVMEEAIGYFEQQLAM*LQQLSGN#ESVLDR.G | 0.5175 |
| IPI00171768 | R FVEGTNIN#RSLLALGNVINALADSK.R | 0.5511 |
| IPI00171791 | S LSNIVRN#LTPAPLFSTPPLRS.- | 0.7344 |
| IPI00171921 | R GHN#LSRDELR G | 0.6891 |
| IPI00171928 | R I FLGN#YTGNVGNDALQYHN#NTAFSIK D | 0 5025 |
| IPI00172422 | K YKPLN#TTPN#ATK.E | 0 7977 |
| IPI00172530 | P DFRN#MTGLVDLTLSRNAITRIGAR A | 0 5919 |
| IPI00172636 | K LXGAILTTMLATRN#FSAAK S | 0 8405 |
| IPI00173346 | M.ASYLETMN#IFLKQQLVKVYEK.Y | 0 5949 |
| IPI00173359 | K.LLKEQAHN#LTIEM*K N | 0 7443 |
| IPI00173359 | M MSNQYVPVKTHEEVKMTLN#DTLAKTNR E | 0 5604 |
| IPI00173448 | M N#NSLDYLAYPVIVSNHRQSTTTK K | 0 9527 |

74

| | | |
|---|---|---|
| IPI00173492 | G.DYEPIDATGFIN#ISSLRLK.E | 0.579 |
| IPI00173844 | K EMEEFVQSSGENGVVVFSLGSMVSN#M*TAER.A | 0.778 |
| IPI00173934 | K.HTGPGILSM*ANAGLNTN#GSQFFICTAK.T | 0.6632 |
| IPI00173934 | K.GSCFHSIIPGFMCQGGDFTLLN#GTGGK.S | 0.9033 |
| IPI00174153 | R.LGTFTTQN#ASAPRNPETPGSPVPPSGR P | 0.5624 |
| IPI00174771 | M HSSN#FSSSN#GSTEDLFR D | 0.5344 |
| IPI00174772 | K.QEN#SSQENEN#KT.K | 0.6531 |
| IPI00174837 | K.NIKHSGN#ITFDEIVNLAR.Q | 0.6773 |
| IPI00174865 | M.ERESLKSPFTGDTSM*NNLETVHHN#NSKADKLK.E | 0.772 |
| IPI00174978 | P.FKYIYELNN#VTPLDNLLN#LSNEILN#AS - | 0.976 |
| IPI00175108 | K.ALRTDYN#ASVSVPDSSGPEHILSISAGIDTIGEIL.K | 0.523 |
| IPI00175146 | M.DQMAVLLVSNIN#ESK.G | 0.6722 |
| IPI00175151 | R.DLHNMQN#GSTLVCTLTR E | 0.9403 |
| IPI00175296 | K.SFNCN#SSLIKHWRVHTGER.P | 0.5582 |
| IPI00175421 | K.SFSLN#RTLTVHQRIHTGEK.P | 0.8543 |
| IPI00175439 | K.GDFESQN#SSLESSISQVINLEK.N | 0.7617 |
| IPI00175448 | R.ESTGAQVXM*AGDMLPN#STEQAITIAGIPQSIIECVK.Q | 0.6594 |
| IPI00176188 | -.MDPN#CSCAASDSCTCAGSCK.C | 0.68 |
| IPI00176196 | L LKAN#NTLLKMGYHFELPGPRM*VVTNLLTR N | 0.8041 |
| IPI00176210 | K.HGNLRNVLILMDQSAWDSN#ATLR.Q | 0.5051 |
| IPI00176376 | K.N#GSGNAIIIVVGGAAESLSSM*PGK.N | 0.9906 |
| IPI00176482 | K.GTSSSPLAVASGPAKSSSMTTLAKN#VTN K | 0.8454 |
| IPI00176482 | I LGKNEEAN#VTIPLQGFPRK.E | 0.5105 |
| IPI00176568 | R.WHIN#FTTFFIDCM*AAFGLAYDQK K | 0.6687 |
| IPI00176590 | R GGN#FSGRGGFGGSHGGGGYGGSGDGYNG F | 0.5039 |
| IPI00176709 | K.KLSN#GSIVPLEDSLNLIEVATEVPKRK.T | 0.7248 |
| IPI00176843 | R.SQSANQVCGYVKSNSLLSSN#CSTWKYFICEK.Y | 0 9477 |
| IPI00177323 | E.NIM*AGATVLFLN#ATDLDR.S | 0.581 |
| IPI00177394 | M SQSMGGDN#LSSLDTNEAEIEPENMR E | 0.5416 |
| IPI00177498 | R.KENSFLTHQHGN#DSEAFGEVVCR.L | 0.5182 |
| IPI00177509 | K.EN#STLNCASFTAGIVEAVLTHSGFPAK.V | 0.6072 |
| IPI00177824 | R.TPN#SSCSTPSRTSSGLFPR I | 0 8676 |
| IPI00177884 | M.QQFLYEISNLDTLTN#SSSFEGY.I | 0.658 |
| IPI00177884 | R LN#SSSVSNLAAVGDLLISSQASLTAALGLR P | 0 5714 |
| IPI00177940 | R.RLEGTN#VTVNVLHPGIVR T | 0.5013 |
| IPI00177967 | D.SFSQASN#VTSQLPGFPK.Y | 0 5069 |
| IPI00178015 | K.LNQLYN#CSSSLSFM*DS C | 0.7591 |
| IPI00178140 | Y.TP TGEPVFGGLPQN#ASLIAHLAR T | 0 5825 |
| IPI00178319 | H.GRYIASIMEN#GSLNIYSVQALTQEINK E | 0.6701 |
| IPI00178324 | M IMIMN#GTLYIAAR D | 0.9495 |
| IPI00178349 | R.EDCNGIFRIN#VSVSKNLNI KLR.P | 0.8474 |
| IPI00178386 | E.VFENLDGDLGN#STEK.Q | 0.7858 |
| IPI00178386 | K YFLFGLQDGN#DTFALLGK.A | 0.8505 |
| IPI00178415 | Q.STM*LDTNSWIFACIN#STSM*CLQGVDLSWK A | 0 8056 |
| IPI00178607 | R.SCRAAQAVI*DCFVNN#GSSLR.D | 0.7522 |
| IPI00178667 | L.APNQYVISGEVAILN#STTIEISELPVRTW TQTYK E | 0.5461 |
| IPI00178673 | R TALFPDLLAQGN#ASLR L | 0.5432 |
| IPI00178675 | M M*LGDAKIGN#NSVSSLK N | 0.554 |
| IPI00178676 | K.N#ITDELGVLGVAGPQARK.V | 0 7784 |
| IPI00178767 | M.LQYYLN#I TEANLKGESIWK L | 0 71 |
| IPI00178926 | R EN#ISDPTSPLR T | 0 9997 |

| | | |
|---|---|---|
| IPI00179053 | P.PNN#VSVPLLM*PLVTLMER.Q | 0 5958 |
| IPI00179057 | K.LN#SSSSSSSN#SSNER.E | 0.8713 |
| IPI00179071 | K.QN#NTNANKPK.K | 0 9727 |
| IPI00179071 | K.QQFNTQN#QSNVM*PGPAQIMRGPTPN.M | 0 8669 |
| IPI00179131 | K.INCIRPDAFQDLQN#LSLLSLYDNK.I | 0.9323 |
| IPI00179193 | K.WSCTEASN#TSPTMSAAQNAE.- | 0.9954 |
| IPI00179193 | N.QAGDTSN#QSSGP.H | 0 635 |
| IPI00179326 | K.LLEEN#ETEAVTVPTPSP.T | 0 5796 |
| IPI00179357 | K.N#ASGTKAVSVMVK.V | 0 5661 |
| IPI00179357 | E.SFVEMSSSSFM*GISN#MTQLESSTSK.M | 0.9308 |
| IPI00179357 | K.WRRPDYDGGSPN#LSYHVERR.L | 0.8427 |
| IPI00179357 | K.VNRYDAGKYTIEAEN#QSGKK.S | 0.6046 |
| IPI00179357 | R.AN#HTPESCPETKYK.V | 0.5494 |
| IPI00179377 | K.LVQDVAN#NTNEETGDGPTTATVLAR.S | 0.7937 |
| IPI00179415 | K.ALTSETN#GTDSN#GSN.S | 0.5723 |
| IPI00179453 | M.FCNQQSVCDPPSQNNAAN#ISMVQAASAGPPSLR.K | 0.7732 |
| IPI00179468 | R.NIYQPPEGN#ASVIQDFTEDGHLLHTFYLGTGRR.V | 0.8474 |
| IPI00179582 | K.LRPVTLTEMN#YSKYGAK.E | 0 7547 |
| IPI00179721 | R.SCNDFGSYNN#QSSNFGPMK.A | 0 9302 |
| IPI00179972 | G.VGAFN#LTLSMLPTR.I | 0.5056 |
| IPI00180034 | R.FN#GSGSGTDFTLK.I | 0.9981 |
| IPI00180178 | K.N#KTTCLRGSDTAALVPVPLATPLLLEGR.S | 0 7197 |
| IPI00180305 | C.ALSLFLMAVNIKTPVVVEN#ITLM*CLR.I | 0.5363 |
| IPI00180305 | K.AM*EEFFSDSGELVQIMMATANEN#LSA.K | 0.5895 |
| IPI00180403 | R.ADKGPVTSILPSQVN#SSPVINIILLLGKK.M | 0 6594 |
| IPI00180404 | K.REEEEEEEGSIM*N#GSTAEDEEQTR.S | 0.9289 |
| IPI00180462 | K.YLKEAPLASSAN#GTEK.N | 0.7163 |
| IPI00180465 | -.MMATPN#QTACNAESPVALEEAK.T | 0.7847 |
| IPI00180466 | R.HPQVLQATQETLQRHGVGAGGTRN#IS.G | 0 5021 |
| IPI00180625 | R.DFDQNM*N#DSCEDALAN.K | 0.7645 |
| IPI00180627 | R.FCTQTLGVDKGYKN#QSFYRK.H | 0.9555 |
| IPI00180687 | R.GNVN#GTFIIHPDSGN#LTVAR.S | 0.6372 |
| IPI00180707 | R.LVLGTPQSNSPFGAAVGEQN#ETLIR.I | 0.7764 |
| IPI00180712 | K.YPLM*QRMTN#SSSSPSLLN#DSAK.P | 0 5241 |
| IPI00180719 | K.GHPN#RSALSLPPGLRIGPSGIPQAGLGVWNEASDLPL.G | 0 921 |
| IPI00180730 | K.DGN#ASGTMLLEALDCILPPTR.P | 0.623 |
| IPI00180919 | G.KGFICEFCQN#TTVIFPFQTATCRR.C | 0.529 |
| IPI00181081 | V.IVGVPPDSQN#LSMNPMLLLTGR.T | 0.9548 |
| IPI00181160 | E.NYLEFGLFIGFTN#FSDSAMQFLEK.Q | 0.7756 |
| IPI00181174 | T.ITMIPNTLTGM*QPLIIFNTFSGGQN#STNLPIIGIISTI.R.V | 0 5768 |
| IPI00181260 | R.NEKCNEN#YTTDFIFNLYSEFGK.G | 0 5859 |
| IPI00181285 | K.GIVVLIDPLAAN#GTTDMHTSVPR.V | 0 9997 |
| IPI00181285 | R.KAASTLSDTKNM*EIIN#STIETLAPDSPFDH.K | 0 6375 |
| IPI00181306 | K.QHGVN#VSVN#ASATPFQQPSG.Y | 0 9438 |
| IPI00181703 | K.IIN#SSSSALLNSPTVITSSCAGASEKKK.F | 0.5105 |
| IPI00181743 | K.LALLN#ASLVKGN#LSR.V | 0.9041 |
| IPI00181743 | R.AN#GTAGPFEDHTDDFLGCLNIPVR.E | 0.876 |
| IPI00181921 | P.TSSM*N#VSMM*FPINDLHFADSLNLAK.G | 0 8415 |
| IPI00181944 | W.AQN#GSMSQPLGESPATAFATATATTRPSPTTPAM*PK.M | 0 7741 |
| IPI00182027 | L.ATLGTFALN#NSNPK.D | 0 9443 |
| IPI00182116 | M.QKSTNSDTSVETLN#STR.Q | 0 5329 |

| | | |
|---|---|---|
| IPI00182164 | K.LASN#GTPM*GTFAPLWEVFR.V | 0.674 |
| IPI00182194 | R.CN#ISLPMENGLNSIEWR.L | 0.5171 |
| IPI00182233 | K.VN#ATNFQALAAEFGGESFTSTFQTQSPPSFYR.A | 0.8446 |
| IPI00182469 | R.YQEAAPNVAN#NTGPHAASCFGAK.K | 0.6337 |
| IPI00182545 | R.ECFNIGNFNSMMAIISGM*N#LSPVAR.L | 0.9661 |
| IPI00182601 | R.DN#TSVYHISGKK.K | 0.8755 |
| IPI00182768 | A.RTTFN#FSIGVLQAECLTSKGR.E | 0.6009 |
| IPI00182811 | K.CPKPM*EEN#HSVSHKKSKK.K | 0.9412 |
| IPI00182840 | R.GRPALPNPEGRAREPCPN#R.T | 0.5505 |
| IPI00183110 | K.EM*YQPEDDN#NSDVTSDDDM*TRNR.R | 0.9663 |
| IPI00183230 | M.VHM*PDSLGGGPEGPCFCPTPCN#LTR.Y | 0.5918 |
| IPI00183414 | A.HVCN#DTNKMTLINPQGAKLNIYKRK.V | 0.9007 |
| IPI00183445 | K.QTESSFM*AGDIN#STPTLNR.G | 0.5272 |
| IPI00183526 | K.TLVLSN#LSYSATEETLQEVFEK.A | 0.5981 |
| IPI00183568 | K.KDAENHEAQLKN#GSLDQGSR.I | 0.9744 |
| IPI00183606 | R.GRPFPLALLGWAPSN#ITFALLFGRR.F | 0.689 |
| IPI00183706 | K.SLIEGVISGYN#ATVFAYGPTGCGK.T | 0.7301 |
| IPI00183804 | K.TFKN#ESENTCQDMTFSTWTPPPGVHSQTLSR.F | 0.9775 |
| IPI00183933 | D.VN#LSKTEKM*GNTVESEHLSELTEEEYEAHYIR.R | 0.727 |
| IPI00183965 | R.LGSSKSGDN#SSSSLGDVVTGTRRPT.P | 0.8717 |
| IPI00184048 | K.LLVNLADHNGNTALHYSVSHSN#FSIVK.L | 0.6851 |
| IPI00184048 | R.N#FSLPDICEEDPGAPAGAVELPGAWVPGAGQR.H | 0.8796 |
| IPI00184160 | R.SMPEASDQEEHLSPLDFLHSAN#FSLGSINQRLNKR.E | 0.8483 |
| IPI00184441 | K.KHELKPNN#PTEEGLASIHSVLFRKDP.F | 0.6678 |
| IPI00184533 | K.QLFTLQTVNSN#GTSDR.T | 0.6054 |
| IPI00184997 | K.SLTTECHLLDSPGLN#CSNPFTQLER.R | 0.7415 |
| IPI00185036 | K.SHISN#HTALENCVSLLCIRADEL.Q | 0.988 |
| IPI00185088 | K.AFHTEISSSDN#NTLTSSNAYNSR.Y | 0.5178 |
| IPI00185198 | K.SVSTSSPAGAAIASTSGASN#NSS.S | 0.9922 |
| IPI00185234 | K.DRIATIN#YTVLTSVLNPFIYSLRNK.D | 0.8774 |
| IPI00185251 | R.RCIIVGNGGVLAN#KSLGSR.I | 0.5341 |
| IPI00185256 | R.N#TTSTCIATVVGLTGAR.L | 0.9065 |
| IPI00185518 | -.MEN#FTALFGAQADPPPPPTALGFGPGK.P | 0.5147 |
| IPI00185526 | D.DSTEAIIEGDPFN#GSGEQSK.T | 0.6032 |
| IPI00185649 | L.LSPN#LTDEQAM*LEDTLVALFDLEK.V | 0.7918 |
| IPI00185878 | R.MSMLASQQN#QSGPSGNN#QSQGNM*QR.E | 0.6421 |
| IPI00185878 | R.N#NSYSGSNSGAAIGWCSASGNGFNGGSGSSMDSQSSGWEM.- | 0.8281 |
| IPI00186101 | G.NYN#NSSNFGTM*KVGNTGGRNSGSYGVGGQYFAKPR.H | 0.5471 |
| IPI00186157 | R.RFN#NSPSNLPR.P | 0.6346 |
| IPI00186315 | R.SVTLQIYN#HSLTLSAR.W | 1 |
| IPI00186315 | R.FDFQGTCEYLLSAPCHGPPLGAFN#FTVTVANEHR.G | 0.9836 |
| IPI00186525 | M.MLQNILQIN#RSK.R | 0.996 |
| IPI00186843 | R.CSHGMVEANGLIYVCGGSLGNN#VSGR.V | 0.7732 |
| IPI00186850 | K.APLN#ETGEVVNFKAK.T | 0.5488 |
| IPI00187002 | K.IEFLEEEENGDSVVQNN#NTSQMSHKK.V | 0.5503 |
| IPI00187149 | R.KMLLWAMSVTLEQN#LTCPGSDLKPFTTR.L | 0.5972 |
| IPI00215608 | K.LLNSN#KSGAAFN#QSKSLTLPQTCNR.E | 0.582 |
| IPI00215613 | R.GENAYSTVLN#ISQSANLQFASLIQK.E | 0.7158 |
| IPI00215615 | I.FTM*IHNN#RSLTIHQAMR.G | 0.9898 |
| IPI00215690 | D.DN#STFN#STQSHMDWGK.V | 0.6856 |
| IPI00215761 | M.LFTNEDNPIIGN#FDSAKASKAR.T | 0.5418 |

| | | |
|---|---|---|
| IPI00215770 | R.RGAQSPGVM*N#GTPSTAGFLVAWP.M | 0.5903 |
| IPI00215869 | K.MEN#ESATEGEDSAMTDMPPTEEVTDIVEM*R.E | 0.8853 |
| IPI00215900 | K.VPWYVLAGNHDHLGN#VSAQIAYSK.I | 0.5019 |
| IPI00215979 | K.HLEGISDEDIIN#ITLPTGVPILLELDENLR.A | 0.6224 |
| IPI00215995 | R CQKLELLLM*DNLRDKLRPIIISMN#YSLPLR.M | 0.6812 |
| IPI00216047 | R HQGTVTEDKNN#ASHVVYPVPGNLEEEEVVRPVM*.K | 0.8674 |
| IPI00216133 | K.MSN#YSLLSVDYVVDK.A | 0.8387 |
| IPI00216142 | K.AIN#NSFAPEKLQELAFQTIQEIR.H | 0.9499 |
| IPI00216143 | R.KN#KSVWITISS.T | 0.6859 |
| IPI00216151 | R.LM*RQLLVIN#ESIESIK W | 0.6777 |
| IPI00216171 | K.LDNLMLELDGTEN#KSK.F | 0.764 |
| IPI00216184 | L.ANLVGNLGIGN#GTTK.N | 0.8756 |
| IPI00216219 | D KAPVN#GTEQTQK.T | 0.9349 |
| IPI00216253 | E.NN#VSKGDNGELAK.E | 0.6856 |
| IPI00216269 | R.TLHSTFQPN#ISR.Y | 0.9532 |
| IPI00216283 | R FGKQAALDPFILLNLLPN#STDK Y | 0.6984 |
| IPI00216311 | -.MPKPINVRVTTMDAELEFAIQPN#TTGK.Q | 0.7673 |
| IPI00216315 | R.N#RTFVLNFIK I | 0.9256 |
| IPI00216315 | K.EVFVHPN#YSK S | 0.9993 |
| IPI00216317 | M.DFN#LSGDSDGSAGVSESR.I | 0.9879 |
| IPI00216362 | Y.RPPDRSAPSWN#TTGEVVVTM*EPEVPIKK.L | 0.7216 |
| IPI00216529 | H.DVTN#ISTPTHVVFSSSTASTTVGFEW.- | 0.7403 |
| IPI00216560 | I.RVGN#ATIDR.E | 0.9979 |
| IPI00216560 | E.FIHLLSN#ITGAIVNTDNVQFHVDK.K | 0.9153 |
| IPI00216560 | R.VLDINDNDPVLLNLPMN#IT.I | 0.6244 |
| IPI00216560 | L.SVIDN#ASDLPERSVSVPNAK L | 0.5203 |
| IPI00216587 | K.TRIIDVVYN#ASNNELVR T | 0.5021 |
| IPI00216702 | R.QNIAIEVDAFGTRN#GTDDPSYNGAIIVSGDEK.D | 0.5627 |
| IPI00216711 | M.SFN#CSTRN#CSSRPIGGR.C | 0.692 |
| IPI00216721 | R.N#CSHWAVGVASWEM*S R | 0.8429 |
| IPI00216722 | D.SAN#YSCVYVDLKPPFGGSAPSER L | 0.9987 |
| IPI00216722 | R EGDHEFLEVPEAQEDVEATFPVHQPGN#YSCSYR.T | 1 |
| IPI00216722 | R.FQSPAGTEALFELIIN#IS.V | 0.8918 |
| IPI00216722 | Q PSLWAESESLLKPLAN#VTLTCQAIILETPDFQLFK N | 0.8198 |
| IPI00216744 | R.RSKSPADSAN#GTSSSQLSTPKSKQSPISTPTSPGSLR.K | 0.7204 |
| IPI00216750 | R IGVSFIDDGSN#ATDLLR.K | 0.9589 |
| IPI00216752 | K.FN#PSLNVVDK I | 0.5155 |
| IPI00216752 | R FYISKGAVVDQLGGDLN#STPIHWAIR.Q | 0.5394 |
| IPI00216798 | K NEEIDEMIKEAPGPIN#FTVFISMFGEKLK.G | 0.9564 |
| IPI00216803 | R KTTSNN#FTIISR A | 0.7493 |
| IPI00216803 | R NSN#YTYPIKPAIENWGSDFLCTEWKAS.N | 0.9624 |
| IPI00216869 | R.SPAAAILELFEEQN#GSLQEI HYLMTVMER L | 0.818 |
| IPI00216889 | K N#ITLLPATAAFTFTVTPSG Q | 0.9734 |
| IPI00216890 | I ELN#DSVNENSDTVGQIVHYIM*K.N | 0.8381 |
| IPI00216984 | R.DGN#GTVDFPEFLGMMARK.M | 0.5129 |
| IPI00216990 | K LMLVSAPSILSSCN#GTAIN#M* I | 0.7066 |
| IPI00216990 | K IGLNIGQAIVN#TSGTVPAIPSINILQN#VTPKGEDK S | 0.8975 |
| IPI00217002 | R LPINGANTVIGSN#NSVQNVPTPQTFGGK.H | 0.5506 |
| IPI00217002 | K QSSNRPAHN#ISHILGHDCSSAV - | 0.8209 |
| IPI00217005 | R IIFKMGSN#ISQLTDKNELLTLQVIIK.A | 0.8253 |
| IPI00217013 | K EVLI K IN#ISGKQS P | 0.9496 |

| IPI00217013 | M.HVLTAPLLAN#TTEDKPSK.D | 0.9588 |
|---|---|---|
| IPI00217032 | -.M*SSKPEPKDVHQLN#GTGPSASPCSSDGPGR.E | 0.5652 |
| IPI00217051 | R.SSTSSIDSN#VSSK.S | 0.5088 |
| IPI00217051 | P.TKIGSGRSSPVTVN#QTDK.E | 0.8504 |
| IPI00217051 | M.EGFNSGLNSGGSTN#SSPK.V | 0.8461 |
| IPI00217051 | R.YATQSN#HSGIATSQ.K | 0.7705 |
| IPI00217051 | K.YHFSNLVSPTN#LSQFNLPGPSMM*R.S | 0.5912 |
| IPI00217055 | R.IVESYFMLN#STLYFSYTHMVCR.T | 0.8907 |
| IPI00217093 | R.RQISQKAFLFN#SSEQVAEFVISR.P | 0.5277 |
| IPI00217110 | R.VGLFCGIFIVLN#ITLVLAAVFK.L | 0.8825 |
| IPI00217162 | K.TKLPEYTREALCPPACRGSTTLYN#CSTCK.G | 0.9307 |
| IPI00217163 | R.N#RSYVFSSLATSAVSFATGALGMWIPLYLHR.A | 0.7188 |
| IPI00217164 | K.LVPSSSYVAVAPVKSSPTTSVPAVSSPPMGN#QSGQSVP.- | 0.8555 |
| IPI00217267 | K.IENYIN#ESTEAQSEQK.E | 0.7729 |
| IPI00217267 | K.LHCNSACLTN#TTHCPEEASVGNPEGAFMKVLQARKN.Y | 0.72 |
| IPI00217272 | M.GHN#FSLPVYKGEIQAR.N | 0.5381 |
| IPI00217309 | R.VLYM*FNQMPLN#LTNAV.A | 0.8516 |
| IPI00217355 | R.EAFFGGNGKIN#LTVFK.L | 0.869 |
| IPI00217355 | K.DN#STACSHPVTK.H | 0.9644 |
| IPI00217370 | K.HELGITAVMNFQTEWDIVQN#SSGCNR.Y | 0.6828 |
| IPI00217378 | K.KVDAQSSAGKEDM*LLSKSPSSLSAN#ISSSPK.G | 0.9212 |
| IPI00217391 | L.FDNAAQPYSN#LSNLDVLNQVIRERDTK.L | 0.8792 |
| IPI00217438 | M.AKSALREN#GTNSETFRQRFR.R | 0.9696 |
| IPI00217442 | S.QELNFVM*DVN#SSK.Y | 0.8408 |
| IPI00217446 | R.VSTVYANN#GSVLQGTSVASVYHGK.I | 1 |
| IPI00217465 | K.ALAAAGYDVEKN#NSR.I | 0.5791 |
| IPI00217542 | R.SCCEGM*ICNVELPTN#HTNAVFAVM*HAQR.T | 0.5205 |
| IPI00217544 | K.HM*PPPN#MTTNERR.V | 0.513 |
| IPI00217652 | K.FN#STQIAAM*APEIIEEPR.I | 0.7554 |
| IPI00217652 | H.PYYGKTGVNSGVMLM*N#M*TRM*RRK.Y | 0.9849 |
| IPI00217669 | C.LQKGSLTIQQVNDLLDSLASN#NSAK.R | 0.9484 |
| IPI00217710 | K.GNSKAGN#GTLENQK.G | 0.6136 |
| IPI00217710 | K.EVDIEGTTVIEVGLDPSNN#MTLAVDCVGILKLR.N | 0.9291 |
| IPI00217766 | K.ANIQFGDN#GTTISAVSNK.A | 0.698 |
| IPI00217797 | K.HSAGSGAEESN#SSSTVQK.Q | 0.9156 |
| IPI00217809 | K.GFN#WSSALTKHK.R | 0.5579 |
| IPI00217851 | K.TVN#LSVTPSPAPR.T | 0.6436 |
| IPI00217872 | K.FN#ISNGGPAPEAITDK.I | 0.8006 |
| IPI00217876 | R.VEN#GSSDEN#ATALPGTWR.R | 0.6182 |
| IPI00217884 | T.ERLLGEASSN#WSQAK.R | 0.8245 |
| IPI00217897 | R.GPVSSDVEEN#DSLNLLGILPN#NSDSAKK.N | 0.7341 |
| IPI00217937 | R.VEYTGHPLEIAVFLNYCTVCN#VTK.K | 0.8099 |
| IPI00217975 | M.N#TSIVNSAR.E | 0.8875 |
| IPI00217991 | K.DKLDETN#NTLRCLK.L | 0.9003 |
| IPI00217998 | R.AAEN#ASLGPTN#GSKLM*NR.Q | 0.5164 |
| IPI00218052 | K.ATAADTGM*N#RSQSEYCNVGTKT.Y | 0.7731 |
| IPI00218064 | K.SWN#KSQNDCAIN#ASYLMVIQDITAM*VR.F | 0.5421 |
| IPI00218081 | R.FIDSSNPGLQISLRVN#NTEHVVS.I | 0.7522 |
| IPI00218093 | K.GRIGVVISSYM*HFTN#VSASADQALDR.F | 0.8862 |
| IPI00218130 | R.GLAGVFN#VTELK.K | 0.9985 |
| IPI00218132 | R.EGGHDVPSNKDVTSLDWNTN#GILLATGSYDGFAR.I | 0.8776 |

| | | |
|---|---|---|
| IPI00218135 | K.KHHHHAVGLN#LSHVRKR.C | 0 9298 |
| IPI00218189 | R.DVMWEN#YSNFISLGPSISKPDVITLLDEER.K | 0.7125 |
| IPI00218192 | K LPTQN#ITFQTESSVAEQEAEFQSPK.Y | 1 |
| IPI00218192 | K.AFITN#FSMNIDGM*TYPGIIK.E | 1 |
| IPI00218288 | R RLRIHNLGLN#CSSQLADLYKSC E | 0 8439 |
| IPI00218337 | R.VFPYISAMVNN#GSLSYDHERDGR.P | 0 9949 |
| IPI00218413 | K.DVQIIVFPEDGIHGFN#FTR.T | 1 |
| IPI00218413 | R YQFNTNVVFSNN#GTLVDR.Y | 1 |
| IPI00218413 | K NPVGLIGAEN#ATGETDPSHSK.F | 1 |
| IPI00218413 | R.FN#DTEVLQR.L | 0 9997 |
| IPI00218413 | K.WNVNAPPTFHSEMMYDN#FTLVPVWGK.E | 0.5565 |
| IPI00218490 | K.CVVEMEGN#QTVLHPPPSNTK.Q | 0.5427 |
| IPI00218490 | D.YQVTLQIPAAN#LSANR.K | 0 6812 |
| IPI00218529 | R.ASLN#HSTAFNPQPQSQMQDTR.Q | 0 6734 |
| IPI00218571 | K.YNN#GSTELHSSSVGLAK A | 0.5342 |
| IPI00218648 | K.NEKN#GTDELDNMN#STERISFLQEKLQEIRK.Y | 0.7548 |
| IPI00218676 | K.FIHNENGAN#YSVTATR S | 0 9775 |
| IPI00218725 | D.LLRTLN#DTLGKLSAIPN#DTAAKLQAVK.D | 0.8184 |
| IPI00218725 | K.N#ESGHLLGSGGTPAPPR R | 0.876 |
| IPI00218725 | R.YM*QN#LTVEQPIEVK.K | 0.7963 |
| IPI00218731 | K.FVDTAGN#FSFPVN#FSLSLL N | 0.7538 |
| IPI00218732 | R.VVAEGFDFANGIN#ISPDGK.Y | 1 |
| IPI00218732 | K.VTQVYAEN#GTVLQGSTVASVYK.G | 1 |
| IPI00218732 | K.HAN#WTLTPLK S | 1 |
| IPI00218762 | R.LSSSGSN#CSSGSEGEPVALHAGICVR.Q | 0.697 |
| IPI00218795 | R.DN#YTDLVAIQNK A | 1 |
| IPI00218795 | K.IGGIWTWVGTN#K.S | 0 9987 |
| IPI00218803 | R.CATPHGDN#ASLEATFVK.R | 1 |
| IPI00218829 | R.FN#LSAAFSRQLNVNAKPFVPNVHAA E | 0.8352 |
| IPI00218832 | K.YHVMAPALSFHMSPWSWSN#CSRK.Y | 0 5001 |
| IPI00218888 | R.SGQVEVN#ITAFCQLIYPGK.G | 0 8154 |
| IPI00218889 | M IN#NTKAFIHHELLAYLYSSADQSSLMEESADQAQR R | 0.8667 |
| IPI00218916 | K.AN#ATGGGGHVQMVQR A | 0.6597 |
| IPI00218924 | G.FN#GTLSR E | 0.551 |
| IPI00218925 | R N#GSLQEKLWAILQATYHSWNLARFVFTYK G | 0 8472 |
| IPI00218964 | K GFSQLSN#LTK.H | 0.5542 |
| IPI00218987 | I FN#ETKN#PTLTR.R | 0 8742 |
| IPI00218987 | S FSWSGGAFLYPPN#MSP.T | 0 6366 |
| IPI00219050 | D.RPPSPTDN#ISRYSFDNLPEK.Y | 0 7622 |
| IPI00219074 | M.TLTN#LSGPYSYCN#TTLDQIGFCWPR.S | 0.8586 |
| IPI00219078 | M.TM*ALSVLVTHMCNALNSLSEN#QSLLR M | 0 5952 |
| IPI00219130 | R.MIRTNFAVPKTAPTN#VSGRSGRR.H | 0.6745 |
| IPI00219131 | K TVVTYHIPQN#SSLENVDSR Y | 1 |
| IPI00219173 | K.FN#PSTVGVER V | 0 8501 |
| IPI00219294 | A DGAAASNAADSAN#ASLVNAK Q | 0.5289 |
| IPI00219314 | R.N#PNSAAPVQSRGGIGASENLENPPKMGELE A | 0 5677 |
| IPI00219336 | K EN#ITDPFRGCVGNTNHWKTGPLFK.R | 0 562 |
| IPI00219336 | R TM*N#ITYEVHLVADGK.F | 0 5344 |
| IPI00219418 | P PTLHATAASVAVPN#KIC - | 0 5068 |
| IPI00219425 | R PVDKPIN#FTLICNPV INALGAR Q | 1 |
| IPI00219458 | R.LVRV FYVSSEGGHSGQTEAPGN#ATSAM*LGPLSSSTTYTVR V | 0 6163 |

| | | |
|---|---|---|
| IPI00219546 | R.AGVVFMAGHVYAVGGFN#GSLR.V | 0 727 |
| IPI00219561 | R.SQSLIFLN#LSTNNLLD.D | 0.7636 |
| IPI00219561 | K.M*N#LTQNTLGYEGIVKLYKVLK.S | 0 6044 |
| IPI00219567 | M.TAM*DN#ASKN#ASEMIDKLTLTFN#R.T | 0 6677 |
| IPI00219616 | R.N#CTIVSPDAGGAKRV.T | 0.6361 |
| IPI00219677 | F.IKTSTGKETVN#ATFPVAIVM*LR.A | 0 5344 |
| IPI00219678 | K.EALRAGLN#CSTENMPIK.I | 0.6587 |
| IPI00219695 | K.LQNAENDYIN#ASLVDIEEAQR.S | 0.7258 |
| IPI00219753 | M.LQYGGRN#RTVATPSHGVWDMRGK.Q | 0 9978 |
| IPI00219778 | G.VSLSSYLEGLMASTISSN#ASKGREAMEWVIHK.L | 0.7098 |
| IPI00220106 | K.EIYHQNVQN#LTHLQVVEVLK.Q | 0.9044 |
| IPI00220113 | R.LATN#TSAPDLK.N | 0.5931 |
| IPI00220279 | K.ARKSIAQSGVNM*CNQN#SSPHK.N | 0.5215 |
| IPI00220289 | K.VLN#HSPMSDASVNFDYK.S | 0.5346 |
| IPI00220289 | K.LILSQN#HSDEEEEEEENEEENLAMAVGM*GE.R | 0.7406 |
| IPI00220289 | R.EHGAQAGEGALKDSNN#DTN.- | 0.5987 |
| IPI00220327 | E.ESRM*SGECAPN#VSVSVSTSHTTISGGGSR.G | 0 9876 |
| IPI00220391 | N.EINN#MSFLTADN#K.S | 0.6322 |
| IPI00220477 | K.WRLSN#NSVVEIASLR.F | 0.5223 |
| IPI00220573 | K.N#PTDEYLDAM*M*NEAPGPIN#FT.M | 0.956 |
| IPI00220630 | K.VLN#GTLLM*APSGCK.S | 0.6459 |
| IPI00220817 | K.GRAN#HSAFLFGFGDGGGGPTQTM*LDR.L | 0 9349 |
| IPI00220830 | K.SPIIPECSTNVQTAAGGSN#SSQYNSN#LTIRLSVSWK.G | 0.7428 |
| IPI00220901 | R.QSSSEQCSN#LSSVR.R | 0 9976 |
| IPI00221035 | K.LGVNN#ISGIEEASNMFT.N | 0 5057 |
| IPI00221055 | T.TN#STN#PSPQGSHSAIGLSGLN#PSTG.- | 0.5081 |
| IPI00221130 | M.FCINICTVYCN#NSFPIHSSN#STK.K | 0.8358 |
| IPI00221193 | R.N#VTVGPPENIEVTPGEGSLIIR.F | 0.682 |
| IPI00221224 | R.N#ATLVNEADKLR.A | 0 9987 |
| IPI00221224 | R.PSAIAAGIIGDYALN#VTGPILNFFAGHYDTPYPLPK.S | 1 |
| IPI00221224 | K.GPSTPLPEDPNWN#VTEFHTTPK.M | 1 |
| IPI00221224 | K.AEFN#ITLIIIPK.D | 0 9998 |
| IPI00221224 | K.VPVTLALN#NTLFLIEER.Q | 0 9152 |
| IPI00221224 | E.KNKNAN#SSPVASTTPSASATTNPASATTLDQSK.A | 0.6663 |
| IPI00221234 | R.EENEGVYN#GSWGGR.G | 0 518 |
| IPI00221246 | H.AATTQYAN#GTVLSGQTTNIVTHR.A | 0.8126 |
| IPI00221307 | R.GTELDDGIQADSGPIN#DTDANPR.Y | 0 6039 |
| IPI00221325 | S.QSGHMLLN#LSR.G | 0 6875 |
| IPI00221332 | K.AENDENGQAFN#FSM*DPQLERQVETIR.N | 0 5238 |
| IPI00221338 | M.I.TTHPSLYRVDN#LSDEGALN#ISDR.T | 0 504 |
| IPI00232047 | R.IVTTN#VTMPEGPPQNCVTGN#ITGK.S | 0 6369 |
| IPI00232311 | L.TKRTNMDFSICISN#ITPADAGTYYCVK.F | 0.6504 |
| IPI00232637 | R.RKLAIENTMAXLVSVGANSAVN#NTAESK.M | 0 6462 |
| IPI00232917 | K.RSPIFFNYLYSPLEIEAIKPNVN#VSSLK.K | 0 5956 |
| IPI00232917 | R.ACLGLIYTVYVDSLN#VSTFSLIANLCACLVPAA.G | 0.6628 |
| IPI00233062 | P.TN#ETTFAK.L | 0 5199 |
| IPI00233501 | R.RIN#MSFVEVKDK.K | 0 5329 |
| IPI00233618 | K.YALIIVM*M*TIM*TATDIQLLN#QTMENTR.Q | 0 8842 |
| IPI00234062 | K.VGAERNVLIFDIGDDTFN#VSILTTLDGIFEVK.S | 0.9519 |
| IPI00234035 | K.KEIYM*HTGN#SSTPKGEGGSC.Y | 0 6357 |
| IPI00234091 | R.IPSYN#LTVSVSDNYGAPPGAWQAR.S | 0 9418 |

| | | |
|---|---|---|
| IPI00234337 | K.YFWN#DTIHNFDFLK.G | 0.869 |
| IPI00234446 | R.QYKDLWN#MSDDKPFLCTAPGCGQ.R | 0.9067 |
| IPI00235307 | K.N#SSLAEFVQN#LSQ.I | 0.7697 |
| IPI00235412 | P.VKLGIIGVVN#RSQLDINNKK.S | 0.516 |
| IPI00235721 | M.RPHEDLSEDN#SSGEVVMRVTSV.- | 0.6184 |
| IPI00235756 | K.ALDPSQPVTFVTN#STYAADKGVNK.E | 0.8329 |
| IPI00235832 | T.DDTN#VTWLQLETEIEALKEELLLM*KK.N | 0.5479 |
| IPI00236481 | R.DRLALAN#ESGVTLMPDGSLHLAALPSR.R | 0.5621 |
| IPI00236852 | K.VLTPEELLYRAVQSVN#VTHDAVH.A | 0.6419 |
| IPI00238209 | M.FN#ISPGAVQF.- | 0.5901 |
| IPI00238575 | R.NSINVFASPAHYTSTTGSCNFETSSGN#WTTA.C | 0.8781 |
| IPI00238781 | R.EACANILIDSGADPNIVGVYGNTAVHYAVNSEN#LSVVAK.L | 0.7623 |
| IPI00239216 | K.N#SSSEQLFSSARLQNEK.K | 0.5697 |
| IPI00239405 | R.TNVLNDAYEN#LTRYK.E | 0.6296 |
| IPI00239992 | M.CVETFSN#YSLLGHFAVR.H | 0.6302 |
| IPI00240401 | V.ILSNNN#HTEIQEISLALR.S | 0.6262 |
| IPI00240812 | R.M*ETVSN#ASSSSN#PSSPGR.I | 0.663 |
| IPI00240988 | K.ECQHGGQCQVEN#GSAVCVC.Q | 0.849 |
| IPI00241148 | R.VLYM*FNQMPLN#LTNAVATAL.Q | 0.9888 |
| IPI00241313 | K.QPVESSEDSTDDSN#SSSGEEE.R | 0.622 |
| IPI00241390 | K.LINYN#NSITNSVYSR.F | 0.5989 |
| IPI00241390 | W.NEDYCKLFKN#ITVEFMNELER.Q | 0.8937 |
| IPI00241802 | R.YTLVFN#SSSERN#VSLTEHKKK.Q | 0.9834 |
| IPI00241809 | R.N#MTLLATIM*SGSTM*SLNHE.A | 0.8965 |
| IPI00242956 | R.SVTLQIYN#HSLTLSAR.W | 1 |
| IPI00242956 | R.FNFQGTCEYLI.SAPCHGPPLGAEN#FTVTVANEHR.G | 0.9999 |
| IPI00242956 | K.VTVRPGESVM*VN#ISAK.A | 0.9997 |
| IPI00242956 | R.VITVQVAN#FTLR.L | 0.9991 |
| IPI00242956 | R.YLPVN#SSLLTSDCSER.C | 0.9957 |
| IPI00242956 | R.VVTVAALGTN#ISIHK.D | 0.7561 |
| IPI00242956 | R.GLCVLSVGAN#LTTFDGARGA.T | 0.6825 |
| IPI00242960 | R.KPSPQDIAQAVLRN#FSGK.D | 0.8685 |
| IPI00242960 | R.SHN#ASLHPTPEQCEAVSKFIGECK.I | 0.6224 |
| IPI00243275 | K.NNGFFQKLN#VTEGAMQDLLKEIIK.V | 0.5003 |
| IPI00243295 | K.LVSSSNAMEN#ASHQASVQVESLQEQLNV.V | 0.5118 |
| IPI00243423 | K.HTVSGII.SM*ANAGPNAN#SSQFFICAAK.T | 0.8311 |
| IPI00243451 | R.YSKEFNIDPSEN#STSNLPNCLINQMLSLN#R.T | 0.7589 |
| IPI00243595 | R.CRELRN#FSSLRAILSALQSNPIYR.L | 0.546 |
| IPI00243984 | K.N#KTSTASSMVASAEQPSGSVEEELSK.K | 0.9238 |
| IPI00244043 | K.HGIEAAFLAMI.GLQGNKQVLDLEAGFGAFYAN#YSPK.V | 0.9357 |
| IPI00244116 | K.NYNDHENN#LSAICLVK.L | 0.7134 |
| IPI00244243 | S.QCGKM*ANKAN#TSGDFEK.D | 0.8987 |
| IPI00244477 | R.RGASVN#RITR.T | 0.7576 |
| IPI00244477 | R.RGASVN#RITRTN#STPLR.A | 0.7564 |
| IPI00244574 | R.NPPAFGN#VSVIALELLNSGYEFDEGSHFNQFK.S | 0.9118 |
| IPI00245135 | K.TNNVN#VSSR.V | 0.9987 |
| IPI00246001 | R.KSEIHGAPVLFQN#LSGVIIWGYFETK.T | 0.504 |
| IPI00246053 | R.HPGFMCQGGGFTCHN#GTGGK.S | 0.5369 |
| IPI00246067 | R.LFSGM*RN#M*SIISK.T | 0.5097 |
| IPI00246067 | R.WLGSTGVTCGVRRQISFMNUN#ISR.I | 0.9315 |
| IPI00246676 | R.DSFGAHTYELLAKPGQFIIT.N#WTGHGGSVSSSSYNA.- | 0.6455 |

| | | |
|---|---|---|
| IPI00246686 | K.IIMLPSALDQLSQLN#IT.Y | 0.7462 |
| IPI00247110 | R.KCQLN#LTDSEN#R.T | 0.8279 |
| IPI00247535 | K.ENIPGDFLCISLVN#SSVQLRYNLGDR.T | 0.8588 |
| IPI00247535 | M.RNLQFTTISLN#FSTTK.T | 0.5147 |
| IPI00247601 | K.YDNSLKIISN#ASCTTNCLAPR.A | 0.659 |
| IPI00247616 | M.DN#VTGGMETSR.Q | 0.9515 |
| IPI00247641 | R.NTFTPGEKVVFTTEINN#QTSKCIK.T | 0.7698 |
| IPI00247659 | -.MDSVAFEDVAVN#FTQEEWALLDPSQK.N | 0.9716 |
| IPI00248101 | K.NAPQN#STQAHSENK.C | 0.605 |
| IPI00248307 | R.SEASN#GSTVAAGTSKSEEGLSSGLGSGVGGK.P | 0.8167 |
| IPI00248651 | G.AEVKFVLKHQN#VSEFASSSGGSQLLFK.Q | 0.8866 |
| IPI00248881 | R.KN#CSQIALFQK.R | 0.7556 |
| IPI00248896 | K.STN#ISFTDMVSADER.L | 0.515 |
| IPI00248930 | K.MN#SSIMAN#VTKAFVGDSK.D | 0.6629 |
| IPI00249283 | K.IIGSNNVGLSEN#LTDGAAAGNGDGGLVPQR.K | 0.8945 |
| IPI00249584 | M.SVTFISN#NTAIQELFRFR.C | 0.8754 |
| IPI00249629 | K.NRVQSKISN#LTDAKNPNLR.K | 0.9153 |
| IPI00249660 | R.NRDLN#NSSIN#LTKVK.I | 0.9934 |
| IPI00249983 | M.ATRTLN#LSFFPR.S | 0.638 |
| IPI00251351 | R.GTRARLLSSFLSFLN#GSSANQAVGQGPEAGEGR.G | 0.7762 |
| IPI00252768 | R.NILDALM*LN#TTR.I | 0.9998 |
| IPI00252944 | L.TRLQLDGNQITN#LTDSSFGGTNLHSLR.Y | 0.6688 |
| IPI00254338 | R.ELATTDSEHSDAEVSCTDN#GTFN#LSR.G | 0.7141 |
| IPI00255107 | K.IKLRSAMYLSN#TTVTILANLVPFTL.T | 0.8915 |
| IPI00255653 | R.DN#LSGLSADM*QDYGLIIDGAALSLIM*KP.R | 0.7665 |
| IPI00256859 | K.EEEELAYDWSDN#NSN#ISAKR.N | 0.9261 |
| IPI00257076 | K.RYGFYN#NSVIIFSSDNGGQTFSGGSNWPLRGR.K | 0.9458 |
| IPI00257239 | R.ESWGQESNAGN#QTVVR.V | 0.7542 |
| IPI00257508 | R.TTQRIVAPPGGRAN#ITSLG.- | 0.5053 |
| IPI00257544 | R.LN#TTNAWDAAPPSLGSQPLYRSSLS.H | 0.8571 |
| IPI00257544 | K.SIGPEHN#GSMVRNK.C | 0.5589 |
| IPI00257717 | T.LTHATNFLNVMLQSN#K.S | 0.5343 |
| IPI00258331 | M.YVDNN#RSWFMIICNSIITN#R.T | 0.5311 |
| IPI00258407 | -.MEN#GSYTSYFILL.G | 0.516 |
| IPI00258462 | K.DSESMSFSDLENWAVAN#SSEPQLEDAKR.E | 0.5437 |
| IPI00258993 | R.VMISAGNLQLPVEAGLVEFTN#ISQK.L | 0.6373 |
| IPI00259549 | K.SNGLMFTNIM*M*QNTN#PSASPEYMFSSNIEPEPK.D | 0.6018 |
| IPI00260178 | R.GGLGGGYGGASGMGGITTVTVN#QSLLSPLNLEVDP.H | 0.6815 |
| IPI00260211 | K.GLYQGFN#MSVQGIIIYR.A | 0.5976 |
| IPI00260230 | K.YTEVTDINSVDANYN#SSVLVSGDDFGI.VKLFK.F | 0.7909 |
| IPI00260367 | K.TPCMPQAASN#TSLGLGDLR.V | 0.9941 |
| IPI00260715 | R.GGSGGGGGGGGGGGYN#R.S | 0.5968 |
| IPI00260916 | R.RGASVN#CTTRTN#STPLR.A | 0.8966 |
| IPI00288960 | -.M*M*QESATEFISN#SSM*NQNGM*STLSSQIDAGSR.D | 0.6344 |
| IPI00289006 | R.DGKEPQPSAEAAAAPSLAN#ISCTTQK.L | 0.9174 |
| IPI00289033 | R.YDN#VTILFSGIVGFNAFCSKHASGEGAM*KIVNL.L | 0.9169 |
| IPI00289082 | R.N#YSKSTELPGKN#ESTIEQIDK.K | 0.526 |
| IPI00289083 | R.FYKFN#TSLAGDLTNLVIIGSH.C | 0.5808 |
| IPI00289083 | A.ENRN#PSCEVIIQEPVTYTAIDPGLQDALHQCVNSR.C | 0.6401 |
| IPI00289123 | R.M*WRRATVAAGNSVVQVVN#VSRLEGDDNPVQI.I | 0.6161 |
| IPI00289169 | R.SMQQQETNLLAN#LTTNDAR.D | 0.9953 |

| | | |
|---|---|---|
| IPI00289171 | R.SITN#ASAAIAPKDNLFIRFLK.P | 0.8389 |
| IPI00289258 | R.CSVGTYN#SSGAYR.F | 0.5269 |
| IPI00289301 | K.IM*CLDEKIDN#FTR.Q | 0.7027 |
| IPI00289334 | K.HVGNQQYN#VTYVVKER.G | 0.876 |
| IPI00289346 | R.VLN#ASAEAQRAAAR.F | 0.7325 |
| IPI00289438 | R.AFSQNAN#LTK.H | 0.7426 |
| IPI00289499 | R.N#LTALGLNLVASGGTAKALR.D | 0.5452 |
| IPI00289561 | K.KHAYCSN#LSFR.L | 0.5106 |
| IPI00289561 | R.VLN#ASTLALALANLN#GSR.Q | 0.9283 |
| IPI00289709 | M.N#NSQGRVTFEDVTVN#FTQGEWQR.L | 0.5278 |
| IPI00289776 | K.VN#GTITFIDEIHNDDGVWLRLN.D | 0.8766 |
| IPI00289787 | K.CNLCLAM*NLQGRHKCIEN#VSR.Q | 0.5624 |
| IPI00289799 | K.IKRN#FSSGTIPGTPGPNGEDGVEQTAIK.V | 0.8639 |
| IPI00289802 | R.HCSVN#GTWTGSDPECLVINCGDPGIPANGLR.L | 0.5171 |
| IPI00289809 | K.LLKSIPLDVVLSNNN#HTEIQEISLALR.S | 0.9424 |
| IPI00289831 | P.RFSILPMSHEIM*PGGNVN#ITCVAVGSPM*PYVK.W | 0.9504 |
| IPI00289866 | M.MQQTPCYSFAPPN#TSLNSPSPNYQK.Y | 0.6239 |
| IPI00289880 | R.GSQSYYTVAHAISEWVEKQSALLIN#GTLK.H | 0.823 |
| IPI00289914 | L.SQFADN#TTYAK.V | 0.931 |
| IPI00289944 | K.FIKNQHCTN#ISELSN#TSEN.D | 0.9668 |
| IPI00289961 | L.LEGQDSGNSNGN#ASIN#ITDISR.N | 0.8835 |
| IPI00290032 | Q.N#ITEEIPM*EVFK.E | 0.8058 |
| IPI00290033 | Q.GWPRPLTPPAAGGLQN#HTVGIIVK.T | 0.5019 |
| IPI00290035 | R.GSSNPLLTTEEAN#LTEK.E | 0.5068 |
| IPI00290043 | Q.ALEN#HTEVQFQK.E | 0.936 |
| IPI00290135 | K.AVSLSVTVPVSHPVLN#LSSPEDLIFEGAK.V | 0.9684 |
| IPI00290155 | R.EHMESNLFLSCATN#QSPVEK.D | 0.7117 |
| IPI00290158 | K.TQLN#SSSLQKLFR.E | 0.5534 |
| IPI00290283 | R.FGYILHTDN#R.T | 0.998 |
| IPI00290283 | K.M*LNN#NTGIYTCSAQGVWM*NK.V | 1 |
| IPI00290283 | R.LEPEGPAPHM*LGLVAGWGISNPN#VTVDEIISSGTR.T | 0.9919 |
| IPI00290292 | R.ELDIPSQDN#VSLTSTETPPPLYV.G | 0.914 |
| IPI00290328 | R.YN#ATVYSQAAN#GTEGQPQAIEFR.T | 1 |
| IPI00290328 | K.IHVAGETDSSNLN#VSEPR.A | 0.9948 |
| IPI00290350 | V.N#LSLIFIIALGSIAGILFVTM*IFVAIKCK.R | 0.6995 |
| IPI00290391 | D.DVNVEIVFLHN#ISPNLELEALFK.R | 0.7585 |
| IPI00290459 | V.M·FLFQGNN#GTVLYTGDFR.L | 0.9278 |
| IPI00290546 | R.KEGN#ISDLK.E | 0.7401 |
| IPI00290547 | R.SYFVVVN#HSQSQDTVTTGFALNVIPGAQEKK.A | 0.7896 |
| IPI00290561 | K.QNPMAN#YSSIPAEIM*DHSISPFM*R.K | 0.5321 |
| IPI00290652 | K.SELQPMDLEN#RSTANVLEETTVK.K | 0.6119 |
| IPI00290671 | A.RDSN#VTLAPSGPK.G | 0.7713 |
| IPI00290837 | R.TENN#ISGENQDITDVCTSPFK.D | 0.6274 |
| IPI00290854 | R.GTSGQPPEGCAAPTVIVSNHN#LTDTVQNK.Q | 0.8369 |
| IPI00290856 | K.ANQQLN#FTEAK.E | 0.9998 |
| IPI00290856 | I.ETKVVKFEKAN#DSNPNEESKK.T | 0.5241 |
| IPI00290889 | K.EMTNEEKNII1N#LSK.C | 0.625 |
| IPI00290928 | R.VFSN#VSIILFLN#K.T | 0.8543 |
| IPI00290952 | T.ATHPPGPAVQLN#KTPSSSK.K | 0.5596 |
| IPI00290954 | K.GVHSFYNN#ISGI TDFGEK.V | 1 |
| IPI00291003 | R.SN#KILADSIDNANDPHDPIVNR.L | 0.9961 |

| | | |
|---|---|---|
| IPI00291170 | T.VKGN#PSSSVEDHIEYHGHR.G | 0 564 |
| IPI00291200 | M.IFETTTKN#ETIAQEDK.I | 0.9155 |
| IPI00291235 | R.RFIPPARMMSTESANSFTLIGEASDGGTMEN#LSR.R | 0 631 |
| IPI00291262 | R.LAN#LTQGEDQYYLR.V | 1 |
| IPI00291262 | K ELPGVCN#ETM*M*ALWEECKPCLK Q | 1 |
| IPI00291262 | R.EILSVDCSTNN#PSQAK L | 0.9997 |
| IPI00291262 | K.MLN#TSSLLEQLNEQFNWVSR L | 0.9994 |
| IPI00291262 | R.HN#STGCLR.M | 0 9992 |
| IPI00291262 | K.EDALN#ETR.E | 0.9978 |
| IPI00291262 | R QLEEFLN#QSSPFYFWMNGDR.I | 0.9732 |
| IPI00291316 | M.CYACN#KSITAKEALICPTCN#VTIHNR.C | 0.5668 |
| IPI00291387 | K NIGDDGGGDDNTFN#FSWK.V | 0.7116 |
| IPI00291410 | K.WFN#NSAASLTM*PTLDNIPFSLIVSQD.V | 0.9022 |
| IPI00291539 | K.QRM*EPLYSLN#VSVSDGLFTSTAQVHIR.V | 0.9622 |
| IPI00291596 | R.QHN#NTGYIYSRDQWDPEVIENHRKK.K | 0.9347 |
| IPI00291827 | R.TIGIFWLN#ASETLVEINTEPAVEYTLTQM*GPVAAKQK V | 0.9627 |
| IPI00291834 | R SASLSSLLITPFPSPN#SSLTRSCASSYQR.R | 0.757 |
| IPI00291860 | R.GPHHLDN#SSPGPGSEARGINGGPSRMSPK A | 0 6965 |
| IPI00291866 | R.DTFVN#ASR T | 0 9997 |
| IPI00291866 | R.VLSN#NSDANLELINTWVAK N | 1 |
| IPI00291866 | K VGQLQLSHN#LSLVILVPQNLK H | 1 |
| IPI00291866 | K.M*LFVEPILEVSSLPTTN#STTNSATK.I | 1 |
| IPI00291866 | R ASSNPN#ATSSSSQDPESLQDR.G | 0 9404 |
| IPI00291867 | R.SIPACVPWSPYLFQPN#DTCIVSGWGR.E | 0.9998 |
| IPI00291867 | K.LISN#CSK.F | 0 7403 |
| IPI00291897 | K.FSIAILPFSIKAMAEAN#VSLRRMK.K | 0.8243 |
| IPI00291910 | R.CN#TTQGNEVTSILRW A | 0.5105 |
| IPI00291916 | K.HSKALNTLSSPGQSSFSHGTRN#NSAK E | 0 8113 |
| IPI00291916 | K.KRN#RSSSVSSSAASSPERK.K | 0.7713 |
| IPI00291919 | K.AN#FSIGPMMPVLAGT Y | 0.9331 |
| IPI00291922 | K.ARVETQNHWFTYN#ETM*TVESVTQAVSNLALQF G | 0.8769 |
| IPI00291929 | K.TN#RSSVKTPKPVEPAASDLE P | 0.9952 |
| IPI00291936 | M.RVN#NSTMLGASGDYADFQYLK.Q | 0.6561 |
| IPI00291990 | R.LLGHSPVLRN#ITNSQAPDGRR.K | 0.8077 |
| IPI00292011 | R N#GSDDPSYNGAIIVSGDQK.D | 0.6457 |
| IPI00292043 | R.ISWEEYN#RTNTRVTHYLPN#VTLEYR.V | 0 6417 |
| IPI00292071 | K LEKN#ATDN#ISK.L | 0.814 |
| IPI00292218 | R GTAN#TTTAGVPCQR W | 0 9841 |
| IPI00292218 | K GTGN#DTVLNVALLNVISNQECNIK H | 1 |
| IPI00292218 | R.AFHYN#VSSHGCQLLPWTQHSPHTR L | 0 9996 |
| IPI00292300 | P.SLLLFIN#SSSQDFVVVLLCK N | 0.9834 |
| IPI00292323 | R NVNFN#GSAGTPVMFNKNGDAPGR Y | 0.6716 |
| IPI00292393 | K EGLLANTM*SKM*YGHENGN#SSSPSPEEK G | 0 8317 |
| IPI00292471 | R SITKNPKIGGLPLIPIQHEGN#ATLAR K | 0.7607 |
| IPI00292487 | M.LQDIQEVLN#RSK S | 0 7645 |
| IPI00292490 | K.MSATHGN#NTAIQELFK R | 0.9893 |
| IPI00292499 | R.IRN#ISNTVM*KVKQHGR.S | 0.6243 |
| IPI00292530 | K.ICDLLVANN#IFAHFFAPQN#LINMNK N | 0 9997 |
| IPI00292530 | R.AN#LSSQALQM*SLDYGFVTPLTSMSIR G | 1 |
| IPI00292537 | K LM*PN#FSDSFGGGSGAGAGGGGMI G S | 0 5399 |
| IPI00292674 | K.SQLGFLN#VTNYCHLAHELRI SCMERK.K | 0 6708 |

| | | |
|---|---|---|
| IPI00292723 | M.FEHFLLHREGMFN#DTLR.L | 0.9872 |
| IPI00292723 | S.VTGN#PSNSWPSPTEPSSETGNPR.H | 0.576 |
| IPI00292737 | L.PLN#ESADITFATLNTKGNEGDIVR.D | 0.5968 |
| IPI00292746 | R.LN#TTNAWGAAPPSLGSQPLYR.S | 0.7378 |
| IPI00292819 | R.EGELCSLLKEN#VSELRILSSGNDHGNWCIIAEKK.G | 0.7925 |
| IPI00292824 | R.NTPWTPWLPVN#VTQGGARQEQR.F | 0.9102 |
| IPI00292859 | K.EAPYFYN#DTVTFK.C | 0.9999 |
| IPI00292859 | K.TPNGN#HTGGNIARF.S | 0.5538 |
| IPI00292907 | Y.LFVIFDFLIGVLIFATIVGNVGSM*ISNM*N#ATR.A | 0.7059 |
| IPI00292928 | R.TKDAGLGVYSLALLNN#VSYNVVEFSK.S | 0.5167 |
| IPI00292946 | K.VTACHSSQPN#ATLYK.M | 1 |
| IPI00292946 | K.TLYETEVFSTDFSN#ISAAK.Q | 1 |
| IPI00292946 | K.TTTVQVPMMHQM*EQYYHLVDM*ELN#CTVLQMDYSK.N | 0.9936 |
| IPI00292950 | K.DFVN#ASSK.Y | 0.9968 |
| IPI00292950 | K.N#LSM*PLLPADFHK.E | 0.9996 |
| IPI00292953 | R.RAELVCLN#NTEISEN#SSDLSQKLK.E | 0.8791 |
| IPI00293057 | K.QVHFFVN#ASDVDNVK.A | 1 |
| IPI00293057 | K.AHLN#VSGIPCSVLLADVEDLIQQQISN#DTVSPR.A | 1 |
| IPI00293086 | R.NN#QTIFEQTINDLTFDGSFVK.E | 0.7167 |
| IPI00293173 | R.GYLQALASKMTEELEALRN#SSLGTR.A | 0.8526 |
| IPI00293183 | M.QAPAFRDKKQGVSAKNQGAHDPDYEN#ITLAFK.N | 0.6116 |
| IPI00293203 | M.FTMATAEHRSN#SSIAGK.M | 0.6155 |
| IPI00293274 | R.LPQDGDN#VTVENGQLLLLDTN#TSILNLLHIK.G | 0.7351 |
| IPI00293274 | R.WQIVPN#ASSPFGFWS.Q | 0.7022 |
| IPI00293328 | R.ETGDN#FSDVAIQGGIMGIE.I | 0.5343 |
| IPI00293381 | M.GMIFTLFTIN#VSTDM*R.H | 0.9229 |
| IPI00293426 | K.RLAYLLQQTDEYVAN#LTELVPQHK.A | 0.685 |
| IPI00293471 | M.NKWAGLLGPISN#HSFGGSFR1ASNK.E | 0.909 |
| IPI00293471 | K.LFSDIEN#ISEETSAEVHPIS.L | 0.9214 |
| IPI00293471 | K.LSNNLNVEGGSSENN#HSLK | 0.6898 |
| IPI00293520 | K.SRMAIWAATDIINVDN#TTEIFR.E | 0.9955 |
| IPI00293565 | K.VLLLHEVHAN#DTGSYVCYYK.Y | 0.9993 |
| IPI00293575 | K.SVN#VSSNLVTQEPSPEETSTKR.S | 0.6597 |
| IPI00293583 | K.AGHSNKYLKM*AN#NTKELEVCEQANK.L | 0.9328 |
| IPI00293590 | K.VLAAKVLNLVLPN#LSLGPIDSSVLSR.N | 0.7033 |
| IPI00293602 | R.NYQRIFQN#LTSTASSGTNVHGS.P | 0.7657 |
| IPI00293616 | R.VGNLGLATSFFNEKNM*N#ITK.D | 0.7543 |
| IPI00293714 | I.DN#ITNSMKKTK.S | 0.6257 |
| IPI00293714 | S.LPMSIN#VTDDIVYISTHPEASSR.T | 0.8565 |
| IPI00293748 | K.FLTEVEKN#ATALYHVEAFK.T | 0.9596 |
| IPI00293748 | R.AN#STSDEL.- | 0.6165 |
| IPI00293773 | R.AGM*VYMAGLVFAVGGFN#GSI.RVR.T | 0.9811 |
| IPI00293849 | L.TV#ISPYIN#VSVKLILMNPEGRK.E | 0.502 |
| IPI00293921 | R.DLN#VSVTHLIAGEVGSKK.Y | 0.5572 |
| IPI00293925 | R.VELEDFNGN#R.T | 0.9981 |
| IPI00293971 | R.VINFYAGAN#QSM*N#VTCAGK.R | 0.5256 |
| IPI00294004 | L.VSGN#NTVPFAVSLVDSTSEK.S | 0.8514 |
| IPI00294005 | R.LEQQM*NSASGSSSN#GSSIN#MSGIDNGEGTRL.R.N | 0.9073 |
| IPI00294073 | R.FRSSGM*LDN#ISR.A | 0.5565 |
| IPI00294125 | K.EN#EFESLQILNAK.T | 0.6675 |
| IPI00294193 | N.QLVDALTFWQN#K.T | 0.9572 |

86

| | | |
|---|---|---|
| IPI00294193 | K.LPTQN#ITFQTESSVAEQEAEFQSPK.Y | 1 |
| IPI00294193 | R.NQALN#LSLAYSFVTPL.T | 1 |
| IPI00294193 | K.AFITN#FSMNIDGMTYPGIIK.E | 0.9988 |
| IPI00294395 | K.EYESYSDFERN#VTEK.M | 1 |
| IPI00294486 | K.SN#ISPNFNFMGQLLDFER.S | 0.9916 |
| IPI00294578 | M.NMGSDFDVFAHITN#NTAEEYVCR.L | 0.6102 |
| IPI00294728 | K.LDDISSN#YTESFSTLDENDLLN#PSEDIIAVQLK.F | 0.9495 |
| IPI00294728 | K.QKPSGLTRSTSMLISSGIIN#KSSNSLK.L | 0.7527 |
| IPI00294739 | K.KEWN#DSTSVQN#PTRLR.E | 0.7366 |
| IPI00294744 | R.SQANGAGALSYVSPN#TSK.C | 0.7598 |
| IPI00294776 | R.HDYILLPEDALTN#TTR.L | 0.992 |
| IPI00294776 | R.APSN#VSTIIHILYLPEDAK.G | 0.813 |
| IPI00294787 | M.PPVSLNHN#LTTPFTSQAGENSLF.M | 0.7359 |
| IPI00294798 | R.TKSNSLSEQLAIN#TSPDAVK.A | 0.9417 |
| IPI00294816 | M.VNN#VTPARAVVSLINGGQR.Y | 0.5376 |
| IPI00294879 | M.QKAFN#SSSFNSNTFLTR.L | 0.712 |
| IPI00294903 | K.KRGTFIEFRNGMLN#ISPIGRSC.T | 0.5352 |
| IPI00294943 | M.QQHN#MSWIEVQFLK.K | 0.5639 |
| IPI00294997 | M.VTEMYSGPCVAM*EIQQNN#ATK.T | 0.7213 |
| IPI00295081 | R.VSGLM*M*AN#HTSISSLFER.T | 0.7492 |
| IPI00295182 | K.GGAVAADGRIEPGDM*LLQVNEINFEN#M*SN.D | 0.8753 |
| IPI00295339 | K.AYSWN#ISR.K | 0.5725 |
| IPI00295376 | -.M*DQNQHLN#KTAEAQPSENK.K | 0.5571 |
| IPI00295380 | R.VVNLEALQMLSVN#TTLEEL.K.I | 0.9781 |
| IPI00295387 | R.N#DSESSGVLYSRAPTYFCGQTLTFR.Q | 0.7118 |
| IPI00295461 | K.DDNLEHYKN#STVMAR.A | 0.9998 |
| IPI00295502 | H.LRQM*GVTEWSVN#GSPIDTLR.F | 0.546 |
| IPI00295503 | K.MM*N#DSILRLQTWDEAVFR.E | 0.5128 |
| IPI00295640 | K.SLKHQNILLEVDDFENRN#GTDGLSYNGAIIVSGK.Q | 0.6525 |
| IPI00295672 | M.KNKRN#VTEFVLTGLTQNPKM*EK.V | 0.7847 |
| IPI00295743 | K.MADALLFGNFGVQN#ITAAIQLYESLAK.E | 0.8936 |
| IPI00295832 | M.TLSITSGM*PNN#FSEM*PQQSITLNLWR.E | 0.7745 |
| IPI00295988 | P.GNLPPSMN#ISQLLGLRK.N | 0.5407 |
| IPI00296053 | R.INKLM*N#ESLMLVTALNPHIGYDK.A | 0.5051 |
| IPI00296063 | K.N#ISNPEAYDIICFFKK.E | 0.5907 |
| IPI00296099 | G.EDTDLDGWPNENLVCVAN#ATYHCKK.D | 0.7692 |
| IPI00296099 | K.VVN#STTGPGEHLR.N | 1 |
| IPI00296099 | K.VSCPIM*PCSN#ATVPDGECCPR.C | 0.9875 |
| IPI00296099 | K.GCSSSTSVLLTLDNNVVN#GSSPAIR.FNYIGHKFK.D | 0.5429 |
| IPI00296161 | K.FIINENGAN#YSVTATR.S | 0.9775 |
| IPI00296165 | N.LLPICLPDN#DTFYDLGLM*GYVSGFGVM*EEK.I | 1 |
| IPI00296165 | K.EHEAQSN#ASIDVFLGHTNVEELM*K.L | 1 |
| IPI00296165 | K.MLLTFHTDFSNFEN#GTIM*FYK.G | 1 |
| IPI00296165 | R.CN#YSIR.V | 0.9967 |
| IPI00296170 | K.MVSHIIN#LTTGATLINFQWLLTFAK.N | 1 |
| IPI00296170 | K.NLFLN#IISLN#ATAK.D | 1 |
| IPI00296210 | K.DKYLHTNCLAALAN#M*SAQFR.S | 0.5587 |
| IPI00296211 | L.LLLLVLAN#LTDASDAPNPYR.Q | 0.8296 |
| IPI00296215 | K.LAAKCLVMKAEMN#GSK.I | 0.503 |
| IPI00296411 | R.YMLASPDVTSILLTYN#LSNTNSCN#VSPKK.F | 0.9247 |
| IPI00296318 | Q.CSSLGAFSII.SGKEN#SSALSPNHR.I | 0.539 |

| | | |
|---|---|---|
| IPI00296362 | R.SPLQACENLAMNEGGPPTEN#NSLILEENK.I | 0.5479 |
| IPI00296421 | R.LRN#SSSFSM*DDPDAGAMGAAAAEGQ.A | 0.8203 |
| IPI00296449 | R.TTSTLSLSAEDSQSTESN#MSVPK.K | 0.9536 |
| IPI00296449 | K.ENN#LTEDNPN#LSM*AQRR.II | 0.603 |
| IPI00296485 | M.VDPEM*LPPKTARQTEN#VSR.T | 0.9937 |
| IPI00296495 | K.CIRCAVVGNGGILN#GSR.Q | 0.9495 |
| IPI00296527 | K.KNSDGM*EAAGVQIQM*VN#ESLG.Y | 0.9403 |
| IPI00296534 | R.NCQDIDECVTGIHN#CSIN#ETCFNIQGGFR.C | 0.9998 |
| IPI00296534 | R.CATPHGDN#ASLEATFVK.R | 1 |
| IPI00296573 | R.SPASERRPLGN#FTAPPTYTETLSTAPLASWVR.S | 0.6423 |
| IPI00296594 | K.DPPSEANSIQSAN#ATTKTSETN#HISR.P | 0.9321 |
| IPI00296608 | R.N#YTLTGR.D | 0.9819 |
| IPI00296608 | K.JNNDFNYEFYN#STWSYVK.H | 1 |
| IPI00296776 | K.ERVEN#YSN#VSIHLKNP.E | 0.6446 |
| IPI00296845 | K.SSSTPFPFRTGLTSGN#VTENLQTYIDK.S | 0.7546 |
| IPI00296858 | R.KTENAYNAIINGEAN#VT.G | 0.523 |
| IPI00296866 | K.EIRVLEFRSPKEN#DSGVDVYYAVTFNGE.A | 0.7294 |
| IPI00296869 | K.ATN#ATLDPR.S | 0.8042 |
| IPI00296936 | M.VSFVSN#YSHTANILPDIENEDFIKDCVRIHNKFR.S | 0.78 |
| IPI00296999 | M.HLTTLCN#TSLDN#PTQR.N | 0.7143 |
| IPI00297089 | K.DISSSEMTN#PSDTLNIETLLN#GSVKRVSENNGNGK.N | 0.5533 |
| IPI00297124 | N.PPIIN#LSVINSEELSSILK.L | 0.5489 |
| IPI00297124 | R.ETHLETN#FTLK.S | 0.9988 |
| IPI00297210 | K.AGKPVVAAPGAGN#LTKFEPR.A | 0.6356 |
| IPI00297223 | H.SDHDN#STSLNGGK.R | 0.6658 |
| IPI00297242 | R.VLN#TSSLESATDEAGSPLAAAAAAAAAER.C | 0.854 |
| IPI00297252 | R.LFPN#ASQHITPSYNYAPNPDK.II | 0.7442 |
| IPI00297257 | T.RGRSISFPALLPIPGSN#RSSVIM*TAK.P | 0.5512 |
| IPI00297263 | R.ALLSITDN#SSSSDIVESSTSYIK.I | 0.7064 |
| IPI00297263 | K.SHAASDAPEN#LTLLAETADAR.G | 1 |
| IPI00297263 | R.SYSESSSTSSSESLN#SSAPR.G | 0.995 |
| IPI00297263 | R.SN#ISSYDGEYAQPS.T | 0.6318 |
| IPI00297277 | K.NWIALIPKGN#CTYR.D | 0.8069 |
| IPI00297366 | K.KM*LYRDFN#MTGWAYK.T | 0.6371 |
| IPI00297570 | K.EEVQN#LTSVLNELQEEIGAYDYDELQSR.V | 0.9997 |
| IPI00297570 | R.LPIIPWSGTGQVVYN#GSIYFNK.F | 0.9999 |
| IPI00297570 | K.VQN#M*SQSIEVLDR.R | 0.9999 |
| IPI00297570 | K.SM*VDFM*N#TDN#FTSHR.L | 0.999 |
| IPI00297622 | K.SDNN#YSTPNER.G | 0.5113 |
| IPI00297626 | A.MAYDLLPIEN#DTYK.Q | 0.9433 |
| IPI00297633 | K.ENMN#LSEAQVQALALSR.Q | 0.7336 |
| IPI00297633 | R.RSSEN#M*TAEPMSESKINTLVQK.L | 0.6802 |
| IPI00297646 | R.LM*STLASQN#ITYHCK.N | 0.8632 |
| IPI00297671 | R.N#LSEGNNAN#YT.E | 0.6528 |
| IPI00297723 | K.DRGVTRFQEN#ASEGKAPAEDVFKK.P | 0.8071 |
| IPI00297763 | M.N#VSGGPITREASKEI.P | 0.7717 |
| IPI00297763 | K.TPSVSPN#ITQITQK.Q | 0.7159 |
| IPI00297763 | M.N#YSVSAGLVVGIFIGFQK.K | 0.5787 |
| IPI00297897 | M.QELSNILN#LSYK.Q | 0.9162 |
| IPI00297910 | R.THHILIDLRHRPFAGAIN#IISDLDAELR.R | 0.5326 |
| IPI00297921 | R.ISCRPQTQISMNYGNNPLN#SSLLPQK.Q | 0.5079 |

| | | |
|---|---|---|
| IPI00297985 | R.EIVSQTTATQEKSQEELPTTN#NSVSK.E | 0.6891 |
| IPI00298031 | K.ISLKIQNCRN#VTSLPCLSLR.K | 0.5194 |
| IPI00298031 | R.APTFLMN#QTDTHIVEK.M | 0.9964 |
| IPI00298216 | K.DTNVQVLM*VLGAGRGPLVN#ASLR.A | 0.6096 |
| IPI00298285 | M.EGTATCN#GSGSDTCAQCAHFR.D | 0.6392 |
| IPI00298337 | R.SLIASGLYGYN#ATLVGVLMAVFSDK.G | 0.8342 |
| IPI00298347 | K.KNPMVETLGTVLQLKQPLN#TTR.I | 0.5988 |
| IPI00298464 | K.M*AAVTLLALAYTQGPVLFN#LTFK.I | 0.7319 |
| IPI00298497 | R.GTAGNALMDGASQLM*GEN#R.T | 0.9775 |
| IPI00298536 | R.DVMLEN#YSNLVSLGLLGPKPDTFSQLEKR.E | 0.7354 |
| IPI00298673 | E.ENTDDN#ITVQGEIRKEDGM*ENLK.N | 0.6622 |
| IPI00298828 | R.DTAVFECLPQHAMFGN#DTITCTTHGN#WTK.L | 0.9998 |
| IPI00298828 | R.VYKPSAGN#NSLYR.D | 1 |
| IPI00298828 | K.LGN#WSAMPSCK.A | 0.9996 |
| IPI00298853 | K.LCDN#LSTK.N | 0.995 |
| IPI00298860 | R.TAGWNVPIGTLRPFLN#WTGPPEPIEAAVAR.F | 0.9959 |
| IPI00298888 | K.NLN#YSVPEEQGAGTVI.G | 0.5438 |
| IPI00298902 | K.KQSNNDLFQVN#STSDDEIPR.K | 0.7548 |
| IPI00298902 | -.M*ASQLQEKCIAFIVDN#FSK.I | 0.9778 |
| IPI00298902 | K.SIHEQDTNVN#NSVLKK.V | 0.8003 |
| IPI00298920 | K.TGGDN#KTLLHLGSSAPGK.E | 0.8688 |
| IPI00298971 | R.N#ISDGFDGIPDNVDAALALPAHSYSGR.E | 1 |
| IPI00298971 | K.NN#ATVHEQVGGPSLTSDLQAQSK.G | 1 |
| IPI00298971 | K.N#GSLFAFR.G | 0.9995 |
| IPI00298980 | N.#TSAPPAVSPN#ITVLAPGK.G | 0.6924 |
| IPI00298994 | R.AATAPLLEAVDN#LS.A | 0.5498 |
| IPI00299059 | R.VTWKPQGAPVEWEEETVTN#HTLR.V | 0.9543 |
| IPI00299059 | R.RYHIYEN#GTLQIN#R.T | 0.9948 |
| IPI00299122 | M.ITMVCCAHSTNEPSN#M*SYVK.E | 0.6482 |
| IPI00299158 | R.VEDEGN#YTCLFVTFPQGSR.S | 0.9958 |
| IPI00299162 | F.GSNMGN#GTVFLGIPGDNK.Q | 0.5758 |
| IPI00299162 | R.IRVDLPLGSPAVN#CTVLPGGIS.V | 0.7538 |
| IPI00299299 | V.LNKNGM*VEFSVTSN#ETITVSPEYVGSR.L | 0.8532 |
| IPI00299377 | R.SVN#GTTSDCLVSLVTSVTN.V | 0.5324 |
| IPI00299435 | R.STERN#VSVEALASALQLLAR.E | 1 |
| IPI00299435 | R.QGGVN#ATQVLIQHLR.G | 0.8233 |
| IPI00299435 | K.DAN#ISQPETTKEGLR.A | 0.548 |
| IPI00299503 | K.LGTSLSSGHVLMN#GTLK.Q | 0.9998 |
| IPI00299503 | K.LNVEAAN#WTVR.G | 1 |
| IPI00299503 | K.FHDVSESTHWTPFLN#ASVHYIR.I | 1 |
| IPI00299503 | R.N#LTTSLTESVDR.N | 0.9996 |
| IPI00299503 | R.NIN#YTER.G | 0.9524 |
| IPI00299503 | R.TLLLVGSPTWKN#ASR.L | 0.5773 |
| IPI00299507 | K.DLQRSIPPVM*AQN#LSIPLAFACLLHLANEK.N | 0.5463 |
| IPI00299512 | R.YMH.MLSPN#NSLDVAAILL | 0.524 |
| IPI00299526 | K.QVPIIPDLN#Q.T | 0.7161 |
| IPI00299526 | K.LRIFYQFLYNN#NTR.Q | 0.6424 |
| IPI00299547 | K.SYN#VTSVLFR.K | 1 |
| IPI00299594 | K.FGPECSQN#YTTPSGVIK.S | 1 |
| IPI00299597 | K.EGFSAN#YSVLQSSVSEDFK.C | 1 |
| IPI00299619 | K.TSATSVN#LSLLTADLYSLFCGLHLF | 0.5243 |

| | | |
|---|---|---|
| IPI00299635 | K.FVLNSN#ITNIP.Q | 0.5209 |
| IPI00299664 | R.VQALDPDEGSNGEVQYSLSN#STQAELR.H | 0.7289 |
| IPI00299778 | R.VSTVYANN#GSVLQGTSVASVYHGKILIGTVFXK.T | 0.603 |
| IPI00299831 | K.N#ASDGALMDDNQNEWGDEDLETKK.F | 0.6047 |
| IPI00299884 | K.LLM*ENPYEGPDSQKEKDSN#SSK.Y | 0.9439 |
| IPI00300020 | K.KVLVAPPPDEEAN#ATSAVVSLLN#ETVTEVPEETK.M | 0.8428 |
| IPI00300078 | K.NN#KSDTLPLATRYN.V | 0.9648 |
| IPI00300078 | R.GNLN#FTCNGNSVISPVGNR.V | 0.9113 |
| IPI00300078 | K.RFEISCN#LSLDAMEEFLNRRK.M | 0.6269 |
| IPI00300117 | P.SSEVMN#KSRCESLLFN#ESMLWENAK.M | 0.6816 |
| IPI00300117 | K.ELQEGN#ETDEAK.T | 0.6902 |
| IPI00300173 | K.LALRN#NSASTTQHLR.L | 0.5401 |
| IPI00300376 | S.AMINSNDDNGVLAGN#WSGTYTGGR.D | 0.8423 |
| IPI00300384 | G.PTQCVN#CSQFLR.G | 0.5345 |
| IPI00300408 | M.KFRN#SSVAMGASLSCSEYSLK.V | 0.7599 |
| IPI00300465 | K.LANN#GTVLR.A | 0.9589 |
| IPI00300573 | R.QFPKLN#ISFVDEQVR.L | 0.5062 |
| IPI00300585 | K.EDGSGSAYDKESMAIIKLN#NTTVLYLK.E | 0.796 |
| IPI00300599 | K.VPLSHSRSN#DTLYIPEWEGR.A | 0.8356 |
| IPI00300631 | R.KRNVDSSGN#KSVLMERL.K | 0.5679 |
| IPI00300813 | M.IN#FSAFLGAATMYTRYK.I | 0.5375 |
| IPI00300838 | R.RLLIKKMPAAATIPAN#SSDAPFIR.P | 0.5065 |
| IPI00300843 | K.QLLRDLSGLQGM*N#GSIQAK.S | 0.5379 |
| IPI00300936 | K.KKDSLHGSTGAVN#ATRPT.L | 0.5997 |
| IPI00301021 | K.DLNGNVFQDAVFN#QTVTVIER.E | 0.8101 |
| IPI00301031 | K.HSSGTSN#TSTAN#RSTHNELEK.N | 0.9056 |
| IPI00301107 | M.GRVLLQN#TSFFSSLLNEMAHK.F | 0.8501 |
| IPI00301143 | K.SLPNTPN#TSATAN#ATGGR.A | 0.9999 |
| IPI00301143 | R.EHYN#LSAATCSPGQM*CGHYTQVVWAK.T | 1 |
| IPI00301180 | -.MPNN#LTDCEDGDGGANPGDGNPK.E | 0.8099 |
| IPI00301180 | K.NM*ALFEEEMDTSPMVSSLLSGLAN#YTNLPQGSR.E | 0.627 |
| IPI00301248 | K.QPTPIAN#TSSQQAVFTSARQLPSAR.T | 0.953 |
| IPI00301248 | K.DQVN#GTSEDSADGSTVGTAVSSSDDADLPPP.P | 0.6801 |
| IPI00301288 | R.CGEPPSIMNGYASGSN#YSFGAMVAYSCNK.G | 0.993 |
| IPI00301480 | K.HPLTAN#ASR.S | 0.9126 |
| IPI00301517 | K.LLYLTTTN#ESGVFITG.H | 0.8678 |
| IPI00301548 | M.SSKHN#MSGGEFQGKR.E | 0.7001 |
| IPI00301548 | M.SGTTTSTNTFPGGPIATLFN#M*SMSIK.D | 0.5976 |
| IPI00301610 | R.KMAHPAMFPRRGSGSGSASALNAAGTGVGSN#ATSSEDFPPP.S | 0.7245 |
| IPI00301793 | A.AQN#NTVTVPK.N | 0.846 |
| IPI00301793 | R.ISKLIN#SSDEIQDNFR.E | 0.8615 |
| IPI00301968 | R.DVMLEN#YSHLVSVGYLVAK.P | 0.684 |
| IPI00302029 | R.EVN#STDWDSKMGFWAFLVLSHSR.R | 0.5398 |
| IPI00302311 | -.M*EN#FSLLSISGPPISSSALSAFPDIMF.S | 0.7655 |
| IPI00302329 | K.RQAEEAEEQSNAN#LSKFR.K | 0.7454 |
| IPI00302383 | M.NIMSTTQWAVN#SSIDVDSLMRSVSR.V | 0.7038 |
| IPI00302409 | P.AAEHFN#YSVMVDIRELIEVDDVM.E | 0.7079 |
| IPI00302448 | K.DGQLTPSSN#YSNIK.I | 0.9908 |
| IPI00302453 | K.SFGSPPLAVSN#VSAAVMVLMAPRG.R | 0.6889 |
| IPI00302503 | K.N#DSLIQN=DSILLSLLEVL.R | 0.7389 |
| IPI00302557 | K.FECRLLNAPPVPPRGGN#GSGRL | 0.528 |

| IPI00302592 | G.LN#TTGVPASLPVEFTIDAK.D | 0.7334 |
|---|---|---|
| IPI00302652 | R.IDRM*FPEMSIHLSRPN#GTSAM*LLVTLGK.V | 0.5906 |
| IPI00302717 | K.KLSN#FSFLTHR.Q | 0.7542 |
| IPI00302807 | R.TSEHQVDLKVDPSQPSN#VSHKLWTAA.G | 0.6753 |
| IPI00302965 | R.N#ASLSQSPR.V | 0.9955 |
| IPI00303040 | K.WTN#LSDPMPVGQMGTVK.Y | 0.7499 |
| IPI00303053 | R.ERTSSSIVFEDSGCDN#ASSK.E | 0.5301 |
| IPI00303068 | K.FLAEHPN#VTLTISAARLYYYR.D | 0.917 |
| IPI00303112 | K.LELNPHTVEN#VTKNEDSM*TGIEVEKWTQNKK.S | 0.5739 |
| IPI00303117 | R.FLLNDN#LTLPPEMYVYSTNSDHLPM*TSSFR.K | 0.9436 |
| IPI00303135 | K.SEEN#STVFSHLM*K.Y | 0.9301 |
| IPI00303157 | M.ILN#LTQSSGFNGFTPLVTLLLR.H | 0.8116 |
| IPI00303163 | K.GIAILN#TSVAPMLNPFIYTLR.N | 0.9001 |
| IPI00303283 | R.AKWDTANNPLYKEATSTFTN#ITYR.G | 0.5207 |
| IPI00303313 | K.N#VTLILDCKKK.T | 0.7314 |
| IPI00303325 | K.AGN#ETQISEFLLLGFSEK.Q | 0.8122 |
| IPI00303335 | K.YRTKIETLN#FTPVDDRVDYVTAK.Q | 0.6984 |
| IPI00303335 | M.PDTPDILLAKSNSAN#ISQKLYTK.G | 0.5824 |
| IPI00303389 | -.M*VLASGN#SSSHPVSFILLGIPGLESF.Q | 0.9163 |
| IPI00303402 | K.AIELLM*ETAEVEQNGGLFIMN#GSR.R | 0.5426 |
| IPI00303431 | -.MNLDSFFSFLLKSLIM*ALSN#SSWR.L | 0.7922 |
| IPI00303452 | R.N#LSTCFSSGDL.F | 0.7174 |
| IPI00303455 | N.VGQN#TTR.F | 0.9348 |
| IPI00303458 | -.M*TVRNIASICNMGTN#ASALEK.D | 0.7048 |
| IPI00303463 | M.DTTISIN#NTVITPMLNPHYSLR.N | 0.9337 |
| IPI00303553 | M.TFENPN#QTVVSHFFLEGLR.Y | 0.9434 |
| IPI00303560 | K.EDSGKIKLLLHWMPEDILPDVWVN#ESER.H | 0.6882 |
| IPI00303581 | M.HLAGIN#FTYSLTVIISYLFILIAILRM*R.S | 0.5177 |
| IPI00303582 | R.RN#CTLVTEFILLGLTS.R | 0.8218 |
| IPI00303583 | R.VKKM*AMFVVAGFN#LSSSLHILLSYLFIFAAIFR.I | 0.8499 |
| IPI00303587 | K.IIPMAN#ITWMAN#HTGWSDFILLGLFR.Q | 0.5496 |
| IPI00303699 | K.KQPGQPRPTSKPPASGAAAN#VSTSGITPGQAAAIASTTIM*VPF.G | 0.5648 |
| IPI00303813 | K.CSEN#ATMTLPGIHPPTLNQIM*DWICLLLDAN#FTV.V | 0.6397 |
| IPI00303868 | R.LNYLLRVN#GSEQTVVAFFIM*PARTNNFN.V | 0.839 |
| IPI00303875 | -.M*N#NTAASPMSTAT.S | 0.9316 |
| IPI00303963 | K.LTDTICGVGN#M*SAN#ASDQER.T | 1 |
| IPI00303963 | K.QSVPAHFVALN#GSK.L | 0.9995 |
| IPI00303963 | R.LGSYPVGGN#VSFFCEDGFILR.G | 0.9985 |
| IPI00303963 | K.TMFPN#LTDVR.E | 0.9745 |
| IPI00303963 | K.DHEN#GTGTNTYAALNSVYLMM*NNQM*R.I | 0.6754 |
| IPI00303980 | K.RFN#GSFSIKSSWN#ISVVKFLLEK.L | 0.8393 |
| IPI00304023 | -.M*N#ISGSSCGSPNSADTSSDFK.D | 0.5777 |
| IPI00304023 | S.SNKQH.INKN#ISESLGEQN#R.T | 0.5867 |
| IPI00304030 | R.VHLTPVEGVPDSDYIN#ASHNGYQEK.N | 0.9071 |
| IPI00304379 | R.NKESSDQTGIN#ISGFENK.I | 0.889 |
| IPI00304379 | K.CESDN#TTNGCGLFSPGNTVTPVNVNEVK.P | 0.7172 |
| IPI00304452 | K.SSLSASTNPELGSN#VSGTQTYPVVTGR.D | 0.6096 |
| IPI00304481 | R.THGRN#GTENINIR.G | 0.8711 |
| IPI00304481 | K.RHN#SSDGFDSAIGRPNGGNFGRKEK.N | 0.6298 |
| IPI00304527 | K.FVNSCTTGSSN#STHGSQGSETPK.E | 0.5151 |
| IPI00304587 | K.KLLFLSPDLELN#SSSSHNT.L | 0.6422 |

| IPI00304654 | R.N#STDENFHASLMSEISPISTSPEISEASLM*SNLP.L | 0 7347 |
| IPI00304661 | K.FHHLIPAHTFTN#ISTKNPQGSK.S | 0 5884 |
| IPI00304670 | R.TPM*N#SSWLPGSPMPQAQSPEEGQR.P | 0.7905 |
| IPI00304706 | H.RVLHNSLGN#ISM*LPCSSFTPNWPVQNPDSR.K | 0.5641 |
| IPI00304710 | K.IM*N#LTEFSM*VDGMWQAQGYPRNR.L | 0.8813 |
| IPI00304849 | R RHKDM*DVDILALVN#DTVGTM*M*TC.A | 0 9665 |
| IPI00304895 | R.QLN#TSDENGKEELFSLK.D | 0.5674 |
| IPI00304911 | K NFINQGPYEN#RSMFESLDLSWK.L | 0.7486 |
| IPI00304926 | K.HVTM*DQVGTFAGLLIGLAQLN#CSELKLK.R | 0.9847 |
| IPI00304972 | V.KEM*HTCTVEGCN#ATFPSR.R | 0.9354 |
| IPI00304992 | K.HSGGGGGGGGGGGADPAWTSALSGN#SS.G | 0.8687 |
| IPI00305022 | -.M*ILSN#TTAVTPFL.T | 0 6979 |
| IPI00305349 | P.SSLIQAN#VTVGTCSGFCSQKEFPLAILR.G | 0 6627 |
| IPI00305349 | R.SLVPTLREM*VAFLN#VSVSEER.L | 0.5169 |
| IPI00305374 | K.TGEDEDEEDNDALLKEN#ESPDVRR.D | 0.848 |
| IPI00305383 | K.TIAQGN#LSNTDVQAAKNK L | 0.726 |
| IPI00305442 | M.LENPLN#STQWM*NDPETGPVMLQISR.I | 0.5715 |
| IPI00305457 | K.ADTHDEILEGLNFN#LTEIPEAQIHEGFQELLR.T | 1 |
| IPI00305457 | R.QLAHQSN#STNIFFSPVSIATAFAMLSLGTK.A | 1 |
| IPI00305457 | K YLGN#ATAIFFLPDEGK.L | 1 |
| IPI00305461 | K.VVN#NSPQPQNVVFDVQIPK.G | 1 |
| IPI00305461 | K GAFISN#FSMTVDGK.T | 1 |
| IPI00305461 | K.ENIQDN#ISLFSLGMGFDVDYDFLK R | 0.8276 |
| IPI00305622 | R.VISAM*VNSLDDNGVLIGN#WSGDYSR G | 0.5299 |
| IPI00305656 | K LETKFKSGLN#GSILAER.E | 0 5948 |
| IPI00305698 | K.EKVEN#GSETGPLPPELQPLLEGEVK.G | 0.698 |
| IPI00305715 | K.VSGYLNLLANTIDN#FTHGIAVAASFLVSK.K | 0.5026 |
| IPI00305725 | R.AVNALPQN#MTSQMF.S | 0.6692 |
| IPI00305894 | R.TSRKSALRAGN#DSAM*ADGEGYR N | 0.8772 |
| IPI00305901 | K.EEQHAN#TSANYDVELLHHK.D | 0.8009 |
| IPI00305945 | R NWAHN#SSVEASSNLEAPGNER.K | 0.7208 |
| IPI00305945 | K.CPYSQEAMQRALIGRCQN#VSALPK G | 0.8236 |
| IPI00306152 | M EVNGQNFEN#ITFMK A | 0.7124 |
| IPI00306196 | F.EN#KTNLKNQLILGVMGVDVSLEDIKR L | 0.8677 |
| IPI00306196 | K.IDVNSWIEN#FTK.T | 1 |
| IPI00306196 | K.ISDN#NTEFLLNFNEFIDR K | 0.8316 |
| IPI00306346 | R.LLAEGVCDN#DTVPSVSSINR.I | 0.5678 |
| IPI00306400 | R.ETQAIN#SSLSTLGLVIMALSNK E | 0 7104 |
| IPI00306471 | K.EAN#PTPLTPGIASSLSQLGAYLDSDDSN#GSN.- | 0.5005 |
| IPI00306599 | R.SCLSCPEN#ISTVKR.G | 0 8955 |
| IPI00306642 | K AN#ASEKLGVLTSR.F | 0.7759 |
| IPI00306718 | K IYIFDN#LSNSNEVEMEEK G | 0.5926 |
| IPI00306718 | R.EGKELILYIDASLEITN*VTQKT | 0 7976 |
| IPI00306723 | R NI N#DSSLFVSAEEFGHLLDENM*GSK F | 0 6316 |
| IPI00306845 | K WSM*VCLLMN#GSSHSPTAI N | 0 925 |
| IPI00306851 | R KVLIN#NSLDEPR.A | 0 827 |
| IPI00306869 | K.SLN#DSIQPDPLCLHN#SSLFALQNLQP W | 0 9805 |
| IPI00306929 | M SLDFN#ATGRITAAQLQIMLLEKSR V | 0 8807 |
| IPI00306967 | R YGETYGN#LTRPDITFTYFQFK P | 1 |
| IPI00306984 | R IVDGIEDGN#SSEESQIFDFGSER I | 0 704 |
| IPI00307017 | R.ELRSAI LEFACTHNLGN#CSTTAMK L | 0 5199 |

| | | |
|---|---|---|
| IPI00307093 | K.HYPKN#HSIEVQ.L | 0.9248 |
| IPI00307150 | K.AVTVMM*DPN#STQRYR.L | 0.665 |
| IPI00307317 | R.FGLLFNQEN#TTYSK.T | 0.7814 |
| IPI00307405 | R.RTSMHSSLN#TSPNQQPD.T | 0.5914 |
| IPI00307591 | R.GKRM*RPNSNTPVN#ETATASDSKGTS.N | 0.7204 |
| IPI00307591 | R.KNKPLSDMELN#SSSEDSK.G | 0.8995 |
| IPI00307592 | K.VSQQLGLDAPN#GSDSSPQAPPPR.R | 0.7343 |
| IPI00307611 | S.GNKVSITTTPFEN#TSIKTGPARR.N | 0.5148 |
| IPI00307611 | R.SFSCLN#RSLSSGESLPGSPTHSLSPR.S | 0.6572 |
| IPI00307649 | R.LHNGN#ASPPR.V | 0.7793 |
| IPI00307660 | R.CFLQLQPDN#STLTWVKPTTASPASSKAK.L | 0.6972 |
| IPI00307665 | T.AANN#LSVSNSASSLQK.D | 0.7301 |
| IPI00307713 | R.KPPLFNMNAM*SALYHIAQN#DSPTLQSNEWTDSFR.R | 0.6905 |
| IPI00307829 | R.AAGSAQGNNQACN#STSEVK.D | 0.7026 |
| IPI00328090 | M.TN#TSYSEPAQNSKLSLK.Q | 0.5474 |
| IPI00328094 | W.VSREPALLCTFPN#PSAPRK.D | 0.7148 |
| IPI00328115 | P.FLSDIHN#ISTLK.I | 0.7296 |
| IPI00328131 | R.STVGLSLISPNN#MSFATK.K | 0.578 |
| IPI00328142 | K.EALGSVVAHSTFSAMKANTMSN#YTLLPPSLLDHR.R | 0.5048 |
| IPI00328147 | K.RTIN#SSQEPAPGMKPNWPRSR.Y | 0.6458 |
| IPI00328183 | R.GSGAVAEGN#RTEAGSQDYSLLQAYYSQESK.V | 0.676 |
| IPI00328183 | K.N#ESSPAPPDSDADK.L | 0.6272 |
| IPI00328195 | R.YLLQNTALEVFMAN#R.T | 0.9065 |
| IPI00328195 | R.CYVNGQLVSYGDMAWHVNTN#DSYDK.C | 0.7676 |
| IPI00328195 | K.SM*IN#TTGAVDSGSSSSSSSSSFVNGATSK.N | 0.6068 |
| IPI00328207 | K.QN#NSYWR.E | 0.6204 |
| IPI00328226 | C.YEYFHQDDHNN#LTDK.H | 0.5659 |
| IPI00328267 | R.NRN#LSGGVLM*GFMLNR.I | 0.8592 |
| IPI00328309 | K.GLAQPPQAYFN#GSLPPQTVGHQAHGR.E | 0.5042 |
| IPI00328318 | K.DENSIN#GTPDITVEIR.E | 0.6536 |
| IPI00328355 | K.ENKGDNNCNHNDPLYEN#SS.- | 0.804 |
| IPI00328365 | A.SGPVILSELHPICN#KSILR.Q | 0.9445 |
| IPI00328434 | M.SLFLSN#LSTN#DSSLWK.E | 0.914 |
| IPI00328450 | R.FN#NSLI.PTEPSQQLQAYVAWVNAQLKKR.P | 0.8225 |
| IPI00328460 | K.EYVKSEVIKLLPNAN#GSNVQLQLK.F | 0.7791 |
| IPI00328493 | K.GLEWIGYIYYSGSTNYN#PSLKSR.V | 0.6115 |
| IPI00328550 | K.YRCN#DTIPEDFQEFQTQNFDRFDN.- | 0.9769 |
| IPI00328555 | R.NLLETGLN#VSDTVTLPTAPNM*NSEPTLQPQTGHITNR.M | 0.6081 |
| IPI00328555 | M.DLTLN#SSTATPVSPGSVTK.E | 0.885 |
| IPI00328584 | R.AS.AGLN#SSATSTAN#NSRCE.G | 0.7296 |
| IPI00328609 | R.SQILEGLGFN#LTFLSESDVHR.G | 1 |
| IPI00328609 | K.DFYVDEN#TTVR.V | 1 |
| IPI00328609 | K.FLN#DTMAVYEAK.L | 0.9998 |
| IPI00328609 | D.GESCSN#SSHQQILETGEGSPSLK.I | 0.998 |
| IPI00328629 | K.KPKGINSN#STAN.L | 0.8143 |
| IPI00328658 | M.TN#YSFHCNVCHHSGNTYFLR.K | 0.9698 |
| IPI00328704 | K.EQYQVLIQAKDM*GCQLGGLAGTTIVN#ITLIDVN.D | 0.5168 |
| IPI00328706 | R.N#SSLVLHHR.T | 0.7457 |
| IPI00328715 | K.SGKGDSTLQVSSGLNEN#LTVNGGGWNEK.S | 0.5142 |
| IPI00328736 | K.SNGI.MFINIMMQNTN#PSASPEYMFSSNIFPEPKD.L | 0.6152 |
| IPI00328746 | R.ARGN#SSSNHLYGVAFAGAPPADPSTLYR.D | 0.6046 |

| IPI00328752 | R AWHN#LTVLATELDSSAQASR.V | 0 6333 |
|---|---|---|
| IPI00328762 | M SGTLVMLLN#DSADLRDLATSMDSIVK.L | 0.61 |
| IPI00328762 | R.TNGDILVLYN#LSK H | 0 8388 |
| IPI00328809 | K.VELPPPDLGPSSALN#QTLMLLR.E | 0.9487 |
| IPI00328911 | L.KDENTISPYEMCSSGLVQALLTVLNN#VSIFRATK Q | 0 7087 |
| IPI00328911 | K.EAASQRPLSSSASNRLSVSSLLAAGAPM*SSSASVPN#LSSR.E | 0 5238 |
| IPI00328943 | K.AQNGIAIMVYTN#SSNTLYWELNQAVR.T | 0.9689 |
| IPI00328960 | K.TPASN#ISTQVSHTKLSVEAPDSK.F | 0.764 |
| IPI00329007 | K.GFN#WSSTLTKHR.R | 0.884 |
| IPI00329028 | D.AKAQLALSSSAN#QSK.E | 0 6857 |
| IPI00329038 | A.GLGNGVLPN#VSEETVSPTR.A | 0.7932 |
| IPI00329054 | R.LCQTCYPLFQQVVSKMDN#ISR.A | 0.974 |
| IPI00329070 | R.TESQLTPCIRN#VTSPTR Q | 0.5361 |
| IPI00329083 | M.QTCGN#VSNQFQLGTCR L | 0.797 |
| IPI00329130 | M AN#VTWPQGPFTTWSTTGDAPV.I | 0.639 |
| IPI00329192 | R.HVN#LSSLVSCLCVNLCSPYLLLRR A | 0.5882 |
| IPI00329205 | K.EPVGCVNN#ISFLASLAGSTSR N | 0.8038 |
| IPI00329244 | K.DFAN#MTSLVDLTLSR.N | 0.6419 |
| IPI00329264 | K VSIIPPIALN#STDSD G | 0.5697 |
| IPI00329318 | S GEKDESEVISQN#ETCSPAEVESNEK.D | 0.8434 |
| IPI00329345 | K.GGM*NGYHVNGAIN#DTESVDSLSEGLETLSIDAR.E | 0 8529 |
| IPI00329351 | K.NIGAKLVQDVAN#NTNEEAG.D | 0 8142 |
| IPI00329367 | R.LDLSGNALTEDM*AALMLQN#LSSLR.S | 0.8238 |
| IPI00329420 | R.SKDGPSYFTVSFN#RTFLMMITNK.A | 0.5126 |
| IPI00329472 | -.MDPN#CSCSTSSSCTCTTSSKSR.E | 0.682 |
| IPI00329488 | R.SN#STSSMSSGLPEQDRM*AM*TLPR.N | 0.9182 |
| IPI00329528 | R.RQAPIN#FTSRLNRR.A | 0.5127 |
| IPI00329536 | N N#ESSSEGFICPQCMK.S | 0.6904 |
| IPI00329536 | K ELVQVQTLMDN#MTLER.E | 0.854 |
| IPI00329577 | K.KVTCPPTVTVKDEQSGGGN#VSSTLLK Q | 0.5474 |
| IPI00329600 | K.M*N#GTLTAVESFL1IISGPEGLSIHDG.T | 0.9825 |
| IPI00329603 | K.M*EVGIEDCLHIEFEYN#KSK.Y | 0.5164 |
| IPI00329628 | R.LN#GSAAGHV.L | 0.5541 |
| IPI00329631 | K.GTN#VSAPDQLSLALAWNR V | 0 8281 |
| IPI00329637 | R QLVEM*EYTM*QQCN#ASVYM*EAKNR G | 0.6213 |
| IPI00329638 | R SRNKYGRGSISLN#SSPRGR.Y | 0.8113 |
| IPI00329662 | A KENEN#SSPVAGAFGVFSTISTAVQSTGK S | 0.5474 |
| IPI00329695 | K.ALDFSLDGNIN#LTELTLALEYELLVTK N | 0.6113 |
| IPI00329708 | R.YDAQLILEN#NSGIPK.L | 0.5184 |
| IPI00329784 | D.M*VVM*LLSLLFGNVVN#GTIGK Q | 0.7234 |
| IPI00329784 | D.LNKN#CTVTVTLGDERGR V | 0 6606 |
| IPI00332067 | K QM*LIVITDGFSIIDHDQLN#DTALELRNK G | 0 7899 |
| IPI00332082 | K.HTGTIPGAQGLM*N#SSLLHQD I | 0 8619 |
| IPI00332158 | K GKNTNDN#HSFLTNDELAVLP V | 0 6801 |
| IPI00332161 | R.EEQYN#STYR V | 1 |
| IPI00332277 | K.EVN#SSLHLGHAGSSPIALA S | 0 7475 |
| IPI00332318 | S.YFKCGEN#VSQK N | 0 5774 |
| IPI00332333 | C FEN#VTSIMFLVALSLYDRVLVESDNENRM*EESK.A | 0 5939 |
| IPI00332345 | R.DNAN#NSPYLQMNSLR A | 0 9671 |
| IPI00332346 | - N#ASLLIQN#VFQEDTGSYTLHIIKR.G | 0 6811 |
| IPI00332370 | K.AGM*NIARLN#FSHGSHEYHAESIANVRFAVFSF A | 0 5309 |

| | | |
|---|---|---|
| IPI00332380 | R.NGGTNEESN#SSGNTNTDPPAEDSQK.S | 0.5529 |
| IPI00332466 | L.CADDAKTHHWN#ITAVKLALVCSSEGSPGGTAR.G | 0.9991 |
| IPI00332512 | R.SSTSSIDSN#ISSK.S | 0.9998 |
| IPI00332512 | Q.HSLN#LTESTSLDM*LLDDTGECSAR.K | 0.9436 |
| IPI00332565 | -.MDPN#CSCAAGDSCTCAGSCKCKECK.C | 0.7704 |
| IPI00332722 | R.TPYRDMMLEN#YSLLLS.V | 0.6869 |
| IPI00332729 | R.WEYCN#LTR.C | 0.9997 |
| IPI00332845 | K.SSSGNENDEQDSDNAN#MSTQSPVSSEEYDR.T | 0.9962 |
| IPI00332864 | R.KM*SAPAQPPAEGTEGTAPGGGPPGPPPN#MTSNR.R | 0.7569 |
| IPI00332961 | K.SFSLN#RTLTVHQRIHTGEK.P | 0.8543 |
| IPI00333002 | R.HAQN#VTVDEVIG.A | 0.7239 |
| IPI00333041 | R.TGPNPGAGQN#PTRTGPNPGTGQN#PTRTGPNPGTGQN#PTR.T | 0.9711 |
| IPI00333112 | K.EAFAAALNAN#NSMSK.K | 0.6958 |
| IPI00333198 | N.M*IN#ATIKQDDPFNIDLGQQSQR.S | 0.5736 |
| IPI00333279 | K.DVISN#TSDVIGTCEAADVAQKVDEDSAEDTQSND.G | 0.937 |
| IPI00333289 | K.M*NELENKAEPGTHLCIDVEDAMN#ITR.K | 0.7228 |
| IPI00333310 | R.AWPKM*HTVNGYVN#RSLPGLIGCHR.K | 0.7641 |
| IPI00333334 | R.CITCAVVGNGGILN#NSHIGQEIDSH.D | 0.6226 |
| IPI00333382 | M.IYIPN#ATASLNLALSLLLFLEIYNER.V | 0.6007 |
| IPI00333575 | R.N#KSSMVVIDVKM*LSGFTPTM*SSIEELENKGQVMK.T | 0.8104 |
| IPI00333585 | R.RLCTNLVVNCWVLGFIWFLIPIVN#ISQ.M | 0.7531 |
| IPI00333592 | D.PFDN#SSRPSQVVAETR.K | 0.661 |
| IPI00333761 | -.M*AAFSVGTAMN#ASSYSAEMTEPKSV.C | 0.8233 |
| IPI00333770 | Q.VN#QSATALKHVFASLR.L | 0.5469 |
| IPI00333825 | L.EEDSVVHSVEN#DSQNMMESLSPKK.Y | 0.7121 |
| IPI00333858 | M.KGLTTTGN#SSLN#STSNTK.V | 0.6112 |
| IPI00333858 | A.ANN#CTVN#TSSVATSSM*K.G | 0.8885 |
| IPI00333870 | K.TWN#QSIALR.L | 0.9053 |
| IPI00333876 | K.DPKEKQIEPAMTSQNSKRN#T.S | 0.5583 |
| IPI00333913 | K.AVQEDEVGVPGSNSADLLRWTTATTMKVLSN#TTTT.T | 0.9611 |
| IPI00333982 | R.EEQYN#STFR.V | 0.9984 |
| IPI00333985 | N.GRENVGIYN#LSKGVNR.F | 0.8303 |
| IPI00333985 | R.VTNSNANAASPLIVAGYN#VSGSVRSDGEPM.K | 0.6708 |
| IPI00334012 | M.NTQAPPYSM*APAMVN#SSAASVGLADM*MSPGESK.L | 0.6306 |
| IPI00334015 | R.HLTSLNLVQNN#FSPK.G | 0.5146 |
| IPI00334125 | R.DLAELKSSLVN#ESEGAAGG.A | 0.7751 |
| IPI00334168 | R.GGLGGGYSGASGMGGITAVMVN#QS.L | 0.6832 |
| IPI00334245 | K.LVGFPAYGHSFLLSN#PSNHGIDAPTTGPGPAGPYTR.Q | 0.5359 |
| IPI00334271 | S.AN#TTIEDEDAKARK.Q | 0.5975 |
| IPI00334273 | R.RLTIEGVLDHPWLN#STEALDNVLPSAQLMMDK.A | 0.606 |
| IPI00334280 | R.RIWEETGN#YTFSS.D | 0.8651 |
| IPI00334281 | R.FCHEVKIN#YSPYVNYFTRVYWN#R.S | 0.6989 |
| IPI00334291 | K.VLFICTAN#VTDTIPEPLR.D | 0.9053 |
| IPI00334466 | K.GRN#TSSAVEMPFRNSKRSR.L | 0.8392 |
| IPI00334524 | R.IPNTYPN#SSSPGPGGLGGSVHYATMARSAVRPA.S | 0.5261 |
| IPI00334587 | N.QNGAEGDQIN#ASK.N | 0.9303 |
| IPI00334721 | R.TLIAPQGYPNPFN#FSWT.E | 0.5063 |
| IPI00334743 | R.SLAEANN#LSFPLEPLSR.E | 0.5787 |
| IPI00334813 | G.LCGKCFNN#NSN#LSKHK.K | 0.7625 |
| IPI00334829 | R.GNCDSSGM*NLNN#ISFLHSN#RS.S | 0.9642 |
| IPI00334930 | R.SHFLSPQPKNFN#RTATGWRLQ.- | 0.8543 |

| | | |
|---|---|---|
| IPI00334985 | M.LRN#VTQM*S.K | 0.9463 |
| IPI00334996 | K.M*SISPN#TTYPSLLEDGR.V | 0.9998 |
| IPI00335009 | R.LTCN#ATGAPSPTLMWLK.D | 0.7553 |
| IPI00335085 | R.KFLQEN#ASGR.G | 0.7603 |
| IPI00335108 | K.TKATQSQRRN#SSK.T | 0.9491 |
| IPI00335121 | K.RPNEN#SSADISGK.T | 0.5276 |
| IPI00335163 | K.NEIQSFLVSDPEN#TTWADIEAMVSVTL.- | 0.5605 |
| IPI00335210 | R.KYGSCSTILLDN#STASQPDLR.H | 0.97 |
| IPI00335216 | Y.N#FTYTGDGDITLITDNNGNMVNVRR.D | 0.6097 |
| IPI00335256 | K.NVIFSPLSISTALAFLSLGAHN#TTLTEILK.G | 1 |
| IPI00335256 | R.TLN#QSSDELQLSMGNAMFVK.E | 1 |
| IPI00335256 | K.FN#LTETSEAEIHQSFQHLLR.T | 1 |
| IPI00335256 | K.YTGN#ASALFILPDQDK.M | 1 |
| IPI00335256 | S.PLDEEN#LTQENQDR.G | 1 |
| IPI00335256 | K.LINDYVKN#GTR.G | 0.9998 |
| IPI00335356 | K.STGKPTLYN#VSLVMSDTAGTC.Y | 0.5542 |
| IPI00335426 | M.NTGMNAGM*NPGMLAAGNGQGIM*PNQVM*N#GSIGAGR.G | 0.799 |
| IPI00335543 · | K.KENVAADIPITETEAYQLLKKATLQDNTN#QTEN.R | 0.9668 |
| IPI00335587 | K.SSLVN#ESETNQN#SSSDSEAERR.P | 0.7004 |
| IPI00335823 | K.IDRLDGTPQEPLCGFSKQMVEIVHKHN#ISLAVLM*S.L | 0.9407 |
| IPI00335859 | K.GKN#LSLSLDALFM*GK.S | 0.9266 |
| IPI00335933 | M.EN#YSSLVSLETHTGEK.L | 0.816 |
| IPI00336019 | K.ANQENQALSKKLN#DTHNELNDIKQK.V | 0.5412 |
| IPI00336075 | K.SSKDGNSVM*SPLFISTFTL.N#ISHTASEGATGENLAK.V | 0.8814 |
| IPI00336156 | R.NDMTYNYANRQSTGSAPQGPAYHGVN#RTDEVLHTDQR.A | 0.557 |
| IPI00337426 | K.N#ISNPPDMSGWNPFGEDN#FSK.L | 0.8699 |
| IPI00337454 | K.KKKALSSM*GAN#YSSYLA.K | -0.5174 · |
| IPI00337558 | K.GELN#TSIFSSR.P | 1 |
| IPI00337558 | K.LLHALGGDDFLGM*LN#R.T | 0.9978 |
| IPI00337662 | R.LAENSGN#ASTER.N | 0.5455 |
| IPI00337691 | M.KFRGNGALSN#ISDLPFLAENSAFPK.M | 0.6824 |
| IPI00337766 | K.RADKYWEYTFKVN#WSDLSVTTVTK.T | 0.7534 |
| IPI00339361 | R.GHKISDYFEYQGGN#GSSPVR.G | 0.6822 |
| IPI00339366 | K.VGMHCSGPLGGLLQLAAEVN#VTSR.V | 0.8747 |
| IPI00339381 | K.KEYNVNDDSMKLGGN#NTSEK.A | 0.8946 |
| IPI00339381 | K.AGGVGLN#LSAASRVFLM*DPAWNPAAEDQ.C | 0.6808 |
| IPI00373782 | R.YDFDLFAN#ESVPDHVGYAK.V | 0.9424 |
| IPI00373787 | R.DDFRQN#PSDVM*VAVGEPAVM*TCQPPR.G | 0.5036 |
| IPI00373797 | R.KKKIN#GSSPDTATSGGYHSPGDSAT.G | 0.9247 |
| IPI00373835 | R.YSWQCVN#QSVLCGPSGN#HTDIETK.Q | 0.6195 |
| IPI00373875 | M.TIWILKVMN#FTIDGMGNLRITEK.G | 0.6142 |
| IPI00373895 | K.EWFNTDSM*TLN#NTAY.L | 0.6588 |
| IPI00373923 | M.FPVLFPEN#PSSLTM*DSIHIPACLNLEFLNEV.- | 0.5603 |
| IPI00373928 | L.AIAN#SSQANDCLDSFASPN#K.T | 0.8228 |
| IPI00373928 | T.LVM*AAFQGPGEDTIGGSIFVN#VTM*FSSGGEMVQAETSGVK.I | 0.9188 |
| IPI00373943 | K.NM*AQFTN#QTPGPMILCSTGCGFYG.N | 0.915 |
| IPI00373947 | R.CCYSLGN#GSSGFRFLKYGGCGFPSLSYGSR.F | 0.5023 |
| IPI00373966 | K.NGQDHLN#ISSMTAAQFGTYT.C | 0.7317 |
| IPI00374007 | K.TTFFDTN#STDIALK.V | 0.9575 |
| IPI00374029 | R.SPSPSKN#DSFFTPDSNHNSLSQSTTGHLSLPQK.Q | 0.6176 |
| IPI00374053 | K.ALTN#GSFSPSGNN#GSVNWR.T | 0.8303 |

| IPI00374046 | K.GLRGPGSIGSEPDFWN#GSGSSRVK.G | 0.588 |
|---|---|---|
| IPI00374078 | K.LEEYKEAFAVALKAN#NSM*SK.K | 0 646 |
| IPI00374080 | K.HTGPGILSMANAGPNTN#G.S | 0.9915 |
| IPI00374113 | K.HTGPGILSMANAGPNMN#GSQFFICTAK.T | 0.9857 |
| IPI00374128 | K.RSHN#ASILDMGE.E | 0.565 |
| IPI00374136 | L.QLAN#HSGYIK.V | 0.5262 |
| IPI00374154 | R.N#YSSIHSQSRST.S | 0.5242 |
| IPI00374218 | K.ADETVTEMN#FSNEYN#KSELMLQENQMIADGK.E | 0.9694 |
| IPI00374218 | R.RGSEVISN#TTEDTQLTSETQSLTGNK.K | 0.8453 |
| IPI00374218 | R.EAYSPLELLDN#LSGADVR.Q | 0.7892 |
| IPI00374218 | K.ITKN#FSEVGFPDILK.A | 0.5008 |
| IPI00374219 | K.QIKN#SSLLSFDNEDENE.- | 0.6397 |
| IPI00374227 | R.FARHPFYGSAGVNSGVM*LMN#LTR.I | 0.5068 |
| IPI00374341 | M.STISCHQDVILSMSFNTN#GSLLATTCKDR.K | 0.8314 |
| IPI00374355 | K.SPPRKIN#SSPNVN#TTASGVEDLNIIQVTIPDDDNERL.S | 0 7092 |
| IPI00374359 | L.FKN#ATFTFTWAFQR.T | 0.6616 |
| IPI00374378 | K.NSLSGIAMNVPASRGSNLN#SSGAN#RTSLSGGTGSGTQGATK.P | 0.6461 |
| IPI00374389 | K.SSSSLGN#ATSDEDPNTNIM*NINENK.N | 0 6864 |
| IPI00374435 | R.QHNVIN#LSSLDAM*M*.D | 0.6392 |
| IPI00374461 | M.GYLCTHQLLFLQLN#QSSFNSK.N | 0.8437 |
| IPI00374532 | K.FSPSDTDEN#ATNTQSIT.- | 0.9426 |
| IPI00374572 | T.TAQLSWRPGPDN#HSPITMYVIQAR.T | 0.5576 |
| IPI00374646 | R.SRDFSAAPQN#TTQNFLVNGRIR.K | 0.896 |
| IPI00374681 | K.N#MSVTFALDEPMKEGECSR.R | 0 7049 |
| IPI00374711 | R.NSRMN#FTYQIADCNR.D | 0.7444 |
| IPI00374729 | R.MDVSSN#SSPSCQASPSQEDVSADMQERR.G | 0 6969 |
| IPI00374741 | K.N#LSEM*QDLEEIRKITGVCPQFNVQF.D | 0.9168 |
| IPI00374749 | K.VDM*HDDSLN#TTANLIWNK.L | 0.6647 |
| IPI00374755 | R.VRRASCEPAN#GSGR.S | 0.9067 |
| IPI00374756 | K.ILHN#VSEDPSFVISQHIR.K | 0 6567 |
| IPI00374770 | K.DYN#ASASTISPPSSM*EEDK.F | 0.6448 |
| IPI00374793 | R.FLAGN#TSSPPLSPGR.P | 0 6648 |
| IPI00374836 | K.VN#PTLVIQPTN#LSARLETDVECLK.L | 0.7367 |
| IPI00374844 | M.TN#ASNSQQSISMQQFSQTSN#PSAHFHK.C | 0 9382 |
| IPI00374967 | K.AIESGTEWN#LSLLK.L | 0 6066 |
| IPI00374984 | V.FDASAPAHCGVRVGLSAQPCPN#KSSK.A | 0.6035 |
| IPI00375011 | K.LKM*LTN#PSTANSNLLLHQ.S | 0 6447 |
| IPI00375121 | K.AAIQGNGDVGAAAATAHNGFSCSN#CSMLSER.K | 0 7765 |
| IPI00375139 | R.VEGLFLTLSGSN#LTVK.V | 0 5812 |
| IPI00375143 | R.FAVQLYNTNQN#TTF.K | 0 9519 |
| IPI00375144 | N.IAPN#ISRAEHSLCK.R | 0.8502 |
| IPI00375152 | K.ISNN#ITLR.E | 0.6838 |
| IPI00375174 | K.KSNQLFN#ITTIVGTR.S | 0 6108 |
| IPI00375179 | R.RTGILSNCN#HTYCLKCIRK.W | 0.8100 |
| IPI00375210 | V.YIVQSGCGEIN#DSLMEILIMINACK.I | 0.6179 |
| IPI00375216 | K.VARLI.QN#GSPMGARGRPNGAVAK.A | 0 8406 |
| IPI00375220 | R.M*ENANLPTKQEPSWIN#QSLQGIK.E | 0.5284 |
| IPI00375253 | K.N#VITSPSFSAGDAPPVLFSSDFRLS | 0 676 |
| IPI00375266 | K.TGHRMERPDN#CSEEM*YR.L | 0.5967 |
| IPI00375294 | R.NRKQGVLAVIDAYN#TSNKET.K | 0 9321 |
| IPI00375294 | R.TIN#ASNLYVGGHPEGEGTSLLTNIRR.S | 0 7631 |

97

| | | |
|---|---|---|
| IPI00375294 | R.QTN#ESLLILRAIPEGIRDK.G | 0.5806 |
| IPI00375442 | K.VDSEENTLNSQTN#ATSGMNPDGVLSK.M | 0.8037 |
| IPI00375455 | R.GCNVN#STSSAGNTALHVAVMRNR.F | 0.9969 |
| IPI00375473 | K.ECCNAFN#QSSALTNHK.R | 0.7395 |
| IPI00375498 | K.FEEN#TSNSQWHVSLSVSFK.K | 0.546 |
| IPI00375506 | R.GLN#VTLSSTGR.N | 1 |
| IPI00375506 | R.FSDGLESN#SSTQFEVK.K | 1 |
| IPI00375506 | K.N#TTCQDLQIEVTVK.G | 0.9998 |
| IPI00375506 | K.N#LTVSVHVSPVFGLCLAGGGGLAQQVLVPAGSAR.P | 0.8835 |
| IPI00375507 | K.LPCSENPRDTEDVPWITLN#SSIQK.V | 0.9122 |
| IPI00375559 | R.NEMLEIQVFN#YSKVFSNK.L | 0.9579 |
| IPI00375628 | R.SSDMDQQEDMISGVENSN#VSENDIPFNVQYPGQTSK.T | 0.7843 |
| IPI00375662 | D.EPTVVPTTSARMESQATSASIN#NSN#PST.S | 0.6812 |
| IPI00375674 | K.LQLWTN#GSVAYSVAR.E | 0.9176 |
| IPI00375747 | Q.NSELQAKTN#ETEK.A | 0.5652 |
| IPI00375757 | R.DGKN#ATTDALTSVLTK.I | 0.8482 |
| IPI00375757 | K.DEHAQSNEIVVN#DSGSDNVK.K | 0.6865 |
| IPI00375772 | K.HTGPGILSMANAGPITN#SSQFFICTAK.T | 0.7151 |
| IPI00375814 | A.GQVLENLPPIGVFWDIEN#CSVPSGR.S | 0.7383 |
| IPI00375823 | V.IHN#ASIMNAEAAGGYR.Y | 0.8084 |
| IPI00375835 | M.ILLN#NSQKLLVLYKPLAWSIPESLK.V | 0.88 |
| IPI00375881 | K.VPPTVCPFHSLNN#VTKAGEGSWLESK.R | 0.8137 |
| IPI00375881 | K.ASGQVIDEIAGN#FSR.A | 0.7828 |
| IPI00375936 | K.GLVEGVYCN#LTEAFEIPACK.Y | 0.9419 |
| IPI00375947 | K.QM*ESSEGSSN#TTEATSGSGVR.G | 0.7925 |
| IPI00375951 | R.STN#HSTQSALN#QSLHTVGAQPITAHSR.R | 0.7436 |
| IPI00375986 | R.RRN#ITVGLAVFATGR.F | 0.8536 |
| IPI00376019 | R.VGFCSCQVSLMLQN#SSAR.A | 0.9243 |
| IPI00376094 | K.IN#SSSVCVSSISENDNGISFTCRLGR.D | 0.579 |
| IPI00376147 | R.GEN#VSTTEVEGVLSRLLGQTDVAVYGVAVPGK.L | 0.9584 |
| IPI00376190 | K.KVIFSEEITN#LSQMTLNVQGPSCILK.K | 0.575 |
| IPI00376192 | K.IN#TTISTTSKGTLLPNSIM*TSTLKDQGGISR.T | 0.6101 |
| IPI00376199 | P.PTLVPLMN#GSATPLPTALGLGGR.A | 0.6961 |
| IPI00376202 | R.FFVELVGHYSLN#M*TVT.E | 0.6697 |
| IPI00376235 | M.N#ATNHAILQSLVHLM*KPNAVPKACCAPTK.L | 0.5799 |
| IPI00376252 | R.FIIFQGPCGRMLPEPLAGHEN#ETVS.- | 0.755 |
| IPI00376258 | R.LYM*LSFLPFLVLLVLIRNLRILTIFSM*LAN#ISML.V | 0.7017 |
| IPI00376259 | R.RRRPGIIGSLTN#ISR.H | 0.9843 |
| IPI00376288 | G.VN#ATATADR.L | 0.8447 |
| IPI00376298 | R.FTASTNM*N#ASSSR.S | 0.9793 |
| IPI00376301 | R.SISWTM*GMEGLLQN#STNFV.L | 0.7409 |
| IPI00376327 | R.LKYQAQN#ITSGDTFTILPAACCIM*K.S | 0.5565 |
| IPI00376383 | K.LDKTEKERELN#LSYNK.I | 0.6894 |
| IPI00376436 | K.NEN#ETILNPEEVALLEEYIPTR.H | 0.7622 |
| IPI00376539 | K.YTGSGILSMANAVLNTN#GSIFFICTAK.T | 0.5376 |
| IPI00376550 | R.RPAAVGAGLQNMGNTCYVN#ASLQC.L | 0.6503 |
| IPI00376566 | R.QVYN#ATIALHAPVG.H | 0.5385 |
| IPI00376572 | K.N#PTDACLDAVM*NEAPGPIN#FTMLLAM*IGKK.I | 0.6754 |
| IPI00376639 | P.FRMVN#CTHLDEIFASNTFAPILTKGLLAK.K | 0.6338 |
| IPI00376647 | -.M*VN#HTM*FFDVAVDSIPLDIIVSFELFAEK.F | 0.5373 |
| IPI00376672 | K.AREDIFMETLNNIM*EYYN#DSNGQ.- | 0.8235 |

98

| | | |
|---|---|---|
| IPI00376675 | - M*DSSCHN#ATTKM*LATAPARGNM*MSTSK.P | 0 8437 |
| IPI00376706 | P.EVLSPLTLVN#TSGEAQGTVDR V | 0.9205 |
| IPI00376711 | R RPAAVGAGLQNMGNTCYEN#ASLQCL T | 0 7832 |
| IPI00376742 | K.AQLIGPLVFGGMN#LTRDELGWK L | 0 6307 |
| IPI00376747 | K.HMGSASEDSMGPPRVGRVLPTTN#GTFPVCIWR.G | 0.6576 |
| IPI00376770 | K.KNTFN#FTLISWHSGLK D | 0 5759 |
| IPI00376784 | R.IAIHALATNM*GAGTEGAN#ASYILIR D | 1 |
| IPI00376784 | R YGEEYGN#LTRPDITFTYFQPK.P | 1 |
| IPI00376817 | K.FLVHDINELEVLMMCN#KSYCAKIAHN#VSSK N | 0 8307 |
| IPI00376829 | R.TEGNIFDSLIGGN#ASAEGPEGK.G | 0.831 |
| IPI00376832 | V LEM*EEAGSIFACNM*EGRSN#SSGEVK.Y | 0.6631 |
| IPI00376877 | R.WN#TTYRR Y | 0 8726 |
| IPI00376890 | M RDSEATGSASSAQDSTSEN#SSSVGGR.C | 0.7392 |
| IPI00376964 | R.VAVVQHAPSESVDN#ASM*PPVK.V | 0.9999 |
| IPI00377042 | R KQPMTLTVTSFN#ASTGRVN#ATLSNSNMELLLSGVY K | 0 7414 |
| IPI00377042 | R.CEALCGGN#ITAM*N#GTIY.S | 0.5896 |
| IPI00377076 | K.FVSDATDYAAGFN#LTYK.A | 0.956 |
| IPI00377111 | P.TFSLPLQLPPPVN#TSK.L | 0.7058 |
| IPI00377116 | K.AFRN#HSFLLIHQ.R | 0.8205 |
| IPI00377188 | K.GEERSSCISGNN#FSWSLQWNGK E | 0.6599 |
| IPI00377202 | M DSSLVSQQPPDNQEKERLN#TSIPQKR K | 0.9893 |
| IPI00382394 | R.RTPEEAAAGEVN#LS S | 0.7237 |
| IPI00382397 | R.SPCSIN#ASISSITSYTCF.- | 0.6137 |
| IPI00382411 | L.FNSNNFDLGCKQN#GTK.L | 0 6054 |
| IPI00382432 | K.LNNPKDFQELNKQTKKN#M*TIDGK.E | 0.991 |
| IPI00382485 | M.LN#LSSYPIWVSLFRGSPR.R | 0.7819 |
| IPI00382512 | M PQPGCNLLN#GTQEIGPVGMTENCNRK.D | 0.6219 |
| IPI00382532 | R.SRHEN#TSQVPLQESRTRKR.R | 0.7304 |
| IPI00382556 | R.KN#YTSTELTVEPEEPSDSSGIN#LSGFGR.N | 0.5475 |
| IPI00382595 | C.TADNSYGPVQSM*VLN#VTVR.E | 0 5487 |
| IPI00382618 | Q.PANSNN#GTSTATSTNNNAK R | 0.5111 |
| IPI00382623 | S.AYDSNDPDVESN#SSSGISSPSR.Q | 0 8752 |
| IPI00382628 | I.FPLLYVELN#DSAK.Q | 0.6415 |
| IPI00382629 | K.YKDQWGQQGLYHCPN#FSDVMGN#K.I | 0.657 |
| IPI00382631 | K.VYEN#TTLGFIVEVEGLPVPG.V | 0 5381 |
| IPI00382631 | K.EKIPSPETLQPDTHN#ISK.S | 0 8112 |
| IPI00382631 | K.CVAEN#NSGAVLSVSDLTVEPVTYR.E | 0.6931 |
| IPI00382681 | K.YCGLGLQIN#HSIFSKG.N | 0.6898 |
| IPI00382705 | I QGLPGSSGN#M*T N | 1 |
| IPI00382717 | R.LAFAIMFN#SSEQSQK.G | 0.5353 |
| IPI00382750 | K.YEFCPFHN#VIQHEQTFR.W | 1 |
| IPI00382792 | K.VSLGAVYFFMN#GTYGLATWYGTSLILNGEPGYTIGTVLAVKR K | 0.5824 |
| IPI00382818 | K.HVEVN#GSKTAGAN#ITIDK.E | 0 7617 |
| IPI00382824 | F QLN#FQTLAEM*KAQEVAELKRK K | 0.6018 |
| IPI00382843 | P MDFYSNQNNFVHDCVN#ITIK Q | 0 5824 |
| IPI00382870 | R TFTN#VSDEVSSEFGPETGYSLRR H | 9 8717 |
| IPI00382926 | S.AAQNPM*M*TN#ASATQATLTAQR F | 0 7783 |
| IPI00382937 | R IYTSGSTNYN#PSLK S | 0 9376 |
| IPI00382937 | R.GLTFQQN#ASSMCGPDQDTAIR V | 0.9865 |
| IPI00382990 | K.NLNLELNPN#QSVK V | 0 7498 |
| IPI00382995 | M.IPCVLQYLN#FSTHGVGVGAGAYILAR Y | 0 7145 |

| | | |
|---|---|---|
| IPI00383015 | T.TTATFTTN#TTTTITSGFTVNQNQLLSR.G | 0.5639 |
| IPI00383032 | K.GDVSLTIFN#VTLADSGIYCCR.I | 0.8603 |
| IPI00383040 | E.GEKDPWGVSMM*N#TSFAGGQIHQDL.- | 0.6457 |
| IPI00383078 | R.SAPVPVTTQNPPGAPPN#VSGRR.H | 0.9704 |
| IPI00383119 | M.IFEN#PSLCASNSEPLK.L | 0.5794 |
| IPI00383151 | F.TVFTN#NT.H | 0.7675 |
| IPI00383197 | R.SSN#LTTHKIIHTGEK.P | 0.7828 |
| IPI00383221 | R.LGPVSTAN#MSR.P | 0.7114 |
| IPI00383222 | K.QN#TTLGLSER.P | 0.99 |
| IPI00383233 | R.YDLTGWLHRAKPN#LSALDAPQVLHQSK.R | 0.9657 |
| IPI00383317 | K.SPLFMGKVVN#PTQK.- | 1 |
| IPI00383323 | R.NGYGFNRN#DTKEDVFVHQTAIK.K | 0.6899 |
| IPI00383351 | -.M*SRINKYLFSNSDSN#FSHFSVSN#V.S | 0.7104 |
| IPI00383371 | E.ILGFNSKGEVHGIN#GTQWGQTLR.M | 0.9636 |
| IPI00383425 | K.VFISN#STN#SSNPCERR.S | 0.8844 |
| IPI00383454 | R.SN#LTSFGADQVM*DLGR.D | 0.6593 |
| IPI00383455 | R.ETALCSLN#SSHGIVAFPSRSR.S | 0.8133 |
| IPI00383469 | R.FDGILADAGQTVEN#MSWMLVCDR.P | 0.7854 |
| IPI00383482 | R.EN#M*SDGDTSATESGDEVP.V | 0.7247 |
| IPI00383482 | K.EMN#KSDLNTNNLLFKPPVESHIQKNK.K | 0.8527 |
| IPI00383505 | R.LLSNPLLQTYLPN#STK.K | 0.5013 |
| IPI00383541 | R.YSGVN#MTGF.R | 0.5346 |
| IPI00383543 | R.KN#STFPQVQM*.R | 0.7637 |
| IPI00383580 | M.SSDN#DSYHSDEFLTNSK.S | 0.5329 |
| IPI00383585 | S.PKVTKQNSLNDEGCQSDLEDN#VSQGSSDALLLR.G | 0.5234 |
| IPI00383614 | K.LN#NTMNACAAIAALERVK.I | 0.5876 |
| IPI00383750 | R.QDN#GTLLSLEDLNGGILVDVNTAEHST.V | 0.7695 |
| IPI00383786 | K.ESLIM*NHVLN#TSQLASSYQYKKK.Y | 0.7004 |
| IPI00383786 | R.VKRNQEN#FSSVLYK.E | 0.8509 |
| IPI00383786 | K.GRGLNAM*AN#ETPDFMR.A | 0.5193 |
| IPI00383794 | V.GKM*QSTQTTN#TSN#STN#K.S | 0.5906 |
| IPI00383828 | R.ELGGAIDFGAAYVLFQASSHIGN#STQATVR.D | 0.5605 |
| IPI00383888 | M.TATIHFSVDLN#ASRSLSQVAM*DLHEAVSM*KLHRVR.E | 0.5144 |
| IPI00383931 | Q.TVQRSM*AN#LSVLFGQVVR.G | 0.9317 |
| IPI00383973 | R.VLYM*FNQMPLN#LTNAVATAR.Q | 0.507 |
| IPI00383973 | M.IIN#CSLIASALTISNIQAGSTGNWGCHVQTK.R | 0.595 |
| IPI00384004 | K.AQAEAN#ATAISNLLPFM*EYEVHTQLMNK.L | 0.5412 |
| IPI00384051 | -.M*PSSTLPGWPGSSGGPVSRPSSLESSRN#TSSN#SSPLNLK.G | 0.982 |
| IPI00384063 | R.YLFPEVDMTSTN#FTGL.S | 0.9761 |
| IPI00384138 | K.TLTFLAVMLIVLN#STGPAHQPGR.G | 0.7809 |
| IPI00384159 | M.KPVN#QTAASNKGLR.G | 0.7011 |
| IPI00384159 | R.QLEDPN#GSFSNAEMSELSVAQKPFK.L | 0.7079 |
| IPI00384793 | K.DIKKKNTNLQPN#WQTLPVEQDTSN#VTEM*K.V | 0.5399 |
| IPI00384202 | K.N#SSNWKLFK.E | 0.8565 |
| IPI00384210 | K.KEDYN#ETAPMLEQV.- | 0.5565 |
| IPI00384238 | R.N#SSLLEPQKSGNN#ET.T | 0.9755 |
| IPI00384277 | K.LIAFLSFKISN#LTIVQAEIK.D | 0.9181 |
| IPI00384277 | K.TWSNRTEKQDILN#NSLTTLSQDTK.V | 0.6615 |
| IPI00384280 | K.M*SN#ITFLNFDPPIEEFHQYYQIHVTTLVK.G | 0.9985 |
| IPI00384367 | K.LGVSSVPSCYLIYPN#GSHGLINVVKPLR.A | 0.82 |
| IPI00384413 | R.AVQPHNGCFN#WTSR.A | 0.947 |

| | | |
|---|---|---|
| IPI00384416 | K.KINLNHLLN#FTFFPR.G | 0 6975 |
| IPI00384441 | R.GKMADSSGRGAGKPATGPTN#SSSAK.K | 0 5636 |
| IPI00384450 | V.TAGILEM*RNALGN#QSTPAPPTGEVADTPLEPGK.V | 0.6518 |
| IPI00384456 | K.GTQTYSVLEGDPSEN#YSKYLLSLK.E | 0.5345 |
| IPI00384470 | R.N#FSSVAASSGN#TTL.N | 0.5266 |
| IPI00384496 | G.CGGGGSYSASSSSAALPVN#KSK.M | 0.8121 |
| IPI00384508 | K.MAAAN#LTFSQEVVWQR.G | 0.838 |
| IPI00384532 | V.HEEFNPNQAN#GSYASR.R | 0.5909 |
| IPI00384543 | K.NTNQGFSSAN#VSEEEER.K | 0.5445 |
| IPI00384556 | R.ELSEMRAPPAATN#SSK.K | 0.5522 |
| IPI00384651 | R.LYQQFNEN#NSIGETQARKLSK.L | 0.69 |
| IPI00384717 | K.LLQAENCDIFQN#LSAKQR.L | 0 6254 |
| IPI00384772 | R.N#LTGVM*NVAR.P | 0.6516 |
| IPI00384785 | M.FKM*AKN#WSAAGNAFCQAAKLHM*QLQSK.H | 0.8306 |
| IPI00384840 | L.SMLN#GTKVLS.D | 0.7724 |
| IPI00384877 | K.HKGLIEIPSLSEENEIN#DTEVN#VSK.K | 0.5927 |
| IPI00384969 | K.SSISNNYLN#LTFPRKR.T | 0.7225 |
| IPI00384984 | K.TDNSLLDQALQN#DTVFLNM*R.G | 1 |
| IPI00384984 | K.TVVTYHIPQN#SSLENVDSR.Y | 1 |
| IPI00385095 | R.FEKTNEM*LLNFNN#LSSARLQQMSER.F | 0.6078 |
| IPI00385247 | K.N#NTIAPKK.A | 0.5642 |
| IPI00385263 | R.DSFKVYCN#FTAGGSTCVFPDK.K | 0.7212 |
| IPI00385264 | K.THTN#ISESHPN#ATFSAVGEASICEDDWDSGER.F | 1 |
| IPI00385264 | K.STGKPTLYN#VSLVMSDTAGTCY.- | 1 |
| IPI00385264 | R.GLTFQQN#ASSMCGPDQDTAIR.V | 0 9865 |
| IPI00385267 | R.GGN#NSFTHEALTLK.Y | 0.6823 |
| IPI00385280 | M.PSN#SSASISKLR.E | 0.6522 |
| IPI00385287 | R.N#LSSLQPPPPGFK.R | 0.9481 |
| IPI00385291 | K.QEM*GGIVTEPIRDYN#SSR.E | 0.6259 |
| IPI00385317 | K.GSTPRNDPSVSVDYN#TTEPAVR.W | 0.8716 |
| IPI00385321 | R.ISLSDMPRSPMSTN#SSVHTGSDVEQDAEK.K | 0.8106 |
| IPI00385334 | Q.SLEDILHQVEN#K.T | 0.9617 |
| IPI00385341 | R.MLRFIQEVN#TTTR.S | 0.5871 |
| IPI00385343 | R.EHEKLLMEVCRN#CS.V | 0.7115 |
| IPI00385358 | R.GNYGGGGNYNDFGN#YSGQQQSNYG.- | 0.8778 |
| IPI00385362 | R.VFFAGFSN#ATVDNSILLR.L | 0.5241 |
| IPI00385404 | K.KAVSPLLLTTTN#SSLGLSMGNYMGLINRLAQKKR.L | 0.9145 |
| IPI00385446 | K.TFAN#SSYLAQHR.I | 0 5827 |
| IPI00385511 | L.KGGNN#DSWMNPLAKQFSNMGLLSQTEDN#PSSK.M | 0.61 |
| IPI00385511 | R.KGALETDNSN#SSAQVSTVGQTSR.E | 0.9344 |
| IPI00385511 | K.M*WKNHISSRN#TTPLPRPPPGLTN.P | 0 7333 |
| IPI00385539 | R.NLN#HTKQRLI EVANYVDQVVNSAGDAHG.- | 0.6411 |
| IPI00385684 | R.GFRHGDIIAIN#RTENWIGSQYKK.V | 0 8302 |
| IPI00385729 | K.N#DSDLFGLGLEEAGPK.E | 0 7373 |
| IPI00385743 | S.LRENSISPEGAQAIAHALCAN#STLK.N | 0.879 |
| IPI00385756 | P.NHRPTGRGNN#ISSIH.- | 0 8353 |
| IPI00385894 | K.NYNTN#ITTETSK.I | 0.761 |
| IPI00385962 | R.DIEIFYN#TSIEEM*PPM*IL.- | 0 8287 |
| IPI00385973 | L.ITEGN#GTESLNSVHSMK.FGELEK.E | 0 7951 |
| IPI00385980 | K.KNKN#SSKPQKNN#GSTWANVPLPPPP.V | 0 8485 |
| IPI00385980 | K.YKIWCLGN#ETR.F | 0 6971 |

| | | |
|---|---|---|
| IPI00385980 | K.TVRTTEEAPSAPPQSVTVLTVGSYN#STS I | 0.561 |
| IPI00386028 | R.KLGYSSLILDSTGSTLFAN#CTDDNIYMFN#MT G | 0.6334 |
| IPI00386099 | K.VNAPILTN#TTLNVIR L | 0.5699 |
| IPI00386134 | Y.CGTWN#NSLSGWVFGGGTK.L | 0.7368 |
| IPI00386139 | M.VYN#CTTGSTNPFHWGEVGMILPVFLNVR.I | 0.5525 |
| IPI00386145 | R.GAAAAPGN#WSSRQRPAHPR.T | 0.7285 |
| IPI00386225 | R.KALPMEFEAYIN#ASGEHGIVVFSLGSM*VS.E | 0.8915 |
| IPI00386236 | K.N#NSDISSTR.G | I |
| IPI00386236 | R.GLTFQQN#ASSMCVPDQDTAIR.V | I |
| IPI00386236 | K.THTN#ISESHPN#ATFSAVGEASICEDDWNSGER.F | I |
| IPI00386257 | R.IYPGPTRLAN#STIKDESPPR.Y | 0.5319 |
| IPI00386279 | F.NQIM*HAFSVAPFDQN#LSIDGK.I | 0.6244 |
| IPI00386327 | K.GETWATPN#CSEATCEGNNVISLS.P | 0.7424 |
| IPI00386389 | -.MRQNNN#ITEFVLLGFSQDLDVQK.A | 0.5718 |
| IPI00386421 | G.NIIN#M*SSVASSVKGGSVSFRGLR C | 0.7712 |
| IPI00386442 | K.ADILLDCLLDEDPEN#QTLRKDYEK.T | 0.9765 |
| IPI00386532 | K.GPIGPGEPLELLCN#VSGALPPAGR.H | 0.7379 |
| IPI00386553 | M.WSHM*QPHLFHN#QSVLAEQMALNKK.F | 0.784 |
| IPI00386566 | K.EN#QSIRAFNSEHK.I | 0.6513 |
| IPI00386567 | R.FWN#GTYHCIFR.Y | 0.6039 |
| IPI00386567 | M.KVMCDNNPVSLNCCSQGNVN#WSK.V | 0.9231 |
| IPI00386567 | K.VLQQQWTN#QSSQLLHSVER F | 0.8635 |
| IPI00386731 | K.NLPFLEHLELIGSN#FSSAMPR N | 0.8123 |
| IPI00386732 | M SHFPDRGSEN#GTPMDVKAGVR.V | 0.7764 |
| IPI00386764 | R.TAADN#FSTQYVLDGSGHILSQKPSHLGQGKK.V | 0.8748 |
| IPI00386928 | K.WQSAIQDFRSN#ATALCHIR.N | 0.9172 |
| IPI00386953 | R.RAFM*LEPEGMSPM*EPAGVSPMPGTQN#DTGRT E | 0.9674 |
| IPI00387050 | R.RARHDSPDPSPPRRPQHN#SSGDCQK A | 0.6349 |
| IPI00394642 | T.VTPVSPSFAHNPKRHNSASVEN#VSLRK.S | 0.9196 |
| IPI00394646 | R.RRKN#M*SEFLGEASIPGQEPP.T | 0.6674 |
| IPI00394652 | M EITWTPMN#ATSAFGPNLR.Y | 0.8107 |
| IPI00394718 | K.RQPATLTVDWFN#ATSSKVN#ATFSEASPVELK L | 0.8689 |
| IPI00394738 | K.SN#ISPNFNFM*GQLLDFER.T | 0.8923 |
| IPI00394801 | M.ENPQEPDAPIVTFFPLIN#DTTR.K | 0.8947 |
| IPI00394816 | K.N#LSGPDDLLIDK.N | 0.9721 |
| IPI00394823 | K PLGPLQTLM*EN#LSSNR.F | 0.5646 |
| IPI00394824 | K CQAHSQN#VTFVLRK V | 0.6463 |
| IPI00394845 | K N#LSINNDLNLR.Y | 0.8772 |
| IPI00394866 | R.SLCCGDISQSAVLFLCQGTLAMLDWQN#GSM*GR S | 0.8122 |
| IPI00395010 | M LQDDN#TSAGLHFMASVK K | 0.8773 |
| IPI00395323 | K.ANEQVVQSLN#QTYK.M | 0.9987 |
| IPI00395400 | N PDASYNLGVLHLDGIPPGVPGRN#QTLAGFIFHK A | 0.5633 |
| IPI00395458 | R LPASLAEYTVFQLRQN#ATYSVCVM*PLGPGR V | 0.9999 |
| IPI00395488 | R.IAQLRPEDLAGLAALQELDVSN#LSLQALPGDLSGLFPR.L | 1 |
| IPI00395488 | R.LHEITN#ETFR G | 0.9599 |
| IPI00395511 | K AVEVATVVIQPTVLRAAVPKN#VSVAEGK.E | 0.5478 |
| IPI00395595 | P.SRPSNSN#ISKGESRPK.W | 0.7249 |
| IPI00395632 | R VN#KSVHEWAGLGGGGGGGATYVFK M | 0.8947 |
| IPI00395659 | K.NKIARLGN#GSQDLNHGVDNENGGR R | 0.9099 |
| IPI00395659 | K IARLGN#GSQDLNHGVDNENGGRRGPN#R T | 0.5352 |
| IPI00395737 | A DKASDTSSETVFGKRGHVLGN#GSQVTQAANSGCSK.A | 0.8702 |

| IPI00396050 | R.RAEMSQTN#FTPDTLAQNEGK.A | 0.9957 |
| IPI00396080 | K.GRTFN#LTAGNDDSIVMK.A | 0.8603 |
| IPI00396096 | L.N#RSDSDSSTLAK.K | 0.6886 |
| IPI00396103 | M.LN#GTTLEAAMLFHGISGGHIQGIMEEMER.R | 0.5147 |
| IPI00396166 | R.EN#FTQTLPK.M | 0.6497 |
| IPI00396200 | R.VAGAPAPWAAAHGGAM*MDVN#SSGR.P | 0.9741 |
| IPI00396341 | K.HYQTVFLM*RSN#STLNKHNENYKQK.K | 0.8381 |
| IPI00396433 | P.LLPKSSTIEEEEN#M*SGHK.C | 0.6574 |
| IPI00396464 | Q.N#STQDSGPQESEGSAGNSLTVAK.D | 0.6878 |
| IPI00396485 | K.GDIGIVPLGLVETAILKPSMWSTFAPVN#TTT.E | 0.9221 |
| IPI00396500 | K.GQSVSSPPNDCN#ISPAR.V | 0.7878 |

## Table 8. N-linked glycosylation sites identified from human serum/plasma which do not contain the consensus.

N-X-T/S glycosylation motif.

| Protein IP # (Version 2.21) | Peptide Sequences | Peptide Probability |
|---|---|---|
| IPI00004574 | K.SGTASVVCLLN*N*FYPR.E | 0.9956 |
| IPI00004617 | V.SVLTVLHQN*WLDGKEYK.C | 0.9847 |
| IPI00006143 | K.N#GREVN#GCSGVN#R.Y | 0.9503 |
| IPI00009464 | K.FDPSTKIYEISN#R.W | 0.9255 |
| IPI00010740 | R.DM#RM#GGGGAM#N*M#GDPYGSGGQK.F | 0.9572 |
| IPI00017648 | R.LLPPN*TVNLPSKVRAFTFPSEVPSK.A | 0.827 |
| IPI00018311 | R.RVTVN*TAYGSN*GVSVLR.I | 0.9984 |
| IPI00019571 | R.N*AN*FKFTDHLK.Y | 0.9542 |
| IPI00019571 | K.SPVGVQPILN*EHTFCAGM#SK.Y | 0.9972 |
| IPI00019571 | K.LRTEGDGVYTLN*NEKQWINK.A | 1 |
| IPI00019943 | K.DLLRN*CCNTENPPGCYR.Y | 1 |
| IPI00021841 | R.LAARLEALKEN*GGAR.L | 0.9896 |
| IPI00021841 | K.LREQLGPVTQEFWDN#LEKETEGLR.Q | 0.9969 |
| IPI00021841 | K.LLDN*WDSVTSTFSK.L | 0.9913 |
| IPI00022229 | R.EYSGTIASEAN*TYLNSK.S | 1 |
| IPI00022229 | Q.FN#N#NEYSQDLDAYNTKDKIGVELTGR.T | 0.9947 |
| IPI00022229 | K.SNTVASLHTEKNTLELSN#GVIVK.I | 0.9107 |
| IPI00022391 | R.AYSLFSYN*TQGRDNELLVYK.E | 0.9993 |

| | | |
|---|---|---|
| IPI00022394 | K.TNQVN*SGGVLLR.L | 0.9935 |
| R.AIEDYIN*EFSVR.K | | 0.991 |
| IPI00022395 | K.TSNFN*AAISLK.F | 0.9665 |
| IPI00022417 | K.LQELHLSSN#GLESLSPEFLRPVPQLR.V | 0.9997 |
| IPI00022432 | R.YTIAALLSPYSYSTTAVVTN*PKE.- | 1 |
| IPI00022432 | R.GSPAIN*VAVHVFR.K | 0.9987 |
| IPI00022463 | R.SM#GGKEDLIWELLN*QAQEHFGKDK.S | 0.9891 |
| IPI00022463 | R.SAGWN#IPIGLLYCDLPEPR.K | 0.9865 |
| IPI00022463 | R.N*TYEKYLGEEYVK.A | 1 |
| IPI00022463 | R.LKCDEWSVN#SVGK.I | 0.9999 |
| IPI00022463 | R.KPVEEYAN#CHLAR.A | 0.9997 |
| IPI00022463 | K.SASDLTWDN*LKGK.K | 0.9901 |
| IPI00022463 | K.LCM*GSGLNLCEPNN#KEGYYGYTGAFR.C | 0.978 |
| IPI00022463 | K.LCM*GSGLN#LCEPNNKEGYYGYTGAFR.C | 0.9989 |
| IPI00022463 | K.IN#HCRFDEFFSEGCAPGSK.K | 0.9473 |
| IPI00022463 | K.IM*N#GEADAMSLDGGFVYIAGK.C | 0.994 |
| IPI00022463 | K.GDVAFVKHQTVPQN#TGGK.N | 0.9999 |
| IPI00022488 | R.YYCFQGN*QFLR.F | 0.9957 |
| IPI00022488 | R.WKN*FPSPVDAAFR.Q | 0.9983 |
| IPI00022488 | L.PPTSAHGN#VAEGFTKPDPDVTER.C | 0.9996 |
| IPI00022488 | K.SLGPN#SCSAN#GPGLYLIHGPNLYCYSDVEK.L | 0.9868 |
| IPI00025426 | R.SSGSLLNN*AIK.G | 0.9617 |
| IPI00025426 | R.N*QGN*TWLTAFVLK.T | 0.9985 |
| IPI00025426 | K.ATVLN*YLPK.C | 0.917 |
| IPI00027482 | K.M#N*TVIAALSR.D | 0.9953 |
| IPI00032179 | R.FATTFYQHLADSKNDNDN*IFLSPLSISTAFAM#TK.L | 0.9994 |
| IPI00032179 | R.EVPLN*TIIFMGR.V | 0.9764 |
| IPI00032179 | Q.PLDFKEN#AEQSR.A | 0.9972 |
| IPI00032220 | R.AAM#VGM#LAN*FLGFR.I | 0.9991 |
| IPI00032220 | A.SDLDKVEGLTFQQNSLN*WM#KK.L | 0.9653 |
| IPI00032256 | R.TEVSSN*HVLIYLDK.V | 1 |
| IPI00032256 | R.SLFTDLEAEN*DVLHCVAFAVPK.S | 0.9792 |
| IPI00032256 | R.SASN*M#AIVDVK.M | 0.9209 |
| IPI00032256 | R.HNVYIN#GITYTPVSSTNEKDM*YSFLEDM*GLK.A | 0.9991 |
| IPI00032256 | K.SSSN#EEVM*FLTVQVKGPTQEFK.K | 0.9994 |
| IPI00032256 | K.SKAIGYLN*TGYQR.Q | 0.9968 |
| IPI00032256 | K.QQN#AQGGFSSTQDTVVALHALSK.Y | 0.9999 |
| IPI00032256 | K.N#EDSLVFVQTDK.S | 0.9997 |
| IPI00032256 | K.GVPIPN*KVIFIR.G | 0.9169 |
| IPI00032256 | K.FSGQLN*SHGCFYQQVK.T | 0.9998 |
| IPI00032256 | K.FRVVSMDEN*FHPLNLLIPLVYIQDPK.G | 0.9951 |

| | | |
|---|---|---|
| IPI00032256 | K.EQAPHCICAN#GR.Q | 0.9933 |
| IPI00032256 | K.ALLAYAFALAGN*QDK.R | 1 |
| IPI00032256 | K.AIGYLN*TGYQR.Q | 0.9984 |
| IPI00032328 | R.IASFSQN#CDIYPGKDFVQPPTK.I | 1 |
| IPI00032328 | R.DIPTN*SPELEETLTHTITK.L | 0.9999 |
| IPI00152059 | R.VEN*SYGQERRCHLM.- | 0.9348 |
| IPI00164623 | R.TVM#VNIEN*PEGIPVK.Q | 0.9888 |
| IPI00164623 | R.TM*QALPYSTVGN#SNNYLHLSVLR.T | 1 |
| IPI00164623 | R.TM#QALPYSTVGN*SNN*YLHLSVLR.T | 1 |
| IPI00164623 | R.AVLYNYRQN*QELK.V | 0.9544 |
| IPI00164623 | K.VHQYFN*VELIQPGAVK.V | 0.9942 |
| IPI00164623 | K.AGDFLEAN*YM#NLQR.S | 0.9398 |
| IPI00167498 | V.QRLAHGLHKVN*TLALK.Y | 0.9251 |
| IPI00175649 | M.KLM*IVGN#TGSGKTTLLQQLM*KTK.K | 0.8308 |
| IPI00216315 | K.IPVVPHN#ECSEVM*SNM*VSENM*LCAGILGDR.Q | 0.944 |
| IPI00216722 | H.LETPDFQLFKN#GVAQEPVHLDSPAIK.H | 0.987 |
| IPI00216773 | K.VFDEFKPLVEEPQNLIKQN#CELFEQLGEYK.F | 1 |
| IPI00216773 | K.VFDEFKPLVEEPQN*LIK.Q | 0.9949 |
| IPI00216773 | K.LVN*EVTEFAK.T | 0.996 |
| IPI00216773 | K.KVPQVSTPTLVEVSRN*LGK.V | 0.9626 |
| IPI00216773 | K.FQN*ALLVR.Y | 0.9105 |
| IPI00218017 | R.YN*RQIGEFIVTR.A | 0.8035 |
| IPI00218199 | K.DKQIITFFSPLTILVGPN#GAGK.T | 0.9318 |
| IPI00218732 | K.GIFTGSEDLEILPN#GLAFISSGLKYPGIK.S | 0.9999 |
| IPI00220120 | K.NTLYLQMN*SLR.A | 0.9772 |
| IPI00220591 | E.RAN*SHIFLYGDLR.S | 0.9594 |
| IPI00250430 | M.VTFSN#TLPRAN#TPSVEDPVR.R | 0.8977 |
| IPI00292530 | R.VQSWKGSLVQASEAN*LQAAQDFVR.G | 0.9955 |
| IPI00292530 | R.GRFPLYNLGFGHNVDFN#FLEVMSM*ENNGR.A | 1 |
| IPI00292530 | K.GSLVQASEAN*LQAAQDFVR.G | 1 |
| IPI00294193 | R.AISGGSIQIEN#GYFVIYFAPEGLTTM*PK.N | 0.9971 |
| IPI00294193 | A.FKN*GIDIYSLTVDSR.V | 0.9145 |
| IPI00296608 | R.YSAWAESVTN*LPQVIK.Q | 0.9949 |
| IPI00298828 | R.VCPFAGILEN*GAVR.Y | 0.9929 |
| IPI00298828 | K.CPFPSRPDN#GFVN#YPAKPTLYYK.D | 0.9996 |
| IPI00299475 | K.SSTLKPTIEALPN#VLPLNFDVN#K.Q | 0.9435 |
| IPI00305457 | R.TLNQPDSQLQLTTGN*GLFLSEGLK.L | 1 |
| IPI00305457 | N.KITPN*LAEFAFSLYR.Q | 0.9594 |
| IPI00305457 | N.ATAIFFLPDEGKLQHLEN#ELTHDIITK.F | 0.9995 |
| IPI00305457 | L.PDEGKLQHLEN#ELTHDIITK.F | 0.9999 |
| IPI00305457 | K.TDTSHHDQDHPTFN*KITPNLAEFAFSLYR.Q | 1 |

| IPI00305457 | K.QIN*DYVEKGTQGK.I | 0.9336 |
| IPI00305457 | K.LQHLEN*ELTHDIITK.F | 0.9989 |
| IPI00305457 | K.ITPN*LAEFAFSLYR.Q | 0.9708 |
| IPI00305457 | K.ELDRDTVFALVN#YIFFK.G | 0.998 |
| IPI00305457 | I.FFLPDEGKLQHLEN*ELTHDIITK.F | 0.9924 |
| IPI00305457 | A.TAIFFLPDEGKLQHLEN#ELTHDIITK.F | 0.9957 |
| IPI00305461 | R.KLWAYLTIN*QLLAER.S | 1 |
| IPI00328609 | R.GFQHLLHTLN*LPGHGLETR.V | 0.9993 |
| IPI00328609 | K.IAPAN*ADFAFR.F | 0.9553 |
| IPI00332161 | V.VSVLTVLHQDWLN*GK.E | 1 |
| IPI00332161 | K.N*QVSLTCLVK.G | 0.9708 |
| IPI00332161 | K.GFYPSDIAVEWESN*GQPEN*NYK.T | 1 |
| IPI00332161 | K.FNWYVDGVEVHN*AK.T | 0.9507 |
| IPI00373776 | K.NSLYLQMN*SLR.A | 0.9973 |
| IPI00375506 | R.GLESQTKLVN#GQSHISLSK.A | 0.9999 |
| IPI00375506 | K.AEFQDALEKLN#M*GITDLQGLR.L | 0.9973 |
| IPI00382950 | R.FFESFGDLSTPDAVM#GN*PK.V | 0.995 |
| IPI00383035 | R.MKGLIDEVN*QDFTNR.I | 0.9957 |
| IPI00383317 | V.FSN#GADLSGVTEEAPLKLSK.A | 0.9915 |
| IPI00383317 | K.VFSN*GADLSGVTEEAPLKLSK.A | 1 |
| IPI00383317 | K.SVLGQLGITKVFSN*GADLSGVTEEAPLKLSK.A | 0.9986 |
| IPI00383317 | K.FNKPFVFLM#IEQN*TK.S | 0.9901 |
| IPI00384391 | R.DNSKN*SLYLQM#NSLR.A | 1 |
| IPI00385298 | K.HFLMEN*INNEN*KGSIN*LKRKHI.T | 0.9997 |
| IPI00385332 | K.DSLYLQMN*SLR.V | 0.9943 |

## Example 6

### Identification of early disease biomarkers using tissue specimens and body fluids

This example describes the identification of disease biomarkers from prostate cancer tissues.

Proteins expressed on the cell surface or secreted-from cells in disease tissues are likely to leak to body fluids at an early stage of disease development and can be detected in body fluids as diagnostic markers. This is demonstrated by several tumor biomarkers currently in clinical use (see Table 9). In general, identification of cell surface proteins or secreted proteins of cells is difficult due to contamination with extremely complex mixtures of intracellular proteins of high abundance.

Table 9. Known tumor markers.

| Current tumor markers | | | |
| --- | --- | --- | --- |
| Tumor markers | Protein Name/Function | Cancer Site | Glycosylation |
| CEA | Carcinoembryonic antigen | Colon, Lung | glycoprotein |
| AFP | A-Fetoprotein | Liver, germ ceil cancer of ovanes or testis | glycoprotein |
| PAP | Prostatic acid phosphatase | Prostate, myeloma, lung cancer; osteogenic sarcoma | glycoprotein |
| HCG | Human chorionic gonadotropin | Gestational trophoblastic tumor | glycoprotein |
| PSA | Prostate specific antigen | Prostate | glycoprotein |

(continued)

**Current tumor markers**

| Tumor markers | Protein Name/Function | Cancer Site | Glycosylation |
|---|---|---|---|
| CA125 | Ovarian cancer marker CA125 | Ovarian | glycoprotein |

Several approaches have been used in an attempt to enrich for cell surface proteins or secreted proteins from cultured cells. These methods includes (I) differential centrifugation (Han et al., Nat. Biotechnol. 19:946-951 (2001)), (2) chemical labeling of cell surface proteins to introduce tags attached to cell surface proteins before lysing cells, and (3) extraction of secreted proteins in cell culture medium secreted from cells (Martin et al., Cancer Res. 64:347-355 (2004)). However, none of these methods can be applied to tissue specimens, which contain potential biomarkers for human diseases.

Most proteins expressed on the cell surface and/or secreted by cells are glycosylated (Durand and Seta, Clin. Chem. 46:795-805 (2000)). Glycoproteins from disease specimens were isolated using a glycopeptide capture method (see Examples 1-5)(Zhang et al., Nat. Biotechnol. 21:660-666 (2003)) Tissue specimens can be obtained from fresh tissues, in which case tissues are minced and digested with collagenase in serum free medium. Both single cells from tissue specimens and cell free supernatants are collected and subjected to glycopeptide capture to enrich for cell surface proteins from cells and/or secreted proteins in the extracellular matrix that arc released to the supernatant after collagenase digestion. Tissue specimens can be obtained from frozen sections, in which case tissues are ground with a blender or tissues homogenizer, and the proteins in the microsomal fraction and supernatant are collected. The glycopeptide capture method is then used to isolate membrane proteins from microsomal fractions and/or secreted proteins from the supernatant.

Using the glycopeptide capture method, isolated formerly glycosylated peptides were identified and quantified by tandem mass spectrometry. The expression of these proteins in body fluids was further determined by antibody based assays or stable isotope labeled synthetic peptides originally identified from tissues. The identification of proteins from disease tissues and detection of these proteins in body fluids can be used to determine specific protein changes related to certain disease states or cancer for diagnostic biomarkers or immunotherapy targets.

Proteins in body fluids have been used to discover biomarkers related to disease states for years, and the advancement of proteomic technologies provides the opportunity to identify additional disease markers and/or potential therapeutic targets. Despite the efforts to identify biomarkers in body fluids, most protein markers identified in body fluids using proteomic approaches are abundant proteins and not specific to a particular disease (Diamandis, Mol. Cell. Proteomics 3:367-378 (2004)). This is due to the peculiar protein content of most body fluids, which are highly complex but contain a few abundant proteins representing most of the protein content. In addition, the protein content of body fluids varies over time in an individual due to different physiological and pathological influences. The protein content of body fluids also varies among individuals in a population. Due to these factors, identifying biomarkers in body fluids for specific disease is extremely challenging.

Using the glycopeptide capture method, secreted proteins and cell surface proteins were identified from tissues. It was determined whether the glycoproteins were present in body fluids using targeted proteomic approaches, including antibody based method and synthetic heavy-isotope labeled peptides (Pan et al., Mol. Cell. Proteomics 4:182-190 (2005)). Since cell surface proteins and secreted proteins are highly glycosylated and likely to leak to body fluids in an early stage of disease development, cell surface proteins or secreted proteins can be identified as potential targets for candidate biomarkers in body fluids. A more sensitive and targeted approach can further be used to determine their diagnostic value in body fluids.

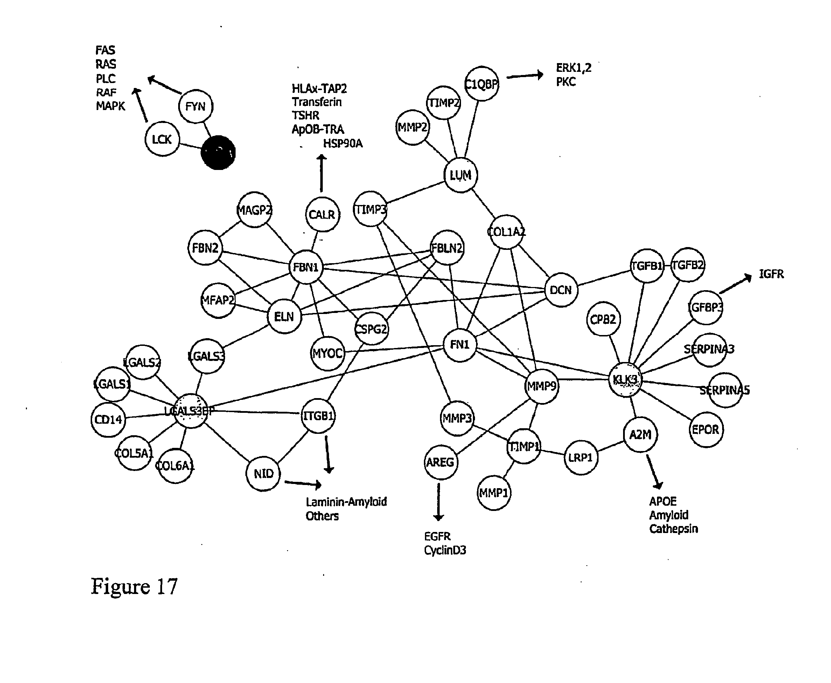

Glycoproteins have been isolated from cell free supernatant of prostate cancer tissues. Over 100 proteins have been identified and quantified (Table 10). Most proteins identified are proteins located in the extracellular environment in spite of the high contamination of intracellular proteins in cell free supernatant during tissue sample preparation. It was found that 61% of the identified proteins were secreted proteins, 18% of the proteins were extracellular matrix proteins, 10% of the proteins were transmembrane proteins, and only 11% of the proteins were from intracellular proteins. Protein network analysis showed that 5 out of 6 protein changes were located in one network (Figure 17). Heavy isotope labeled peptides were synthesized and mixed with N-linked glycopeptides isolated from serum (see Figure 4). The relative abundances of these peptides were quantified among individuals (see Figure 16). The protein TIMPl has also been found to be decreased in prostate cancer patients relative to normal patients (Liu et al., J. Urol. 173:73-78 (2005).

Table 10. Identification of proteins and relative abundance changes in cancer tissues compared to patient matched normal tissues.

| Protein IPI # (Version 2.28) | Peptide Sequences | Ratio (Cancer/Normal) |
|---|---|---|
| IPI00004573 | R.LSLLEEPGN*GTFTVILNQLTSR D | |
| IPI00004617 | R.EQQFNSTFR V | |
| IPI00004618 | R EEQFN*STYR V | |
| IPI00004641 | R.LAGKPTIIVN*VSVV.M | |
| IPI00004641 | V QGFFPQEPLSVTWSESGQN*VTAR N | |
| IPI00005794 | K.IVVYNQPYIN*YSR.T | |
| IPI00005794 | R.GKIVVYNQPYINYSR.T | |
| IPI00006154 | R.LQNNENN*ISCVER.G | |
| IPI00006662 | R ADGTVNQIEGEATPVN*LTEPAKLEVK.F | |
| IPI00007244 | R.SCPACPGSN*ITIR.N | |
| IPI00007778 | A.NAPYN*QTLTGYNDYIK M | |
| IPI00009030 | R.VQPFN*VTQGK.Y | |
| IPI00009802 | R.TLYRFEN*QTGFPPPDSR F | |
| IPI00010858 | R.NKSVILLGR.H | down |
| IPI00010949 | R.ALAYGEKN*LTFEGPLPEKIELLAHK G | |
| IPI00011229 | K.YYKGSLSYLN*VTR.K | |
| IPI00011302 | K IAVNCSSDFDACLITK.A | |
| IPI00011302 | S LQCYNCPNPTADCK.T | |
| IPI00012503 | R.NLEKN*STKQEII AALEK G | |
| IPI00012837 | K YSVANDTGFVDIPKQEK A | |
| IPI00013179 | K.SVVAPATDGGLN*LTSTFLR K | |
| IPI00013179 | K.SVVAPATDGGLN*LTSTFLRK N | |
| IPI00013446 | K.AQVSNEDCI QVEN*CTQLGEQCWTAR I | |
| IPI00013449 | K ALKQYN*STGDYR.S | |
| IPI00013698 | K ILAPAYFILGGN*QSGEGCVITR D | |
| IPI00015976 | T AASEFTLFN*ASQR I | |
| IPI00015028 | R SFMFVN*WTHAPGNVEK.Y | |

| | | |
|---|---|---|
| IPI00015102 | K.IIISPEEN*VTLTCTAENQLER.T | |
| IPI00017601 | K.EHEGAIYPDNTTDFQR.A | |
| IPI00017601 | K.ELHIILQEQNVSNAFLDK.G | |
| IPI00019571 | K.VVLHPNYSQVDIGLIK.L | |
| IPI00019591 | R.SPYYN*VSDEISFHCYDGYTLR.G | |
| IPI00020986 | R.NNQIDHIDEKAFENVTD.L | down |
| IPI00021891 | K.VDKDLQSLEDILHQVEN*KTSEVK.Q | |
| IPI00022255 | R.VNLTTN*TLAVTQTLPNAAYNNR.F | |
| IPI00022418 | K.LDAPTNLQFVN*ETDSTVLVR.W | |
| IPI00022429 | R.QDQCIYNTTYLNVQR.E | |
| IPI00022431 | K.AALAAFNAQNN*GSNFQLEEISR.A | |
| IPI00022431 | K.VCQDCPLLAPLNDTR.V | |
| IPI00022463 | R.QQQHLFGSNVTDCSGNFCLFR.S | |
| IPI00022488 | R.SWPAVGNCSSALR.W | |
| IPI00022488 | K.ALPQPQNVTSLLGCTH.- | |
| IPI00022792 | R.VDLEDFENNTAYAK.Y | |
| IPI00022892 | R.LDCRHEN*TSSSPIQYEFSLTR.E | up |
| IPI00023673 | R.ALGFENATQALGR.A | down |
| IPI00023673 | R.DAGVVCTN*ETR.S | |
| IPI00023673 | K.GLNLTEDTYKPR.I | |
| IPI00023673 | R.TVIRPFYLTN*SSGVD.- | |
| IPI00023673 | R.YKGLN*LTEDTYKPR.I | |
| IPI00024284 | R.SLTQGSLIVGDLAPVN*GTSQGK.F | |
| IPI00024284 | R.IQGEEIVFHDLN*LTAHGISHCPTCR.D | |
| IPI00024284 | R.NLHQSN*TSRAELLVTEAPSKP.I | |
| IPI00024284 | R.VAQQDSGQYICN*ATSPAGHAEAT.I | |
| IPI00027482 | R.AQLLQGLGINLTER.S | |
| IPI00027827 | R.AKLDAFFALEGFPTEPN*SSSR.A | |
| IPI00027851 | K.SAEGTFFIN*KTEIEDFPRFPHR.G | |
| IPI00028908 | R.IHQN*ITYQVCR.H | |
| IPI00029739 | K.IPCSQPPQIEHGTINSSR.S | |
| IPI00031008 | R.CIN*GTCYCEEGFTGEDCGKPTCPHACHTQGR.C | |
| IPI00032256 | K.SLGNVNFTVSAEALESQELCGTEVPSVPEHGRK.D | |
| IPI00032292 | R.SHN*RSEEFLIAGK.L | down |
| IPI00032292 | R.AKFVGTPEVNQTTLYQR.Y | |
| IPI00032292 | K.FVGTPEVNQTTLYQR.Y | |
| IPI00032292 | K.FVGTPEVN*QTTLYQRYEIK.M | |
| IPI00032328 | K.LNAENNATFYFK.I | |
| IPI00043715 | K.KLRLPDTGLYNMTDSG.T | |
| IPI00098026 | R.LIINQLLPN*VTTVER.N | |
| IPI00163563 | K.VISLLPKENKTR.Q | |
| IPI00164623 | K.TVLTPATNHM#GN*VTTTIPANR.E | |
| IPI00166729 | R.FGCEIENN*R.S | |
| IPI00166729 | K.DIVEYYN*DSN*GSHVLQGR.F | |
| IPI00166729 | R.FGCEIENNRSSGAFW.K.Y | |
| IPI00168520 | V.AN*FSQIETLTSVFQK.K | |
| IPI00168728 | R.EEQFN*STFR.V | |
| IPI00169285 | K.SDI.NPAN*GSYFFKAI.R.Q | |
| IPI00171411 | R.LQQDVLGFQKN*QTNLFR.K | |
| IPI00178617 | R.KFDVNQLQNTTIKR.I | |
| IPI00178926 | R.HVPLNNREN*ISDPISPLR.I | |

| | |
|---|---|
| IPI00215998 | R.QQMENYPKNNHTASILDR.M |
| IPI00215998 | K.NRVPDSCCIN*VTVGCGINFNEK.A |
| IPI00217503 | R.TATESFPHPGFN*NSLPNKDHR.N |
| IPI00221224 | K.AEFNITLIHPK.D |
| IPI00221224 | K.GPSTPLPEDPNWN*VTEFHTTPK.M |
| IPI00221224 | K.KLNYTLSQGHR.V |
| IPI00221224 | V.LLNLNVTGYYR.V |
| IPI00221224 | R.N*ATLVNEADKLR.A |
| IPI00221224 | V.TLALNNTLFLIEER.Q |
| IPI00247063 | R.SCIN*ESAIDSR.G |
| IPI00289489 | R.AQQLLAN*STALEEAMLQEQQR.L |
| IPI00289489 | R.KQELSRDN*ATLQATLHAAR.D |
| IPI00289489 | G.LAN*ASAPSGEQLLR.T |
| IPI00289489 | R.LHRLNASIADLQSQLR.S |
| IPI00289489 | K.RLNTTGVSAGCTADLLVGR.A |
| IPI00289983 | R.KFLN*ESYK.H |
| IPI00289983 | K.FLNESYKHEQVYIR.S |
| IPI00289983 | R.KFLNESYKHEQ.V |
| IPI00289983 | R.KFLNESYKHEQVYIR.S |
| IPI00291262 | R.LANLTQGEDQYYLR.V |
| IPI00291866 | K.VGQLQLSHN*LSLVILVPQNLK.H |
| IPI00292069 | K.GSQWSDIEEFCN*R.S |
| IPI00292732 | R.LYLDHN*NLTR.M |
| IPI00293088 | R.GVFITNETGQPLIGK.V |
| IPI00293088 | K.VTVLGVATAPQQVLSN*GVPVSN*FTYSPDTK.A |
| IPI00296141 | R.ALAGLVYN*ASGSEHCYDIYR.L |
| IPI00296141 | R.FGN*KTFPQR.F |
| IPI00296170 | K.NLFLNHSEN*ATAK.D |
| IPI00296170 | K.MVSHHN*LTTGATLINEQWLLTTAK.N |
| IPI00296922 | R.CAPNFWN*LTSGHGCQPCACHPSR.A |
| IPI00298281 | R.TLAGENQTAFEIEELNR.K |
| IPI00298281 | R.EGFVGNRCDQCEENYFYNR.S |
| IPI00298281 | R.IASAVQKNATSTKAEAER.F |
| IPI00298281 | R.KIPAINQTITEANEK.T |
| IPI00298281 | K.LLNN*LTSIK.I |
| IPI00298281 | K.QVLSYGQNLSFSFR.V |
| IPI00298281 | K.TANDTSTEAYNLLR.T |
| IPI00298828 | R.VYKPSAGNNSLYR.D |
| IPI00298828 | K.LGN*WSAM#PSCK.A |
| IPI00298860 | R.TAGWNIPMGLLFN*QTGSCK.F |
| IPI00298860 | K.FGRN*GSDCPDKFCLFQSETK.N |
| IPI00298971 | R.PQPPAEEELCSGKPFDAFTDLKN*GSLEAER.G |
| IPI00299547 | K.SYN*VFSVFR.K |
| IPI00301579 | K.GQSYSVNVTFTSNIQSK.S |
| IPI00305964 | K.AFN*STLPTM#AQMEK.A |
| IPI00305457 | K.YLGNATAIFFLPDEGK.L |
| IPI00328113 | R.VIPVNVTDYCQLVR.Y |
| IPI00328113 | R.CDSGEALDSLERN*CTDIDECR.I |
| IPI00328113 | K.AWGTPCEM#CPAVNTSFYK.I |
| IPI00328113 | R.NYYADN*QTCDGEI LFN*MIKK.M |
| IPI00328488 | R.RPYVSYVNN*SIAR.N |

down

down

110

IPI00329482 · I.SAQYAN*FTGCISNAYFTR.V
IPI00329482 R.DAVRN*LTEVVPQLLDQLR.T
IPI00329573 R.NLQVYNATSNSLTVK.W
IPI00329573 P.LTDQGTTLYLN*VTDLK.T
IPI00332161 R.EEQYNSTYR.V
IPI00332161 K.TKPREEQYNSTYR.V
IPI00333982 R.EEQYN*STFR.V
IPI00333982 K.TKPREEQYNSTFR.V
IPI00335256 K.FN*LTETSFAEIHQSFQHLLR.T
IPI00374091 R.LHNKLLPNVTTVER.N
IPI00375506 R.GLNVTLSSTGR.N
IPI00375506 R.FSDGLESNSSTQFEVK.K
IPI00375947 S.EGSSNTTEATSGSGVR.G
IPI00382512 K.M#SIQGCVAQPSSFLLNHTR.Q
IPI00383517 R.AVCGGVLVHPQWVLTAAHCIRN*K.S
IPI00383981 R.FVN*VTVTPEDQCRPNNVCTGVLTR.R
IPI00385514 H.VSNVTVN*YN*VTVERM#NR.M
IPI00386236 K.YKNNSDISSTR.G
IPI00386236 R.GLTFQQN*ASSM#CVPDQDTAIR.V

## Example 7

### Identification of Proteotypic N-linked Glycopeptides for Serum Protein Analysis

Theoretically, one unique peptide per protein that can be observed by mass spectrometry is sufficient for unambiguously identifying and quantifying a parent protein. If such proteotypic peptides can be isolated and selectively analyzed by mass spectrometry, the complexity of proteome profiling could be reduced by one or two orders of magnitude. This increases the sensitivity, throughput and reproducibility of scoring phase studies. Protein glycosylation, especially N-linked glycosylation, is very common post-translational modification for proteins expressed on extracellular surfaces, in cell secretions, and for proteins contained in body fluids. These are the most easily accessible human proteins for diagnostic purposes, and the literature confirms that glycoproteins constitute a large number of clinical biomarkers Table 11). A method was developed to isolate N-linked glycopeptides (Zhang et al., Nat. Biotechnol. 21:660-6G6 (2003)). By selectively isolating a subset of N-linked glycopeptides, the procedure achieves a significant reduction in analyte complexity and increases the sensitivity for serum proteomic analysis at two levels: first, a reduction of the total number of peptides due to the fact that every serum protein on average only contains a few N-linked glycosylation sites, and second, a reduction of pattern complexity by removing the oligosaccharides that contribute significantly to the peptide pattern heterogeneity. It is therefore believed that the quantitative analysis of these formerly N-linked glycopeptides has potential use for detecting new diagnostic markers (Zhang et al Nat. Biotechnol. 21:660-666 (2003); Zhang et al., Mol. Cell. Proteomics 4:144-155 (2005).

Table 11. Most known clinical tumor markers are glycoproteins

| Tumor markers | Protein Name/Function | Cancer Site | Glycosylation |
|---|---|---|---|
| CEA | Carcinoembryonic antigen | Colon, Lung | glycoprotein |
| AFP | A-Fetoprotein | Liver, germ cell cancer of ovaries or testis | glycoprotein |
| NSE | Neuron specific enolase | Neuroblastoma, small cell lung cancer | unknown |
| PAP | Prostatic acid phosphatase | Prostate, myeloma, lung cancer, osteogenic sarcoma | glycoprotein |
| HCG | Human chorionic gonadotropin | Gestational trophoblastic tumor | glycoprotein |
| PSA | Prostate specific antigen | Prostate | glycoprotein |
| CA125 | Ovarian cancer marker CA125 | Ovarian | glycoprotein |

For chromatography procedures, HPLC-grade reagents were purchased from Fisher Scientific (Pittsburgh, USA). PNGase F was purchased from New England Biolabs (Beverly, MA) and hydrazide resin from Bio-Rad (Hercules, CA). All other chemicals and the human serum used in this study were purchased from Sigma (St. Louis, USA).

Purification and Fractionation of Formerly N-linked Glycosylaled Peptides from Serum by SCX. 0.75 ml serum was used to isolate N-linked glycopeptides (Zhang et at Nat. Biotechnol. 21:660-666 (2003)), which were fractionated by strong cation-exchange chromatography into 43 fractions as described previously (Han et al., Nat. Biotechnol. 19: 946-951 (2001)).

Analysis of Peptides by Mass Spectrometry. Fractionated peptides were analyzed using a LCQ ion trap mass spectrometer. (Finnigan; San Jose, CA) or ESI-qTOF mass spectrometer (Waters. Milford, MA) as described previously (Pieper et al., Proteomics 3:422-432 (2003)). Using SEQUEST, acquired MS/MS spectra were compaired against the International Protein-Index (IPI) human protein database (version 2.28). For MS/MS spectra acquired by the ESI-qTOF mass spectrometer, the mass window of each peptide was given a tolerance of 0.4 Da between the measured monoisotopic mass and calculated monoisotopic mass; the b and y ion series of database peptides were included in the SEQUEST analysis. For MS/MS spectra acquired via the Finnigan mass spectrometer, the mass window for each peptide was given a tolerance of 3 Da between the measured average mass and calculated average mass; the b and y ion series were included in the SEQUEST analysis: The database sequence tool was set to the following modifications: carboxymethylated cysteines, oxidized methionines, and an enzyme-catalyzed conversion of Asn to Asp at the site of carbohydrate attachment. No other constraints were included in the SEQUEST searches. Search results were further analyzed with a suite of software tools that included INTERACT (Han et al., Nat. Biotechnol. 19:946-951 (2001)) and PeptideProphet (Keller et at., Anal. Chem. 74:5383-5392 (2002)). All MS/MS spectra were manually checked to verify the validity of the database search results.

Amino acid preference around the glycosylation sites. Position-independent amino acid abundance ratios were first calculate for each protein corresponding to one or more peptides from the set of all identified N-linked glycopeptides using the sequence information contained in the human International Protein Index version 2.28. This yielded a mean abundance and variance for each amino acid in the set of all identified proteins. The relative abundance of each amino acid was then calculated for all positions plus or minus twenty residues from the asparagine in the NX(T/S) motif using the set of identified N-linked glycopeptides, where the asparagine was taken to be at position zero. A "probability" score describing the bias for each amino acid at each position was generated by calculating the deviation of the observed abundance for that amino acid at that position from its position-independent abundance, then-dividing by the standard-devialion-in the position-independent abundances for that amino acid in all identified proteins.

Subcellular Localization of Identified Proteins. Signal peptides were predicted using signal P 2.0 (19). Transmembrane (TM) regions were predicted using TMHMM(version 2.0) (Krogh et al., J. Mol. Biol. 305:567-580 (2001)). The TMHMM program predicts protein topology and the number of TM helices. Information from signalP and TMHMM were combined to separate proteins into the categories: 1) membrane bound, 2) souluable, 3) secreted and 4) membrane proteins anchored by an uncleaved signal peptide also predicted to be a trans membrane helix. All protein sequences were taken from IPI version 2.28.

Identification of Serum Peptides Using SPEG and Tandem Mass

Spectrometry. To assess the potential of the proposed glycopeptide capture method for serum protein profiling, four 0.75 ml of serum was processed using SPEG as described previously (Zhang et al., Nat. Biotechnol. 21:660-666 (2003)). Formerly N-linked glycosylated peptides were fractionated by two dimensional chromatography using cation exchange fractionation and reverse phase liquid chromatography (RP-LC). Peptide mixtures were sequentially analyzed by electrospray ionization tandem mass spectrometry (ESI-MS/MS) (Han et al., Nat. Biotechnol..19:946-951 (2001)). The resulting collision induced dissociation (CID) spectra were used to perform searches within the human International Protein Index sequence database (IPI version 2.28 with 41100 entries); database search results were statistically analyzed using PeptideProphet (Keller et al., Anal. Chem. 74:5383-5392 (2002)).

Most cancer-specific serum biomarkers consist of low-abundance serum proteins. According to these results; a significant number of proteins identified in the present study belong to low-abundance serum protein groups such as growth factors, cell surface receptors, and channel or transporter proteins. Several previously identified serum markers were also identified, such as the MIAC-2 binding protein and the ovarian cancer-related tumor marker CA125. The MAC-2 binding protein belongs to a family of beta-gahictoside-binding proteins (also known as *galectins)* that are thought to modulate call-cell and cell-matrix interactions. MAC-2 binding protein is present in normal serum in the μg/ml range and elevated levels of MAC-2 binding protein (≥11 μg/ml) have been found in the sera of cancer patients (Bresalier et al., Gastroenterology 127, 741-748 (2004): Marchetti et al., Cancer Res. 62:2535-2539 (2002)). An increase in serum CA 125 is considered an accurate and reliable measure of responses to treatment and relapses in ovarian cancer patients (Guppy et al., Oncologist 7:437-443 (2002)).

Identification of Nonredundant N- linked Glycopeptides as Proteotypic Peptides. N-linked glycosylation sites in peptide sequences are generally fall into the N-X-T/S sequon (Bause, Biochem. J. 209:331-336 1983)). From the set of all peptides identified above, a list of non-redundant-*N*-linked- glycopeptides for each sequon was generated as follows. First, the identified sequences were filtered for the presence of the N-X-T/S sequon to remove peptides not containing the sequon. Non-sequon-containing peptides can come from two sources. The first is peptides from non-specific.isolation of *N*-linked glycopeptides (selectivity of the method) and the second is peptides that are incorrectly identified by SEQEST search (false positive identifications). In the present analysis, the false positive error rate was estimated by the PeptideProphet statistical model (Zhang et al., Mol. Cell. Proteomics 4:144-155 (2005)). Second, a minimum probability score of 0.5 was used to filter out low probability sequon-containing peptides. And finally, redundant peptides with overlapping sequences containing the same sequons were resolved in favor of those sequences which contained the greater number oftryptic ends. Using the two-dimensional peptide separation protocol for analyzing formerly *N*-linked glycopeptides, we identified 3244 nonredundant N-linked glycosylation sites were identified, representing 2585 unique proteins with a PeptideProphet score at least 0.5 (Table 7). 2106 peptides are unique to a single database entry, and thus selected as experimentally identified proteotypic peptides, representing a total of 1671 proteins.

This indicates that the combination of solid-phase *N*-linked glycopeptide capture and tandem mass spectrometry is able to identify a large number of peptides with consensus *N*-linked glycosylation sites from scrum with high confidence. These peptides can now be used as standard peptides to identify and quantify the same peptides from samples isolated by glycopeptide capture method with different biological or physiological relevance (Pan et al., Mol. Cell. Proteomics 4: 182-190 (2005)).

Determination of amino acid preference around N-linked glycosylation sequon using the experimentally identified N-linked glycopeptides. White each protein containing multiple *N-linked* glycosylation sequons can generate multiple possible tryptic peptides not all potential *N*-linked tryptic glycopeptide were identified by the large scale mass spectrometry analysis (Table 7). The reasons for.this are that not all NXT/S sequons are occupied (Petrescu et al., Glycobiology 14: 103-114 (2004)) and that only a portion of protein's possible peptides exhibit particular physico-chemical properties such that they are consistently observed by a mass spectrometer (Mallick, P. et al., In Preparation (2005)). Determining the amino acid specificity around *N*-linked glycopeptides detected by a mass spectrometer offers the possibility of developing a refined *N*-linked- glycosylation motif to predict occupation of the glycosylation sequon. This refined motif can then be used to scan protein databases to computationally predict proteotypical glycopeptides for the subsequent use in the scoring phase analysis of serum proteins. In general, a proteotypic *N*-linked glycopeptide is determined by a short linear sequence motif that occurs around the *N*-linked glycosylation and trypsin cleavage sites. The large number of *N*-linked glycopeptide sequences identified here allows statistical characterization of the preference for each amino acid at each position around the NXT/S motif. Specifically, the relative occurrence of each amino acid at each position around identified *N*-linked glycosylation sites, from -20 to +20 (where position 0 is taken to be the asparagine that oligosaccharide is formerly attached to), has been calculated. This region was chosen to include residues immediately around the glycosylation site that may interact with the translocon complex where glycosylation occurs (glycosylation occurring approximately 30 residues from the ribosome (Varki, Essentials of Glycobiology, Cold Spring Harbor Laboratory Press, Cold Spring Harbor, NY, (1999).). The occurrence of finding each amino acid at each sequence position ($F_{Pos}$) was compared with the average occurrence ($F_{ave}$) of each amino acid at any position in the set of identified proteins. The probabilities (P) of each amino acid occurrence in each position were determined in standard deviations (s) relative to the average occurrence:

$$P = \left( F_{pos} - F_{ave} \right) / s$$

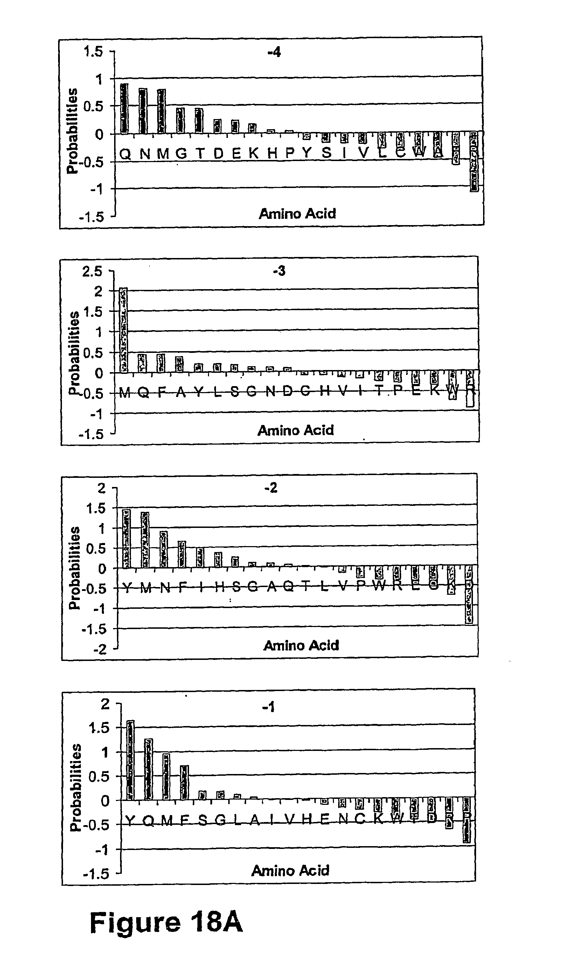

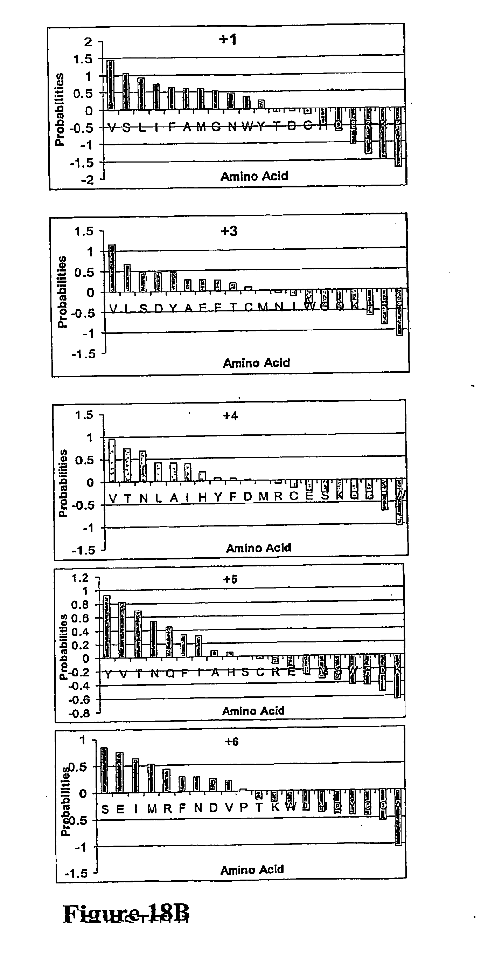

It was found that significant biases in amino acid occurrence only appear in the immediate vicinity of the glycosylation site (-3 to +5). There is a marked preferences for non-charged amino acids and discrimination against charged amino acids (D.E.R.K) as well as proline on either side of the glycosylation site (Figure 18A and 18B). With the exception of W, there is also an increased probability of finding bulky hydrophobic amino acids, such as M, F, and Y, immediately before a glycosylation site (-3, -2, and -1), and there is an increased probability of finding small, non-charged amino acids (I... S, V, 1, A) at positions +1, +3, +4, and +5. In addition, at either side of the glycosylation site, the identified *N*-linked glycopeptides appear selective against amino acids that arc likely to be modified (W and C) at either sides of the glycosylation site. These data indicate that there is well-defined specificity for a protetypical *N*-linked glycopeptide that is likely to be detected by mass spectrometry.

Computational prediction of N-linked glycopeptides as proteotypical peptides. Next the large number of *N*-linked glycopeptides identified in this study was used to generate predictors to score all the theoretical tryptic N-linked glycopeptides from human IPI database (version 2.28). It allowed us to predict the likelihood of occupancy for an N-X-T/S sequon (Yaffe et al., Nat. Biotechnol. 19:348-353 (2001)) and its detection possibility by mass spectrometer (Mallick, P.

et al., In Preparation (2005)). A web interface, UniPep, was developed for displaying N-X-T/S sequon containing peptides in the human IPI database for predicted or experimentally identified proteotypic *N*-linked glycopeptides. This is of particular relevance with respect to those genes or proteins that have been shown to change their abundance in disease tissues compared to normal tissues using genomic or proteomic approaches. The detection of these proteins in serum, especially secreted proteins or extracellular surface proteins which are most likely to make their way into blood serum, is a critical step in the development of these proteins as disease biomarkers. In this case, the proteotypical *N*-linked glycopeptides are predicted by their sequences and heavy isotopic labeled peptides can be synthesized as candidates to determine their presence and quantify their abundance in serum.

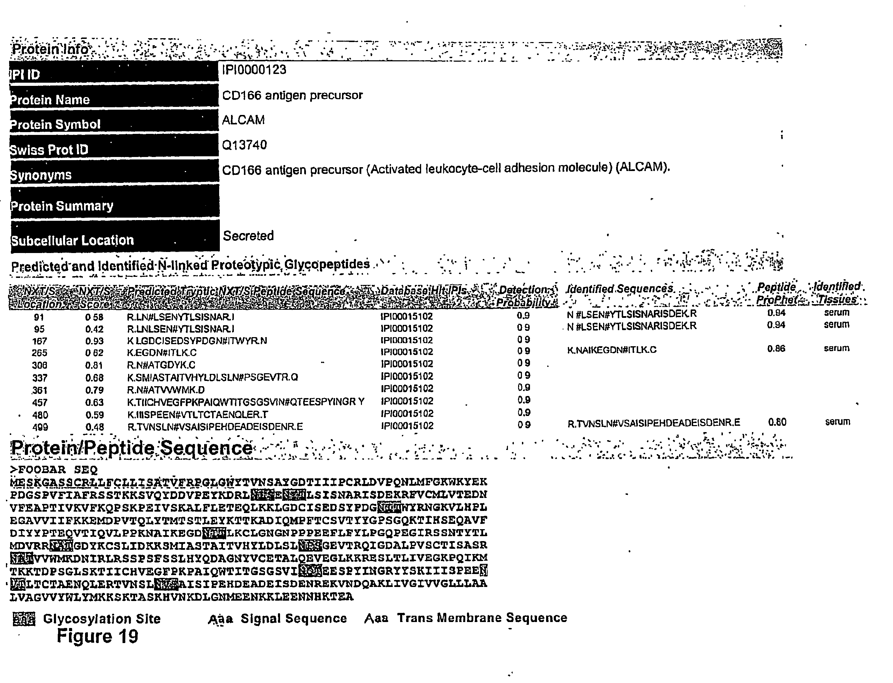

Four different types of information were used to predict proteotypical *N*-linked glycopeptides when scanning the IPI protein database. First, since *N*-linked glycosylation is likely to occur on the extracellular surface or secreted proteins, the subcellular localization of each protein was predicted using a combination of hidden Markov model (HMM) algorithms,Nielsen et al., Protein Eng. 10:1-6 (1997) and transmembrane (TM) region predictions using a commercial version of the TMHMM algorithm Kroah et al., J. Mol. Biol. 305:567-580 (2001).. By so doing, the subcellular localizations of each protein was able to be categorizes as being either a) *extracellular—proteins* that contained predicted non-cleavable signal peptides and no predicted transmembrane segments; b) *secreted*—proteins that contained predicted cleavable signal peptides and no predicted transmembrane segments: c) *transmembrane*—proteins that contained predicted transmembrane segments and extracellular loops and intracellular loops; and d) *intracellular*—proteins that contained neither predicted signal peptides nor predicted transmembrane regions. The predicted protein subcellular localization is displayed in UniPep along with other protein information from database annotations (Figure 19, Protein infor), and the signal peptides and transmembrane sequences are highlighted in the protein sequence to give a general indication of the protein topology. NXS/T score for predicted peptides). For all predicted peptides that have also been experimentally identified in the dataset, the Peptide ProPhet score and the tissue resources from which the peptides were identified are indicated as well. Third, the experimentally identified *N* linked glycopeptides were used to calculate peptide frequencies that are likely to be detected by mass spectrometry and identify a set of physico-chemical properties that distinguish observed peptides by MS from unobserved peptides (Mallick, P. et al., In. Preparation (2005)). The physico-chemical properties determined from the identified peptides were used to score the likelihood of a potential *N*-linked glycopeptide to be detected by MS (Figure 19, detection probability). Fourth, the uniqueness of each predicted *N*-linked glycopeptide was determined by searching for each sequence within the entire IPI protein database. Peptides present in multiple proteins were indicated by multiple database hits (Figure 19, Number of other proteins with the peptide). Uniqueness of a peptide sequence to a particular protein was taken to be a necessary condition for being a proteotypic peptide.

Applying the protein subcellular localization prediction method to all 40,110 protein entries in the IPI database, it was predicted that 14041 proteins are exposed to extracellular environment as secreted, transmembrane, or Extracellular surface proteins (Table 12). Of these, 76% contain at least one *N*-linked glycosylation sequon that is potentially *N*-linked glycosylated and can be detected by a proteotypic *N*-linked glycopeptides (Table 14). In other words, profiting proteotypical *N*-linked glycopeptides can capture a large number of proteins in the extracellular environment that derive from variety of cells and tissues. The glycopeptide capture method significantly enriches in extracellular,-secreted , and are the same proteins considered most easily accessible in the human body for diagnostic and therapeutic purposes.

Table 12 Predicted subcellular localizations of proteins from human protein database and their potential *N*-linked glycosylation

|  | Total | NXT/S | % |
|---|---|---|---|
| All proteins in database | 40110 | 29908 | 75% |
| Extracellular/secreted/ membrane proteins | 14041 | 10664 | 76% |
| Secreted proteins | 3947 | 3017 | 76% |
| Extracellular proteins | 3415 | 2358 | 69% |
| Transmembrane proteins | 6679 | 5289 | 79% |

Since analyses of serum proteins using SPEG focus on information-rich subproteomes, it should be pointed out that non-glycosylated proteins are transparent to this system. While it is believed that the majority of serum-specific proteins are glycosylated (Durand and Seta, Clin. Chem. 46:795-805 (2000)), intracellular proteins that are non-glycosylated may represent a rich source of biomarkers in the dead cells of diseased tissue. The data suggest that cell-specific surface proteins that are mostly glycosylated are also released into serum and are therefore identifiable using the glycopeptide capture method.

Validation of the experimentally identified or computational predicted glycopeptides by synthetic peptide standard. Peptide identification using tandem mass spectrometry is based on the matching of experimentally determined CID spectra with theoretical spectra generated from all possible peptide sequence from a protein database. Therefore, the peptide sequence assignments using database search algorithms includes a certain degree of error, and in this study, statistical methods were used to objectively estimate the probability of an identified -peptide being a correct peptide (Keller et al.. Anal .Chem. 74:5383-5392 (2002)). An identified peptide with a peptide probability of 0.9 indicates that this peptide has a 90% chance to be correct. Using medium probability score cut-off, 3244 unique glycosylation sites were identified. Of these peptides, it is estimated that 2502 unique N-linked glycopeptides are predicted to be correct identifications.

The identified peptides were validated with additional evidence. Since MS/MS spectra from the same peptide sequence are highly reproducible (Rush et al., Nat. Biotechnol. 23:94-101 (2005)), a heavy isotope labeled peptide corresponding to the identified peptide of interest was synthesized. The MS/MS spectrum of the synthetic peptide was then compared with the one that was used to make the sequence assignment. The heavy isotope labeled peptide versus regular peptide was synthesized, which allowed differentiation of the synthetic standard from its native form by mass spectrometry and could be used to quantify the same peptide from biological sample using the high throughput platform we developed recently (Pan et al., Mol. Cell. Proteomics 4:182-190 (2005)).

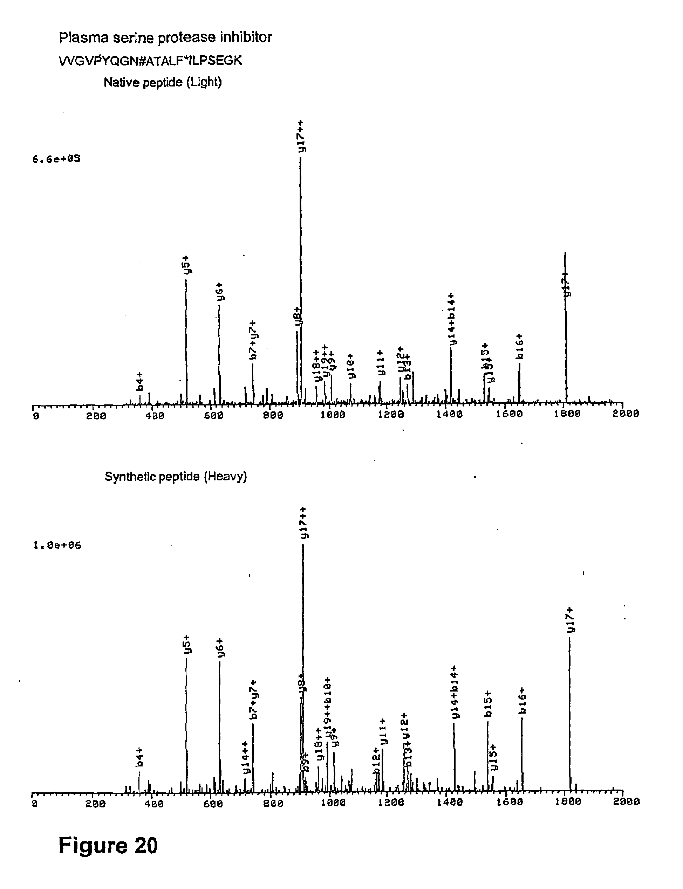

One of the identified N-linked glycopeptides from plasma serine protease inhibitor shown in Figure 19 was synthesized and the phenylalanine residue was replaced with heavy .Phenylalanine (using $^{13}$C) in the peptide sequence. This heavy isotope labeled peptide standard produces 9 mass unit difference from the native peptide. The signature of the CID spectra of the native light and synthetic heavy forms of the peptide were highly reproducible, and the correctness of the sequence assignment of this peptide can be determined by 1) the co-elution of this heavy isotope labeled peptide with its light form of native peptide and 2) the similarity of the CID spectra (Figure 20).

In the present study, a list of a large number of serum proteins and their N-linked alycopeptides from serum were identified using SPEG followed by MS/MS. The list of identified proteins confirmed the presence of a number of candidate marker proteins in plasma and serum, indicating that the quantitative analysis of serum proteins using SPEG increases the sensitivity and has the potential to identity disease markers. The increased sensitivity is achieved by focusing on only N-linked glycopeptides versus all tryptic peptides from whole proteins and avoiding the analysis of highly abundance proteins such as albumin.

As demonstrated in our recent publication (Pan et al., Mol. Cell. Proteomics 4:182-190 (2005)), this list of glycopeptides can be synthesized as a heavy isotope labeled standard and used to identity and quantify native glycopeptides using the recently developed mass spectrometry-based screening technology. This allows specific targeting of certain peptides/proteins with biological significance in a complex sample for identification and quantification. For each candidate marker, the identified formerly N-linked glycopeptide was chemically synthesized; labeled with at least one heavy isotope amino acid, and spiked into peptides isolated from serum using SPEG. During MS analysis, this representative stable isotope labeled peptide standard distinguishes itself from the corresponding native peptide by a mass difference corresponding to the stable isotope label. Knowing the exact mass, sequence and quantity of the standard peptide, the peptide standard and its isotopic pair isolated from serum can be located and selectively sequenced for identification. The quantification of the native peptide is determined by the ratio of the abundance of the spiked peptide, whose identify and quantity are known, to that of the native peptide (Pan et al., Mol. Cell. Proteomics 4:182-190 (2005)). Since this approach transforms proteomic analysis from traditional data dependant discovery phase to validation scoring phase and directly focusing on interesting peptides/proteins for identification and quantification, it technically increases the sample loading capacity, avoids some difficult issues associated with sample complexity and thus significantly improves the throughput and sensitivity.

Using the identified N-linked glycosylation sites from this study, further refinement was performed on the N-linked glycosylation motif by identifying amino acid preference around the glycosylation sites that are likely to be identified by mass spectrometry. It was found that amino acid positions -3 to +5 showed a significant bias for non-charged amino acids and against charged amino acids (K, R, D, E), as well as proline, tryptophan, and cysteine. The amino acid sequence before the glycosylated Asparagine (-3, -2, and -1) had preference for bulky hydrophobic amino acid (Y, F, M), and amino acid sequence after the glycosylated Asparagine (+1 to +5) had preference for small non-charged amino acids such as V, L, I, S and A. These amino acid preferences are in general agreement with the previous studies with the exception that the K, and R are less preferred around the NXT/S sequon (Petrescu et al., Glycobiology 14:103-114 (2004) : Apweiler et al., Biochim Biophys Acta 1473:4 8 (1999)). The less represented K and R might be due to less efficient cleavage of tryptic digest around the N-linked glycosylation sites.

The selected amino acid preference around N-linked glycosylation site and the physico-chemical properties of the identified peptides by mass spectrometry in this study allow us to predier proteotypical N- linked glycopeptides from proteins exposed to extracellular environment that likely to be detected by mass spectrometry. A software tool, UniPep, has developed to output the known and unknown proteotypical N-linked glycopeptides from queried proteins in database.

Theoretically, the experimentally identified or computationally determined proteotypic *N*-linked glycopeptides for quantitative analysis of serum proteins will capture majority of proteins designated to extracellular environment, which is likely to be detected in serum as disease biomarkers.

Throughout this application various publications have been referenced. The disclosures of these publications in their entireties are hereby incorporated by reference in this application in order to more fully describe the state of the art to which this invention pertains. Although the invention has been described with reference to the examples provided above, it should be understood that various modifications can be made without departing from the spirit of the invention.

**Claims**

1. A method for identifying glycopolypeptides in a serum or plasma sample, comprising:

    (a) immobilizing said sample glycopolypeptides to a solid support;
    (b) cleaving said immobilized sample glycopolypeptides, thereby releasing non-glycosylated peptide fragments and retaining immobilized sample glycopeptide fragments;
    (c) labeling said immobilized sample glycopeptide fragments with an isotope tag;
    (d) releasing said sample glycopeptide fragments from said solid support, thereby generating released sample glycopeptide fragments;
    (e) adding to said released sample glycopeptide fragments a plurality of standard peptides selected from peptides containing the glycosylation sites referenced as SEQ ID NOS 2-3482, wherein said plurality of standard peptides comprises at least one peptide containing the glycosylation site referenced as SEQ ID NO 1064, wherein said standard peptides correspond to peptides cleaved as in step (b), and released as in step (d), and wherein said standard peptides are differentially labeled with a corresponding isotope tag as used in step (c);
    (f) analyzing said released sample glycopeptide fragments using mass spectrometry; and
    (g) identifying released sample glycopeptide fragments that correspond to standard peptides added in step (e).

2. The method of claim 1, further comprising the initial step of derivatizing glycopolypeptides in said sample.

3. The method of claim 1, or claim 2, further comprising quantifying the amount of said sample glycopeptide fragments identified in step (g).

4. The method of claim 3 when dependent upon claim 2, wherein the control serum or plasma sample is normal serum or plasma obtained from a healthy individual or individuals.

5. The method of claim 4, wherein said method is for identifying one or more diagnostic markers for a disease.

6. The method of claim 5, wherein the disease is cancer.

7. The method of any of claims 1, 2, 3, or 5, wherein said solid support comprises a hydrazide moiety.

8. The method of any of claims 1, 2, 3, or 5, wherein said glycopeptides are released from said solid support using a glycosidase, optionally wherein said glycosidase is an N-glycosidase or an O-glycosidase, and optionally further wherein said glycopeptides are released from said solid using sequential addition of N-glycosidase and O-glycosidase.

9. The method of any of claims 1, 2, 3, or 5, wherein said glycopeptides are one of:

    released from said solid support using chemical cleavage;
    oxidized with periodate;
    cleaved with a protease; or
    cleaved with trypsin.

10. A composition comprising a plurality of peptides containing the glycosylation sites referenced as SEQ ID NOS 2-3482, wherein said plurality of peptides comprises at least one peptide containing the glycosylation site referenced as SEQ ID NO 1064, and wherein said peptides each correspond to peptide fragments derived by cleavage of polypeptides using the same cleavage reagent.

11. The composition of claim 10, wherein said cleavage reagent is a protease, and optionally, wherein said protease is trypsin.

12. A kit comprising a plurality of peptides containing the glycosylation sites referenced as SEQ ID NOS 2-3482, wherein said plurality of peptides comprises at least one peptide containing the glycosylation site referenced as SEQ ID NO 1064, and wherein said peptides each correspond to peptide fragments derived by cleavage of polypeptides using the same cleavage reagent.

13. The kit of claim 12, further comprising a pair of differentially labeled isotope tags.

14. The kit of claim 12, further comprising the cleavage reagent corresponding to said peptide fragments, optionally wherein said cleavage reagent is a protease, and further optionally wherein said protease is trypsin.

15. The kit of claim 12, further comprising a hydrazide resin or a glycosidase.

Figure 1

labeling reagents        chemical structure[a]

Succinic-anhydride

Isatoic-anhydride

N-Methyl-isatoic-anhydride

Glyceraldehyde

**Figure 2A**

EP 2 302 394 A1

| labeling reagents | chemical structure[a] | |
|---|---|---|
| Boc-Phe-OH | | |
| Benzaldehyde | | |
| Salicylaldehyde | | |

**Figure 2B**

EP 2 302 394 A1

Figure 3

## Quantitative analysis of serum proteins by MALDI-TOF

Biological sample

N-linked glycopeptides

'Normal'    'Heavy'    Standard Isotopic labeled peptides

Separate

RP-μLC

+ α-cyano

Sample plate

MALDI-TOF

MS 1) quantification

MS/MS 2) identification

**Figure 4**

EP 2 302 394 A1

Spiking stable isotope-labeled peptide: VVGVPYQGDATAL_FILPSEGK

MS spectrum:

MSMS spectrum of spiked peptide:

spiked peptide: 2170 m/z

Figure 5

Figure 6

Figure 7

Figure 8

Figure 9

**1. Sample preparation:**

```
        ┌──────────────────────────┐
        │   Protein tryptic digest  │
        └──────────────────────────┘
                     │
                     ▼
┌────────────────────────────────────────────┐
│ Spike with stable isotope-labeled peptides  │
└────────────────────────────────────────────┘
                     │
                     ▼
        ┌──────────────────────────┐
        │   Fractionate peptides onto│
        │    a MALDI sample plate    │
        └──────────────────────────┘
```

**2. Mass spectrometry and data analysis:**

Peptide array

MS analysis

**B.** Export, process, and re-organize the MS raw data

**C.** Select interested peptides for MS/MS analysis (optional)

**A.** Identify peptides using the accurate mass of isotope-peptide pairs

Sum peptide intensity across the continue elution spots

Validate the peptide identification using MS/MS data and database search

Peptide/Protein identification & quantification

Quantify peptide concentration using the intensity ratio of spike-native pair

# Figure 10

Figure 11

Figure 12

A

Base peak chromatograph

B

MS spectrum at spot 99:

Figure 13

A

B

Figure 14

**Figure 15**

Figure 16

Figure 17

Figure 18A

Figure-18B

## Protein Info

| | |
|---|---|
| IPI ID | IPI0000123 |
| Protein Name | CD166 antigen precursor |
| Protein Symbol | ALCAM |
| Swiss Prot ID | Q13740 |
| Synonyms | CD166 antigen precursor (Activated leukocyte-cell adhesion molecule) (ALCAM). |
| Protein Summary | |
| Subcellular Location | Secreted |

## Predicted and Identified N-linked Proteotypic Glycopeptides

| NXT/S Location | NXT/S Score | Predicted Tryptic NXT/S Peptide Sequence | Database Hit IPIs | Detection Probability | Identified Sequences | Peptide ProPhet | Identified Tissues |
|---|---|---|---|---|---|---|---|
| 91 | 0 58 | R.LN#LSENYTLSISNAR.I | IPI00015102 | 0.9 | N #LSEN#YTLSISNARISDEK.R | 0.94 | serum |
| 95 | 0.42 | R.LNLSEN#YTLSISNAR.I | IPI00015102 | 0 9 | N #LSEN#YTLSISNARISDEK.R | 0.94 | serum |
| 167 | 0.93 | K LGDCISEDSYPDGN#ITWYR.N | IPI00015102 | 0 9 | | | |
| 265 | 0 62 | K.EGDN#ITLK.C | IPI00015102 | 0 9 | K.NAIKEGDN#ITLK.C | 0.86 | serum |
| 306 | 0.81 | R.N#ATGDYK.C | IPI00015102 | 0 9 | | | |
| 337 | 0.68 | K.SMIASTAITVHYLDLSLN#PSGEVTR.Q | IPI00015102 | 0 9 | | | |
| 361 | 0.79 | R.N#ATVVWMK.D | IPI00015102 | 0.9 | | | |
| 457 | 0.63 | K.TIICHVEGFPKPAIQWTITGSGSVIN#QTEESPYINGR Y | IPI00015102 | 0.9 | | | |
| 480 | 0.59 | K.IIISPEEN#VTLTCTAENQLER.T | IPI00015102 | 0.9 | | | |
| 499 | 0.48 | R.TVNSLN#VSAISIPEHDEADEISDENR.E | IPI00015102 | 0 9 | R.TVNSLN#VSAISIPEHDEADEISDENR.E | 0.80 | serum |

## Protein/Peptide Sequence

```
>FOOBAR SEQ
MESKGASSCRLLFCLLISATVFRPGLGWYTVNSAYGDTIIIPCRLDVPQNLMFGKWKYEK
PDGSPVFIAFRSSTKKSVQYDDVPEYKDRLNISENITLSISNARISDEKRFVCMLVTEDN
VFEAPTIVKVFKQPSKPEIVSKALFLETEQLKKLGDCISEDSYPDGNITWYRNGKVLHPL
EGAVVIIFKKEMDPVTQLYTMTSTLEYKTTKADIQMPFTCSVTYYGPSGQKTIHSEQAVF
DIYYPTEQVTIQVLPPKNAIKEGDNISLKCLGNGNPPPEEFLFYLPGQPEGIRSSNTYTL
MDVRRNATGDYKCSLIDKKSMIASTAITVHYLDLSLNISGEVTRQIGDALPVSCTISASR
NISVVWMKDNIRLRSSPSFSSLHYQDAGNYVCETALQEVEGLKKRESLTLIVEGKPQIKM
TKKTDPSGLSKTIICHVEGFPKPAIQWTITGSGSVINQTEESPYINGRYYSKIIISPEEN
VTLTCTAENQLERTVNSLNVSAISIPEHDEADEISDENREKVNDQAKLIVGIVVGLLLAA
LVAGVVYWLYMKRSKTASKHVNKDLGNMEENKKLEENNHKTEA
```

▓ Glycosylation Site   Aaa Signal Sequence   Aaa Trans Membrane Sequence

## Figure 19

Plasma serine protease inhibitor

VVGVPYQGN#ATALF*ILPSEGK

Native peptide (Light)

Synthetic peptide (Heavy)

# Figure 20

**EUROPEAN SEARCH REPORT**

Application Number

EP 10 01 2637

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | WO 03/102018 A (THE INSTITUTE FOR SYSTEMS BIOLOGY; AEBERSOLD, RUDOLF, H; ZHANG, HUI) 11 December 2003 (2003-12-11) * page 1 * * pages 4,42,119 * * example XIII * * sequences 170,185,186 * ----- | 1-15 | INV. G01N33/68 |
| X | US 6 407 210 B1 (BAMBER BRUCE A [US] ET AL) 18 June 2002 (2002-06-18) * figures 4B-1; sequence 20 * ----- | 10-15 | |
| X | WO 92/22652 A1 (MERCK SHARP & DOHME [GB]) 23 December 1992 (1992-12-23) * page 7, last paragraph; figure 6 * ----- | 10-15 | |

TECHNICAL FIELDS SEARCHED (IPC)

G01N

The present search report has been drawn up for all claims

| Place of search | Date of completion of the search | Examiner |
|---|---|---|
| The Hague | 15 February 2011 | Gundlach, Björn |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

    ................................................................................

& : member of the same patent family, corresponding document

EPO FORM 1503 03.82 (P04C01)

**ANNEX TO THE EUROPEAN SEARCH REPORT
ON EUROPEAN PATENT APPLICATION NO.**

EP 10 01 2637

This annex lists the patent family members relating to the patent documents cited in the above-mentioned European search report.
The members are as contained in the European Patent Office EDP file on
The European Patent Office is in no way liable for these particulars which are merely given for the purpose of information.

15-02-2011

| Patent document cited in search report | | Publication date | Patent family member(s) | | Publication date |
|---|---|---|---|---|---|
| WO 03102018 | A | 11-12-2003 | AU | 2003249692 A1 | 19-12-2003 |
| | | | CA | 2487314 A1 | 11-12-2003 |
| | | | EP | 1514107 A2 | 16-03-2005 |
| | | | JP | 4583168 B2 | 17-11-2010 |
| | | | JP | 2006507476 T | 02-03-2006 |
| | | | JP | 2010096769 A | 30-04-2010 |
| US 6407210 | B1 | 18-06-2002 | US | 2003065144 A1 | 03-04-2003 |
| | | | US | 6406872 B1 | 18-06-2002 |
| WO 9222652 | A1 | 23-12-1992 | AT | 234355 T | 15-03-2003 |
| | | | AU | 663090 B2 | 28-09-1995 |
| | | | AU | 1921192 A | 12-01-1993 |
| | | | CA | 2109193 A1 | 12-12-1992 |
| | | | DE | 69232953 D1 | 17-04-2003 |
| | | | DE | 69232953 T2 | 11-12-2003 |
| | | | DK | 589930 T3 | 22-04-2003 |
| | | | EP | 0589930 A1 | 06-04-1994 |
| | | | ES | 2192554 T3 | 16-10-2003 |
| | | | JP | 6508023 T | 14-09-1994 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20040023306 A, Aebersold and Zhang
- WO 0011208 A
- US 5143854 A
- US 5986076 A
- US 5917016 A
- US 5489678 A
- US 5405783 A
- WO 02052259 A
- US 5264563 A, Huse

**Non-patent literature cited in the description**

- **KELLER et al.** *Anal. Chem.,* 2002, vol. 74, 5383-5392
- **ZHANG et al.** *Nat. Biotechnol.,* 2003, vol. 21, 660-666
- **GYGI et al.** *Nature Biotechnol.,* 1999, vol. 17, 994-999
- **HELENIUS ; AEBI.** *Science,* 2001, vol. 291, 2364-2369
- **RUDD et al.** *Science,* 2001, vol. 291, 2370-2375
- **VARKI et al.** Essentials of Glycobiology. Cold Spring Harbor Laboratory, 1999
- **ROTH.** *Chem. Rev.,* 2002, vol. 102, 285-303
- **SHEPARD et al.** *J. Clin. Immunol.,* 1991, vol. 11, 117-127
- **DURAND ; SETA.** *Clin. Chem.,* 2000, vol. 46, 795-805
- **FREEZE.** *Glycobiology,* 2001, vol. 11, 129R-143R
- **SPIRO.** *Glycobiology,* 2002, vol. 12, 43R-56R
- **HERMANSON.** Bioconjugate Techniques. Academic Press, 1996, 3-166
- **GLAZER et al.** Laboratory Techniques in Biochemistry and Molecular Biology: Chemical Modification of Proteins. Elsevier Biomedical Press, 1975, 68-120
- **ZHOU et al.** *Nat. Biotechnol.,* 2001, vol. 19, 375-378
- **DORMAN ; PRESTWICH.** *Trends Biotech.,* 2000, vol. 18, 64-77
- **GREENE ; WUTS.** Protective Groups in Organic Synthesis. John Wiley & Sons, 1991
- **YATES.** *J.Mass Spect.,* 1998, vol. 33, 1-19
- **KINTER ; SHERMAN.** Protein Sequencing and Identification Using Tandem Mass Spectrometry. John Wiley & Sons, 2000
- **AEBERSOLD ; GOODLETT.** *Chem. Rev.,* 2001, vol. 101, 269-295
- **YATES et al.** *Methods Mol. Biol.,* 1999, vol. 112, 553-.569
- **GOODLETT et al.** *Anal. Chem.,* 2000, vol. 72, 1112-1118
- **YATES.** *J. Mass Spec.,* 1998, vol. 33, 1-19
- **AUSUBEL et al.** Current Protocols in Molecular Biology. John Wiley & Sons, 2001
- **SCOPES.** Protein Purification: Principles and Practice. Springer-Verlag, 1993
- **FRANCIS et al.** *Curr. Opin. Chem. Biol,* 1998, vol. 2, 422-428
- **TIETZE et al.** *Curr. Biol.,* 1998, vol. 2, 363, 371
- **SOFIA.** *Mol. Divers.,* 1998, vol. 3, 75-94
- **EICHLER et al.** *Med. Res. Rev.,* 1995, vol. 15, 481-496
- **GORDON et al.** *J. Med. Chem,* 1994, vol. 37, 1233-1251
- **GORDON et al.** *J. Med. Chem,* 1994, vol. 37, 1385-1401
- **GORDON et al.** *Acc. Chem. Res.,* 1996, vol. 29, 144-154
- Combinatorial Chemistry: Syntheses and Application. John Wiley & Sons, 1997
- **WULFKUHLE et al.** *Nat. Rev. Cancer,* 2003, vol. 3, 267-275
- **ANDERSON ; ANDERSON.** *Proc. Natl. Acad. Sci. USA,* 1977, vol. 74, 5421-5425
- **MERRIL et al.** *Science,* 1981, vol. 211, 1437-1438
- **MERRIL et al.** *Proc. Natl. Acad. Sci. USA,* 1979, vol. 76, 4335-4339
- **AEBERSOLD ; MANN.** *Nature,* 2003, vol. 422, 198-207
- **DIAMANDIS.** *Mol. Cell. Proteomics,* 2004, vol. 3, 367-378
- **PETRICOIN et al.** *Lancet,* 2002, vol. 359, 572-577
- **ADKINS et al.** *Mol. Cell. Proteomics,* 2002, vol. 1, 947-955

- **TIRUMALAI et al.** *Mol. Cell. Proteomics,* 2003, vol. 2, 1096-1103
- **SHEN et al.** *Anal. Chem.,* 2004, vol. 76, 1134-1144
- **WANG ; HANASH.** *J. Chromatogr. B Analyt. Technol. Biomed. Life Sci.,* 2003, vol. 787, 11-18
- **SHIN et al.** *J. Mammary Gland Biol. Neoplasia,* 2002, vol. 7, 407-413
- **VILLANUEVA et al.** *Anal. Chem.,* 2004, vol. 76, 1560-1570
- **ANDERSON ; ANDERSON.** *Mol. Cell. Proteomics,* 2002, vol. 1, 845-867
- **PIEPER et al.** *Proteomics,* 2003, vol. 3, 1345-1364
- **WRIGHT et al.** *Genome Biol.,* 2003, vol. 5, R4
- **FLORY ; AEBERSOLD.** *Prog. Cell Cycle Res.,* 2003, vol. 5, 167-171
- **GUINA ; WU, M. ; MILLER, S. L ; PURVINE, S. O. ; YI, E. C. ; ENG, J. et al.** *J. Am. Soc. Mass Spectrom.,* 2003, vol. 14, 742-751
- **AEBERSOLD.** *Nature,* 2003, vol. 422, 115-116
- **FLORY ; GRIFFIN, T. J. ; MARTIN, D. ; AEBERSOLD et al.** *Trends Biotechnol.,* 2002, vol. 20, 23-29
- **SHIIO, Y. ; DONOHOE, S. ; YI, E. C. ; GOODLETT, D. R. ; AEBERSOLD, R. ; EISENMAN, R. N.** *EMBO J.,* 2002, vol. 21, 5088-5096
- **RABILLOUD et al.** *J. Biol. Chem.,* 2002, vol. 277, 19396-19401
- **BRAND et al.** *Nat. Struct. Mol. Biol.,* vol. 11, 73-80
- **HIMEDA et al.** *Mol. Cell. Biol.,* 2004, vol. 24, 2132-2143
- **RANISH et al.** *Nat. Genet.,* 2004, vol. 36, 707-713
- **RANISH, J. A. ; YI, E. C. ; LESLIE. D. M. ; PURVINE, S. O. ; GOODLETT, D. R. ; ENG, J. et al.** *Nat. Genet.,* 2003, vol. 33, 349-355
- **AEBERSOLD.** *J. Am. Soc. Mass Spectrom.,* 2003, vol. 14, 685-695
- **AEBERSOLD.** *J. Infect. Dis.,* 2003, vol. 187 (2), 315-320
- **PATTERSON ; AEBERSOLD.** *Nat. Genet.,* 2003, vol. 33, 311-323
- **GRIFFRIN et al.** *Anal. Chem.,* 2003, vol. 75, 867-874
- **HALE et al.** *Brief Funct. Genomic Proteomic,* 2003, vol. 2, 185-193
- **SHAU et al.** *Brief Funct Genomic Proteomic,* 2003, vol. 2, 147-158
- **FLORY et al.** *Trends Biotechnol.,* 2002, vol. 20, 23-29
- **AEBERSOLD, R. ; CRAVATT.** *Trends Biotechnol.,* 2002, vol. 20, 1-2
- **TAO ; AEBERSOLD.** *Curr. Opin. Biotechnol.,* 2003, vol. 14, 110-118
- **LI et al.** *Anal. Chem.,* 2004, vol. 76, 3856-3860
- **NESVIZHSKII ; AEBERSOLD.** *Drug Discov. Today,* 2004, vol. 9, 73-181
- **NESVIZHSKII et al.** Anal. Chem. 2003, vol. 75, 4646-4658
- **DOMON ; BRODER.** *J. Proteome Res.,* 2004, vol. 3, 253-260
- *Adv. Exp. Med. Biol.,* 2004, vol. 547, 21-30
- **AEBERSOLD.** *Nature,* 2004, vol. 422, 115-116
- **SHEN et al.** Anal. Chem. 2004, vol. 76, 1134-1144
- **ANDERSON et al.** *Mol. Cell. Proteomics,* 2004, vol. 3, 311-326
- **PIEPER et al.** *Proteomics,* 2003, vol. 3, 422-432
- **TIRUMALAI et al.** *Mol. Cell. Proteomics,* 2003, vol. 2, 1096-1 103
- **GUPPY et al.** *Oncologist,* 2002, vol. 7, 437-443
- **PAN et al.** *Mol. Cell. Proteomics,* 2005, vol. 4, 182-190
- **ENG et al.** *J. Am. Soc. Mass. Spectrom.,* 1994, vol. 5, 976-989
- **KELLER et al.** Anal. Chem. 2002, vol. 74, 5383-5392
- **HAN et al.** *Nat. Biotechnol.,* 2001, vol. 19, 946-951
- **ENG et al.** *J. Am. Soc. Mass Spectrom.,* 1994, vol. 5, 976-989
- **KEMP et al.** *Cell,* 1993, vol. 74, 813-822
- **GYGI et al.** *Nat. Biotechnol.,* 1999, vol. 17, 994-999
- **YI et al.** *Rapid Commun. Mass Spectrom.,* 2003, vol. 17, 2093-2098
- **GRIFFIN et al.** Anal. Chem. 2003, vol. 75, 867-874
- **LI et al.** Anal. Chem. 2003, vol. 75, 6648-6657
- **EISEN et al.** *Proc. Natl. Acad. Sci. USA,* 1998, vol. 95, 14863-14868
- **ZHANG et al.** *Nat. Biotechnol.,* 2003, vol. 2, 660-666
- **PETRESCU et al.** *Glycobiology,* 2004, vol. 14, 103-114
- **PUTNAM.** The plasma proteins: Structure, Function, and Genetic Control. Academic Press, 1975
- **LUM ; GAMBINO.** *Am. J. Clin. Pathol.,* 1974, vol. 61, 108-113
- **DUAN et al.** *Electrophoresis,* 2004, vol. 25, 3055-3065
- **LI et al.** Anal. Chem. 2004, vol. 76, 3856-3860
- **BROWN ET AL., K. et al.** *Cell,* 1986, vol. 46, 447-456
- **QUINTANILLA et al.** *Nature,* 1986, vol. 322, 78-80

- **MOLE et al.** *J. Immunol.,* 1988, vol. 141, 3642-3646
- **ZHANG et al.** *Curr. Opin. Chem. Biol.,* 2004, vol. 8, 66-75
- **ANDERSON ; ANDERSON.** *Mol..Cell. Proteomics,* 2002, vol. 1, 845-867
- **DIAMANDIS.** *Mol. Cell. Proteomics,* 2004, vol. 3, 3.67-378
- **KAJI et al.** *Nat. Biotechnol.,* 2003, vol. 21, 66 7-672
- **SHEN et al.** Anal. Chem. 2004, vol. 76, 1134-1114
- **SULLIVAN PEPE et al.** *J. Natl. Cancer Inst.,* 2001, vol. 93, 1054-1061
- **ADAM et al.** *Cancer Res,* 2002, vol. 62, 3609-3614
- **VEJDA et al.** *Mol. Cell. Proteomics,* 2002, vol. 1, 387-393
- **ENG.** *J. Am. Soc. Mass Spect.,* 1994, vol. 5, 976-989
- **ZHANG et al.** *Nat. Biotechnol,* 2003, vol. 21, 660-666
- **MARTIN et al.** *Cancer Res.,* 2004, vol. 64, 347-355
- **LIU et al.** *J. Urol.,* 2005, vol. 173, 73-78
- **ZHANG et al.** *Nat. Biotechnol.,* 2003, vol. 21, 660-6G6
- **ZHANG et al.** *Mol. Cell. Proteomics,* 2005, vol. 4, 144-155
- **ZHANG.** *Nat. Biotechnol.,* 2003, vol. 21, 660-666
- **HAN et al.** *Nat. Biotechnol,* 2001, vol. 19, 946-951
- **KELLER.** Anal. Chem. 2002, vol. 74, 5383-5392
- **KROGH et al.** *J. Mol. Biol.,* 2001, vol. 305, 567-580
- **BRESALIER et al.** *Gastroenterology,* 2004, vol. 127, 741-748
- **MARCHETTI et al.** *Cancer Res,* 2002, vol. 62, 2535-2539
- **BAUSE.** *Biochem. J.,* 1983, vol. 209, 331-336
- **MALLICK, P. et al.** *Preparation,* 2005
- **VARKI.** Essentials of Glycobiology. Cold Spring Harbor Laboratory Press, 1999
- **YAFFE et al.** *Nat. Biotechnol.,* 2001, vol. 19, 348-353
- **NIELSEN et al.** *Protein Eng.,* 1997, vol. 10, 1-6
- **KROAH et al.** *J. Mol. Biol,* 2001, vol. 305, 567-580
- **KELLER et al.** Anal .Chem. 2002, vol. 74, 5383-5392
- **RUSH et al.** *Nat. Biotechnol.,* 2005, vol. 23, 94-101
- **APWEILER et al.** *Biochim Biophys Acta,* 1999, vol. 1473, 4-8